# EUROPEAN PATENT SPECIFICATION

(11) **EP 1 793 841 B1**
(45) Date of publication and mention of the grant of the patent: **03.04.2013**
(21) Application number: 05773610.0
(22) Date of filing: 22.08.2005
(51) Int. Cl.: C07K 14/00, C07K 7/08, A61K 38/04

(54) **PEPTIDE INHIBITORS OF C-JUN DIMERIZATION AND USES THEREOF**
PEPTIDHEMMER DER C-JUN-DIMERISIERUNG UND IHRE VERWENDUNGEN
INHIBITEURS PEPTIDIQUES DE LA DIMERISATION AVEC C-JUN ET UTILISATIONS CORRESPONDANTES

(30) Priority: 20.08.2004 US 603525 P
(43) Date of publication of application: 13.06.2007
(73) Proprietor: Phylogica Limited, Subiaco WA 6008 (AU)
(72) Inventor: WATT, Paul, Michael, Mount Claremont, Western Australia 6010 (AU); FEAR, Mark, Kardinya, Western Australia 6161 (AU)
(74) Representative: Plougmann & Vingtoft A/S
(86) International application number: PCT/AU2005/001255
(87) International publication number: WO 2006/017913

(56) References cited:
- WO-A-2004/074479
- WO-A1-2004/074479
- YAO S ET AL: "UNCOILING C-JUN COILED COILS: INHIBITORY EFFECTS OF TRUNCATED FOS PEPTIDES ON JUN DIMERIZATION AND DNA BINDING IN VITRO" BIOPOLYMERS, NEW YORK, NY, US, vol. 47, 1 January 1998 (1998-01-01), pages 277-283, XP002924577 ISSN: 0006-3525
- ZHOU X-F ET AL: "LIGAND-ACTIVATED RETINOIC ACID RECEPTOR INHIBITS AP-1 TRANSACTIVATION BY DISRUPTING C-JUN/C-FOS DIMERIZATION" MOLECULAR ENDOCRINOLOGY, BALTIMORE, MD, US, vol. 13, 1 January 1999 (1999-01-01), pages 276-285, XP008057705 ISSN: 0888-8809
- BAINS NARESH P S ET AL: "Zipping up transcription factors: Rational design of anti-Jun and anti-Fos peptides" LETTERS IN PEPTIDE SCIENCE, vol. 4, no. 2, 1997, pages 67-77, XP009118164 ISSN: 0929-5666
- ESTUS S ET AL: "ALTERED GENE EXPRESSION IN NEURONS DURING PROGRAMMED CELL DEATH: IDENTIFICATION OF C-JUN AS NECESSARY FOR NEURONAL APOPTOSIS" THE JOURNAL OF CELL BIOLOGY, ROCKEFELLER UNIVERSITY PRESS, US, vol. 127, no. 6, PART 01, 1 December 1994 (1994-12-01), pages 1717-1727, XP000674679 ISSN: 0021-9525
- DATABASE GENPEPT [Online] REN S.X. ET AL: 'Unique physiological and pathogenic features of Leptospira interrogans revealed by whole-genome sequencing', XP008112696 Retrieved from NCBI Database accession no. (AAN49594) & NATURE vol. 422, no. 6934, 2003, pages 888 - 893
- DATABASE GENPEPT [Online] NASCIMENTO A.L. ET AL: 'Comparative Genomics of Two Leptospira interrogans Serovars Reveals Novel Insights into Physiology and Pathogenesis', XP008113919 Retrieved from NCBI Database accession no. (AAS7019) & J. BACTERIOL. vol. 186, no. 7, 2004, pages 2164 - 2172
- DATABASE GENPEPT [Online] BOLOTIN A. ET AL: 'Complete sequence and comparative genome analysis of the dairy bacterium Streptococcus thermophilus', XP008112697 Retrieved from NCBI Database accession no. (AAV59791) & NAT. BIOTECHNOL. vol. 22, no. 12, 2004, pages 1554 - 1558
- DATABASE GENPEPT [Online] STRAUSBERG R.L. ET AL: 'Generation and initial analysis of more than 15,000 full-length human and mouse cDNA sequences', XP008113916 Retrieved from NCBI Database accession no. (AAH36335) & PROC.NATL.ACAD.SCI. U.S.A. vol. 99, no. 26, 2002, pages 16899 - 16903
- DATABASE GENPEPT [Online] KATINKA M.D. ET AL: 'Genome sequence and gene compaction of the eukaryote parasite Encephalitozoon cuniculi', XP008113920 Retrieved from NCBI Database accession no. (CAD25932) & NATURE vol. 414, no. 6862, 2001, pages 450 - 453
- DATABASE GENPEPT [Online] BLOECKER H. ET AL, XP008113921 Retrieved from NCBI Database accession no. (CAH10659)

## Description

### Field of the invention

The present invention relates to isolated or recombinant peptides or peptide analogues for use in the treatment of ischemia as well as pharmaceutical compositions comprising such peptides. Also described are methods for the diagnosis and treatment of stroke using peptide inhibitors of Jun dimerization that have been identified using the screening methods described herein.

### Background of the invention

### 1. General information

This specification contains nucleotide and amino acid sequence information prepared using PatentIn Version 3.3, presented herein after the claims. Each nucleotide sequence is identified in the sequence listing by the numeric indicator <210> followed by the sequence identifier (e.g. <210>1, <210>2, <210>3, etc). The length and type of sequence (DNA, protein (PRT), etc), and source organism for each nucleotide sequence, are indicated by information provided in the numeric indicator fields <211>, <212> and <213>, respectively. Nucleotide sequences referred to in the specification are defined by the term "SEQ ID NO:", followed by the sequence identifier (eg. SEQ ID NO: 1 refers to the sequence in the sequence listing designated as <400>1).

The designation of nucleotide residues referred to herein are those recommended by the IUPAC-IUB Biochemical Nomenclature Commission, wherein A represents Adenine, C represents Cytosine, G represents Guanine, T represents thymine, Y represents a pyrimidine residue, R represents a purine residue, M represents Adenine or Cytosine, K represents Guanine or Thymine, S represents Guanine or Cytosine, W represents Adenine or Thymine, H represents a nucleotide other than Guanine, B represents a nucleotide other than Adenine, V represents a nucleotide other than Thymine, D represents a nucleotide other than Cytosine and N represents any nucleotide residue.

As used herein the term "derived from" shall be taken to indicate that a specified integer may be obtained from a particular source albeit not necessarily directly from that source.

Throughout this specification, unless the context requires otherwise, the word "comprise", or variations such as "comprises" or "comprising", will be understood to imply the inclusion of a stated step or element or integer or group of steps or elements or integers but not the exclusion of any other step or element or integer or group of elements or integers.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

Each embodiment described herein is to be applied *mutatis mutandis* to each and every other embodiment unless specifically stated otherwise.

The present invention is performed without undue experimentation using, unless otherwise indicated, conventional techniques of molecular biology, microbiology, virology, recombinant DNA technology, peptide synthesis in solution, solid phase peptide synthesis, and immunology. Such procedures are described, for example, in the following texts:
1. Sambrook, Fritsch & Maniatis,, whole of Vols I, II, and III;
2. DNA Cloning: A Practical Approach, Vols. I and II (D. N. Glover, ed., 1985), IRL Press, Oxford, whole of text;
3. Oligonucleotide Synthesis: A Practical Approach (M. J. Gait, ed., 1984) IRL Press, Oxford, whole of text, and particularly the papers therein by Gait, pp1-22; Atkinson *et al.,* pp35-81; *Sproat et al.,* pp 83-115; and *Wu et al.,* pp 135-151;
4. Nucleic Acid Hybridization: A Practical Approach (B. D. Hames & S. J. Higgins, eds., 1985) IRL Press, Oxford, whole of text;
5. Animal Cell Culture: Practical Approach, Third Edition (John R.W. Masters, ed., 2000), ISBN 0199637970, whole of text;
6. Immobilized Cells and Enzymes: A Practical Approach (1986) IRL Press, Oxford, whole of text;
7. Perbal, B., A Practical Guide to Molecular Cloning (1984);
8. Methods In Enzymology (S. Colowick and N. Kaplan, eds., Academic Press, Inc.), whole of series;
9. J.F. Ramalho Ortigão, "The Chemistry of Peptide Synthesis" In: Knowledge database of Access to Virtual Laboratory website (Interactiva, Germany);
10. Sakakibara, D., Teichman, J., Lien, E. Land Fenichel, R.L. (1976). Biochem. Biophys. Res. Commun. 73 336-342
11. Merrifield, R.B. (1963). J. Am. Chem. Soc. 85, 2149-2154.
12. Barany, G. and Merrifield, R.B. (1979) in The Peptides (Gross, E. and Meienhofer, J. eds.), vol. 2, pp. 1-284, Academic Press, New York.
13. Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart.
14. Bodanszky, M. (1984) Principles of Peptide Synthesis, Springer-Verlag, Heidelberg.
15. Bodanszky, M. & Bodanszky, A. (1984) The Practice of Peptide Synthesis, Springer-Verlag, Heidelberg.
16. Bodanszky, M. (1985) Int. J. Peptide Protein Res. 25, 449-474.
17. Handbook of Experimental Immunology, Vols. I-IV (D. M. Weir and C. C. Blackwell, eds., 1986, Blackwell Scientific Publications).
18. McPherson et al., In: PCR A Practical Approach., IRL Press, Oxford University Press, Oxford, United Kingdom, 1991.
19. Methods in Yeast Genetics: A Cold Spring Harbor Laboratory Course Manual (D. Burke et al., eds) Cold Spring Harbor Press, New York, 2000 *(see whole of text).*
20. Guide to Yeast Genetics and Molecular Biology. In: Methods in Enzymology Series, Vol. 194 (C. Guthrie and G.R. Fink eds) Academic Press, London, 1991 2000 *(see whole of text*).

### 2. Description of the related art.

### Peptide therapeutics

As a response to the increasing demand for new lead compounds and new target identification and validation reagents, the pharmaceutical industry has increased its screening of various sources for new lead compounds having a unique activity or specificity in therapeutic applications, such as, for example, in the treatment of neoplastic disorders, infection, modulating immunity, autoimmunity, fertility, etc.

It is known that proteins bind to other proteins, antigens, antibodies, nucleic acids, and carbohydrates. Such binding enables the protein to effect changes in a wide variety of biological processes in all living organisms. As a consequence, proteins represent an important source of natural modulators of phenotype. Accordingly, peptides that modulate the binding activity of a protein represent attractive lead compounds (drug candidates) in primary or secondary drug screening. For example, the formation of a target biological interaction that has a deleterious effect (eg. replication of a pathogen or of a cancer cell), can be assayed to identify lead compounds that antagonize the biological interaction.

It is widely recognized that there is a need to develop methods for determining novel compounds, including nucleic acid-based products and peptide-based products, that modulate an activity or function of a particular target. In such approaches, an activity of a target protein or nucleic acid is screened in the absence and presence of a potential lead compound, which is a peptide, and modified activity of the target is determined.

Similarly, peptides can be used as dominant negative inhibitors or the validation of prospective drug targets using assays such as observing the phenotype resulting from over-expression of the peptides in ex-vivo assays or in transgenic mice.

### Screening methods

In one known approach to identify novel lead compounds, random peptide (synthetic mimetic or mimotope) libraries are produced using short random oligonucleotides produced by synthetic combinatorial chemistry. The DNA sequences are cloned into an appropriate vehicle for expression and the encoded peptide is then screened using one of a variety of approaches. However, the ability to isolate active peptides from random fragment libraries can be highly variable with low affinity interactions occurring between the peptide-binding partners. Moreover, the expressed peptides often show little or none of the secondary or tertiary structure required for efficient binding activity, and/or are unstable. This is not surprising, considering that biological molecules appear to recognize shape and charge rather than primary sequence (Yang and Honig J. Mol. Biol 301(3), 691-711 2000) and that such random peptide aptamers are generally too small to comprise a protein domain or to form the secondary structure of a protein domain. The relatively unstructured 'linear' nature of these peptide aptamers also leads to their more rapid degradation and clearance following administration to a subject *in vivo,* thereby reducing their appeal as therapeutic agents.

To enhance the probability of obtaining useful bioactive peptides or proteins from random peptide libraries, peptides have previously been constrained within scaffold structures, eg., thioredoxin (Trx) loop *(*Blum et al. Proc. Natl. Acad. Sci. USA, 97, 2241-2246, 2000) or catalytically inactive staphylococcal nuclease (Norman et al, Science, 285, 591-595, 1999), to enhance their stability. Constraint of peptides within such structures has been shown, in some cases, to enhance the affinity of the interaction between the expressed peptides and its target, presumably by limiting the degrees of conformational freedom of the peptide, and thereby minimizing the entropic cost of binding.

It is also known to tailor peptide expression libraries for identifying specific peptides involved in a particular process, eg., antigen-antibody-binding activity. For example US Patent No 6,319,690 (Dade Behring Marburg GmBH) teaches a PCR-based method of amplifying cDNA sequences encoding a population of antibodies, wherein oligonucleotide primers that are homologous to conserved regions of antibody-encoding cDNAs derived from a mixture of non-activated B- lymphocytes are used to amplify nucleic acids that encode antibody variable regions. The amplified sequences are expressed using a bacterial display system, for screening with selected antigens to determine those antibody fragments that bind the antigens. However, the expression libraries described in US Pat. No. 6,319,690 show limited diversity, because the amplified fragments were all antibody-encoding fragments derived from a single complex eukaryote. Additionally, the antibody-encoding libraries described in US Pat. No. 6,319,690 were screened for antigen-binding activity rather than for a novel bioactivity (ie. the expressed peptides were not mimotopes).

Several attempts have been made to develop libraries based on naturally occurring proteins (eg genomic expression libraries). Libraries of up to several thousand polypeptides or peptides have been prepared by gene expression systems and displayed on chemical supports or in biological systems suitable for testing biological activity. For example, genome fragments isolated from *Escherichia coli* MG1655 have been expressed using phage display technology, and the expressed peptides screened to identify peptides that bind to a polyclonal anti-Rec A protein antisera (Palzkill et al. Gene, 221 79-83, 1998). Such expression libraries are generally produced using nucleic acid from single genomes, and generally comprise nucleic acid fragments comprising whole genes and/or multiple genes or whole operons, including multiple linked protein domains of proteins. Additionally, as many bacteria comprise recA-encoding genes, the libraries described by Palzkill *et al.,* were screened for an activity that was known for the organism concerned, rather than for a novel bioactivity (ie. the expressed peptides were not necessarily mimotopes).

US Patent No. 5,763,239 (Diversa Corporation) describes a procedure for producing normalized genomic DNA libraries from uncharacterized environmental samples containing a mixture of uncharacterized genomes. The procedure described by Diversa Corp. comprises melting DNA isolated from an environmental sample, and allowing the DNA to reanneal under stringent conditions. Rare sequences, that are less likely to reanneal to their complementary strand in a short period of time, are isolated as single-stranded nucleic acid and used to generate a gene expression library. However, total normalization of each organism within such uncharacterized samples is difficult to achieve, thereby reducing the biodiversity of the library. Such libraries also tend to be biased toward the frequency with which a particular organism is found in the native environment. As such, the library does not represent the true population of the biodiversity found in a particular biological sample. In cases where the environmental sample includes a dominant organism, there is likely to be a significant species bias that adversely impacts on the sequence diversity of the library. Furthermore, as many of the organisms found in such samples are uncharacterized, very little information is known regarding the constitution of the genomes that comprise such libraries. Accordingly, it is not possible to estimate the true diversity of such libraries. Additionally, since the Diversa Corp. process relies upon PCR using random primers to amplify uncharacterized nucleic acids, there is no possibility of accounting for biasing factors, such as, for example, a disproportionate representation of repeated sequences across genomes of the organisms in the environmental sample.

Accordingly, there remains a need to produce improved methods for constructing highly diverse and well characterized expression libraries wherein the expressed peptides are capable of assuming a secondary structure or conformation sufficient to bind to a target protein or nucleic acid, such as, for example, by virtue of the inserted nucleic acid encoding a protein domain.

As used herein, the term "protein domain" shall be taken to mean a discrete portion of a protein that assumes a secondary structure or conformation sufficient to permit said portion to perform a specific function in the context of a target protein or target nucleic acid and, in particular, to bind with high affinity to the target protein or nucleic acid. Preferred protein domains are not required to be constrained within a scaffold structure to bind to the target nucleic acid or target protein, or for said binding to be enhanced.

The term "protein domain" or "domain" or similar shall be taken to include an independently folding peptide structure (ie. a "subdomain") unless the context requires otherwise. For example, protein subdomain consisting of a 19-residue fragment from the C-loop of the fourth epidermal growth factor-like domain of thrombomodulin has been described by Alder et al, J. Biol. Chem., 270: 23366-23372, 1995. Accordingly, the skilled artisan is aware of the meaning of the term "protein subdomain".

There also remains a need to screen such libraries to identify those peptides that modulate the activity of a target protein or nucleic acid by virtue of assuming or presenting a secondary and/or tertiary structure that is compatible with the target albeit not necessary iterative of a structure in the target. Selection based on such conformational features, rather than mere primary structure, provides the advantage of indicating a wide range of useful therapeutic and diagnostic compounds that are checmically unrelated, yet modulate activity of the same target.

### Ischemia/stroke

Stroke is the second leading cause of death and the leading single cause of disability in Australia. As used herein, the term "stroke" includes any ischemic disorder e.g., a peripherial vascular disorder, a venous thrombosis, a pulmonary embolus, a myocardial infarction, a transient ischemic attack, lung ischemia, unstable angina, a reversible ischemic neurological deficit, adjunct thromolytic activity, excessive clotting conditions, reperfusion injury, sickle cell anemia, a stroke disorder or an iatrogenically induced ischemic period such as angioplasty.

The direct and indirect cost of stroke to the Australian community is estimated to be over $2 billion annually. Currently, there is no effective clinical agent that inhibits the delayed neuronal cell death associated with stroke, and thought to be the major cause of long term brain damage associated with stroke. Treatment of acute ischemic stroke has focused on the disruption of the formed clot. Drugs such as Activase (genetically engineered tissue plasminogen activator; Genentech), Abciximab (a platelet inhibtor; Centocor), and Ancrod (fibrinogenolytic) have had limited success if administered soon after the stroke occurs. Even alternative approaches that target the glutamate receptor antagonists to prevent neuronal damage have shown no significant or consistent improvements in patient outcome, most likely due to the need to target these events early in stroke.

### Involvement of the MAPK kinase pathway in ischemia

Various types of evidence indicate that c-Jun N-Terminal Kinase (JNK or SAPK) is involved in neuronal cell death during or following ischemia, via activation of the c-Jun N-Terminal Kinase (JNK) pathway.

Components of the JNK pathway associate with scaffold proteins that modulate their activities and cellular localization. Similar to other mitogen-activated protein kinases (MAPKs), JNK activity is controlled by a cascade of protein kinases and by protein phosphatases, including dual-specificity MAPK phosphatases. For example, the JNK-interacting protein-1 (JIP-1) scaffold protein specifically binds JNK, MAPK kinase 4 (MKK4) and MAPK kinase 7 (MKK7), and members of the mixed lineage kinase (MLK) family, and regulates JNK activation in neurons. Distinct regions within the N termini of MKK7 and the MLK family member dual leucine zipper kinase (DLK) mediate their binding to JIP-1. JNK binds to c-Jun, and this appears to be required for efficient c-Jun phosphorylation.

Several members of the death-related JNK/c-Jun pathway acting upstream of JNK have been defined. The most distal of these are the Rho small GTPase family members Rac1 and Cdc42. Over expression of constitutively active forms of Rac1 (i.e., Rac1V12) and Cdc42 (i.e., Cdc42V12) leads to activation of the JNK pathway and to death of Jurkat T lymphocytes, PC 12 cells, and sympathetic neurons. Conversely, over expression of dominant-negative mutants of Cdc42 (i.e., Cdc42N17) and Rac1 (i.e., Rac1N17) in sympathetic neurons prevents elevation of c-Jun and death evoked by nerve growth factor (NGF) withdrawal (Bazenet et al., Proc. Natl. Acad. Sci. USA 95, 3984-3989, 1998; Chuang et al., Mol. Biol. Cell 8, 1687-1698, 1997). Over expression of the dominant negative mutant Rac1N17 also reverses the induction of death by Cdc42V12, whereas Cdc42N17 has no effect on Rac1V12-induced death, suggesting that Cdc42 lies upstream of Rac1 (Bazenet et al., Proc. Natl. Acad. Sci. USA 95, 3984-3989, 1998). Similar approaches have indicated that mitogen-activated protein kinase kinases 4 and 7 (MKK4 and MKK7) lie downstream of Cdc42 and Rac1 and directly upstream of the JNKs *(*Foltz et al., J Biol. Chem. 273, 9344-9351, 1998; Holland et al., J. Biol. Chem. 272, 24994-24998, 1997; Mazars et al., Oncogene 19, 1277-1287, 2000; Vacratsis et al., J Biol. Chem. 275, 27893-27900, 2000; Xia et al., Science 270, 1326-1331, 1995; Yamauchi et al., J Biol. Chem. 274, 1957-1965, 1999). Studies using constitutively active and dominant-negative constructs have also implicated apoptosis signal-regulating kinase 1 (ASK1) as an additional participant in the pathway that lies between Cdc42 and the downstream MKKs and JNKs (Kanamoto iet al., Mol. Cell. Biol. 20, 196-204, 2000).

MLKs have been shown to function as MKK kinases and lead to activation of JNKs via activation of MKKs *(*Bock et al., J. Biol. Chem. 275, 14231-1424, 2000; Cuenda et al., Biochem. J. 333, 11-159, 1998; Hirai et al., J. Biol. Chem. 272, 15167-15173, 1997; Merritt et al., J Biol. Chem. 274, 10195-10202, ;1999; Rana et al., J Biol. Chem. 271, 19025-19028, 1996; Tibbles et al., EMBO J 15, 7026-7035, 1996; Vacratsis et al., J Biol. Chem. 275, 27893-27900, 2000). Members of the family include MLK1, MLK2 (also called MST), MLK3 (also called SPRK or PTK1), dual leucine zipper kinase (DLK; also called MUK or ZPK), and leucine zipper-bearing kinase (LZK). Constitutively active mutants of Rac1 and Cdc42 have been found to bind to and to modulate the activities of MLK2 and -3, and co-expression of MLK3 and activated Cdc42 leads to enhanced MLK3 activation.

In animal models of ischemia or stroke, apoptotic neurons have enhanced phosphorylation of the transcription factor c-Jun by JNK. Additionally, neuronal c-Jun levels are elevated in response to trophic factor withdrawal, and dominant-negative forms of this transcription factor are at least partially-protective against neuronal cell death evoked by selective activation of JNKs (Eilers et al., J Neurosci. 18, 1713-1724, 1998; Ham et al., Neuron 14, 927-939).

The transcriptional activating activity of c-Jun is regulated at the post-translational level by its phosphorylation by JNK (SAPK) at two residues within the amino-terminal trans-activation domain, serines 63 and 73, in response to a variety of cellular stresses. Phosphorylation of these two residues is critical for the transcriptional activating activity of c-Jun, since mutation of them markedly decreases this activity. JNKs (SAPKs) readily phosphorylate c-Jun at Ser 63/73, and at a rate that is about 10 times faster than ERK-1 and ERK-2. The JNKs (SAPKs) account for the majority of c-Jun trans-activation domain (Ser 63/73) kinase activity after reperfusion, suggesting that they trigger part of the kidney's very early genetic response to ischemia by enhancing the transcriptional activating activity of c-Jun. Since induction of c-Jun is auto-regulated, it is likely that activation of the JNKs (SAPKs) is, at least in part, responsible for the induction of c-Jun following myocardial or renal ischemia.

The role of JNKs (SAPKs) in the control of gene expression during and/or following ischemia extends well beyond the regulation of c-Jun by JNK. It is known that c-Jun functions primarily as a heterodimer with c-Fos or ATF-2 (a member of the CREB family). When complexed with c-Fos, the dimer is targeted to promoters, such as that of the collagenase gene, containing canonical AP-1 elements. When complexed with ATF-2, however, the dimer appears to prefer CRE sequences, and AP-1 variants such as that contained in the c-Jun promoter which controls induction of c-Jun in response to a variety of stimuli. After ischemia and reperfusion, ATF-2 and c-Jun are targeted as a heterodimer to both ATF/CRE motifs and the Jun2 TRE within the c-Jun promoter. This suggests that, following reperfusion of ischemic tissue, the JNKs (SAPKs) target ATF-2/c-Jun heterodimers to various promoters, including the c-Jun promoter, and enhance transcriptional activating activity of both components of the c-Jun/ATF-2 dimer. This may provide a potent mechanism for the induction of a large number of genes regulated by promoters containing ATF/CRE sites or AP-1 variants to which the heterodimer binds.

Dimerization of c-Jun also leads to apoptosis in neurons in response to ischemia (Tong et al., J. Neurochem 71, 447-459, 1998; Ham et al., Biochem. Pharmacol. 60, 1015-1021, 2000).

A homodimer of c-Jun is also known to activate the c-Jun transcription factor via binding to the transcriptional regulatory element (TRE) in the c-Jun promoter.

As used herein unless specifically stated otherwise or the context requires otherwise, the term "c-Jun dimerization" shall be taken to include homo-dimerization of c-Jun monomers and the partnering of c-Jun with another peptide or polypeptide e.g., JNK, c-Fos, ATF-2.

Similarly, unless specifically stated otherwise or the context requires otherwise, the term "c-Jun dimer" shall be taken to include homo-dimer of c-Jun monomers and a heterodimer of c-Jun with another peptide or polypeptide e.g., JNK, c-Fos, ATF-2.

### Summary of the invention

The present invention is based upon the understanding of the present inventors that proteins that fold well in nature have non-random hydrophobicity distributions (Irback et al., Proc Natl Acad Sci. USA 93, 9533 - 9538, 1996). In any native peptide, the distribution of amino acid residues according to their chemical properties (e.g., hydrophobicity, polarity, etc) is also non-random (Baud and Karlin, Proc Natl Acad. Sci. USA 96, 12494-12499, 1999). Accordingly, the present inventors realized that random peptide libraries have a low frequency of naturally occurring or native peptide conformational structures, secondary structures and/or tertiary structure, such as, for example, formed by protein domains.

In work leading up to the present invention, the inventors sought to take advantage of expression libraries produced, for example, as described in International Patent Application No. PCT/AU00/00414 and US Patent Publication No. 2003-0215846 A1. Additional libraries are described herein. Those expression libraries are well-characterized and highly diverse by virtue of comprising nucleic acid fragments from diverse and well-characterized prokaryotic genomes and/or compact eukaryotic genomes. In particular, the use of combinations of nucleic acid fragments from one or two or more well characterized genomes controls the degree the diversity of peptides/proteins expressed in such expression libraries, to enhance the possibility of isolating novel peptides having the ability to bind to a desired protein or nucleic acid.

For the isolation of modulatory peptides it is to be understood that the bioactive peptides or proteins expressed by individual library clones of such libraries are screened for an activity of the encoded peptide, particularly a binding activity, which said encoded protein has not been shown to possess in the context of the protein from which it was derived (i.e., in its native environment). For example, local BLAST searching of the peptide sequence against a database of sequences comprised from the source genome used to produce the library identified the organism from which the peptide is derived and the function, if any, ascribed to the peptide in nature. Any library clone encoding a peptide that has the same activity as it would have in its native environment is excluded during the screening process.

The present inventors have now found that is it possible to identify highly conserved specific secondary and/or tertiary structures for peptides identified in such screens, notwithstanding that the primary amino acid sequences of the peptides bear no significant identity to each other or to the target protein or nucleic acid against which they were screened. This provides for improved screening assays based on the selection of peptides for their specific conformation, rather than merely selection peptides on the basis of their not having the desired activity in their native environment. The low probability that peptides having very different amino acid sequences and highly conserved structures, as well as the low probability that peptides having conserved structural features and inhibitory activity against a target protein or nucleic acid, enhances the structural consideration, e.g., secondary and/or tertiary structure of the modulatory peptide.

More particularly, the present invention enables to the use of the expression libraries to isolate a nucleic acid that encodes a peptide or protein domain, in particular, a peptide having a conformation sufficient for binding to a target protein or target nucleic acid. This conformation is a product of secondary and/or tertiary structural features and must, by virtue of the peptide binding to its target protein or nucleic acid, be compatible albeit not iterative necessity, of the target protein or target nucleic acid. In accordance with this aspect of the invention, the expression library is screened to identify a peptide encoded by an inserted nucleic acid fragment of the library that binds to a target protein or target nucleic acid, such as, for example to modulate a specific protein:DNA or protein:protein interaction or a structure such as a cell wall or a membrane transport component.

For example, the present inventors have identified a large number of peptides that inhibit Jun dimerization, in a screen of a yeast library comprising combined gene fragments from microorganisms and compact eukaryotes genomes. The identified peptides are useful for preventing or treating stroke or stroke-associated damage in humans and animals, as determined by their deliverability, stability, and efficacy in animal models of stroke (i.e., a focal ischemic model in which stroke caused by embolism is mimicked, and a global ischemic model in which stroke and brain damage associated with cardiac arrest, severe hypotension and head injury are mimicked). In primary screens, selection of peptides was based on their ability to disrupt Jun protein dimerization in a modified yeast reverse two hybrid screening platform and sequence analysis to determine those peptides having sequences not known to be involved in the Jun/JNK interactions in nature (i.e. their native environment).

Those peptides which disrupt Jun dimerization and do not possess this function in nature were further subjected to structural analysis e.g., by searching for secondary and/or tertiary structural features. For example, structural features are determined using appropriate software available on the website of the National Center for Biotechnology Information (NCBI) at the National Institutes of Health, 8600 Rockville Pike, Bethesda MD 20894 such as, for example, through the NCBI Molecules Modeling Database (MMDB) including three-dimensional biomolecular structures determined using X-ray crystallography and/or NMR spectroscopy. The NCBI conserved domain database (CDD) includes domains from the well-known Smart and Pham collections, with links to a 3D-structure viewer (Cn3D). The NCBI Conserved Domain Architecture Retrieval Tool (CDART) uses precalculated domain assignments to neighbor proteins by their domain architecture. By *such in silico* neighboring of peptide inhibitors, the present inventors identified a class of Jun dimerization inhibitory peptides that form a leucine zipper-like structure capable of binding to the leucine zipper of c-Jun thereby inhibiting Jun dimerization. Such peptides may also include an acidic domain capable of binding to the DNA-binding domain of c-Jun thereby preventing docking of c-Jun or Jun dimerization.

*In silico* analysis have also identified a second class of Jun dimerization inhibitory peptides that form novel structures and folds that appear to interact with c-Jun. Precise structural determination of these peptides is performed by a process comprising X-ray crystallography, NMR or circular dichroism.

As used herein, the term "leucine zipper-like" shall be taken to mean a subdomain of an α-helical structure that resembles a classical leucine zipper or a part thereof capable of binding to a protein having a leucine zipper motif (e.g., c-Jun). It is to be understood that a leucine zipper-like subdomain may comprise leucine residues or any combination of leucine-like residues, e.g., isoleucine, valine or methionine, of similar hydrophobicity and/or polarity leucine or leucine-like residues spaced at most about 6-12 residues apart, preferably spaced about 2-6 residues apart or 3-6 residues or 2-4 residues apart, and surrounded by a hydrophobic core. As a single turn of an α-helix consists of about 3.6 amino acid residues, a leucine zipper-like subdomain may have the hydrophobic residues spaced about 3 or 4 residues from each leucine-like residue, to maintain the core. Optimally, each leucine-like residue will be spaced 6 or 7 residues apart, and interspersed by a hydrophobic residue spaced 3 or 4 residues from each leucine-like residue.

Preferably, an acidic domain comprises clustered aspartate or glutamate residues, such as, for example Asp-Asp-Asp-Asp, which interacts with the leucine zipper-like subdomain. In the exemplified embodiment, the acide domain comprises the sequence Asp-Asp-Asp-Asp which interacts with Arg-276, Lys-273 and Arg-270 of the c-Jun leucine zipper.

Accordingly, a method is provided for determining a peptide that binds to a target nucleic acid or target protein comprising:
(a) screening an expression library to identify a peptide expressed by the library that binds to the target protein or target nucleic acid;
(b) selecting any one or more peptides from (a) that do not bind to said target protein or nucleic acid in their native environment; and
(c) selecting one or more peptides from (a) or (b) having conserved secondary structure and/or tertiary structure.

Screening approaches suitable for performing the method include for example, a method selected from the group consisting of yeast-2-hybrid, n-hybrid, reverse-2-hybrid, reverse n-hybrid, split two hybrid, bacterial display, minicell display, phage display, retroviral display, covalent display and *in vitro* display. In a preferred embodiment, the expression library is screened using a phage display method.

Preferably, the screening method further comprises constructing the expression library by a method described herein. Any library produced by such a method, including any of the exemplified expression libraries, is suitable for this purpose. Alternatively or in addition, any suitable expression library is obtained for screening according to the method.

Optionally, a secondary screen is performed, e.g., using Surface Plasmon Resonance (SPR/Biacore) or isothermal calorimetry (ITC) to measure binding of the selected peptides to the immobilized target and selecting those peptides that bind at a specific desired affinity (e.g. high affinity).

Alternatively or in addition, the method further enables determining the ability of a peptide to interact with a target protein or nucleic acid in a heterologous system to that in which the peptide was selected. By "heterologous system" is meant a different cell and/or using a different reporter gene and/or by measuring the interaction of the target protein or nucleic acid with a different binding partner to the interaction of the primary screen. For example, peptides that block c-Jun dimerization in primary yeast reverse hybrid screens can be expressed in mammalian cells in which an expression of different reporter gene (e.g., luciferase) is placed under operable control of AP-1 enhancer elements and dependent on c-Jun dimerization.

The present invention enables the use of any *in silico* analytical method and/or industrial process for carrying the screening methods described herein into a pilot scale production or industrial scale production of a compound identified in such screens. This invention also provides for the provision of information for any such production.

Accordingly, a process is also provided for identifying or determining a compound or modulator *supra,* said method comprising:
(i) performing a method as described herein to thereby identify or determine a peptide capable of forming a conformation sufficient for binding a target protein and/or nucleic acid; and
(ii) providing the compound or the name or structure of the peptide such as, for example, in a paper form, machine-readable form, or computer-readable form.

Optionally, the process further comprises determining the amount of the peptide after (i). Optionally, the process further comprises determining the structure of the peptide after (i).

As used herein, the term "providing the peptide" shall be taken to include any chemical or recombinant synthetic means for producing said compound (with or without derivitisation) or alternatively, the provision of a compound that has been previously synthesized by any person or means.

The compound or the name or structure of the compound can be provided with an indication as to its use e.g., as determined by a screen described herein.

A process is also provided for producing a compound *supra,* said method comprising performing a process for identifying or determining a peptide *supra,* said method comprising:
(i) performing a method as described herein to thereby identify or determine a peptide capable of forming a conformation sufficient for binding a target protein and/or nucleic acid;
(ii) optionally, determining the amount of the peptide;
(iii) providing the name or structure of the peptide such as, for example, in a paper form, machine-readable form, or computer-readable form; and
(v) providing the peptide.

Optionally, the process further comprises determining the structure of the peptide after (i).

Preferably, the method further comprises providing a chemical derivative of the peptide by protection of the amino-or carboxy-terminus, cyclisation of the peptide or construction of the peptide as a retro-inverted peptide.

In a preferred embodiment, the synthesized peptide or the name or structure of the peptide is provided with an indication as to its use e.g., as determined by a screen described herein.

A method is also provided for manufacturing a peptide identified by a method as described above for use in medicine comprising:
(i) performing a method as described herein to thereby identify or determine a peptide capable of forming a conformation sufficient for binding a target protein and/or nucleic acid; and
(ii) using the peptide in the manufacture of a therapeutic or prophylactic for use in medicine.

In one embodiment, the method comprises the additional step of isolating the peptide. Alternatively, a compound is identified and is produced for use in the manufacture of a compound for use in medicine.

The present invention generally relates to the field of an isolated peptide or protein domain that blocks an interaction between two c-Jun proteins, i.e., c-Jun self-dimerization or between c-Jun and another protein e.g., ATF-2, c-Fos or JNK and preferably between c-Jun and ATF-2 or between c-Jun and c-Fos (i.e., a c-Jun heterodimer) or an analogue of said isolated peptide or protein domain. Preferably, the isolated peptide comprises a leucine zipper-like domain or sub-domain and optionally, further comprises an acidic domain or sub-domain as hereinbefore described. Even more preferably, the isolated peptide or protein domain blocks c-Jun dimerization in a cell.

It will be understood from the disclosure herein that the sequence set forth in comprises fusions between a peptide encoded by the phage vector used to rpoduce the expression library and a peptide encoded by a compact eukaryote or prokaryote genomic DNA inserted into the vector. Thus, the combination of this encoded peptide moiety into novel fusion peptides is one means by which the present invention enables the inhibition of c-jun dimerization.

Alternatively, the amino acid sequence set forth in

The present invention relates to an isolated or recombinant peptide or peptide analogue for use in the treatment of ischemia comprising an amino acid sequence selected from the group consisting of:
(i) the sequence set forth in SEQ ID NO: 134, or SEQ ID NO: 136, and
(ii) a retro-inverted peptide analogue of (i), wherein said analogue comprises the amino acid sequence set forth in SEQ ID NO: 182.

Yao, S. et al; Biopolymers, vol. 47, 1998-01-01, pages 277-283, discloses certain truncated fos peptides which inhibit c-Jun dimerization.

SEQ ID NO: 136 is encoded by the compact eukaryote or prokaryote genome DNA inserted into the vector. Such a peptide also has utility in inhibiting c-Jun dimerization and the present invention clearly encompasses all such peptides (i.e., without flanking phage vector sequences).

The present invention clearly extends to a peptide analogue of an exemplified c-Jun dimerization inhibitory peptide. Particularly preferred analogues of such peptides are retro-inverted (retro-inverso) peptides as indicated, a retro-inverted peptide comprises an amino acid sequence set forth in SEQ ID NO: 182.

The present invention is clearly enabled by any isolated nucleic acid encoding the peptide or protein domain that partially or completely inhibits or antagonizes or blocks c-Jun dimerization in a cell. Exemplary nucleic acids provided herein comprise a nucleotide sequence selected from the group consisting of SEQ ID NO: 133 and SEQ ID NO: 135.

As with the peptide inhibitors of the invention, the present invention clearly extends to sub-groups of the exemplified peptides that comprise the flanking sequence derived from the phage vector, or alternatively, omit such flanking sequences, in accordance with the grouping shown in Table 5 herein.

The present disclosure provides a database comprising the nucleotide sequences of isolated nucleic acid fragments. Preferably, the database incorporates information regarding the secondary structure of the peptides, including predicted structure or a structure as determined by X-ray crystallography or other empirical means.

The present invention also enables of a peptide that inhibits c-Jun dimerization, said analogue comprising a reversed amino acid sequence of a c-Jun dimerization inhibitory peptide of the present invention wherein every amino acid residue inverted (i.e., substituted with a corresponding D-amino acid residue).

The present invention also enables an analogue of a peptide that inhibits c-Jun dimerization, said analogue comprising a reversed amino acid sequence of a c-Jun dimerization inhibitory peptide of the present invention wherein an amino acid residue in said sequence other than glycine is inverted (i.e., substituted with a corresponding D-amino acid residue). Preferably, all amino acid residues other than glycine are inverted.

In a particularly preferred embodiment, the present invention enables an analogue of a peptide that capable of inhibiting c-Jun dimerization, wherein said analogue comprises a complete or partial reverse of an amino acid sequence set forth in SEQ ID NO: 136 and wherein one or more amino acid residues of the reversed amino acid sequence are D-amino acid residues. More preferably, the present invention enables an analogue of a peptide that capable of inhibiting c-Jun dimerization, wherein said analogue comprises (i) a first peptidyl moiety comprising a sequence that consists of complete or partial reverse of an amino acid sequence set forth in SEQ ID NO: 136 and wherein one or more amino acid residues of the reversed amino acid sequence are D-amino acid residues; and (ii) a protein transduction domain optonally separated from (i) by an amino acid spacer.

The present invention also enables a method for determining or validating a target comprising
(a) screening an expression library to identify a peptide expressed by the library that binds to a target protein or target nucleic acid;
(b) selecting one or more peptides from (a) that do not bind to said target protein or nucleic acid in their native environment;
(c) selecting one or more peptides from (a) or (b) having conserved secondary structure and/or tertiary structure; and
(d) expressing a selected peptide in an organism and determining a phenotype of the organism that is modulated by the target protein or target nucleic acid.

The present invention also enables a method for identifying a therapeutic or prophylactic compound comprising
(a) screening an expression library to identify a peptide expressed by the library that binds to a target protein or target nucleic acid;
(b) selecting one or more peptides from (a) that do not bind to said target protein or nucleic acid in their native environment;
(c) selecting one or more peptides from (a) or (b) having conserved secondary structure and/or tertiary structure;
(d) expressing a selected peptide in an organism and determining a phenotype of the organism that is modulated by the target protein or target nucleic acid; and
(e) optionally, identifying a mimetic compound of a peptide that modulated the phenotype of the organism.

The present invention also enables a method for determining the efficacy of a compound in treating or preventing an ischemic disorder such as stroke in a subject, comprising: a) inducing an ischemic disorder in an animal model for ischemic disorders; b) measuring the stroke outcome in said animal, c) comparing the stroke outcome at (b) with the stroke outcome of the animal model in the absence of the compound so as to identify a compound capable of treating or preventing an ischemic disorder in a subject.

The present invention also enables a method of treatment of a disease or disorder comprising administering an effective amount of a peptide identified by a screening method of the present invention or an analogue of said peptide to a subject suffering from the disease and/or disorder or at risk of developing and/or suffering from the disease and/or disorder.

The present invention also enables a method for preventing or treating ischemia or an ischemic event (e.g., stroke) in a subject comprising administering a peptide inhibitor of c-Jun dimerization according to any embodiment described herein or an analogue of said peptide to a subject in need of treatment.

In a preferred embodiment, the present invention enables a method for preventing or treating ischemia or an ischemic event (e.g., stroke) in a subject comprising administering to a subject in need of treatment a peptide that comprises an amino acid sequence selected from the group consisting of SEQ ID NO: 134 and SEQ ID NO: 136, or an analogue of said peptide.

In a related embodiment, the present invention enables the use of a peptide that inhibits the dimerization of c-Jun according to any embodiment described herein or an analogue of said peptide in medicine. Preferred uses in medicine are, for example, in the manufacture of a medicament for the treatment of ischemia or an ischemic event (e.g., stroke) in a subject.

The present invention also enables a method for preventing or treating ischemia or an ischemic event (e.g., stroke) in a subject comprising administering an isolated nucleic acid encoding a c-Jun dimerization inhibitory peptide according to any embodiment described herein or an analogue of said peptide to a subject in need of treatment.

Preferred nucleic acid encoding a c-Jun dimerization inhibitory peptide will comprise a sequence selected from the group consisting of SEQ NO: 133 and SEQ ID NO: 135.

In a related embodiment, the present invention enables the use of an isolated nucleic acid encoding a peptide that inhibits the dimerization of c-Jun according to any embodiment described herein or an analogue of said peptide in medicine. Preferred uses in medicine are, for example, in the manufacture of a medicament for the treatment of ischemia or an ischemic event (e.g., stroke) in a subject.

The present invention clearly encompasses the use of multiple or a plurality of isolated c-Jun dimerization inhibitory peptides or analogues thereof or nucleic acids encoding same in medicine, such as, for example, in the manufacture of a medicament for the treatment of ischemia or an ischemic event (e.g., stroke) in a subject.

### Brief description of the drawings

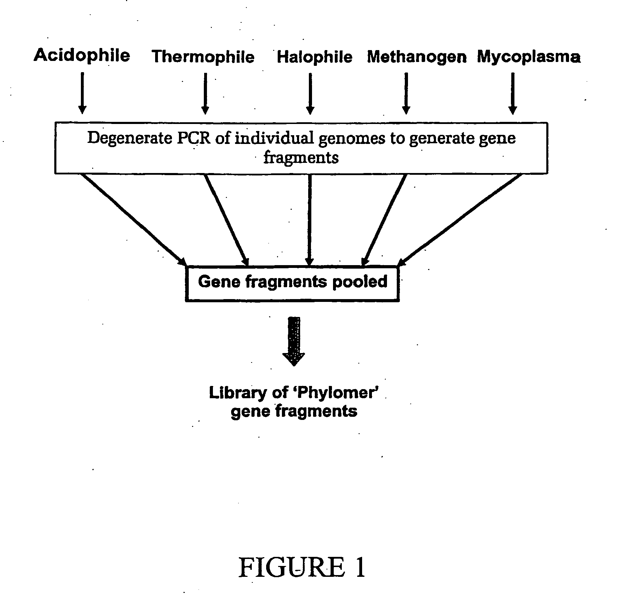

Figure 1 is a schematic representation showing a simplified method of generating an expression library, said library comprising nucleic acid fragments from multiple evolutionary diverse organisms. Initially nucleic acids are isolated from such organisms and pooled in such away as to ensure equal representation of each of the genomes. Degenerate PCR is then used to amplify sequences from the pool of the genomes, before specific PCR is used to further amplify these nucleic acid fragments in such a way that they may be cloned into an expression vector.

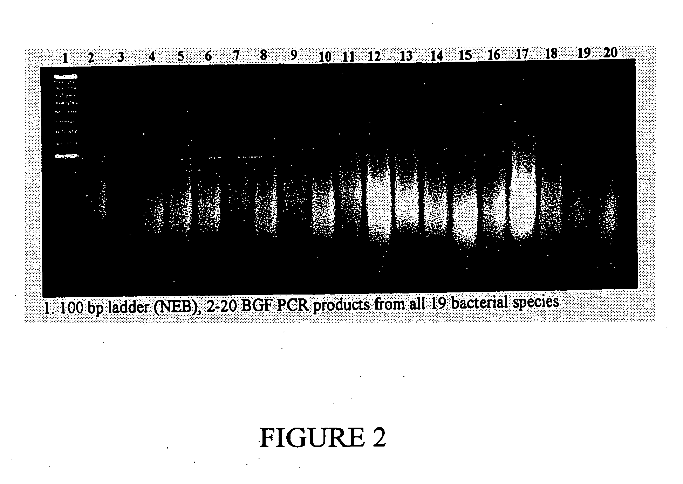

Figure 2 is a photographic representation showing amplification products of random PCR amplification of genomic DNA isolated from *Archaeoglobus fulgidis, Aquifex aeliticus, Aeropyrum pernix, Bacillus subtilis, Bordetella pertussis TOX6, Borrelia burgdorferi, Chlamydia trachomati, Escherichia coli K12, Haemophilus influenzae (rd), Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis PCC 6803, Thermoplasma volcanium,* and *Thermotoga maritima.* The molecular weight marker is shown on the far left.

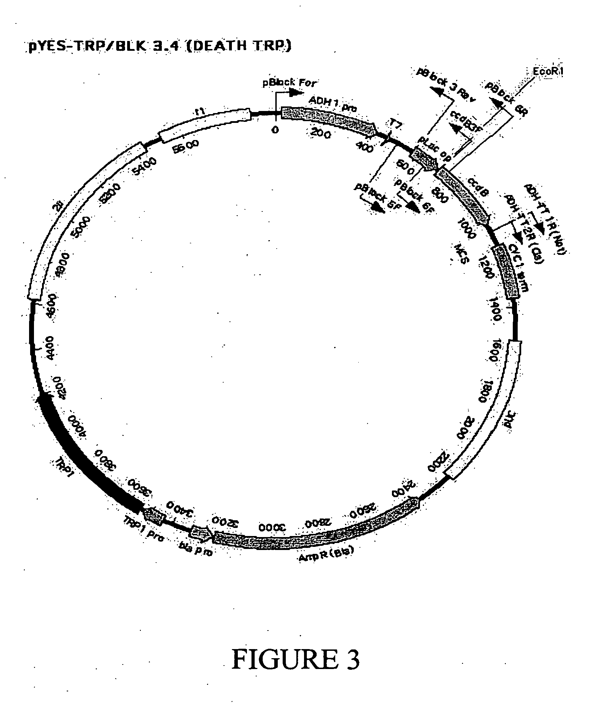

Figure 3 is a schematic representation of the pDEATH-Trp vector (SEQ ID NO: 36). The pDEATH-Trp vector comprises a minimal ADH promoter for constitutive expression of a nucleic acid inserted into the vector in yeast cells; a T7 promoter for expression of a nucleic acid fragment in bacterial cells; a nucleic acid encoding a SV-40 nuclear localization signal to force any expressed polypeptide into the nucleus of a yeast cell; a CYC1 terminator, for termination of transcription in yeast cells; a nucleic acid encoding a peptide conferring ampicillin resistance, for selection in bacterial cells; a nucleic acid encoding TRP1 which allows auxotrophic yeast to grow in media lacking tryptophan; a pUC origin of replication, to allow the plasmid to replicate in bacterial cells; and a 2µ origin of replication, to allow the plasmid to replicate in yeast cells.

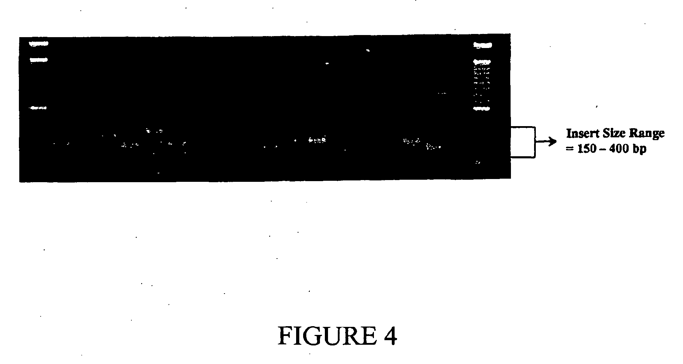

Figure 4 is a photographic representation showing nucleic acid fragments isolated from bacterial clones carrying the pDEATH-Trp vector. The isolated vector was digested with the restriction endonuclease EcoRI and the resulting fragments electrophoresed. The molecular weight marker is shown on the far left and far right, and the text indicates the size range of the nucleic acid fragments in base pairs.

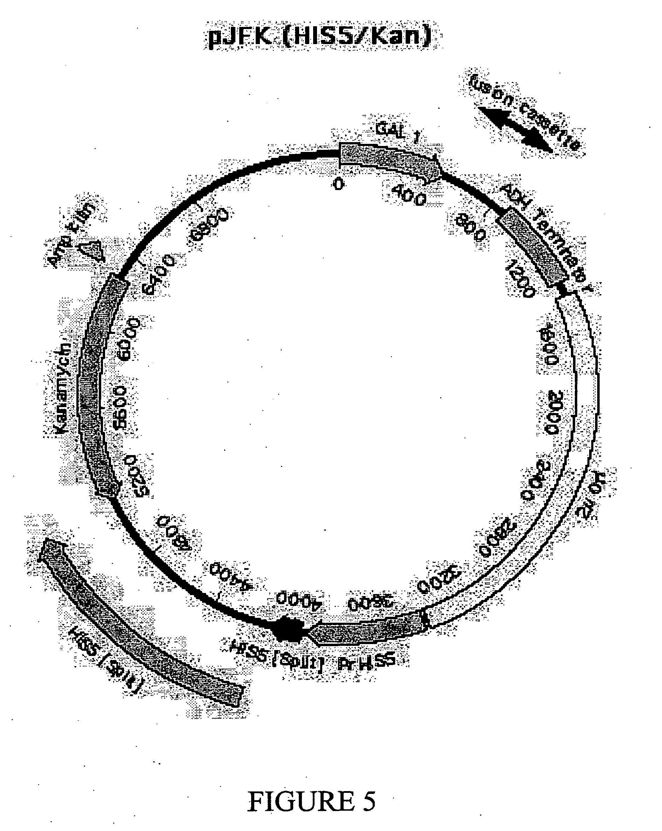

Figure 5 is a schematic representation of the pJFK vector (SEQ ID NO: 60). The pJFK vector comprises a GAL1 promoter for inducible expression of a nucleic acid fragment in yeast cells; a nuclear localization signal to force any expressed polypeptide into the nucleus of a yeast cell; a nucleic acid encoding an activation domain derived from the B42 protein, to be expressed as a fusion with a polypeptide of interest in a "n"-hybrid screen; an ADH terminator or termination of transcription in yeast cells; a 2µ origin of replication, to allow the plasmid to replicate in yeast cells; an HIS5 gene to allow auxotrophic yeast to grow in media lacking histidine; a nucleic acid encoding a peptide conferring ampicillin resistance, for selection in bacterial cells; and a nucleic acid encoding a peptide conferring kanamycin resistance.

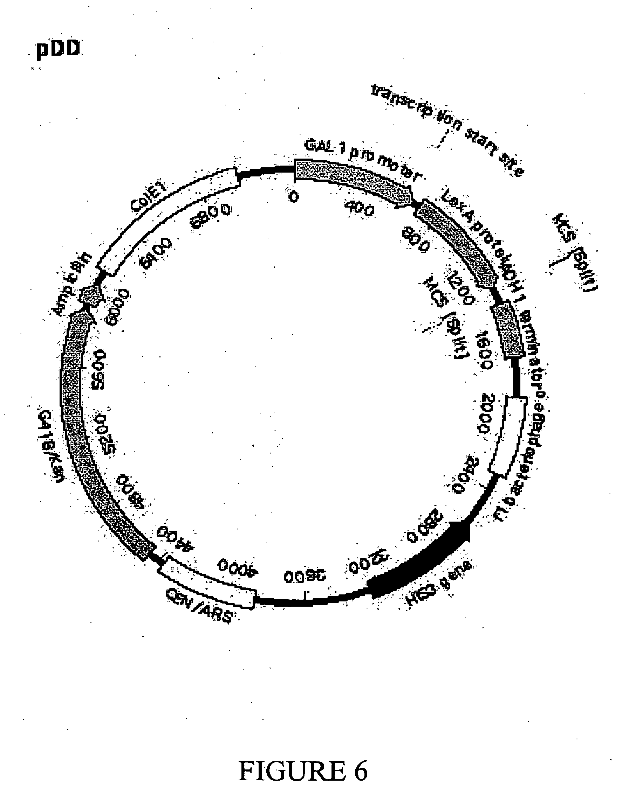

Figure 6 is a schematic representation of the pDD vector (SEQ ID NO: 61). The pDD vector comprises a GAL1 promoter for inducible expression of a nucleic acid fragment in yeast cells; a nucleic acid encoding a LEXA1 protein, to be expressed as a fusion with a polypeptide of interest in a "n"-hybrid screen; an ADH terminator or termination of transcription in yeast cells; a 2µ origin of replication, to allow the plasmid to replicate in yeast cells; an HIS5 gene to allow auxotrophic yeast to grow in media lacking histidine; a nucleic acid encoding a peptide conferring ampicillin resistance, for selection in bacterial cells; and a nucleic acid encoding a peptide conferring kanamycin resistance.

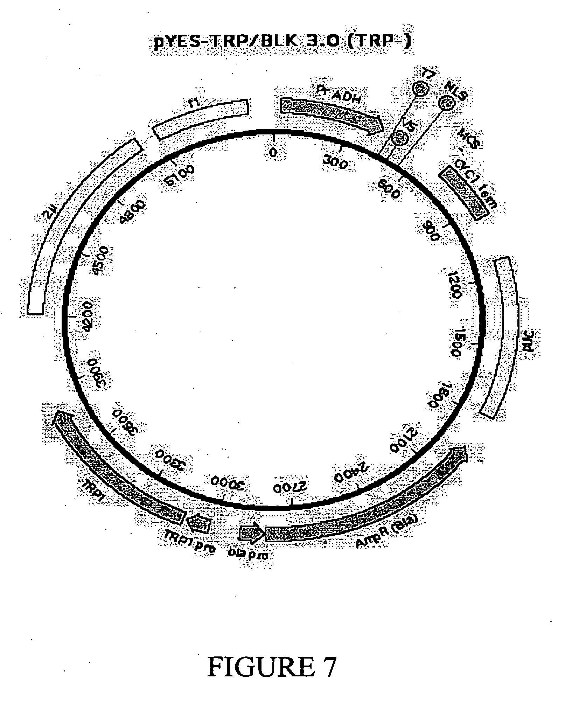

Figure 7 is a schematic representation of the pYTB3 vector (SEQ ID NO: 62). The pYTB vector comprises a minimal ADH promoter for constitutive expression of a nucleic acid fragment in yeast cells, a nuclear localization signal, to target an expressed peptide to the nuclecuis of a yeast cell, a CYC1 terminator for termination of transcription in yeast cells; a 2µ origin of replication, to allow the plasmid to replicate in yeast cells; a TRP1 gene to allow auxotrophic yeast to grow in media lacking tryptophan; a nucleic acid encoding a peptide conferring ampicillin resistance, for selection in bacterial cells; and a pUC origin of replication to allow for replication in bacterial cells. The pYTB3 vector also comprises a T7 promoter to facilitate expression of peptides in bacterial cells and using *in vitro* transcription/translation systems.

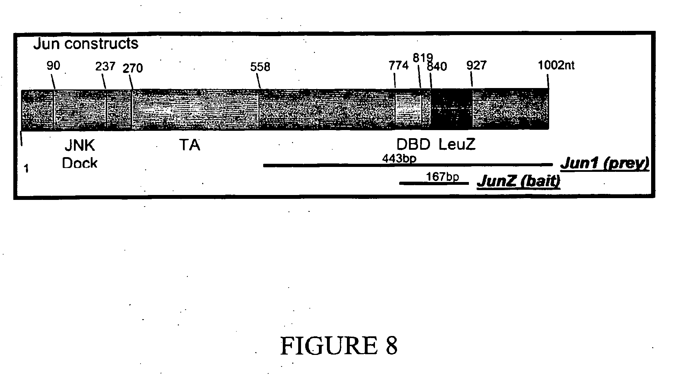

Figure 8 is a schematic representation of a JUN polypeptide. As shown the constructs JUN1 and JUNZ both encompass the DNA binding domain (DBD) and leucine zipper (LeuZ) domain of JUN. The leucine zipper domain is important for homo-dimerization of JUN.

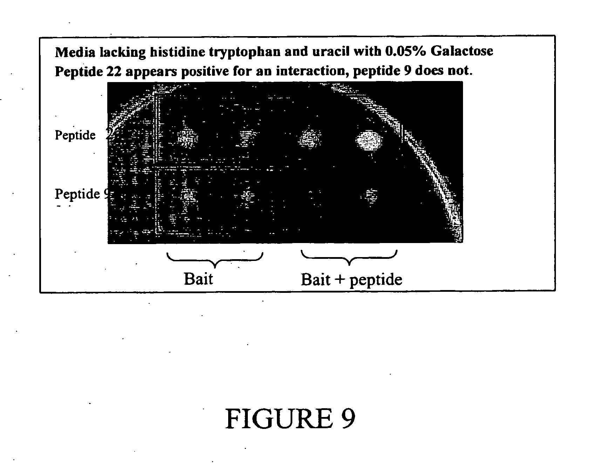

Figure 9 is a graphical representation of a photograph showing yeast colonies expressing JUN1 and a peptide thatinterats with JUN1 (Peptide 22) or JUN1 and a peptide that does not interact with JUN1 (Peptide 9). Also shown are cells expressing only the bait(ie JUN1). Note the increased growth in those cell expressing the interacting polypeptides.

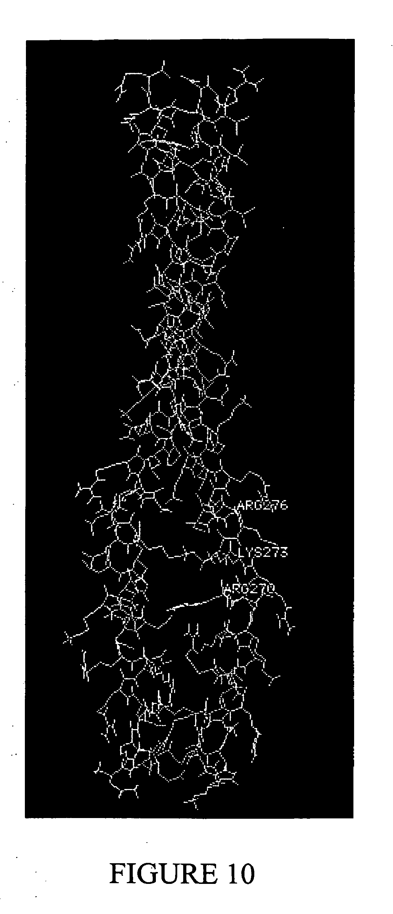

Figure 10 is a graphical representation showing the structure of peptide 22 as determined by threading using the structure of a Jun dimer. The peptide is shown interacting with the leucine zipper of the Jun protein and, in particular, with residues Arg-276, Lys-273 and Arg-270 as indicated.

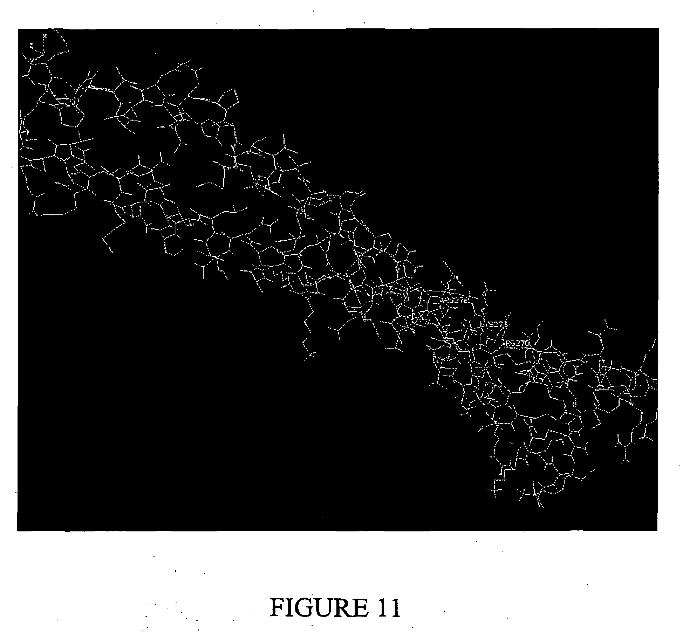

Figure 11 is a graphical representation showing the structure of peptide 22 as determined by threading using the structure of a Jun dimer. Non-polar amino acids that form the core of the peptide that comprises two α-helices are highlighted in blue. The peptide is shown interacting with the leucine zipper of the Jun protein and, in particular, with residues Arg-276, Lys-273 and Arg-270 as indicated.

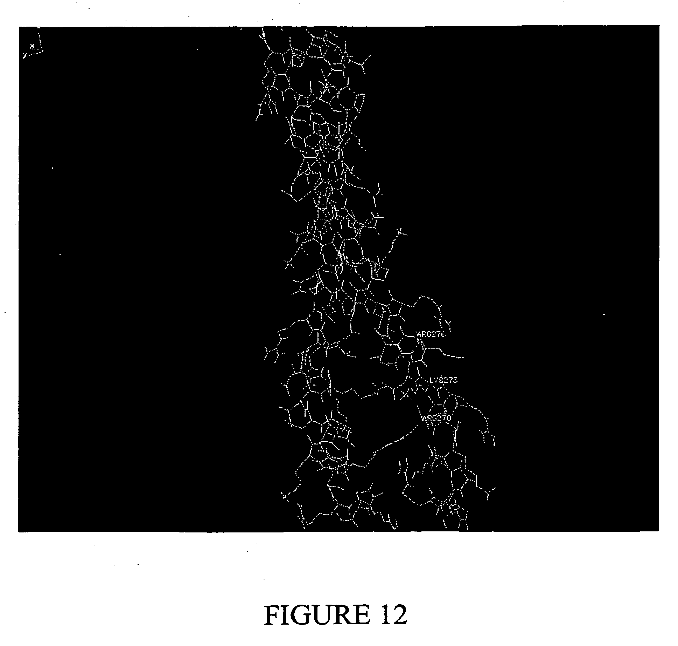

Figure 12 is a graphical representation showing the structure of peptide 22 as determined by threading using the structure of a Jun dimer. Acidic amino acids are highlighted in blue. Amino acids from the FLAG epitope of peptide 22 are shown interacting with residues Arg-276, Lys-273 and Arg-270 of Jun.

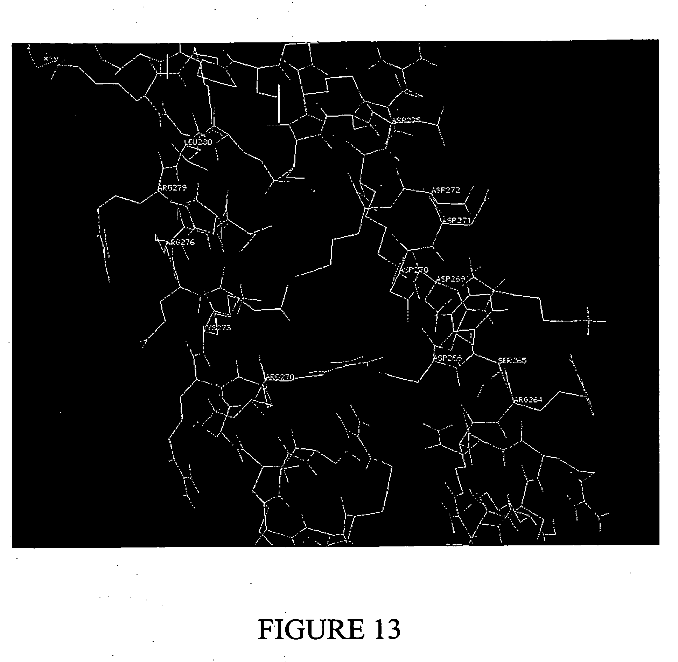

Figure 13 is a graphical representation showing a the FLAG epitope of peptide 22 interacting with residues Arg-276, Lys-273 and Arg-270 of Jun. The structure of the FLAG epitope was determined by threading the sequence of peptide 22 onto the structure of a Jun dimer.

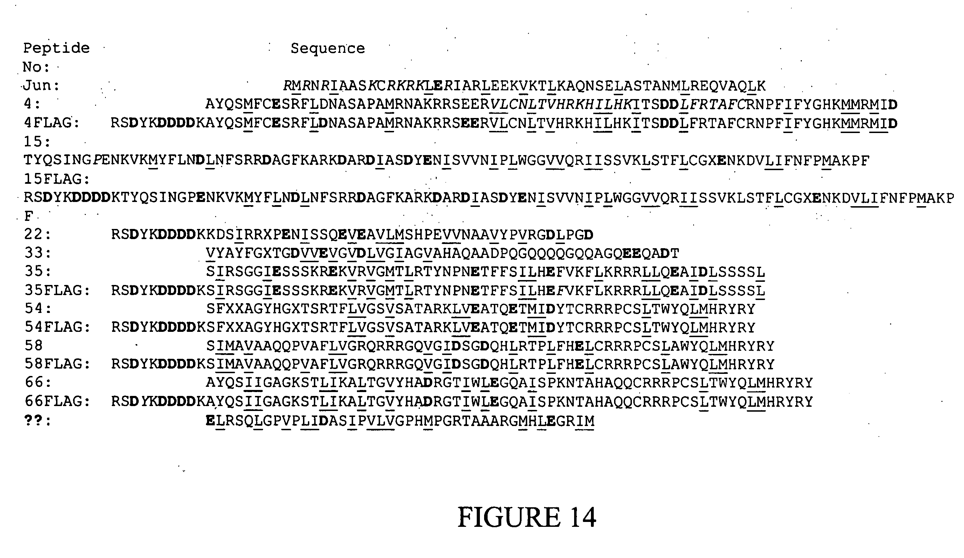

Figure 14 is a graphical representation showing the sequence of several of the c-Jun dimerization inhibitory peptides. Also shown in the location of the amino acid leucine or an equivalent (i.e. valine, isoleucine or methionine) involved in the formation of a leucine zipper like domain (underline). Text in bold font indicates the location of acidic residues involved in interacting with the basic residues of Jun that bind to DNA. The basic residues in Jun are indicated in *italics.*

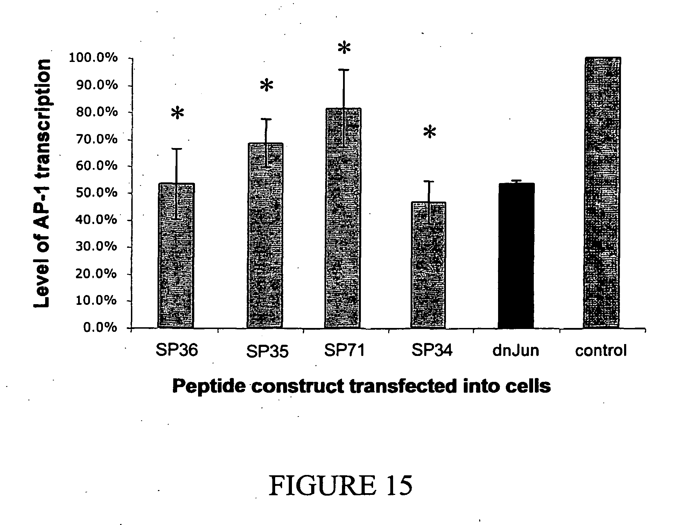

Figure 15 is a graphical representation showing the level of expression of a reporter gene placed operably under control of an AP-1 regulatory element in the presence of a number of peptides identified using the method of the invention. The level of expression is shown as a percentage of control (no peptide). The level of expression identified in cells expressing the following peptides is shown SP35 (SEQ ID NO: 130), SP36 (SEQ ID NO: 134), SP71 (SEQ ID NO: 158), SP34 (SEQ ID NO: 126) and positive control dnJun. Columns representing results from each peptide are indicated. *, p<0.05.

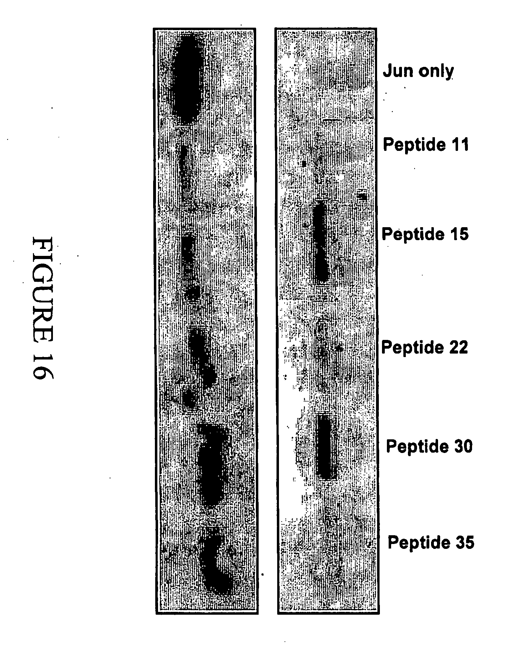

Figure 16 is a copy of a photographic representation showing immunoprecipitation of c-Jun bound to a peptide of the invention. Peptides were captured with an anti-FLAG antibody and proteins separated by SDS-PAGE. c-Jun was then detected with an anti-c-Jun antibody (Top Panel). The total level of c-Jun in each cell is indicated in the Bottom Panel. Peptide identity is indicated at the top of the Top Panel.

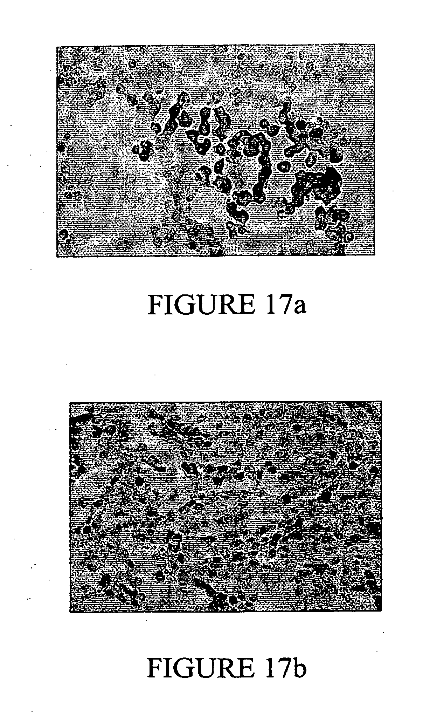

Figure 17a is a copy of a photomicrograph showing the level of TNF-α induced cell death in PC-12 cells. Cells were treated with TNFα and apoptosis determined using TUNEL. Dark stained cells are those undergoing apoptosis.
Figure 17b is a copy of a photomicrograph showing the level of TNF-α induced cell death in PC-12 cells expressing peptide SP36 (SEQ ID NO: 134). Cells were treated with TNFα and apoptosis determined using TUNEL.

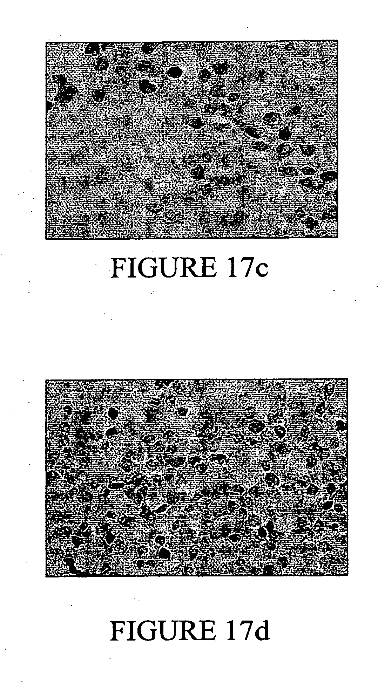

Figure 17c is a copy of a photomicrograph showing the level of TNF-α induced cell death in PC-12 cells expressing peptide SP71 (SEQ ID NO: 158). Cells were treated with TNFα and apoptosis determined using TUNEL.
Figure 17d is a copy of a photomicrograph showing the level of TNF-α induced cell death in PC-12 cells expressing peptide SP34 (SEQ ID NO: 126). Cells were treated with TNFα and apoptosis determined using TUNEL.

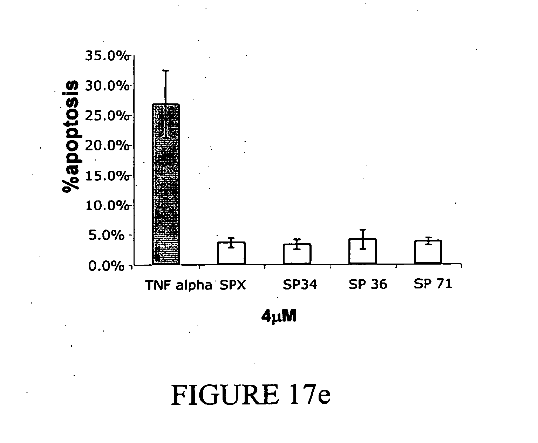

Figure 17e is a graphical representation showing the percentage of PC12 cells undergoing apoptosis following TNFα treatment (i.e., percentage of total cells). Results from control cells are labeled TNF alpha. Results from cells expressing peptide SP34 (SEQ ID NO: 126), SP36 (SEQ ID NO: 134) or SP71 (SEQ ID NO: 158) are indicated.

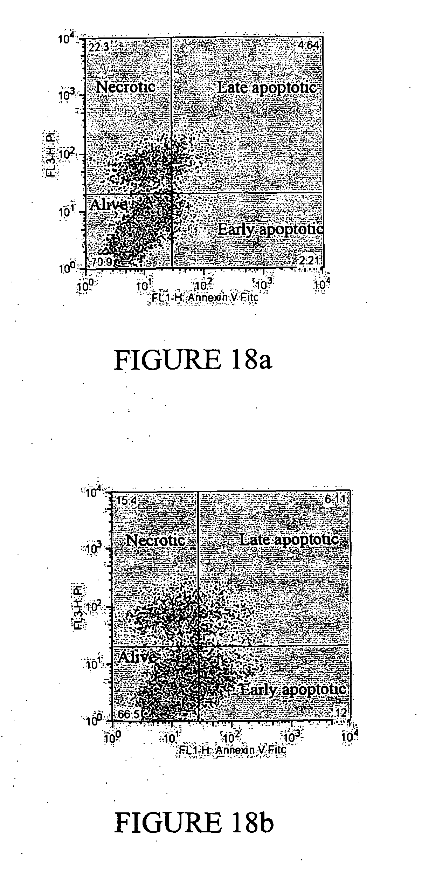

Figure 18a is a graphical representation showing the results of FACS analysis to detect propidium iodide and Annexin V expression to determine the level of cell death in a sample of SIRC cells. Live cells and cells undergoing various forms of cell death are indicated.
Figure 18b is a graphical representation showing the results of FACS analysis to detect propidium iodide and Annexin V expression to determine the level of cell death in a sample of SIRC cells exposed to UV B radiation for 10 minutes. Live cells and cells undergoing various forms of cell death are indicated.

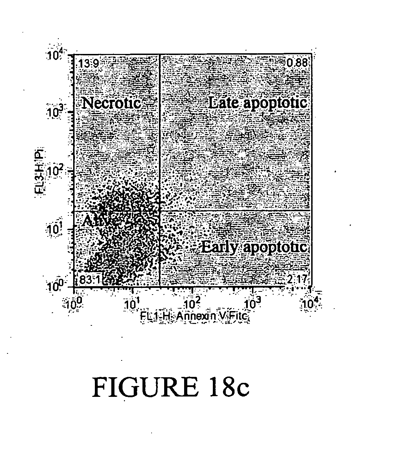

Figure 18c is a graphical representation showing the results of FACS analysis to detect propidium iodide and Annexin V expression to determine the level of cell death in a sample of SIRC cells expressing the peptide SP36 (SEQ ID NO: 134) and exposed to UV B radiation for 10 minutes. Live cells and cells undergoing various forms of cell death are indicated.

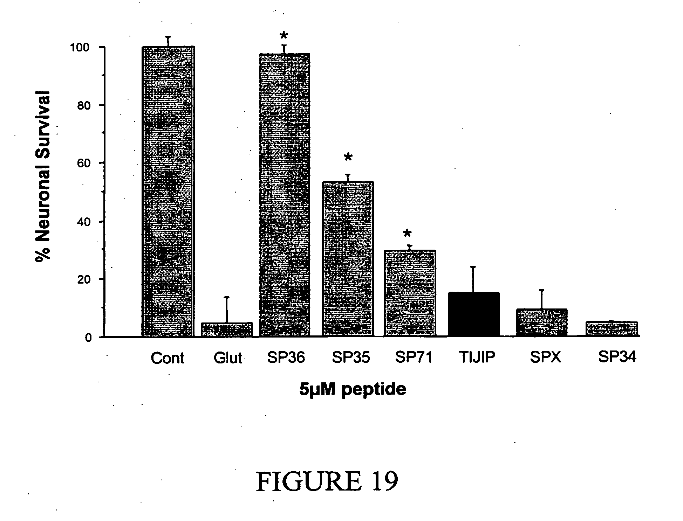

Figure 19 is a graphical representation showing the percentage of primary neurons surviving following exposure to glutamate (relative to control - no glutamate). Results are presented for control (Co), glutamate treated cells (glu), glutamate treated cells expressing SP35 (SEQ ID NO: 130), glutamate treated cells expressing SP36 (SEQ ID NO: 134), glutamate treated cells expressing SP71 (SEQ ID NO: 158), TIJIP and SP34 (SEQ ID NO: 126). *, p<0.05

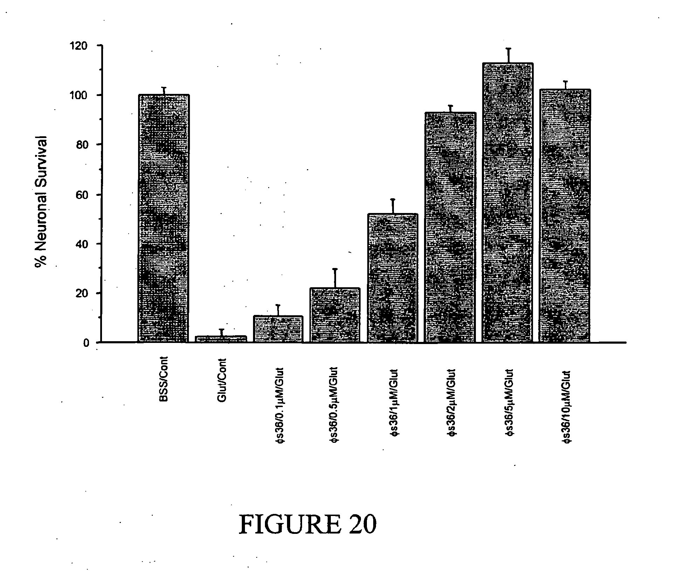

Figure 20 is a graphical representation showing the percentage of primary neurons surviving following exposure to glutamate (relative to control - no glutamate). Results are presented for various doses of peptide SP36 (SEQ ID NO: 134) as indicated.

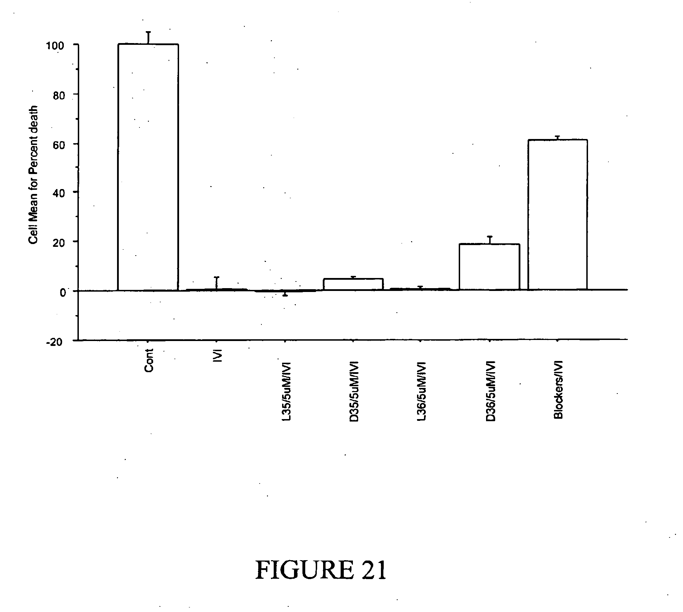

Figure 21 is a graphical representation showing the percentage of cells rescued from glutamate induced cell death (relative to control cells that have not been treated with glutamate). As indicated cells were treated with various concentrations of peptide 35 comprising L amino acids (L35) (SEQ ID NO: 130); peptide 35 comprising D amino acids (D35) (SEQ ID NO: 130); peptide 36 comprising L amino acids (L36) (SEQ ID NO: 134); peptide 36 comprising D amino acids (D36) (SEQ ID NO: 136); TiJIP or known glutamate receptor blockers MK801 and CNQX (blocker).

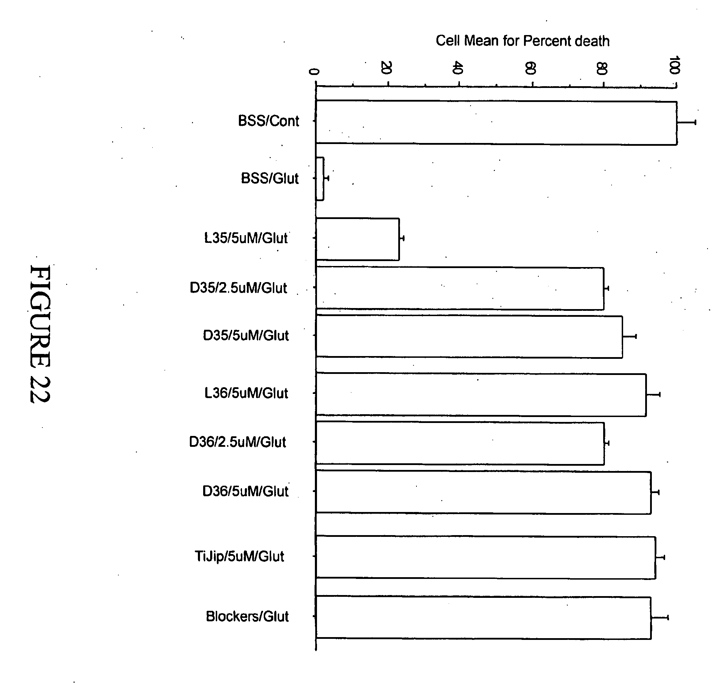

Figure 22 is a graphical representation showing the percentage of cells rescued from hypoxia (exposure to acute anaerobic conditions) induced cell death (relative to control cells that have not been exposed to anaerobic conditions). As indicated cells were treated with various concentrations of peptide 35 comprising L amino acids (L35); peptide 35 comprising D amino acids (D35); peptide 36 comprising L amino acids (L36); peptide 36 comprising D amino acids (D36); or known glutamate receptor blockers MK801 and CNQX (blocker).

### Detailed description of the preferred embodiments

### Suitable expression libraries

Expression libraries for expressing a polypeptide having a conformation sufficient for binding to and/or that binds to a target protein or nucleic acid are constructed as described below.

As used herein, the term "expression library" shall be taken to mean a plurality of nucleic acids cloned into a recombinant expression vector such that the cloned DNA fragments are expressed to produce peptides or proteins. As used herein, the terms "expression", "expressed" or "express" shall be taken to mean at least the transcription of a nucleotide sequence to produce a RNA molecule. The term "expression" "expressed" or "express" further means the translation of said RNA molecule to produce a peptide, polypeptide or protein.

As used herein, the term "having a conformation sufficient for binding to a target protein or nucleic acid" shall be taken to mean that an expressed peptide is capable of achieving a secondary structure and/or tertiary structure sufficient for it to bind to a particular target protein or peptide or polypeptide, or alternatively, a target nucleic acid, preferably in the absence of a constraining peptide such as, for example a Trx loop. Such an affinity is to be interpreted in its broadest context to include, for example, the formation of a peptide:peptide complex, a peptide:protein complex, an antigen:antibody complex, and a peptide:nucleic acid complex.

Accordingly, a peptide "that binds to a target protein or nucleic acid" also achieves the secondary and/or tertiary structure required for such binding to occur.

A preferred means for producing a suitable expression library comprises producing nucleic acid fragments from the genome of one or two or more prokaryotes and/or compact eukaryotes, each of said prokaryotes (and/or microorganisms) and/or compact eukaryotes having a substantially sequenced genome.

The term "fragment" as used herein, shall be understood to mean a nucleic acid that is the same as part of, but not all of a nucleic acid that forms a gene. The term "fragment" also encompasses a part, but not all of an intergenic region.

As used herein, the term "gene" means the segment of nucleic acid, specifically DNA, capable of encoding a peptide or polypeptide, in the present context, a "nucleic acid fragment" is include regions preceding and/or following the coding region of a naturally occurring gene, eg. 5' untranslated or 3' untranslated sequences, as well as intervening sequences between individual coding sequences.

It will be apparent from the disclosure herein that the nucleic acid fragments used to produce the expression libraries in accordance with the present invention do not necessarily encode the same protein or peptide as in their native context (ie. the gene from which they were derived). In fact, in some situations the nucleic acid fragments will encode a hitherto unknown peptide, particularly if derived from a non-coding region of a native gene. All that is required is an open reading frame of sufficient length to encode a peptide or protein domain.

Nucleic acid fragments are generated by one or more of a variety of methods known to those skilled in the art. Such methods include, for example, a method of producing nucleic acid fragments selected from the group consisting of mechanical shearing (e.g., by sonication or passing the nucleic acid through a fine gauge needle), digestion with a nuclease (eg Dnase 1), digestion with one or more restriction enzymes, preferably frequent cutting enzymes that recognize 4-base restriction enzyme sites and treating the DNA samples with radiation (eg. gamma radiation or ultra-violet radiation). Suitable methods are described, for example, in Ausubel et al (In: Current Protocols in Molecular Biology. Wiley Interscience, ISBN 047 150338, 1987) or Sambrook *et al.,* (*In*: ).

In another embodiment, nucleic acid fragments derived from one or two or more organisms are generated by polymerase chain reaction (PCR) using, for example, random or degenerate oligonucleotides. Preferably, such random or degenerate oligonucleotides include restriction enzyme recognition sequences to allow for cloning of the amplified nucleic acid into an appropriate nucleic acid vector. Methods of generating oligonucleotides are known in the art and are described, for example, in Oligonucleotide Synthesis: A Practical Approach (M. J. Gait, ed., 1984) IRL Press, Oxford, whole of text, and particularly the papers therein by Gait, pp1-22; Atkinson *et al.,* pp35-81; Sproat *et al.,* pp 83-115; and *Wu et al.,* pp 135-151. Methods of performing PCR are also described in detail by McPherson et al., In: PCR A Practical Approach., IRL Press, Oxford University Press, Oxford, United Kingdom, 1991.

In a preferred embodiment, the nucleic acid fragment comprises or consists of an open reading frame of nucleotides having a length sufficient to encode a protein domain and preferably, one or two protein domain(s). Examples of protein domains include, for example protein domains selected from the group comprising, helix-loop helix (HLH), leucine zipper, zinc finger, SH2 domain, SH3 domain, WW domain, C2 domain, and proline rich region (PRR), amongst others. However, the present invention is not to be limited to such protein domains. Rather, the present invention contemplates any domain that comprises a sequence of amino acids capable of forming a secondary and/or tertiary structure. Preferably, said structure is stable, more preferably, said structure is stable in the absence of a structural scaffold.

Several studies have shown that the smallest natural domains that are able to fold autonomously consist of about 19 amino acids to about 87 amino acids in length (Gegg et al., Protein Science, 6: 1885-1892, 1997, Yang, Biochemistry 38, 465, 1999, Alder et al., J Biol. Chem., 270: 23366-23372, 1995, Homg. Biochemistry, 41:13360, 2002, Neidigh, Nature Structural Biology, 9:425, 2002). In this context, the term "autonomous" means independent of controlling factors, thus a protein that is able to fold autonomously does so in the absence of factors such as, for example disulphide bonds, ligand binding, or the use of a constraint such as, for example a Trx loop. Accordingly, in one preferred embodiment of the present invention, the nucleic acid fragments of the expression library will consist of an open reading frame sufficient to encode a peptide of at least about 30-50 amino acids in length.

It is also known that factors such as disulphide bonds control the folding of the peptides. US Patent No. 6,361,969 and US Patent No. 6,083,715 describe the expression of protein disulphide isomerases to induce disulphide bond formation in proteins. Studies by Vranken (In: Proteins, 47:14-24, 2002) have suggested that natural protein domains stabilized by disulphide bonding can be as small as 15 to 25 amino acids in length. Accordingly, an alternative embodiment of the present invention uses nucleic acid fragments that consist of an open reading frame sufficient to encode a peptide of at least about about 15 amino acids to about 25 amino acids in length.

As for an upper limit of peptide size, it is preferred that the peptide does not comprise or consist of an entire protein that occurs in nature. Preferably, the peptide comprises one or two or three or four protein domains or folds or sub-domains. More preferably, the peptide comprises one or two protein domains or folds or sub-domains. Accordingly, it is preferable that the peptide comprises fewer than about 200 amino acids, more preferably fewer than about 150 amino acids and even more preferably, fewer than about 120 amino acids. For example, the present inventors have identified a peptide comprising about 99 amino acids that is capable of binding to c-Jun and inhibiting c-Jun dimerization. Furthermore, the present inventors have identified a peptide comprising about 75, 70, 65, 60, 50, 40, 30, 20 or 15 amino acids in length.

It will be apparent from the preceding description that the present invention preferably utilizes nucleic acid fragments having a length of about 45 to about 600 nucleotides in length or about 300 nucleotides in length. However, it is to be understood that some variation from this range is permitted, the only requirement being that, on average, nucleic acid fragments generated encode a protein domain or a peptide comprising about at least about 15 to about 100 amino acids in length, and more preferably at elast about about 20 to about 100 amino acids in length and still more preferably at least about 30 to about 100 amino acids in length.

Methods of producing nucleic acid fragments and separating said fragments according to their molecular weight are known in the art and include, for example, the fragmentation methods *supra* and a method of separation selected from the group consisting of, agarose gel electrophoresis, pulse field gel electrophoresis, polyacrylamide gel electrophoresis, density gradient centrifugation, size exclusion chromatography and mixtures thereof. A number of other methods for separating DNA fragments by their size are known in the art and are described, for example in Sambrook *et al* (In: ).

The genomic nucleic acid is isolated from a variety of sources. In one preferred embodiment, genomic DNA is isolated from a prokaryotic organism. Exemplary prokaryotic sources of nucleic acid fragments include, *Aeropyrum pernix, Agrobacterium tumeficians, Aquifex aeolicus, Archeglobus fulgidis, Baccilus halodurans, Bacillus subtilis, Borrelia burgdorferi, Brucella melitensis, Brucella suis, Bruchnera sp., Caulobacter crescentus, Campylobacter jejuni, Chlamydia pneumoniae, Chlamydia pneumoniae, Chlamydia trachomatis, Chlamydia muridarum, Chlorobium tepidum, Clostridium acetobutylicum, Deinococcus radiodurans, Escherichia coli, Haemophilus influenzae Rd, Halobacterium sp., Helicobacter pylori, Methanobacterium thermoautotrophicum, Lactococcus lactis, Listeria innocua, Listeria monocytogenes, Methanococcus jannaschii, Mesorhizobium loti, Mycobacterium leprae, Mycobacterium tuberculosis, Mycoplasma genitalium, Mycoplasma penetrans, Mycoplasma pneumoniae, Mycoplasma pulmonis, Neisseria meningitidis, Oceanobacillus iheyensis, Pasteurella multocida, Pseudomonas aeruginosa, Pseudomonas putida, Pyrococcus horikoshii , Rickettsia conorii, Rickettsia prowazekii, Salmonella typhi, Salmonella typhimurium, Shewanella oneidensis MR-1, Shigella flexneri 2a, Sinorhizobium meliloti, Staphylococcus aureus, Streptococcus agalactiae, Streptococcus agalactiae, Streptococcus mutans, Streptococcus pneumoniae, Streptococcus pyogenes, Streptomyces avermitilis, Streptomyces coelicolor, Sulfolobus solfataricus, Sulfolobus tokodaii, Synechocystis sp., Thermoanaerobacter tengcongensis, Thermoplasma acidophilum, Thermoplasma volcanium, Thermotoga maritima, Treponema pallidum, Ureaplasma urealyticum, Vibrio cholerae, Xanthomonas axonopodis pv., Citri, Xanthomonas campestris pv., Campestris, Xylella fastidiosa,* and *Yersinia pestis.*

Methods of isolating genomic DNA from prokaryotic organisms are known in the art and are described, for example, in Ausubel et al (In: Current Protocols in Molecular Biology. Wiley Interscience, ISBN 047 150338, 1987) or (Sambrook *et al., In*: ).

In an alternative embodiment, genomic nucleic acid is from a compact eukaryote. As used herein the term "compact eukaryote" shall be taken to mean any organism of the superkingdom Eukaryota that has a haploid genome size of less than about 1700 mega base pairs (Mbp), and preferably, less than 100 Mbp. Exemplary compact eukaryotes that are suitable for this purpose include, for example, *Arabidopsis thaliana, Anopheles gambiae, Brugia malayi, Caenorhabditis elegans, Danio rerio, Drosophila melanogaster, Eimeria tenella, Eimeria acervulina, Entamoeba histolytica, Oryzias latipes, Oryza sativa, Plasmodium falciparum, Plasmodium vivax, Plasmodium yoelii, Sarcocystis cruzi, Saccharomyces cerevesiae, Schizosaccharomyces pombe, Schistosoma mansoni, Takifugu rubripes, Theileria parva, Tetraodon fluviatilis, Toxoplasma gondii, Tryponosoma brucei,* and *Trypanosoma cruzi.*

Furthermore, it is preferred that said eukaryotes having a compact genome have less repetitive nucleotide sequences in their genome than, for example humans. Such information can be determined, for example, from information from NCBI or TIGR.

As used herein the term "NCBI" shall be taken to mean the database of the National Center for Biotechnology Information at the National Library of Medicine at the National Institutes of Health of the Government of the United States of America, Bethesda, MD, 20894.

As used herein the term "TIGR" shall be taken to mean the database of The Institute of Genomic Research, Rockville, MD, 20850.

By way of example, an organism having a compact genome is the Japanese puffer fish, *Takifugu rubripes. T rubripes* has a haploid genome size of approximately 400Mbp, with a gene density of about 16%. This is compared to the human genome, which has a size in excess of 3000Mbp of which only about 3% of nucleotide sequences encode proteins. The absolute number of native genes in the *T. rubripes* genome is comparable to that in the human genome, suggesting fewer repetitive sequences occur in *T. rubripes.* This feature makes *T. rubripes* particularly useful as a source of nucleic acid fragments of the expression libraries. This is because a nucleic acid fragment derived from the genome of a compact eukaryote has an increased probability of encoding a protein domain that is contained within a naturally occurring protein in its native context, compared to a sequence derived from a non-compact eukaryote.

It is to be understood that, whilst such a native domain of a protein is expressed by a library disclosed herein, the invention is not limited to the expression of known protein domains. Moreover, it is to be understood that the expression library is screened using a process that excludes the selection of clones that encode a known protein domain having its native function. Accordingly, the present invention is directed to products and processes for isolating peptides having new or enhanced functions.

Methods of isolating genomic DNA from eukaryotic organisms are known in the art and are described in, for example, Ausubel et al (In: Current Protocols in Molecular Biology. Wiley Interscience, ISBN 047 150338, 1987) or (Sambrook *et al* (In: ).

In a further embodiment of the present invention, the nucleic acid fragments are derived from complementary DNA (cDNA). Those skilled in the art will be aware that cDNA is generated by reverse transcription of RNA using, for example, avian reverse transcriptase (AMV) reverse transcriptase or Moloney Murine Leukemia Virus (MMLV) reverse transcriptase. Such reverse transcriptase enzymes and the methods for their use are known in the art, and are obtainable in commercially available kits, such as, for example, the Powerscript kit (Clontech), the Superscript II kit (Invitrogen), the Thermoscript kit (Invitrogen), the Titanium kit (Clontech), or Omniscript (Qiagen). Such cDNA may then be used to produce nucleic acid fragments, for example, using a method described herein.

Methods for isolating mRNA from a variety of organisms are known in the art and are described for example in, Ausubel et al (In: Current Protocols in Molecular Biology. Wiley Interscience, ISBN 047 150338, 1987) or Sambrook *et al* (*In:* ).

Methods of generating cDNA from isolated RNA are also commonly known in the art and are described in for example, Ausubel et al (In: Current Protocols in Molecular Biology. Wiley Interscience, ISBN 047 150338, 1987) or (Sambrook *et al* (*In*: ).

In a preferred embodiment, the nucleic acid fragments generated from RNA or cDNA are normalized to reduce any bias toward more highly expressed genes. Methods of normalizing nucleic acids are known in the art, and are described, for example, in Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001) and Soares et al Curr. Opinion Biotechnol 8, 542-546, 1997, and references cited therein. One such method (described by Soares) uses reasssociation-based kinetics to reduce the bias of the library toward highly expressed sequences.

Alternatively, cDNA is normalized through hybridization to genomic DNA that has been bound to magnetic beads, as described in Kopczynski et al, Proc. Natl. Acad Sci. USA, 95(17), 9973-9978, 1998. This provides an approximately equal representation of cDNA sequences in the eluant from the magnetic beads. Normalized expression libraries produced using cDNA from one or two or more prokaryotes or compact eukaryotes are clearly contemplated by the present invention.

In a particularly preferred embodiment, the nucleic acid fragments are derived from a prokaryote and/or compact eukaryote having a substantially sequenced genome. An advantage of using such fragments is that bioinformatic data can be assembled and used to provide more complete information about the composition of a library than would be possible using uncharacterized libraries. This facilitates, for example, the generation of DNA arrays containing sequences derived from many or all of the nucleic acid fragments of the library. Methods used in the generation and screening of DNA arrays are known in the art and are described in for example, Schena (In: Microarray Analysis, John Wiley and Sons, ISBN: 0471414433, 2002). The use of a DNA array in the high-throughput analysis of the screening of a biodiverse nucleic acid fragment to determine the sequences of positive clones is contemplated.

As used herein "substantially sequenced genome" shall be taken to mean that at least about 60% of the genome has been sequenced. More preferably at least about 70% of the genome has been sequenced, and more preferably at least about 75% of the genome has been sequenced. Even more preferably at least about 80% of the genome has been sequenced.

Methods for determining the amount of a genome that has been sequenced are known in the art. Furthermore, information regarding those sequences that have been sequenced is readily obtained from publicly available sources, such as, for example, the databases of NCBI or TIGR, thereby facilitating determination of the diversity of the genome.

Organisms having a substantially sequenced genome include, for example, an organism selected from the group consisting of *Actinobacillus pleuropneumoniae serovar, Aeropyrum pernix, Agrobacterium tumeficians, Anopheles gambiae, Aquifex aeolicus, Arabidopsis thaliana, Archeglobus fulgidis, Bacillus anthracis, bacillus cereus, Baccilus halodurans, Bacillus subtilis, Bacteroides thetaiotaomicron, Bdellovibrio bacteriovorus, Bifidobacterium longum, Bordetella bronchiseptica, Bordetella parapertussis, Borrelia burgdorferi, Bradyrhizobium japonicum, Brucella melitensis, Brucella suis, Bruchnera aphidicola, Brugia malayi, Caenorhabditis elegans, Campylobacter jejuni, Candidatus blochmannia floridanus, Caulobacter crescentus, Chlamydia muridarum, Chlamydia trachomatis, Chlamydophilia caviae, Chlamydia pneumoniae, Chlorobium tepidum, Chromobacterium violaceum, Clostridium acetobutylicum, Clostridium perfringens, Clostridium tetani, Corynebacterium diphtheriae, Corynebacterium efficiens, Corynebacterium glutamicum, Coxiella burnetii, Danio rerio, Dechloromonas aromatica, Deinococcus radiodurans, Drosophila melanogaster, Eimeria tenella, Eimeria acervulina, Entamoeba histolytica, Enterococcus faecalis, Escherichia coli, Fusobacterium nucleatum, Geobacter sulfurreducens, Gloeobacter violaceus, Haemophilis ducreyi, Haemophilus influenzae, Halobacterium, Helicobacter hepaticus, Helicobacter pylori, Lactobacillus johnsonii, Lactobacillus plantarum, Lactococcus lactis, Leptospira interrogans serovar lai, Listeria innocua, Listeria monocytogenes, Mesorhizobium loti, Methanobacterium thermoautotrophicum, Methanocaldocossus jannaschii, Methanococcoides burtonii, Methanopyrus kandleri, Methanosarcina acetivorans, Methanosarcina mazei Goel, Methanothermobacter thermautotrophicus, Mycobacterium avium, Mycobacterium bovis, Mycobacterium leprae, Mycobacterium tuberculosis, Mycoplasma gallisepticum strain R, Mycoplasma genitalium, Mycoplasma penetrans, Mycoplasma pneumoniae, Mycoplasma pulmonis, Nanoarchaeum equitans, Neisseria meningitidis, Nitrosomonas europaea, Nostoc, Oceanobacillus iheyensis, Onion yellows phytoplasma, Oryzias latipes, Oryza sativa, Pasteurella multocida, Photorhabdus luminescens, Pirellula, Plasmodium falciparum, Plasmodium vivax, Plasmodium yoelii, Porphyromonas gingivalis, Prochlorococcus marinus, Prochlorococcus marinus, Prochlorococcus, Pseudomonas aeruginosa, Pseudomonas putida, Pseudomonas syringae, Pyrobaculum aerophilum, Pyrococcus abyssi, Pyrococcus furiosus, Pyrococcus horikoshii, Ralstonia solanacearum, Rhodopseudomonas palustris, Rickettsia conorii, Rickettsia prowazekii, Rickettsia rickettsii, Saccharomyces cerevisiae, Salmonella enterica, Salmonella typhimurium, Sarcocystis cruzi, Schistosoma mansoni, Schizosaccharomyces pombe, Shewanella oneidensis, Shigella flexneri, Sinorhizobium meliloti, Staphylococcus aureus, Staphylococcus epidermidis, Streptococcus agalactiae, Streptococcus agalactiae, Streptococcus mutans, Streptococcus pneumoniae, Streptococcus pyogenes, Streptomyces avermitilis, Streptomyces coelicolor, Sulfolobus solfataricus, Sulfolobus tokodaii, Synechocystis sp., Takifugu rubripes, Tetraodon fluviatilis, Theileria parva, Thermoanaerobacter tengcongensis, Thermoplasma acidophilum, Thermoplasma volcanium, Thermosynechococcus elongatus, Thermotoga maritima, Toxoplasma gondii, Treponema denticola, Treponema pallidum, Tropheryma whipplei, Tryponosoma brucei, Trypanosoma cruzi, Ureaplasma urealyticum, Vibrio cholerae, Vibro parahaemolyticus, Vibro vulnificus, Wigglesworthia brevipalpis, Wolbachia endosymbiont of Drosophilia melanogaster, WOlinella succinogenes, Xanthomonas axonopodis pv. Citri, Xanthomonas campestris pv. Campestris, Xylella fastidiosa* and *Yersinia pestis.*

In an alternative embodiment, the library is produced from the genomic DNA of one or more publicly available bacteria having substantially sequenced genomes and being selected from the group consisting of *Acidithiobacillus ferrooxidans, Campylobacter jejuni* subsp. *Jejuni, Caulobacter vibrioides, Colwellia psychrerythraea, Corynebacterium diphtheriae, Desulfovibrio vulgaris* subsp. *Vulgaris, Enterococcus faecalis, Escherichia coli, Geobacter sulfurreducens, Haemophilus actinomycetemcomitans, Haemophilus influenzae, Halobacterium salinarum, Haloferax volcanii, Helicobacter pylori, Klebsiella pneumoniae* subsp. *pneumoniae, Lactobacillus plantarum, Mannheimia haemolytica, Methanococcus jannaschii, Methanococcus maripaludis, Methylobacterium extorquens, Neisseria gonorrhoeae, Neisseria meningitidis, Nitrosomonas europaea, Nostoc* sp., *Novosphingobium aromaticvorans, Oenococcus oeni, Pectobacterium atrosepticum, Porphyromonas gingivalis, Pseudomonas aeruginosa, Pyrococcus furiosus, Pyrococcus horikoshii, Rhizobium radiobacter, Rhodopseudomonas palustris, Salmonella enterica* subsp. *Diarizonae, Salmonella enterica* subsp. *enterica* serovar *Paratyphi A, Salmonella enterica* subsp. *enterica* serovar *Typhi, Salmonella enterica* subsp. *enterica* serovar *Typhimurium, Shewanella oneidensis, Shigella flexneri, Silicibacter pomeroyi, Staphylococcus epidermidis, Streptomyces violaceoruber, Thermoplasma volcanium, Thermotoga maritima, Thermus thermophilus, Thiobacillus ferrooxidans, Ureaplasma urealyticum, Vibrio fischeri, Wautersia metallidurans and Xylella fastidiosa* and combinations thereof.

In an alternate, and/or additional embodiment, nucleic acid fragments are derived from a virus having a substantially sequenced genome. Virus' with a substantially sequenced genomes are known in the art and include, for example, a virus selected from the group consisting of T7 phage, HIV, equine arteritis virus, lactate dehydrogenase-elevating virus, lelystad virus, porcine reproductive and respiratory syndrome virus, simian hemorrhagic fever virus, avian nephritis virus 1, turkey astrovirus 1, human asterovirus type 1, 2 or 8, mink astrovirus 1, ovine astrovirus 1, avian infectious bronchitis virus, bovine coronavirus, human coronavirus, murine hepatitis virus, porcine epidemic diarrhea virus, SARS coronavirus, transmissible gastroenteritis virus, acute bee paralysis virus, aphid lethal paralysis virus, black queen cell virus, cricket paralysis virus, Drosophila C virus, himetobi P virus, kashmir been virus, plautia stali intestine virus, rhopalosiphum padi virus, taura syndrome virus, triatoma virus, alkhurma virus, apoi virus, cell fusing agent virus, deer tick virus, dengue virus type 1, 2, 3 or 4, Japanese encephalitis virus, Kamiti River virus, kunjin virus, langat virus, louping ill virus, modoc virus, Montana myotis leukoencephalitis virus, Murray Valley encephalitis virus, omsk hemorrhagic fever virus, powassan virus, Rio Bravo virus, Tamana bat virus, tick-borne encephalitis virus, West Nile virus, yellow fever virus , yokose virus, Hepatitis C virus, border disease virus, bovine viral diarrhea virus 1 or 2, classical swine fever virus, pestivirus giraffe, pestivirus reindeer, GB virus C, hepatitis G virus, hepatitis GB virus, bacteriophage M11, bacteriophage Qbeta, bacteriophage SP, enterobacteria phage MX1, enterobacteria NL95, bacteriophage AP205, enterobacteria phage fr, enterobacteria phage GA, enterobacteria phage KU1, enterobacteria phage M12, enterobacteria phage MS2, pseudomonas phage PP7, pea enation mosaic virus-1, barley yellow dwarf virus, barley yellow dwarf virus-GAV, barley yellow dwarf virus-MAW, barley yellow dwarf virus-PAS, barley yellow dwarf virus-PAV, bean leafroll virus, soybean dwarf virus, beet chlorosis virus, beet mild yellowing virus, beet western yellows virus, cereal yellow dwarf virus-RPS, cereal yellow dwarf virus-RPV, cucurbit aphid-borne yellows virus, potato leafroll virus, turnip yellows virus, sugarcane yellow leaf virus, equine rhinitis A virus, foot-and-mouth disease virus, encephalomyocarditis virus, theilovirus, bovine enterovirus, human enterovirus A, B, C, D or E, poliovirus, porcine enterovirus A or B, unclassified enterovirus, equine rhinitis B virus, hepatitis A virus, aichi virus, human parechovirus 1, 2 or 3, ljungan virus, equine rhinovirus 3, human rhinovirus A and B, porcine teschovirus 1, 2-7, 8, 9, 10 or 11, avian encephalomyelitis virus, kakugo virus, simian picornavirus 1, aura virus, barmah forest virus, chikungunya virus, eastern equine encephalitis virus, igbo ora virus, mayaro virus, ockelbo virus, onyong-nyong virus, Ross river virus, sagiyama virus, salmon pancrease disease virus, semliki forest virus, sindbis virus, sindbus-like virus, sleeping disease virus, Venezuelan equine encephalitis virus, Western equine encephalomyelitis virus, rubella virus, grapevine fleck virus, maize rayado fino virus, oat blue dwarf virus, chayote mosaic tymovirus, eggplant mosaic virus, erysimum latent virus, kennedya yellow mosaic virus, ononis yellow mosaic virus, physalis mottle virus, turnip yellow mosaic virus and poinsettia mosaic virus.

Information regarding those viral sequences that have been sequenced is readily obtained from publicly available sources, such as, for example, the databases of VirGen and/or NCBI, thereby facilitating determination of the diversity of the genome.

As used herein, the term "VirGen" shall be taken to mean the viral genome resource of the Bioinformatics Centre, University of Pune, Pune 411 007, India.

In a particularly preferred embodiment, nucleic acid fragments are selected that have sufficiently different or divergent nucleotide sequences to thereby enhance nucleotide sequence diversity among the selected fragments compared to the diversity of sequences in the genome from which they were derived.

In one embodiment a nucleic acid fragment is selected such that the encoded polypeptide varies by one or more amino acids with regard to the amino acid sequence of the polypeptide encoded by another fragment in the library, a process that is facilitated using genomes that are substantially sequenced.

In an alternative embodiment, the nucleotide sequence of a nucleic acid fragment is mutated by a process such that the encoded peptide varies by one or more amino acids compared to the "template" nucleic acid fragment. The "template" may have the same nucleotide sequence as the original nucleic acid fragment in its native context (ie. in the gene from which it was derived). Alternatively, the template may itself be an intermediate variant that differs from the original nucleic acid fragment as a consequence of mutagenesis. Mutations include at least one nucleotide difference compared to the sequence of the original fragment. This nucleic acid change may result in for example, a different amino acid in the encoded peptide, or the introduction or deletion of a stop codon. Accordingly, the diversity of the nucleic acids of the expression library and the encoded polypeptides is enhanced by such mutation processes.

In one embodiment, the nucleic acid fragments are modified by a process of mutagenesis selected from the group consisting of, mutagenic PCR, expressing the nucleic acid fragment in a bacterial cell that induces a random mutation, site directed mutagenesis and expressing a nucleic acid fragment in a host cell exposed to a mutagenic agent such as for example radiation, bromo-deoxy-uridine (BrdU), ethylnitrosurea (ENU), ethylmethanesulfonate (EMS) hydroxylamine, or trimethyl phosphate amongst others.

In a preferred embodiment, the nucleic acid fragments are modified by amplifying a nucleic acid fragment using mutagenic PCR. Such a method includes, for example, a process selected from the group consisting of: (i) performing the PCR reaction in the presence of manganese; and (ii) performing the PCR in the presence of a concentration of dNTPs sufficient to result in misincorporation of nucleotides.

Methods of inducing random mutations using PCR are known in the art and are described, for example, in Dieffenbach (ed) and Dveksler (ed) (In: PCR Primer: A Laboratory Manual, Cold Spring Harbour Laboratories, NY, 1995). Furthermore, commercially available kits for use in mutagenic PCR are obtainable, such as, for example, the Diversify PCR Random Mutagenesis Kit (Clontech) or the GeneMorph Random Mutagenesis Kit (Stratagene).

In one embodiment, PCR reactions are performed in the presence of at least about 200µM manganese or a salt thereof, more preferably at least about 300µM manganese or a salt thereof, or even more preferably at least about 500µM or at least about 600µM manganese or a salt thereof. Such concentrations manganese ion or a manganese salt induce from about 2 mutations per 1000 base pairs (bp) to about 10 mutations every 1000 bp of amplified nucleic acid (Leung et al Technique 1, 11-15, 1989).

In another embodiment, PCR reactions are performed in the presence of an elevated or increased or high concentration of dGTP. It is preferred that the concentration of dGTP is at least about 25µM, or more preferably between about 50µM and about 100µM. Even more preferably the concentration of dGTP is between about 100µM and about 150µM, and still more preferably between about 150µM and about 200µM. Such high concentrations of dGTP result in the misincorporation of nucleotides into PCR products at a rate of between about 1 nucleotide and about 3 nucleotides every 1000 bp of amplified nucleic acid (Shaflthani et al BioTechniques 23, 304-306, 1997).

PCR-based mutagenesis is preferred for the mutation of the nucleic acid fragments, as increased mutation rates is achieved by performing additional rounds of PCR.

In another preferred embodiment, the nucleic acid of the expression library is mutated by inserting said nucleic acid into a host cell that is capable of mutating nucleic acid. Such host cells are deficient in one or more enzymes, such as, for example, one or more recombination or DNA repair enzymes, thereby enhancing the rate of mutation to a rate that is rate approximately 5,000 to 10,000 times higher than for non-mutant cells. Suitable bacterial strains carry, for example, alleles that modify or inactivate components of the mismatch repair pathway. Examples of such alleles include alleles selected from the group consisting of *mutY, mutM, mutD, mutT, mutA, mutC* and *mutS.* Bacterial cells that carry alleles that modify or inactivate components of the mismatch repair pathway are known in the art, such as, for example the XL-1Red, XL-*mutS* and XL*-mutS-*Kan^{r} bacterial cells (commercially available from Stratagene).

Alternatively, nucleic acid fragments are cloned into a nucleic acid vector that is preferentially replicated in a bacterial cell by the repair polymerase, *Pol I.* By way of exemplification, a *Pol I* variant strain will induce a high level of mutations in the introduced nucleic acid vector, thereby enhancing sequence diversity of the nucleic acid used to generate the expression library. Such a method is described by Fabret et al (In: Nucl Acid Res, 28, 1-5 2000), which is incorporated herein by reference.

In a further preferred embodiment the mutated nucleic acid fragments are combined with the non-mutated fragments from which they were derived, for subcloning into an expression vector. In this way, the nucleotide diversity of the expression library is enhanced, as is the diversity of the conformations of the expressed peptides and proteins.

In another embodiment, the sequence diversity of a nucleic acid fragment is increased, such as, for example, using a synthetic shuffling technique, such as, for example, the process described by Ness et al, Nature Biotechnology, 20, 1251-1255, 2002, which is incorporated herein by reference. In adapting such a technique to the present invention, functionally homologous nucleic acid fragments are selected from the expression library, using methods described herein. By "functionally homologous" in this context means that the selected fragments bind to the same target protein or target nucleic acid. The amino acid sequence of each peptide that binds to the target is determined using methods known in the art, and the sequences are aligned using an algorithm known in the art. A consensus sequence is determined from the alignment that provides for highly conserved residues, as well as elucidating those residues that are structurally similar albeit not strictly conserved. The structural features of the peptides are also derived using X-ray crystallography and/or computer-based modelling procedures. Accordingly, the divergence in the identified peptides from an individual screen permits the identification of both primary and secondary structural features that are required for binding to the target protein or target nucleic acid to occur. Based upon the bioinformatic data obtained, oligonucleotides (e.g., degenerate oligonucleotides or non-degenerate oligonucleotides as appropriate) are designed that encode all of the possible peptides that bind to the target protein or target nucleic acid. These oligonucleotides are then assembled using PCR employing multiple rounds of amplification, to generate a plurality of nucleic acids encoding all possible peptide combinations. Accordingly, an amino acid sequence that is not normally found in nature is produced.

In one embodiment, nucleic acid fragments are cloned into a gene construct in at least two forward open reading frames, and preferably three forward open reading frames, to thereby enhance the number of divergent peptides or proteins that are encoded by a particular nucleic acid fragment. Preferably, a significant proportion of the nucleic acid fragments are cloned into a gene construct in at least two forward open reading frames, and preferably three forward open reading frames, to thereby enhance the number of divergent peptides or proteins that are encoded by a particular nucleic acid fragment. In this context, the term "significant proportion" means at least about 30% to 50%, preferably at least about 40% to 60%, more preferably at least about 50% to 70%, still more preferably at least about 60% to 80% and still more preferably greater than about 70% or 80% of the total nucleic acid fragments that are subcloned successfully into a suitable gene construct such that more than one open reading frame can be utilized for expression. As will be known to those skilled in the art, procedures for cloning a single nucleic acid into a gene construct in multiple reading frames are known.

A preferred method of subcloning nucleic acid fragment(s) in multiple reading frames comprises a process selected from the group consisting of:
(a) ligating a nucleic acid fragment to a linker or adaptor, such as for example, one or more linkers modified to contain an additional one or two or three base pairs, or a multiple of one or two or three nucleotides;
(b) Placing a nucleic acid fragment operably under the control of a Kozak consensus sequence and at different distances therefrom (eg. one or two or three nucleotides or a multiple of one or two or three nucleotides) from said Kozak consensus sequence;
(c) Placing a fragment under control of a sequence that confers transcriptional and/or translational slippage.

By ligating the nucleic acid fragment to a linker or adaptor, the number of introduced nucleotides can be varied such that a significant proportion of the nucleic acid fragments are introduced into an expression vector or gene construct in at least two and preferably three reading frames. Linkers or adaptors are ligated to the 5'-end of the nucleic acid fragment such that, on average, a different length linker or adaptor is added to each nucleic acid fragment having the same sequence. This is generally achieved by varying the relative proportions of each linker/adaptor to the nucleic acid fragments. Naturally, each linker/adaptor of differing length is generally in equimolar concentration in the ligation reaction, and the total concentration of linker/adaptor 3'-ends is held in equimolar concentration to the total concentration of 5'-ends of the nucleic acid fragments being ligated. Methods of ligating adaptors to nucleic acids are known in the art and are described in for example, Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001).

As an alternative to separately adding the linkers/adaptors to the nucleic acid fragments prior to subcloning into a suitable gene construct, a suitable gene construct is used that comprises additional nucleotides 3' of a translation initiation signal, and provides for sub-cloning of nucleic acid fragments in each reading frame. As will be known to those skilled in the art, each reading frame in a gene construct is generally accessed by digesting the gene construct with a different restriction endonuclease and then sub-cloning nucleic acid fragments into the digested, linearized vector. By "sub-cloning" means a process involving or comprising a ligation reaction.

Alternatively, site directed mutagenesis is used to introduce additional nucleotides after the translation initiation site of the gene construct. Methods of site-directed mutagenesis are known in the art, and are described for example, in Dieffenbach (eds) and Dveksler (ed) (In: PCR Primer: A Laboratory Manual, Cold Spring Harbour Laboratories, NY, 1995). Furthermore, kits containing instructions and reagents necessary for site-directed mutagenesis are commercially available, such as, for example, the Quikchange site directed mutagenesis kit (Stratagene).

Furthermore, expression vectors are commercially available that have been modified to include an additional one or two nucleotides after the transcription start codon to allow for cloning of a nucleic acid in at least two and preferably three reading frames. Such vectors include, for example, the pcDNA (A, B, or C) vector suite (Invitrogen).

By positioning each nucleic acid fragment so that expression is placed operably under the control of a Kozak consensus sequence and at different distances therefrom, a significant proportion of the nucleic acid fragments is inserted into the vector in at least two and preferably three reading frames. A preferred Kozak sequence has the core sequence RNNATG (SEQ ID NO: 1), wherein R is a purine (ie. A or G) and N is any nucleotide. A particularly preferred Kozak sequence for expression of a polypeptide in eukaryotic cells comprises the sequence CCRCCATG (SEQ ID NO: 2) or GCCAGCCATGG (SEQ ID NO: 3). A preferred Kozak sequence for the expression of polypeptides in plants is CTACCATG (SEQ ID NO: 4).

A Kozak consensus sequence is generated using synthetic oligonucleotides in a process that is known in the art and described, for example, in, Oligonucleotide Synthesis: A Practical Approach (M. J. Gait, ed., 1984) IRL Press, Oxford, whole of text, and particularly the papers therein by Gait, pp1-22; *Atkinson et al.,* pp35-81; *Sproat et al.,* pp 83-115; and *Wu et al.,* pp 135-151. Alternatively, a Kozac sequence is isolated from a natural or recombinant source using methods known in the art, such as for example using from the group, restriction enzyme digestion or PCR.

In one embodiment, the Kozak sequence is generated as an oligonucleotide or nucleic acid fragment and then ligated 5' of the nucleic acid fragment (i.e., the nucleic acid fragment being sub-cloned). Methods of ligating such oligonucleotides or fragments are known in the art and are described in for example, Ausubel et al (In: Current Protocols in Molecular Biology. Wiley Interscience, ISBN 047 150338, 1987) or (Sambrook et al (In: Molecular Cloning: Molecular Cloning: A Laboratory Manual, Cold Spring Harbor Laboratories, New York, Third Edition 2001). As with other ligations, the total concentration of nucleic acid of each ligating species (ie. the Kozak containing fragment and the nucleic acid) should preferably be equimolar. Naturally, to ensure that a significant proportion of nucleic acid fragments are ligated in each reading frame, the Kozak-containing fragments of differing length should also be present in approximately equimolar concentration.

As an alternative to separately adding the Kozak consensus sequence oligonucleotide or fragment to the nucleic acid fragment prior to subcloning into a suitable vector, an expression vector is used that comprises a translation start site and provides for subcloning of nucleic acid fragments in each reading frame. As will be known to those skilled in the art, each reading frame in such a vector is generally accessed by digesting the vector with a different restriction enzyme and then subcloning fragments into the digested, linearized vector.

When the nucleic acid fragment is to be expressed in prokaryotic cells, it is particularly preferred that the Kozak sequence of the above embodiments is replaced with a ribosome binding sequence, or Shine Dalgarno sequence. A particularly preferred Shine Dalgarno sequence consists of nucleic acids having the nucleotide sequence GAAGAAGATA (SEQ ID NO: 5).

By placing a fragment under control of sequences that confer transcriptional and/or translational slippage is meant that the fidelity of the start site for transcription and/or translation is reduced such that translation is initiated at different sites. Accordingly, such a sequence is cause the expression of several different polypeptides.

In one embodiment translational slippage (or translational frameshifting) is induced using nucleic acid comprising of the consensus sequence N₁N₁N₁N₂N₂N₂N₃, wherein N represents any nucleotide and all nucleotides represented by N₁ are the same nucleotide, all nucleotides represented by N₂ are the same nucleotide. In accordance with this embodiment, N₁ and/or N₂ and/or N₃ are the same or different. A particularly preferred translational slippage sequence for use in a eukaryote will comprise a sequence selected from the group consisting of: AAAAAAC (SEQ ID NO: 6), AAATTTA (SEQ ID NO: 7), AAATTTT (SEQ ID NO: 8), GGGAAAC (SEQ ID NO: 9), GGGCCCC (SEQ ID NO: 10), GGGTTTA (SEQ ID NO: 11), GGGTTTT (SEQ ID NO: 12), TTTAAAC (SEQ ID NO: 13), TTTAAAT (SEQ ID NO: 14), TTTTTA (SEQ ID NO: 15), and GGATTTA (SEQ ID NO: 16). In an alternative embodiment, a sequence that induces translational slippage in yeast is CTTAGGC (SEQ ID NO: 17) or GCGAGTT (SEQ ID NO: 18). In yet another embodiment a sequence that induces translational slippage in mammals is TCCTGAT (SEQ ID NO: 19).

In another embodiment, a translational slippage sequences for use in prokaryotic organisms includes, but is not limited to s sequence selected from the group consisting of AAAAAAG (SEQ ID NO: 20), AAAAAAA (SEQ ID NO: 21), AAAAAAC (SEQ ID NO: 22), GGGAAAG (SEQ ID NO: 23), AAAAGGG (SEQ ID NO: 24), GGGAAAA (SEQ ID NO: 25), TTTAAAG (SEQ ID NO: 26) and AAAGGGG (SEQ ID NO: 27). It is particularly preferred that this translational slippage sequence is positioned about 7 to about 19 nucleotides downstream of a Shine Dalgarno sequence. In an alternative embodiment, a nucleic acid that induces translational slippage in bacterial cells comprises the nucleotide sequence CTT (SEQ ID NO: 28), and is positioned 3 nucleotides upstream of a Shine Dalgarno sequence controlling the expression of the nucleic acid fragment.

A translational slippage sequence is generated using synthetic oligonucleotides, or isolated from a natural or recombinant source, for example the *prfB* gene, the *dnaX* gene, the mammalian ornithine decarboxylase antizyme, in addition to various retroviruses, coronaviruses, retrotransposons, virus-like sequences in yeast, bacterial genes and bacteriophage genes. Such a sequence is isolated using a method that is known in the art, such as for example, restriction enzyme digestion or PCR.

It is preferred that sequences that confer translational slippage are ligated to the 5'-end of the nucleic acid fragment in the same manner as for adaptor addition. Methods of ligating adaptors are known in the art and are described in for example, Ausubel et al (In: Current Protocols in Molecular Biology. Wiley Interscience, ISBN 047 150338, 1987) or (Sambrook et al (In: Molecular Cloning: Molecular Cloning: A Laboratory Manual, Cold Spring Harbor Laboratories, New York, Third Edition 2001).

It is also preferred that the sequences that confer transcriptional or translational slippage are incorporated into the expression vector or gene construct into which the nucleic acid fragment is inserted, such that it is positioned upstream (ie. 5') of the translational start site in the fragment.

In another embodiment, transcriptional slippage is induced by the introduction of a stretch of nucleotides with a sequence such as, for example, T₉ or A₉. Transcriptional slippage sequences are preferably cloned downstream (ie. 3') of the site of initiation of transcription. It is also preferred to position a transcriptional slippage sequence upstream (5') of a translational start site in the nucleic acid fragment. Accordingly, the transcriptional slippage sequence is included in the expression vector or gene construct into which the nucleic acid fragment is inserted.

Accordingly, the nucleic acids that form the transcriptional slippage sequence is ligated to the 5' end of a nucleic acid fragment, in conjunction with a translation start site.

It will be apparent from the preceding description that the transcriptional slippage sequence is incorporated into the expression vector or gene construct upstream of the translation start site, and downstream of the site of initiation of transcription.

Preferably, the nucleic acid fragments derived from the prokaryote or compact eukaryote genome are inserted into a gene construct in both the forward and/or reverse orientation, such that 1 or 2 or 3 or 4 or 5 or 6 open reading frames of said nucleic acid fragments are utilized. Methods of bi-directionally inserting fragments into vectors are known in the art.

It will be apparent to the skilled artisan that, by sub-cloning the nucleic acid fragments in multiple reading frames into a suitable expression vector, it is possible to encode a peptide or protein domain that does not occur in nature, as well as producing a variety of natural peptide domains. Accordingly, the diversity of the nucleic acids of the expression library and their encoded peptides are greatly enhanced in these modified nucleic acid fragment expression libraries.

In a preferred embodiment, the expression libraries are normalized to remove any redundant nucleic acid from the genome. As used herein the term "redundant nucleic acid" shall be taken to mean those nucleic acid fragments having the same or substantially the same nucleotide sequence, such as, for example, high copy number or repetitive sequences. Nucleic acid fragments derived from multiple homologous sequences, whether derived from the same or a different species can be subject to normalization to reduce the presence of redundant sequences in the expression library. Similarly, nucleic acid fragments derived from repetitive DNA and nucleic acid fragments derived from pseudogenes can be subject conveniently to normalization. Methods of normalizing libraries to remove redundant nucleic acid are known in the art and are described, for example, by Ausubel et al., In: Current Protocols in Molecular Biology. Wiley Interscience, ISBN 047 150338, 1987, or Diversa Corporation (US Patent No. 5,763,239), or Sambrook et al., In: Molecular Cloning: Molecular Cloning: A Laboratory Manual, Cold Spring Harbor Laboratories, New York, Third Edition 2001, or Bonaldo et al., Genome Res. 6(9), 791-806, 1997.

In one embodiment, the nucleic acid fragments are subjected to hydroxyapatite chromatography to remove redundant or highly repetitive sequences. The success of such a normalization process can be determined, for example, by hybridizing labelled non-normalized and normalized DNA to Southern blots of genomic DNA and comparing the amount of label bound to each blot. The amount of bound label is comparable to the amount of hybridized DNA. A reduced hybridization signal for normalized libraries indicates that iterative sequences have been reduced in the normalized pool.

In another embodiment of the present invention the nucleic acids are derived from two or more prokaryotes and/or compact eukaryotes including any and all combinations thereof.

It is preferred that the prokaryote(s) and/or compact eukaryote(s) used to produce expression libraries from combined genomes are evolutionally diverse organisms. As used herein the term "evolutionary diverse" shall be taken to mean those organisms that when compared at the genetic level, show a significant degree of genetic diversity. As used herein the term "significant degree of genetic diversity" shall be taken to mean, that the genes of the prokaryotes or compact eukaryotes differ, by at least about 10% to 30% at the nucleic acid level. More preferably the genetic sequences of the prokaryotes or compact eukaryotes differ by at least about 30% to 40% at the nucleic acid level. More preferably the genetic sequences of the prokaryotes or compact eukaryotes differ by at least about 50% at the nucleic acid level. More preferably the genetic sequences of the prokaryote or compact eukaryotes differ by at least about 70% at the nucleic acid level, or more preferably at least about 80% at the nucleic acid level or 90% at the nucleic acid level.

In determining whether or not two nucleotide sequences fall within these defined percentage identity limits, those skilled in the art will be aware that it is possible to conduct a side-by-side comparison of the nucleotide sequences. In such comparisons or alignments, differences will arise in the positioning of non-identical residues depending upon the algorithm used to perform the alignment. In the present context, references to percentage identities and similarities between two or more nucleotide sequences shall be taken to refer to the number of identical and similar residues respectively, between said sequences as determined using any standard algorithm known to those skilled in the art. In particular, nucleotide identities and similarities are calculated using software of the Computer Genetics Group, Inc., University Research Park, Maddison, Wisconsin, United States of America, eg., using the GAP program of Devereaux et al., Nucl. Acids Res. 12, 387-395, 1984, which utilizes the algorithm of Needleman and Wunsch, J. Mol. Biol. 48, 443-453, 1970. Alternatively, the CLUSTAL W algorithm of Thompson et al., Nucl. Acids Res. 22, 4673-4680, 1994, is used to obtain an alignment of multiple sequences, wherein it is necessary or desirable to maximize the number of identical/similar residues and to minimize the number and/or length of sequence gaps in the alignment. Nucleotide sequence alignments can also be performed using a variety of other commercially available sequence analysis programs, such as, for example, the BLAST program available at NCBI.

In an alternative embodiment, the genetic sequences of the prokaryotes or compact eukaryotes fail to cross hybridize in a standard Cot analysis. The skilled artisan will be aware that standard Cot analysis determines the similarity between two nucleotide sequences at the nucleotide level by using renaturation-kinetics of the corresponding nucleic acids (eg., Britten and Kohne Science, 161, 529-540, 1968).

Where more than one substantially sequenced genome is used to produce the expression library, it is also preferred that the fragments from each distinct prokaryote or compact eukaryote are used in an amount proportional to the complexity and size of the genome of said prokaryote or compact eukaryote. As the genomes of the prokaryotes and/or compact eukaryotes are substantially sequenced the approximate size of said genomes is determined. Accordingly, a library is normalized to ensure that the amount of nucleic acids from all of the incorporated genomes to the final expression library is equal.

In a preferred embodiment, the nucleic acid fragment expression libraries are normalized such that nucleic acid fragments from each of the prokaryotes or compact eukaryotes are incorporated in equimolar amounts. In one exemplified embodiment, the sizes (in Mbp or molecular weight) of the genomes to be used in the expression library are compared and nucleic acid from each genome is used in an amount that is proportional to the ratio of genome size to the size of the smallest contributing genome for the library. For example, the genome of *T. rubripes* is about 400Mb in size, compared to the genome of *A. thaliana,* which is only about 120Mb. Accordingly, for a combination of genomic *T. rubripes* and *A. thaliana* nucleic acid fragments, the ration of *T. rubripes* nucleic acid fragments to *A. thaliana* nucleic acid fragments would be about 4:1.2 (w/w). The relative contributions of nucleic acid fragments for constructing expression libraries from multiple genomes are readily calculated from the information presented in Table 1.

**TABLE 1**

| Sizes of genomes of organisms from which nucleic acid fragments are derived for construction of expression libraries | |
|---|---|
| Source of nucleic acid fragments | Approx. genome size (Mb) |
| *Actinobacillus pleuropneumoniae* | 2.2 |
| *Aeropyrum pernix* | 1.6-1.7 |
| *Agrobacterium pernix* | 1.67 |
| *Anopheles gambiae* | 26-27 |
| *Arabidopsis thaliana* | 120 |
| *Aquifex aeolicus* | 1.5-1.6 |
| *Archaeoglobus fulgidis* | 1.7 |
| *Bacillus anthracis* | 5.09 |
| *Acillus cereus* | 5.4 |
| *Bacillus halodurans* | 4.2 |
| *Bacillus subtilis* | 4.2 |
| *Bacteroides thetaiotaomicron* | 6.2 |
| *Bdellovibrio bacteriovorus* | 3.8 |
| *Bifidobacterium longum* | 2.3 |
| *Bordetella bronchiseptica* | 5.34 |
| *Bordetall parapertusis* | 4.77 |
| *Bordetella pertussis* | 3.91 |
| *Borellia afzelii* | 0.95 |
| *Borellia garinii* | 0.95 |
| *Borrelia burgdorferi* | 0.91-0.96 |
| *Bradyrhizobium japonicum* | 9.11 |
| *Brucella melitensis* | 3.2 |
| *Brucella suis* | 3.29 |
| *Brugia malayi* | 100 |
| *Buchnera aphidicola* | 0.64 |
| *Caenorhabditis elegans* | 97-102 |
| *Campylobacter jejuni* | 1.64 |
| *Candidatus blochmannia floridanus* | 0.7 |
| *Caulobacter crescentus* | 4.01 |
| *Chlamydia muridarum* | 1.07 |
| *Chlamydia pneumoniae* | 1.22 |

| Source of nucleic acid fragments | Approx. genome size (Mb) |
|---|---|
| *Chlamydia trachomatis* | 1.0-1.1 |
| *Chlamydophila caviae* | 3.53 |
| *Chlamydophila pneumoniae* | 1.23 |
| *Chlorobium tepidum* | 2.1 |
| *Chlostridium acetobuty*/*icum* | 4.1 |
| *Chromobacterium violaceum* | 4.8 |
| *Clostridium acetobutylicum* | 3.94 |
| *Clostridium perfringens* | 3.03 |
| *Clostridium tetani* | 4.1 |
| *Corynebacterium diphtheriae* | 2.49 |
| *Corynebacterium efficiens* | 3.15 |
| *Corynebacterium glutamicum* | 3.31 |
| *Coxiella burnetii* | 2.0 |
| *Danio rerio* | 1700 |
| *Dechloromonas aromatica* | 4.50 |
| *Deinococcus radiodurans* | 3.28 |
| *Drosophila melanogaster* | 120 |
| *Eimeria acervulina* | 70 |
| *Eimeria tenelia* | 70 |
| *Entamoeba hystolitica* | 40 |
| *Enterococcus faecalis* | 3.36 |
| *Escherichia coli* | 4.6-5.6 |
| *Fusobacterium nucleatum* | 4.33 |
| *Geobacter sulfurreducens* | 3.85 |
| *Gloebacter vio*/*aceus* | 4.7 |
| *Haemophilus ducreyi* | 1.7 |
| *Haemophilus influenzae* | 1.83 |
| *Halobacterium sp.* | 2.57 |
| *Helicobacter hepaticus* | 1.8 |
| *Helicobacter pylori* | 1.66 |
| *Lactobacillus johnsonii* | 2.0 |
| *Lactobacillus plantarum* | 3.3 |
| *Lactococcus lactis* | 2.36 |
| *Leptospira interrogans serovar lai* | 4.6 |
| *Listeria innocua* | 3.01 |
| *Listeria monocytogenes* | 2.94 |
| *Mesorhizobium loti* | 7.59 |
| *Methanobacterium thermoautotrophicum* | 1.75 |
| *Methanocaldococcusjannaschii* | 1.66 |
| *Methanococcoides burtonii* | 2.6 |
| *Methanopyrus kandleri* | 1.69 |
| *Methanosarcina acetivorans* | 5.75 |
| *Methanosarcina mazei Goel* | 4.1 |
| *Methanothermobacter thermautotrophicus* | 1.75 |
| *Mycobacterium avium sp.* | 4.96 |
| *Mycobacterium bovis* | 4.35 |
| *Mycobacterium leprae* | 2.8 |
| *Mycobacterium tuberculosis* | 4.4 |
| *Mycoplasma gallisepticum strain R* | 1.0 |
| *Mycoplasma genitalium* | 0.58 |
| *Mycoplasma penetrans* | 1.36 |
| *Mycoplasma pneumoniae* | 0.81 |
| *Mycoplasma pulmonis* | 0.96 |
| *Nanoarchaeum equitans Kin4* | 0.49 |
| *Neisseria meningitidis* | 2.18-2.27 |
| *Nitrosomonas europaea* | 2.81 |
| *Nostoc sp.* | 6.41 |
| *Oceanobacillus iheyensis* | 3.6 |
| *Onion yellows phytoplasma* | 0.86 |
| *Oryza sativa* | 400 |
| *Pasturella multocida* | 2.4 |
| *Photorhabdus luminescens sp.* | 5.7 |
| *Pirellula sp.* | 7.1 |
| *Porphyromonas gingivalis* | 2.34 |
| *Plasmodium berghei* | 25 |
| *Plasmodium falciparum* | 25 |
| *Plasmodium yoelii* | 23 |
| *Plasmodium vivax* | 30 |
| *Prochlorococcus marinus str.* | 2.41 |
| *Pseudomonas aeruginosa* | 6.3 |
| *Pseudomonas putida* | 6.1 |
| *Pseudomonas syringae* | 6.4 |
| *Pyrobaculum aerophilum* | 2.2 |
| *Pyrococcus abyssi* | 1.77 |
| *Pyrococcus furiosus* | 1.91 |
| *Pyrococcus horikoshii* | 1.74 |
| *Ralstonia solanacearum* | 5.80 |
| *Rhodopseudomonas palustris* | 5.46 |
| *Ricketsia conorii* | 1.27 |
| *Ricketsia prowazekii* | 1.1 |
| *Ricketsia rickettsii* | 1.3 |
| *Saccharomyces cerevesiae* | 13.0 |
| *Salmonella enterica* | 4.8 |
| *Salmonella typhimurium* | 4.8 |
| *Sarcocystis cruzi* | 201 |
| *Schizosaccharomyces pombe* | 13.8-14.0 |
| *Schistosoma mansoni* | 270 |
| *Shewanalla oneidensis* | 5. 4 |
| *Shigella flexneri* | 4.7 |
| *Sinorhizobium meliloti* | 6.7 |
| *Staphylococcus aureus* | 2.8 |
| *Staphylococcus epidermidis* | 2.6 |
| *Streptococcus agalactiae* | 2.21 |
| *Streptococcus mutans* | 2.03 |
| *Streptococcus pneumoniae* | 2.2 |
| *Streptococcus pyogenes* | 1.85 |
| *Streptomyces avermitilis* | 9 |
| *Streptomyces coelicolor* | 8.7 |
| *Sulfolobus solfataricus* | 2.99 |
| *Sumolobus tokodaii* | 2.81 |
| *Synechococcus sp.* | 2.43 |
| *Synechocystis PCC 6803* | 3.57 |
| *Takifugu rubripes* | 400 |
| *Thermoplasma volcanium* | 1.56-1.58 |
| *Thermoanaerobacter tengcongensis* | 2.69 |
| *Thermoplasma acidophilum* | 1.56 |
| *Thermoplasma volcanium* | 1.58 |
| *Thermotoga maritima* | 1.80 |
| *Thermotoga pallidum* | 1.14 |
| *Toxoplasma gondii* | 89 |
| *Treponema denticola* | 3.06 |
| *Treponema pallidum* | 1.14 |
| *Tropheryma whipplei* | 0.93 |
| *Trypanosoma brucei* | 35 |
| *Trypanosoma cruzi* | 40 |
| *Ureaplasma urealyticum* | 0.75 |
| *Vibrio cholerae* | 4 |
| *Vibro parahaemolyticus* | 5.2 |
| *Vibrio vulnificus* | 5.1 |
| *Wigglesworthia brevipalpis* | 0.7 |
| *Wolbachia endosymbiont of Drosophila melanogaster* | 1.27 |
| *Wolinella succinogenes* | 2.1 |
| *Xanthomonas axonopodis* | 5.17 |
| *Xanthomonas campestris* | 5.07 |
| *Xylella fastidiosa* | 2.68 |
| *Yersinia pestis* | 4.65 |

Preferred combinations of genomes are selected from the group consisting of:
a) nucleic acid fragments derived from two organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
b) nucleic acid fragments derived from three organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
c) nucleic acid fragments derived from four organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
d) nucleic acid fragments derived from five organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
e) nucleic acid fragments derived from six organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
f) nucleic acid fragments derived from seven organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
g) nucleic acid fragments derived from eight organisms selected from the group consisting of *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
h) nucleic acid fragments derived from nine organisms selected from the group consisting of *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
i) nucleic acid fragments derived from ten organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
j) nucleic acid fragments derived from eleven organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
k) nucleic acid fragments derived from twelve organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
l) nucleic acid fragments derived from thirteen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
m) nucleic acid fragments derived from fourteen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacteriuin thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
n) nucleic acid fragments derived from fifteen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
o) nucleic acid fragments derived from sixteen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi; Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
p) nucleic acid fragments derived from seventeen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
q) nucleic acid fragments derived from eighteen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
r) nucleic acid fragments derived from nineteen organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio; Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
s) nucleic acid fragments derived from twenty organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
t) nucleic acid fragments derived from twenty one organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
u) nucleic acid fragments derived from twenty two organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
v) nucleic acid fragments derived from twenty three organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
w) nucleic acid fragments derived from twenty four organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces. pombe, Synechocystis* PCC 6803, *Takifugu rubripes, Thermoplasma volcanium,* and *Thermotoga maritima;*
x) nucleic acid fragments derived from twenty five organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima;*
y) nucleic acid fragments derived from' twenty six organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis; Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis.* PCC 6803, *Takifugu rubripes, Thermoplasma volcanium,* and *Thermotoga maritima;* and
z) nucleic acid fragments derived from twenty seven organisms selected from the group consisting of: *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima.*

In a particularly preferred embodiment, the nucleic acid fragments are derived from the organisms *Aeropyrum pernix, Anopheles gambiae, Arabidopsis thaliana, Aquifex aeolicus, Archaeoglobus fulgidis, Bacillus subtilis, Bordetella pertussis, Borrelia burgdorferi, Caenorhabditis elegans, Chlamydia trachomatis, Danio rerio, Drosophila melanogaster, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Saccharomyces cerevesiae, Schizosaccharomyces pombe, Synechocystis* PCC 6803, *Takifugu rubripes, Thermoplasma volcanium,* and *Thermotoga maritima.*

In a particularly preferred embodiment, nucleic acid fragments derived from the following bacteria are combined into a single expression library: *Aeropyrum pernix, Aquifex aeolicus, Archaeoglobus fulgidis, Bacillus subtilis, Bordetella pertussis, Borrelia burgdorferi, Chlamydia trachomatis, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis* PCC 6803, *Thermoplasma volcanium* and *Thermotoga maritima.*

In another particularly preferred embodiment, nucleic acid fragments derived from the following bacteria are combined into a single expression library: *Archaeoglobus fulgidis, Aquifex aeliticus, Aeropyrum pernix, Aquifex aeolicus, Bacillus subtilis, Bordatella pertussis TOX6, Borrelia burgdorferi, Chlamydia trachomatis, Escherichia coli, Haemophilus influenzae, Helicobacter pylori, Methanobacterium thermoautotrophicum, Methanococcus jannaschii, Methanothermobacter thermoautotrophicus, Mycoplasma pneumoniae, Neisseria meningitidis, Pirellula species, Pyrococcus horikoshii, Pseudomonas aeruginosa, Synechosistis sp., Thermoplasma volcanium and Thermotoga maritima.*

In a preferred embodiment, nucleic acid fragments are derived from two or more organisms selected from the group consisting of *Aeropyrum pernix, Aquifex aeolicus, Archaeoglobus fulgidis, Bacillus subtilis, Bordetella pertussis, Borrelia burgdorferi, Chlamydia trachomatis, Desulfovibrio vulgaris, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis* PCC 6803, *Thermoplasma volcanium, Thermus thermophilus* and *Thermotoga maritima.*

In another preferred embodiment, nucleic acid fragments are derived from two or more organisms selected from the group consisting of *Archaeoglobus fulgidus, Aquifex aeolicus, Aeropyrum pernix, Bacillus subtilis, Bordetella pertussis, Borrelia burgdorferi, Chlamydia trachomatis, Escherichia coli K12, Haemophilus influenzae, Helicobacter pylori, Methanobacterium thermoautotrophicum., Methanococcus jannashii, Neisseria meningitidis, Pyrococcus horikoshii, Pseudomonas aeruginosa, Synechocystis PCC 6803, Thermoplasma volcanicum, Thermotoga maritima, Acidobacterium capsulatum, Halobacterium salinarum, Desulfobacterium autotrophicum, Haloferax volcanii, Rhodopirellula baltica, Thermus thermophilus HB27 and Prochlorococcus marinus MED4.*

The nucleic acid fragments, unmodified or modified by the addition of one or more linkers, adaptors, Kozak containing oligonucleotides, Kozak containing fragments, or nucleic acids comprising a sequence that confers transcriptional or translational slippage, are placed in operable connection with a promoter sequence, thereby producing a recombinant gene construct.

The term "gene construct" is to be taken in its broadest context and includes a promoter sequence that is placed in operable connection with a nucleic acid fragment. The nucleic acid cmprising the promoter sequence is isolated using techniques known in the art, such as for example PCR or restriction digestion. Alternatively the nucleic acid comprising the promoter sequence is synthetic, that is an oligonucleotide. The methods of producing oligonucleotides are known in the art and are described, for example, in Oligonucleotide Synthesis: A Practical Approach (M. J. Gait, ed., 1984) IRL Press, Oxford, whole of text, and particularly the papers therein by Gait, pp1-22; Atkinson *et al.,* pp35-81; Sproat *et al.,* pp 83-115; and Wu *et al.,* pp 135-151.

The term "promoter" is to be taken in its broadest context and includes the transcriptional regulatory sequences of a genomic gene, including the TATA box or initiator element, which is required for accurate transcription initiation, with or without additional regulatory elements (ie. upstream activating sequences, transcription factor binding sites, enhancers and silencers) which alter gene expression in response to developmental and/or external stimuli, or in a tissue specific manner. In the present context, the term "promoter" is also used to describe a recombinant, synthetic or fusion molecule, or derivative which confers, activates or enhances the expression of a nucleic acid molecule to which it is operably linked, and which encodes the peptide or protein. Preferred promoters can contain additional copies of one or more specific regulatory elements to further enhance expression and/or alter the spatial expression and/or temporal expression of said nucleic acid molecule.

Placing a nucleic acid molecule under the regulatory control of, i.e., "in operable connection with", a promoter sequence means positioning said molecule such that expression is controlled by the promoter sequence. Promoters are generally positioned 5' (upstream) to the coding sequence that they control. To construct heterologous promoter/structural gene combinations, it is generally preferred to position the promoter at a distance from the gene transcription start site that is approximately the same as the distance between that promoter and the gene it controls in its natural setting, ie., the gene from which the promoter is derived. As is known in the art, some variation in this distance can be accommodated without loss of promoter function. Similarly, the preferred positioning of a regulatory sequence element with respect to a heterologous gene to be placed under its control is defined by the positioning of the element in its natural setting, ie., the gene from which it is derived. Again, as is known in the art, some variation in this distance can also occur.

Typical promoters suitable for expression in bacterial cells, such as, for example, a bacterial cell selected from the group comprising *E*. *coli, Staphylococcus sp, Corynebacterium sp., Salmonella sp., Bacillus sp.,* and *Pseudomonas sp.,* include, but are not limited to, the *lacz* promoter, the Ipp promoter, temperature-sensitive λ_{L} or λ_{R} promoters, T7 promoter, T3 promoter, SP6 promoter or semi-artificial promoters such as the IPTG-inducible *tac* promoter or lacUV5 promoter. A number of other gene construct systems for expressing the nucleic acid fragment in bacterial cells are well-known in the art and are described for example, in Ausubel et al (In: Current Protocols in Molecular Biology. Wiley Interscience, ISBN 047 150338, 1987), US Patent No. 5,763,239 (Diversa Corporation) and (Sambrook et al (In: Molecular Cloning: Molecular Cloning: A Laboratory Manual, Cold Spring Harbor Laboratories, New York, Third Edition 2001).

Typical promoters suitable for expression in yeast cells such as, for example, a yeast cell selected from the group consisting of *Pichia pastoris, S. cerevisiae* and *S. pombe,* include, but are not limited to, the *ADH1* promoter, the *GAL1* promoter, the *GAL4* promoter, the *CUP1* promoter, the *PHO5* promoter, the *nmt* promoter, the *RPR1* promoter, or the *TEF1* promoter.

Typical promoters suitable for expression in insect cells, or in insects, include, but are not limited to, the OPE12 promoter, the insect actin promoter isolated from *Bombyx muri,* the *Drosophila sp. dsh* promoter *(*Marsh et al Hum. Mol. Genet. 9, 13-25, 2000) and the inducible metallothionein promoter. Preferred insect cells for expression of the recombinant polypeptides include an insect cell selected from the group consisiting of BT1-TN-5B1-4 cells, and *Spodoptera frugiperda* cells (eg., sf19 cells, sf21 cells). Suitable insects for the expression of the nucleic acid fragments include but are not limited to *Drosophila sp.* The use of *S. frugiperda* is also contemplated.

Promoters for expressing peptides in plant cells are known in the art, and include, but are not limited to, the *Hordeum vulgare amylase* gene promoter, the cauliflower mosaic virus 35S promoter, the nopaline synthase (NOS) gene promoter, and the auxin inducible plant promoters P1 and P2.

Typical promoters suitable for expression in a mammalian cell, mammalian tissue or intact mammal include, for example, a promoter selected from the group consisting of, retroviral LTR elements, the SV40 early promoter, the SV40 late promoter, the cytomegalovirus (CMV) promoter, the CMV IE (cytomegalovirus immediate early) promoter, the EF_{1α} promoter (from human elongation factor 1α), the EM7 promoter, the UbC promoter (from human ubiquitin C).

Preferred mammalian cells for expression of a nucleic acid fragment include epithelial cells, fibroblasts, kidney cells, T cells, or erythroid cells, including a cell line selected from the group consisting of COS, CHO, murine 10T, MEF, NIH3T3, MDA-MB-231, MDCK, HeLa, K562, HEK 293 and 293T. The use of neoplastic cells, such as, for example, leukemic/leukemia cells, is also contemplated herein.

Preferred mammals for expression of the nucleic acid fragments include, but are not limited to mice (ie., *Mus sp.)* and rats (ie., *Rattus sp.).*

In one embodiment, nucleic acid comprising a promoter sequence is ligated to a nucleic acid fragment from the prokaryote or compact eukaryote, or a modified form thereof, using techniques known in the art.

In another embodiment, nucleic acid comprising a promoter sequence is modified by the addition of one or more linkers, adaptors, Kozak containing oligonucleotides, Kozak containing fragments, or nucleic acids comprising a sequence that confers transcriptional or translational slippage and ligated to a nucleic acid fragment from the prokaryote or compact eukaryote using techniques known in the art.

In yet another embodiment, nucleic acid comprising a promoter sequence is incorporated into an oligonucleotide with or without another nucleic acid comprising one or more spacers, Kozak sequences, or nucleic acids comprising a sequence that confers transcriptional or translational slippage.

Preferably, the oligonucleotide comprises a nucleotide sequence that is complementary or homologous to a region flanking the nucleic acid fragment from the prokaryote or compact eukaryote, such as, for example, an adaptor. Such a complementary or homologous sequence permits oligonucleotide primers to be used for amplifying nucleic acid comprising a promoter region and means for ribosome binding (such as for example a Kozak sequence or Shine-Dalgarno sequence) and the nucleic acid fragment as a single fragment. In this manner, a gene construct comprising a promoter sequence, means for ribosome binding and a nucleic acid fragment is readily constructed using the amplified nucleic acid.

In an alternative embodiment, a nucleic acid comprising a promoter sequence is incorporated into an oligonucleotide with or without another nucleic acid comprising one or more spacers, Kozak sequences, or nucleic acids comprising a sequence that confers transcriptional or translational slippage, and said oligonucleotide is operably linked to a nucleic acid fragment by, for example, ligation.

In one embodiment, the nucleic acid fragments are expressed *in vitro.* According to this embodiment, the gene construct preferably comprises a nucleic acid fragment of the prokaryote or compact eukaryote, and a promoter sequence and appropriate ribosome binding site which is both be present in the expression vector or added to said nucleic acid fragment before it is inserted into the vector. Typical promoters for *the in vitro* expression of the nucleic acid fragments include, but are not limited to the T3 or T7 (Hanes and Plückthun Proc. Natl. Acad Sci. USA, 94 4937-4942 1997) bacteriophage promoters.

In another embodiment, the gene construct optionally comprises a transcriptional termination site and/or a translational termination codon. Such sequences are known in the art, and may be incorporated into oligonucleotides used to amplify the nucleic acid fragment of the prokaryote or compact eukaryote, or alternatively, present in the expression vector or gene construct before the nucleic acid fragment is inserted.

In another embodiment, the gene construct is an expression vector. The term "expression vector" refers to a nucleic acid molecule that has the ability confer expression of a nucleic acid fragment to which it is operably connected, in a cell or in a cell free expression system. Within the context of the present invention, it is to be understood that an expression vector may comprise a promoter as defined herein, a plasmid, bacteriophage, phagemid, cosmid, virus sub-genomic or genomic fragment, or other nucleic acid capable of maintaining and or replicating heterologous DNA in an expressible format. Many expression vectors are commercially available for expression in a variety of cells. Selection of appropriate vectors is within the knowledge of those having skill in the art.

Typical expression vectors for *in vitro* expression or cell-free expression have been described and include, but are not limited to the TNT T7 and TNT T3 systems (Promega), the pEXP1-DEST and pEXP2-DEST vectors (Invitrogen).

Numerous expression vectors for expression of recombinant polypeptides in bacterial cells and efficient ribosome binding sites have been described, such as for example, PKC30 (Shimatake and Rosenberg, Nature 292, 128, 1981); pKK173-3 (Amann and Brosius, Gene 40, 183, 1985), pET-3 (Studier and Moffat, J. Mol. Biol. 189, 113, 1986); the pCR vector suite (Invitrogen), pGEM-T Easy vectors (Promega), the pL expression vector suite (Invitrogen) the pBAD/TOPO or pBAD/thio - TOPO series of vectors containing an arabinose-inducible promoter (Invitrogen, Carlsbad, CA), the latter of which is designed to also produce fusion proteins with a Trx loop for conformational constraint of the expressed protein; the pFLEX series of expression vectors (Pfizer nc., CT,USA); the pQE series of expression vectors (QIAGEN, CA, USA), or the pL series of expression vectors (Invitrogen), amongst others.

Expression vectors for expression in yeast cells are preferred and include, but are not limited to, the pACT vector (Clontech), the pDBleu-X vector, the pPIC vector suite (Invitrogen), the pGAPZ vector suite (Invitrogen), the pHYB vector (Invitrogen), the pYD1 vector (Invitrogen), and the pNMT1, pNMT41, pNMT81 TOPO vectors (Invitrogen), the pPC86-Y vector (Invitrogen), the pRH series of vectors (Invitrogen), pYESTrp series of vectors (Invitrogen). Particularly preferred vectors are the pACT vector, pDBleu-X vector, the pHYB vector, the pPC86 vector, the pRH vector and the pYES vectors, which are all of use in various 'n'-hybrid assays described herein. Furthermore, the pYD1 vector is particularly useful in yeast display experiments in *S. cerevesiae.* A number of other gene construct systems for expressing the nucleic acid fragment in yeast cells are well-known in the art and are described for example, in Giga-Hama and Kumagai (In: Foreign Gene Expression in Fission Yeast: Schizosaccharomyces Pombe, Springer Verlag, ISBN 3540632700, 1997) and Guthrie and Fink (In: Guide to Yeast Genetics and Molecular and Cell Biology Academic Press, ISBN 0121822540, 2002).

A variety of suitable expression vectors, containing suitable promoters and regulatory sequences for expression in insect cells are known in the art, and include, but are not limited to the pAC5 vector, the pDS47 vector, the pMT vector suite (Invitrogen) and the pIB vector suite (Invitrogen).

Furthermore, expression vectors comprising promoters and regulatory sequences for expression of polypeptides in plant cells are also known in the art and include, for example, a promoter selected from the group, pSS, pB1121 (Clontech), pZ01502, and pPCV701 *(*Kuncz et al, Proc. Natl. Acad. Sci. USA, 84 131-135, 1987).

Expression vectors that contain suitable promoter sequences for expression in mammalian cells or mammals include, but are not limited to, the pcDNA vector suite supplied by Invitrogen, the pCI vector suite (Promega), the pCMV vector suite (Clontech), the pM vector (Clontech), the pSI vector (Promega), the VP16 vector (Clontech) and the pDISPLAY vectors (Invitrogen). The pDISPLAY vectors are of particular use in mammalian display studies with the expressed nucleic acid fragment targeted to the cell surface with the Igκ leader sequence, and bound to the membrane of the cell through fusion to the PDGFR transmembrane domain. The pM and VP16 vectors are of particular use in mammalian two-hybrid studies.

Methods of cloning DNA into nucleic acid vectors for expression of encoded polypeptides are known in the art and are described for example in, Ausubel et al (In: Current Protocols in Molecular Biology. Wiley Interscience, ISBN 047 150338, 1987) or Sambrook et al (In: Molecular Cloning: Molecular Cloning: A Laboratory Manual, Cold Spring Harbor Laboratories, New York, Third Edition 2001).

The nucleic acid fragments are also expressed in the cells of other organisms, or entire organisms including, for example, nematodes (eg *C*. *elegans)* and fish (eg *D. rerio,* and *T. rubripes).* Promoters for use in nematodes include, but are not limited to *osm-10 (*Faber et al Proc. Natl. Acad. Sci. USA 96, 179-184, 1999), *unc-54* and *myo-2* (Satyal et al Proc. Natl. Acad. Sci. USA, 97 5750-5755, 2000). Promoters for use in fish include, but are not limited to the zebrafish *OMP* promoter, the GAP43 promoter, and serotonin-N-acetyl transferase gene regulatory regions

In a preferred embodiment, the expression library is transcribed and translated *in vitro.* Methods of transcribing nucleic acid fragments and translating the resulting mRNA are known in the art and are described for example, in Ausubel et al (In: Current Protocols in Molecular Biology. Wiley Interscience, ISBN 047 150338, 1987), US Patent No. 5,763,239 (Diversa Corporation) and (Sambrook et al (In: Molecular Cloning: Molecular Cloning: A Laboratory Manual, Cold Spring Harbor Laboratories, New York, Third Edition 2001), for example the use of E. *coli* S30 lysate (available in kit for from Promega).

In a preferred embodiment the gene construct contains a second nucleic acid in operable connection with a nucleic acid fragment. This second nucleic acid encodes a fusion partner. As used herein the term "fusion partner" shall be understood to mean a polypeptide sequence that is associated with a peptide encoded by a nucleic acid fragment. Such a fusion partner confers a common function or ability upon all polypeptides encoded by the expression library. Suitable fusion partners include, but are not limited to, presentation structures, polypeptides that facilitate the uptake of peptides into target cells, polypeptides that cause nuclear localization, polypeptides that cause secretion, polypeptides that cause mitochondrial localization, polypeptides that cause membrane localization, or a combination of any of these sequences.

Without suggesting that such a process is essential to the invention, a peptide encoded by the expression library can also be expressed such that it is conformationally constrained, or expressed in a "presentation structure". Such constraint, whilst not generally necessary for expressing protein domains or peptides having a conformation sufficient to bind to a target protein or target nucleic acid, is useful for displaying peptides that comprise more highly flexible sequences, or to enhance stability against proteolytic enzymes (Humphrey et al, Chem Rev 97, 2243-2266, 1997).

A presentation structure will generally comprise a first component, i.e., polypeptide, that is fused to the amino terminus of the polypeptide and a second component fused to the carboxyl- terminus of the peptide. Examples of such presentation structures include, but are not limited to, cysteine-linked (disulfide) structures, zinc-finger domains, cyclic peptides, and transglutaminase linked structures.

In a preferred embodiment, the presentation structure is a sequence that contains at least two cysteine residues, such that a disulphide bond is formed between the cysteine residues, resulting in a conformationally constrained peptide.

In another embodiment, a peptide encoded by an expression library is expressed within a second polypeptide as a fusion protein. Polypeptides used for such purposes are capable of reducing the flexibility of another protein's amino and/or carboxyl termini. Preferably, such proteins provide a rigid scaffold or platform for the protein. In addition, such proteins preferably are capable of providing protection from proteolytic degradation and the like, and/or are capable of enhancing solubility. Preferably, conformation-constraining proteins are small in size (generally, less than or equal to about 200 amino acids in length), rigid in structure, of known three-dimensional configuration, and are able to accommodate insertions of proteins without undue disruption of their structures. A key feature of such proteins is the availability, on their solvent exposed surfaces, of locations where peptide insertions can be made (eg., the Trx loop). It is also preferable that conformation-constraining protein producing genes be highly expressible in various prokaryotic and eukaryotic hosts, or in suitable cell-free systems, and that the proteins be soluble and resistant to protease degradation.

Examples of conformation-constraining proteins include the active site of thioredoxin or Trx loop and other thioredoxin-like proteins, nucleases (eg., RNase A), proteases (eg., trypsin), protease inhibitors (eg., bovine pancreatic trypsin inhibitor), antibodies or structurally rigid fragments thereof, conotoxins, and the pleckstrin homology domain. A conformation-constraining peptide can be of any appropriate length and can even be a single amino acid residue.

This technique has been successfully used for bacterial display of peptides in bacteria using a Trx scaffold (Blum et al Proc. Natl. Acad. Sci. USA 97, 2241-2246 2000) in addition to the use in yeast 2 hybrid screening using either a catalytically inactive form of staphylococcal nuclease, or Trx (Norman et al, Science,285, 591-595, 1999; and Colas et al, Nature 380, 548-550, 1996).

In another embodiment the expression vector or gene construct is optionally comprise a transcriptional terminator that is operative in the expression system. Furthermore, the gene construct is also comprise a nucleic acid comprising the sequence of a polyadenylation signal operative in the expression system.

It is preferred that when the gene constructs are to be introduced to and/or maintained and/or propagated and/or expressed in bacterial cells, either during generation of said gene constructs, or screening of said gene constructs, that the gene constructs contain an origin of replication that is operable at least in a bacterial cell. A particularly preferred origin of replication is the ColE1 origin of replication. A number of gene construct systems containing origins of replication are well-known in the art and are described for example, in Ausubel et al (In: Current Protocols in Molecular Biology. Wiley Interscience, ISBN 047 150338, 1987), US Patent No. 5,763,239 (Diversa Corporation) and (Sambrook et al (In: Molecular Cloning: Molecular Cloning: A Laboratory Manual, Cold Spring Harbor Laboratories, New York, Third Edition 2001).

It is also preferred that when the gene constructs are to be introduced to and/or maintained and/or propagated and/or expressed in yeast cells, either during generation of said gene constructs, or screening of said gene constructs, that the gene constructs contain an origin of replication that is operable at least in a yeast cell. One preferred origin of replication is the CEN/ARS4 origin of replication. Another particularly preferred origin of replication is the 2-micron origin of replication. A number of gene construct systems containing origins of replication are well-known in the art and are described for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001).

In another embodiment, the gene construct containing the nucleic acid fragments comprises another nucleic acid cassette comprising a promoter sequence in operable connection with a polynucleotide sequence encoding a selectable marker.

As used herein the term "selectable marker" shall be taken to mean a protein or peptide that confers a phenotype on a cell expressing said selectable marker that is not shown by those cells that do not carry said selectable marker. Examples of selectable markers include, but are not limited to the *dhfr* resistance gene, which confers resistance to methotrexate (Wigler, et al., 1980, Natl. Acad. Sci. USA 77:3567; O'Hare, et al., 1981, Proc. Natl. Acad. Sci. USA 78:1527); the gpt resistance gene, which confers resistance to mycophenolic acid (Mulligan & Berg, 1981, Proc. Natl. Acad. Sci. USA 78:2072); the neomycin phosphotransferase gene, which confers resistance to the aminoglycoside G-418 (Colberre-Garapin, et al., 1981, J. Mol. Biol. 150:1); and the hygromycin resistance gene (Santerre, et al., 1984, Gene 30:147). Alternatively, a marker gene catalyses a reaction resulting in a visible outcome (for example, the production of a blue precipitate when β galactosidase is expressed in the presence of the substrate molecule 5-bromo-4-chloro-3-indoyl-β-D-galactoside) or confer the ability to synthesize particular amino acids (for example the HIS3 gene confers the ability to synthesize histidine).

In one embodiment the peptide encoded by the nucleic acid fragment is expressed as a fusion protein with a peptide sequence capable of enhancing, increasing or assisting penetration or uptake of the peptide by cells either *in vitro* or *in vivo.* For example, the peptide sequence capable of enhancing, increasing or assisting penetration or uptake is the *Drosophila* penetratin targeting sequence (a "protein transduction domain"). This peptide sequence at least comprises the amino acid sequence:
CysArgGlnIleLysIleTrpPheGlnAsnArgArgMetLysTrpLysLys (SEQ ID NO. 29)
further comprising (Xaa)n after the final Lys residue and followed by Cys wherein Xaa is any amino acid and n has a value greater than or equal to 1. Alternatively, a homologue, derivative or analogue of said sequence is used. The use of said sequence is particularly useful when peptides encoded by the nucleic acid fragment are synthesized *in vitro* or secreted from a host cell, and must be taken up by a cell for screening said peptide encoded by the nucleic acid fragment.

Those skilled in the art will also be aware of an analogous use of signals such as for example, the *tat* sequence of HIV to drive import of peptides into cells.

In an alternative embodiment, the peptide encoded by the nucleic acid fragment is mixed with a peptide capable of enhancing, increasing or assisting penetration or uptake by cells *in vitro or in vivo.* A peptide sequence that is able to increase or assist penetration or uptake of cells is the synthetic peptide *Pep 1,* which at least comprises the amino acid sequence:
LysGluThrTipTrpGluThrTrpTrpThrGluTrpSerGlnLysLysLysLysArgLysVal (SEQ ID NO. 30).

The *Pep1* peptide does not need to be conjugated to the peptide encoded by the nucleic acid fragments. Furthermore, *Pep1* dissociates from the peptide encoded by the expression library. Thus *Pep1* will not interfere with the peptide forming a conformation sufficient for binding to a target protein or nucleic acid. *Pep1* is only useful when the peptides encoded by the expression library are isolated prior to the addition to a cell or organism for screening. Thus *Pep1* is particularly useful when in *vitro* libraries are screened.

Other protein transduction domains are known in the art, and are clearly useful in the present invention. For example, amino acids 43-58 of *Drosophila* antennapedia, polyarginine, PTD-5, Transportan and KALA (reviewed in Kabouridis, TRENDS in Biotechnology, 21: 498-503, 2003).

Alternative protein transduction domains are known in the art, and include, for example, TAT fragment 48-60 (GRKKRRQRRRPPQ, SEQ ID NO: 31), signal sequence based peptide 1 (GALFLGWLGAAGSTMGAWSQPKKKRKV, SEQ ID NO: 32), signal sequence based peptide 2 (AAVALLPAVLLALLAP, SEQ ID NO: 33), transportan (GWTLNSAGYLLKINLKALAALAKKIL, SEQ ID NO: 34), amphiphilic model peptide (KLALKLALKALKAALKLA, SEQ ID NO: 35), polyarginine (e.g., RRRRRRRRRRR, SEQ ID NO: 36)

In one embodiment, the expression library is introduced into and preferably expressed within a cellular host or organism to generate the expression library, it is preferred that the gene constructs are introduced into said cellular host or said organism. Methods of introducing the gene constructs into a cell or organism for expression are known to those skilled in the art and are described for example, in Ausubel et al (In: Current Protocols in Molecular Biology. Wiley Interscience, ISBN 047 150338, 1987) and Sambrook et al (In: Molecular Cloning: Molecular Cloning: A Laboratory Manual, Cold Spring Harbor Laboratories, New York, Third Edition 2001). The method chosen to introduce the gene construct in depends upon the cell type in which the gene construct is to be expressed.

In one embodiment, the cellular host is a bacterial cell. Means for introducing recombinant DNA into bacterial cells include, but are not limited to electroporation or chemical transformation into cells previously treated to allow for said transformation.

In another embodiment, the cellular host is a yeast cell. Means for introducing recombinant DNA into yeast cells include a method chosen from the group consisting of electroporation, and PEG mediated transformation.

In another embodiment, the cellular host is a plant cell. Means for introducing recombinant DNA into plant cells include a method selected from the group consisting of *Agrobacterium* mediated transformation, electroporation of protoplasts, PEG mediated transformation of protoplasts, particle mediated bombardment of plant tissues, and microinjection of plant cells or protoplasts.

In yet another embodiment, the cellular host is an insect cell. Means for introducing recombinant DNA into plant cells include a method chosen from the group consisting of, infection with baculovirus and transfection mediated with liposomes such as by using cellfectin (Invitrogen).

In yet another embodiment, the cellular host is a mammalian cell. Means for introducing recombinant DNA into mammalian cells include a means selected from the group comprising microinjection, transfection mediated by DEAE-dextran, transfection mediated by calcium phosphate, transfection mediated by liposomes such as by using Lipofectamine (Invitrogen) and/or cellfectin (Invitrogen), PEG mediated DNA uptake, electroporation, transduction by Adenoviuses, Herpesviruses, Togaviruses or Retroviruses and microparticle bombardment such as by using DNA-coated tungsten or gold particles (Agacetus Inc., WI,USA).

In an alternative embodiment, the expression library is an *in vitro* display library (ie., the peptides encoded by the prokaryote or compact eukaryote nucleic acid fragments of the expression library are displayed using *in vitro* display wherein the expressed peptide is linked to the nucleic acid from which it was expressed such that said peptide is presented in the absence of a host cell). Accordingly, expression libraries produced by *in vitro* display technologies are not limited by transformation or transfection efficiencies. Accordingly any such library is of much higher complexity than an *in vivo* display library. Examples of methods of *in vitro* display include a method selected from the group comprising but not limited to, ribosome display, covalent display and mRNA display.

In one embodiment, the *in vitro* display library is a ribosome display library. The skilled artisan will be aware that a ribosome display library directly links mRNA encoded by the expression library to the peptide that it encodes. Means for producing a ribosome display library require that the nucleic acid fragment be placed in operable connection with an appropriate promoter sequence and ribosome binding sequence, ie. form a gene construct. Preferred promoter sequences are the bacteriophage T3 and T7 promoters.

Preferably, the nucleic acid fragment is placed in operable connection with a spacer sequence and a modified terminator sequence with the terminator sequence removed.

As used herein the term "spacer sequence" shall be understood to mean a series of nucleic acids that encode a peptide that is fused to the peptide. The spacer sequence is incorporated into the gene construct, as the peptide encoded by the spacer sequence remains within the ribosomal tunnel following translation, while allowing the peptide to freely fold and interact with another protein or a nucleic acid.

A preferred spacer sequence is, for example, a nucleic acid that encodes amino acids 211-299 of gene *III* of filamentous phage M13 mp 19.

The display library is transcribed and translated *in vitro* using methods known in the art and are described for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001).

Examples of systems for *in vitro* transcription and translation include, for example, the TNT *in vitro* transcription and translation systems from Promega. Cooling the expression reactions on ice generally terminates translation. The ribosome complexes are stabilized against dissociation from the peptide and/or its encoding mRNA by the addition of reagents such as, for example, magnesium acetate or chloroamphenicol. Such *in vitro* display libraries are screened by a variety of methods, as described herein.

In another embodiment, the expression library is a ribosome inactivation display library. In accordance with this embodiment, a nucleic acid fragment is operably linked to a nucleic acid encoding a first spacer sequence. It is preferred that this spacer sequence is a glycine/serine rich sequence that allows a peptide encoded by the expression library to freely fold and interact with a target protein or nucleic acid.

The first spacer sequence is linked to a nucleic acid that encodes a toxin that inactivates a ribosome. It is preferred that the toxin comprises the ricin A chain, which inactivates eukaryotic ribosomes and stalls the ribosome on the translation complex without release of the mRNA or the encoded peptide.

The nucleic acid encoding the toxin is linked to another nucleic acid that encodes a second spacer sequence. The second spacer is required as an anchor to occupy the tunnel of the ribosoine, and allow both the peptide and the toxin to correctly fold and become active. Examples of such spacer sequences are sequences derived from gene *III* of M13 bacteriophage.

Ribosome inactivation display libraries are generally transcribed and translated *in vitro,* using a system such as the rabbit reticulocyte lysate system available from Promega. Upon translation of the mRNA encoding the toxin and correct folding of this protein, the ribosome is inactivated while still bound to both the encoded polypeptide and the mRNA from which it was translated.

In another embodiment, the expression library is an mRNA display library. In accordance with this embodiment, a nucleic acid fragment is operably linked to a nucleic acid encoding a spacer sequence, such as a glycine/serine rich sequence that allows a peptide encoded by the expression library to freely fold and interact with a target protein or nucleic acid.

The nucleic acid encoding the spacer sequence is operably linked to a transcription terminator.

mRNA display libraries are generally transcribed *in vitro,* using methods known in the art, such as, for example, the HeLaScribe Nuclear Extract *in vitro* Transcription System available from Promega. Encoded mRNA is subsequently covalently linked to a DNA oligonucleotide that is covalently linked to a molecule that binds to a ribosome, such as, for example, puromycin, using techniques known in the art and are described in, for example, Roberts and Szostak, Proc.Natl. Acad. Sci. USA, 94, 12297-12302 (1997). Preferably, the oligonucleotide is covalently linked to a psoralen moiety, whereby the oligonucleotide is photo-crosslinked to a mRNA encoded by the expression library.

The mRNA transcribed from the expression library is then translated using methods known in the art and are described for example, in Ausubel et al (In: Current Protocols in Molecular Biology. Wiley Interscience, ISBN 047 150338, 1987) and (Sambrook et al (In: Molecular Cloning: Molecular Cloning: A Laboratory Manual, Cold Spring Harbor Laboratories, New York, Third Edition 2001). When the ribosome reaches the junction of the mRNA and the oligonucleotide the ribosome stalls and the puromycin moiety enters the phosphotransferase site of the ribosome and thus covalently links the encoded polypeptide to the mRNA from which it was expressed.

In yet another embodiment, the expression library is a covalent display library. In accordance with this embodiment, the nucleic acid fragment is operably linked to a second nucleic acid fragment that encodes a protein that interacts with the DNA from which it was encoded. Examples of a protein that interacts with the DNA from which it interacts include, but are not limited to, the E. *coli* bacteriophage P2 viral A protein (P2A) and equivalent proteins isolated from phage 186, HP1 and PSP3.

The P2A protein is particularly preferred. The P2A protein recognizes a defined initiator sequence TCGGA (SEQ ID NO 31) positioned within the nucleic acid encoding the P2A protein and nicks one of the strands while forming a covalent bond with one of the free end nucleotides. Accordingly, it is preferred that at least the sequence TCGGA (SEQ ID NO 31) is included in the gene construct containing the expression library.

It is particularly preferred that the protein attachment site is positioned such that a nucleic acid fragment is covalently linked to the peptide that it encodes.

A covalent display gene construct is transcribed and translated *in vitro,* using a system such as the rabbit reticulocyte lysate system available from Promega. Upon translation of the fusion of the peptide and the P2A protein, the P2A protein nicks the nucleic acid of the sequence of SEQ ID NO: 31 and forms a covalent bond therewith. Accordingly, a nucleic acid fragment is covalently linked to the peptide that it encodes.

In yet another embodiment, the expression library is a phage display library wherein the expressed peptides or protein domains are displayed on the surface of a bacteriophage, as described, for example, in US Patent No. 5,821,047 and US Patent No. 6,190,908. The basic principle described relates to the fusion of a first nucleic acid comprising a sequence encoding a peptide or protein to a second nucleic acid comprising a sequence encoding a phage coat protein, such as, for example a phage coat proteins selected from the group, M13 protein-3, M13 protein-7, or M13, protein-8. These sequences are then inserted into an appropriate vector, e.g., a vactor capable of replicating in bacterial cells. Suitable host cells, such as, for example E. *coli,* are then transformed with the recombinant vector. Said host cells are also infected with a helper phage particle encoding an unmodified form of the coat protein to which a nucleic acid fragment is operably linked. Transformed, infected host cells are cultured under conditions suitable for forming recombinant phagemid particles comprising more than one copy of the fusion protein on the surface of the particle. This system has been shown to be effective in the generation of virus particles such as, for example, a virus particle selected from the group comprising λ phage, T4 phage, M13 phage, T7 phage and baculovirus. Such phage display particles are then screened to identify a displayed protein having a conformation sufficient for binding to a target protein or nucleic acid.

In yet another embodiment, the expression library is a retroviral display library wherein the expressed peptides or protein domains are displayed on the surface of a retroviral particle. Retroviral display is of particular use as the proteins and peptides displayed in such a system are generated in eukaryotic cells that can carry out a number of post-translational modifications to the peptides or protein domains that are required for activity. Such a retroviral display system is described in US Patent No. 6,297,004 (Cambridge Drug Discovery Holding, Limited). In adapting such a system to the present invention, a nucleic acid fragment is placed in operable connection with an envelope protein of a retrovirus, more preferably a spike glycoprotein. An example of such a protein is the mature envelope protein of Moloney Murine leukemia virus. A gene construct comprising a nucleic acid fragment in operable connection with a retroviral envelope protein is also placed in operable connection with long terminal repeat sequences, a tRNA binding site and a polypurine tract to ensure reverse transcription and integration of the encapsid RNA in an infected mammalian cell. Furthermore, such a gene construct should comprise an encapsidated signal sequence. An encapsidated signal sequence is a nucleic acid that is recognised by a component of the viral particle that mediates the inclusion of the nucleic acid into the viral particle. Such a gene construct is then expressed in an appropriate host cell, such as, for example, a *COS* cell or *NIH3T3* cell, that has been previously infected with a retrovirus encoding an unmodified spike glycoprotein. In such a system chimeric retroviral particles are generated, carrying a mixture of modified and unmodified forms of the spike glycoprotein. These recombinant retrovirus particles are used to identify a displayed peptide that binds to a target protein or nucleic acid.

In yet another embodiment, the expression library is a bacterial display library wherein the expressed peptides or protein domains are displayed on the surface of a bacterial cell. The cells displaying the expressed peptides or protein domains are then used for biopanning as described, for example, in US Patent No. 5,516,637. Bacterial display is based on the finding that heterologous proteins is expressed as a fusion with bacterial surface proteins and assayed for the ability to bind to a target protein or nucleic acid. Accordingly, in such systems a nucleic acid fragment is placed in operable connection with a second nucleic acid that encodes an anchoring motif, or amino acid sequence that directs the incorporation of the encoded peptide on the surface of the bacterial cell surface. Preferred amino acid sequences that direct incorporation of a peptide onto the surface of a bacterial cell include, but are not limited to, the flagella major subunit FliC for localizing a protein on the flagellum of E *coli,* the cell sorting signal of the cell wall proteinase PrtP of *Lactobacillus casei,* the OmpS maltoprotein of *Vibrio cholerae,* Protein A of *Bacillus subtilis,* LysA of *B. subtilis,* and ActA of *B. subtilis.* Expression libraries comprising such gene constructs are then introduced into an appropriate host cell, such as for example E. *coli or B. subtilis* and the expressed peptides displayed on the surface of the bacterial cell. Such displayed libraries are of particular use in screening for peptides that have a conformation sufficient for binding a target protein or nucleic acid.

In an alternative embodiment, the peptides encoded by the nucleic acid fragment is also fused to a second nucleic acid comprising a sequences that encodes a peptide that directs the incorporation of the encoded peptide on the surface of a bacterial spore. Such methods are particularly useful in the display of peptides that are toxic to bacteria when expressed intra cellularly, or when screening conditions are particularly harsh, such as, for example in the presence of organic solvents, or high temperatures.

In yet another embodiment, the expression library is a display library wherein the expressed peptides or protein domains are displayed on the surface of a yeast cell. This method is particularly useful for the display of peptides encoded by nucleic acid derived from eukaryotes, as prokaryotic species are unable to form some structures encoded by eukaryotic sequences. Such a yeast display method is described in US Patent No 6,423,538. In adapting this method to the present invention, a nucleic acid fragment is operably linked to a second nucleic acid fragment encoding the membrane-associated alpha-agglutinin yeast adhesion receptor, encoded by the *aga2* gene. The expression library is introduced into an appropriate host cell, such as for example *S*. *cerevisiae or S. pombe.* Following introduction into an appropriate host cell the fusion protein is secreted from the cell. The fusion protein then binds to the Aga1 protein on the surface of the cell by forming disulfide bonds. Such a yeast cell is screened to determine whether or not it expresses a peptide having a conformation sufficient for binding to a target protein or nucleic acid.

In yet another embodiment, the expression library is a display library wherein the expressed peptides or protein domains are displayed on the surface of a mammalian cell. Such a system is described for example in Strenglin et al EMBO J, 7, 1053-1059, 1988. Mammalian display is particularly useful for the display of peptides derived from eukaryotes, as prokaryotic species and some lower eukaryotic species are unable to form some structures encoded by eukaryotic sequences. The mechanism behind mammalian display relates to the fusion of a nucleic acid fragment to a second nucleotide sequence encoding a peptide leader sequence, which directs the protein to be secreted, such as for example the Ig κ secretion signal. Furthermore, the nucleic acid fragment is placed in operable connection with another nucleic acid, which encodes a peptide that anchors the peptide to the membrane, such as, for example the sequence of the transmembrane domain of PDGFR. An example of a vector containing such a sequence is the pDISPLAY vector available from Invitrogen. Proteins expressed by such a vector are displayed upon the surface of the mammalian cell, making these cells particularly useful for screening for peptides that adopt a conformation sufficient for binding to a target protein or nucleic acid.

In another embodiment, the expression library is an arrayed expression library. As used herein "arrayed expression library" shall be taken to mean that the library is assembled in such a way that an individual peptide and/or nucleic acid encoding same is readily identified. For example, each peptide encoded by the library of the present invention is produced individually (ie. in isolation from other peptides), a number or a plurality of different peptides are then pooled. Two or more of these pools of peptides are then pooled, and if necessary, this process is repeated. Accordingly, pools of several thousands or millions of peptides may be produced. The largest of these pools is then screened to determine whether or not it comprises a peptide with a conformation sufficient for binding to a target protein and/or nucleic acid. Should such a pool comprise a peptide that binds to a target protein or nucleic acid, one or more groups of smaller pools (ie. sub-pools) of peptides are screened to determine which comprise the peptide of interest. Clearly, this process can be iteratively repeated with pools of descending size until the individual peptide of interest is isolated. Alternatively, a pool of a smaller number of peptides (e.g., 10 or 100) is directly screened to determine which, if any, of the peptides have a conformation sufficient for binding a target protein and/or nucleic acid and the sequence of said peptide or encoding nucleic acid (for example using a biosensor chip in conjunction with mass spectrometry).

As will be apparent to the skilled artisan the present invention clearly encompasses the use of multiple different libraries. Accordingly, the present invention also includes screening one or more pooled libraries. For example, the present invention encompasses the pooling of two or more libraries. In one embodiment, the libraries are derived from the same organism/s. In another embodiment, the libraries are derived from different organisms (e.g., a library derived from eukaryotes comprising a compact genome, and another library derived from bacteria).

As will be apparnt to the skilled artisan an arrayed or pooled library may comprise nucleic acid fragments derived from the genome of one or more organisms and/or a vector comprising said fragment and/or the peptides encoded by the nucleic acid fragments and/or cells expressing said peptide.

In another embodiment, an arrayed expression library is produced or bound to or conjugated to a chip for analysis. To produce such a chip, the peptides (and/or nucleic acid encoding said peptide and/or a vector comprising said nucleic acid and/or a cell expressing said peptide) of the present invention are either synthesized on, or synthesized and then bound to, a solid support such as, for example glass, polycarbonate, polytetrafluoroethylene, polystyrene, silicon oxide, gold or silicon nitride. This immobilization is either direct (e.g. by covalent linkage, such as, for example, Schiff's base formation, disulfide linkage, or amide or urea bond formation) or indirect. Methods of generating a protein chip are known in the art and are described in for example U.S. Patent Application No. 20020136821, 20020192654, 20020102617 and U.S. Patent No. 6,391,625. To bind a protein to a solid support it is often necessary to treat the solid support so as to create chemically reactive groups on the surface, such as, for example, with an aldehyde-containing silane reagent or the calixcrown derivatives described in Lee et al, Proteomics, 3: 2289-2304, 2003. A streptavidin chip is also useful for capturing proteins and/or peptides and/or nucleic acid and/or cells that have been conjugated with biotin (eg. as described in Pavlickova et al., Biotechiques, 34: 124-130, 2003). Alternatively, a peptide is captured on a microfabricated polyacrylamide gel pad and accelerated into the gel using microelectrophoresis as described in, Arenkov et al. Anal. Biochem. 278:123-131, 2000.

Methods of determining a peptide on the chip capable of binding a target protein and/or nucleic acid will be apparent to the skilled artisan. For example, a sample to be analyzed using a protein chip is attached to a reporter molecule, such as, for example, a fluorescent molecule, a radioactive molecule, an enzyme, or an antibody that is detectable using methods known in the art. Accordingly, by contacting a protein chip with a labeled sample and subsequent washing to remove any unbound proteins the presence of a bound protein and/or nucleic acid is detected using methods known in the art, such as, for example using a DNA microarray reader.

Alternatively, biomolecular interaction analysis-mass spectrometry (BIA-MS) is used to rapidly detect and characterize a protein present in complex biological samples at the low- to sub-fmole level (Nelson et al. Electrophoresis 21: 1155-1163, 2000 and Needelkov and Nelson, Biosensors and Bioelectronics, 16: 1071-1078, 2001). One technique useful in the analysis of a protein chip is surface enhanced laser desorption/ionization-time of flight-mass spectrometry (SELDI-TOF-MS) technology to characterize a protein bound to the protein chip. Alternatively, the protein chip is analyzed using ESI as described in U.S. Patent Application 20020139751.

### Library Screening Processes

The selection step of the screening process is to identify mimotopes or mimetic peptides, rather than merely selecting peptides that perform a known or expected function. Suitable processes for selecting a peptide that does not bind to the target protein or target nucleic acid in its native environment include, for example, determining the amino acid sequence of the peptide or determining the nucleotide sequence of the corresponding nucleic acid encoding said peptide and deriving the amino acid sequence from said nucleotide sequence, determining a known function of the amino acid sequence and excluding a peptide that binds to a target protein or target nucleic acid associated with the known function.

Alternatively, or in addition, the selection involves using an expression library that comprises nucleic acid fragments from organisms that do not possess a particular biochemical pathway or signal transduction pathway relevant to the binding reaction being assayed.

Alternatively, or in addition, the selection comprises using an expression library that comprises nucleic acid fragments from organisms that do not express one or more of the binding partners of the binding reaction being assayed. The present invention clearly contemplates the combined use of bioinformatic analysis and selection of library components from organisms that are not known to carry out the binding reaction being assayed, to exclude those peptides from the screening process that merely perform their known function. Accordingly, such selection ensures that the selected peptide or protein domain does not bind to the target protein or target nucleic acid in its native environment.

A particularly preferred embodiment of the present invention provides for the identification of a peptide or protein domain that is able to modulate the biological activity of a target protein or nucleic acid, wherein the modulated biological activity is the ability of the target protein or nucleic acid to bind to another protein or nucleic acid and wherein the modulated binding is determined using a reporter molecule. As used herein, the term "reporter molecule" shall be taken to mean a molecule that displays a physically measurable property that alters in a way that can be measured and correlated with changes in the biological activity or a target protein or nucleic acid. Reporter molecules are known in the art, and include, but are not limited to, proteins that fluoresce, for example, green fluorescence protein, proteins that induce a colour change in the presence of a substrate, for example *E coli* β-galactosidase, molecules that confer growth characteristics on the host cells, such as for example *HIS1,* and molecules that induce the death or reduced growth ability of the host cells, such as, for example, *URA3* and *CYH2 or CYH3.*

One embodiment of the present invention relates to the identification of nucleic acids that encode peptides having a conformation capable of binding to a DNA sequence. The one-hybrid assay, as described in Chong and Mandel (In: Bartel and Fields, The Yeast Two-Hybrid System, New York, NY pp 289-297, 1997) is used to determine those peptides able to bind to a target DNA sequence. In adapting the standard one-hybrid technique to the present purpose, the target nucleotide sequence is incorporated into the promoter region of a reporter gene(s), the expression of which can be determined as described above. The peptide encoded by the expression library is expressed in such a manner that it forms a fusion protein with a transcriptional activation domain (for example from the GAL4 protein, the LexA protein, the VP16 protein, the B42 peptide or the mouse NF κB protein). The transcriptional activation domain is recruited to the promoter through a functional interaction between the expressed peptide and the target nucleotide sequence. The transcriptional activation domain subsequently interacts with the basal transcriptional machinery of the cell, activating expression of the reporter genes.

In another embodiment a polypeptide is identified that is able to bind a target protein or peptide using the two-hybrid assay described in US Patent No. 6,316,223 to Payan et al and Bartel and Fields, The Yeast Two-Hybrid System, New York, NY, 1997. The basic mechanism described requires that the binding partners are expressed as two distinct fusion proteins in an appropriate host cell, such as for example bacterial cells, yeast cells, and mammalian cells. In adapting the standard two-hybrid screen to the present purpose, a first fusion protein consists of a DNA binding domain fused to the target protein, and a second fusion protein consists of a transcriptional activation domain fused to the peptide encoded by the expression library. The DNA binding domain binds to an operator sequence which controls expression of one or more reporter genes. The transcriptional activation domain is recruited to the promoter through the functional interaction between the peptide expressed by the expression library and the target protein. Subsequently, the transcriptional activation domain interacts with the basal transcription machinery of the cell, thereby activating expression of the reporter gene(s), the expression of which can be determined.

The three hybrid assay as described in Zhang et al (In: Bartel and Fields, The Yeast Two-Hybrid System, New York, NY pp 289-297, 1997) is used to determine those peptides that bind target RNA sequences. In adapting the described 3-hybrid technique to the present invention, a first fusion protein consists of a DNA binding domain which is fused to a known RNA binding protein, eg. the coat protein of bacteriophage MS2. An RNA hybrid molecule is also formed, consisting of a fusion between a RNA molecule known to bind the RNA binding protein, eg. MS2 binding sequences, and a target RNA binding sequence. A second fusion protein consists of a transcriptional activation domain fused to the peptide encoded by the expression library. The DNA binding domain of the first fusion protein binds to an operator sequence that controls expression of one or more reporter genes. The RNA fusion molecule is recruited to the first fusion protein through the functional interaction between the RNA binding protein and the RNA molecule known to interact with said RNA binding protein. The transcriptional activation domain is recruited to the promoter of one or more reporter molecules through functional interaction between the target RNA sequence of the peptide encoded by the nucleic acid of the present invention.

Other modifications of the two-hybrid screens are known in the art, such as for example a PolIII two hybrid system, a Tribrid system, a ubiquitin based split protein sensor system and a Sos recruitment system as described in Vidal and Legrain Nucl. Acid Res. 27(4), 919-929 (1999). All of these systems are particularly contemplated.

A particularly preferred embodiment of the present invention relates to the identification of peptides that antagonize or inhibit the interaction between the target protein or nucleic acid and another protein or nucleic acid. Accordingly, reverse 'N'-hybrid screens are employed to identify agonist molecules. Reverse hybrid screens differ from the forward hybrid screens *supra* in that they use a counter selectable reporter marker(s), such as for example the URA3 gene, the CYH2 gene or the LYS2 gene, to select against interactions between the target protein or nucleic acid and another protein or nucleic acid. Cell survival or cell growth is reduced or prevented in the presence of a drug or a toxigenic substrate of the counter selectable reporter gene product, which is converted by the counter selectable marker to a toxic compound, such as for example the URA3 gene product which confers lethality in the presence of the drug 5-FOA. Accordingly, cells in which the interaction between the target protein and another protein or nucleic acid is blocked or inhibited survive in the presence of the substance. This is because the counter selectable reporter molecule will not be expressed, and accordingly, the substrate will not be converted to a toxic product or the drug (in the case of cycloheximide) will not be active against the essential target encoded by the reporter gene. Such a result suggests that the peptide encoded by the expression library is an inhibitor of the interaction between the target protein or nucleic acid and another protein or nucleic acid.

In a particularly preferred embodiment, the screening method of the present invention identifies an antagonist of a protein: protein interaction or protein: nucleic acid interaction. In accordance with this embodiment, the present invention provides a reverse two hybrid screening process, such as, for example, essentially as described by Watt et al. (USSN 09/227,652), for identifying an inhibitory amino acid sequence that partially or completely inhibits a target protein-protein interaction or DNA-protein interaction involving one or more protein binding partners said method comprising:
(i) providing cells that each comprise: (a) a nucleic acid comprising a counter-selectable reporter gene encoding a polypeptide that is capable of reducing cell growth or viability by providing a target for a cytotoxic or cytostatic compound (eg., *CYH2* gene that confers susceptibility to cycloheximide) or by converting a substrate to a cytotoxic or cytostatic product (eg., *URA3* gene that converts 5-FOA to a toxic product), said gene being positioned downstream of a promoter comprising a cis-acting element such that expression of said gene is operably under the control of said promoter and wherein a protein binding partner of the protein-protein interaction or the DNA-protein interaction being assayed binds to said cis-acting element; and (b) nucleic acid selected from the group consisting of: (i) nucleic acid encoding a protein of the DNA-protein interaction that binds to said *cis*-acting element to activate expression of the counter-selectable reporter gene; and (ii) nucleic acids encoding two protein binding partners of the protein-protein interaction wherein a protein binding partner binds to the *cis*-acting element and the protein binding partners interact, said binding to the *cis*-acting element and said interaction being required to activate expression of the counter-selectable reporter gene;
(ii) transforming or transfecting the cells or a portion of the cells with an expression library such that a single gene construct of the expression library is present in each transformed or transfected cell;
(iii) culturing the transformed or transfected cells for a time and under conditions sufficient for the protein binding partner(s) to activate expression of the counter-selectable reporter gene in the absence of inhibition of the protein-protein interaction or the DNA-protein interaction by an amino acid sequence encoded by the expression library;
(iv) culturing the transformed or transfected cells under conditions sufficient for an amino acid sequence of the expression library to be expressed in each of said transformed or transfected cells or a proportion of said transformed or transfected cells;
(v) culturing the transformed or transfected cells in the presence of the substrate or the cytotoxic or cytostatic compound such that the expressed counter-selectable reporter gene reduces the growth or viability of the cells unless said expression is reduced by virtue of an amino acid sequence of the expression library inhibiting the target protein-protein interaction or DNA-protein interaction;
(vi) selecting a cell having enhanced growth or viability compared to a cell that does not express the amino acid sequence of the expression library wherein the enhanced growth or viability is indicative of a partial or complete inhibition of the protein-protein interaction or a DNA-protein interaction by the amino acid sequence and
(vii) selecting a peptide expressed by the cell at (vi) that does not bind to a protein or nucleic acid of the protein-protein interaction or a DNA-protein interaction in its native environment.

Preferably, wherein a protein-protein interaction is being assayed, the binding of the two protein binding partners reconstitutes a functional transcriptional regulatory protein, such as, for example, by virtue of the binding partners being expressed as fusion proteins wherein each fusion protein comprises a portion of a transcriptional regulatory protein that does not modulate transcription without the other portion (eg., a fusion protein comprising a transcriptional activator domain and a fusion protein comprising a DNA-binding domain). In a particularly preferred embodiment, one fusion protein comprises a Gal4 DNA-binding domain fused to SCL, and another fusion protein comprises the transcriptional activation domain of the LMO2 protein and a domain that interacts with SCL and, in this embodiment, the *URA3* counter selectable reporter gene is operably under the control of a promoter comprising a Gal4 upstream activator sequence (Gal4 UAS), such that docking of the Gal4/SCL fusion to the Gal4 UAS and binding between SCL and LMO2 is required to activate transcription of the *URA3* gene, thereby conferring lethality on cells grown in the presence of 5-fluoro orotic acid (5-FOA). In screening the expression library, only those cells that survive in the presence of 5-FOA are selected.

For example, a specific receptor is expressed as a DNA binding domain fusion protein, such as with the DNA binding domain of GAL4, and the ligand of said receptor is expressed as an activation domain fusion protein, such as with the GAL4 activation domain. These fusion proteins are expressed in yeast cells in operable connection with the CYH2 counter selectable marker, wherein expression of the CYH2 gene requires a physical interaction between the GAL4 DNA binding domain and the GAL4 activation domain. This physical relation is achieved is achieved, for example, by placing the expression of the marker gene under the control of a promoter comprising nucleotide sequences to which the GAL4 DNA binding domain binds. Cells in which the reporter gene is expressed do not grow in the presence of cycloheximide. The expression libraries are expressed in these yeast cells and those cells that then grow in the presence of cycloheximide are further analyzed, such as, for example, analysis of the nucleic acid encoding the candidate peptide inhibitor(s).

In another particularly preferred embodiment, one fusion protein comprises a Gal4 DNA-binding domain fused to JUN1, and another fusion protein comprises the transcriptional activation domain of the LMO2 protein and a domain that interacts with JUN1 (e.g., JUNZ) and the *URA3* counter selectable reporter gene is operably under the control of a promoter comprising a Gal4 upstream activator sequence (Gal4 UAS), such that docking of the Gal4/JUN1 fusion to the Gal4 UAS and binding between JUN1 and JUNZ is required to activate transcription of the *URA3* gene, thereby conferring lethality on cells grown in the presence of 5-fluoro orotic acid (5-FOA). In screening the expression library, only those cells that survive in the presence of 5-FOA are selected.

As will be known to the skilled artisan, the reverse 'n'-hybrid technique briefly described above is readily modified for use in 1-hybrid, 2-hybrid or 3-hybrid assays.

In an alternative embodiment, the antagonist is identified using a reverse split two hybrid screening process, such as, for example, essentially as described by Erickson *et al.* (WO95/26400, wherein a relay gene that is a negative regulator of transcription is employed to repress transcription of a positive readout reporter gene when the interacting proteins (ie., bait and prey) interact, such that reporter gene expression is only induced in the absence of the protein encoded by the relay gene product. In accordance with this embodiment, there is provided a method for identifying an inhibitory amino acid sequence that partially or completely inhibits a target protein-protein interaction or DNA-protein interaction involving one or more protein binding partners said method comprising:
(i) providing cells that each comprise: (a) a nucleic acid encoding a negative regulator of transcription (eg., Gal80 or *mdm2* oncoprotein-encoding gene), said nucleic acid being positioned downstream of a promoter comprising a cis-acting element and wherein a protein binding partner of the protein-protein interaction or the DNA-protein interaction being assayed binds to said *cis*-acting element; (b) nucleic acid selected from the group consisting of: (i) nucleic acid encoding a protein of the DNA-protein interaction that binds to said *cis*-acting element to activate expression of the negative regulator of transcription; and (ii) nucleic acids encoding two protein binding partners of the protein-protein interaction wherein a protein binding partner binds to the *cis*-acting element and the protein binding partners interact, said binding to the cis-acting element and said interaction being required to activate expression of the negative regulator of transcription; and (c) nucleic acid comprising a positive reporter gene (eg., an antibiotic resistance gene, herbicide resistance gene, or other resistance gene, or a gene which complements an auxotrophic mutation in the screening cells) operably connected to a *cis*-acting element (eg., a *GAL4* binding site capable of binding to Gal80, or Gal80, or the transactivation domain of p53 that binds to mdm2 oncoprotein) to which the negative regulator of transcription binds to thereby inhibit or repress expression of the positive reporter gene;
(ii) transforming or transfecting the cells or a portion of the cells with an expression library such that a single gene construct of the expression library is present in each transformed or transfected cell;
(iii) culturing the transformed or transfected cells for a time and under conditions sufficient for the protein binding partner(s) to activate expression of negative regulator of transcription in the absence of inhibition of the protein-protein interaction or the DNA-protein interaction by an amino acid sequence encoded by the expression library;
(iv) culturing the transformed or transfected cells under conditions sufficient for an amino acid sequence of the expression library to be expressed in each of said transformed or transfected cells or a proportion of said transformed or transfected cells ;
(v) culturing the transformed or transfected cells in the presence of a compound to which the positive reporter gene confers resistance on the cells such that the expressed negative regulator of transcription represses expression of the positive reporter gene thereby reducing the growth or viability of the cells unless said expression is reduced by virtue of an amino acid sequence of the expression library inhibiting the target protein-protein interaction or DNA-protein interaction;
(vi) selecting a cell having enhanced growth or viability compared to a cell that does not express the amino acid sequence of the expression library wherein the enhanced growth or viability is indicative of a partial or complete inhibition of the protein-protein interaction or a DNA-protein interaction by the amino acid sequence and
(vii) selecting a peptide expressed by the cell at (vi) that does not bind to a protein or nucleic acid of the protein-protein interaction or a DNA-protein interaction in its native environment.

Preferably, wherein a protein-protein interaction is being assayed, the binding of the two protein binding partners reconstitutes a functional transcriptional regulatory protein. In a particularly preferred embodiment, one interacting protein comprises a LexA fusion protein, and another interacting protein comprises a VP16 fusion protein which when they interact induce expression of a GAL80 reporter gene regulated by lexA operators. In this embodiment, the positive reporter gene (eg. a gene complementing an auxotrophic mutation) is placed operably under the control of a promoter comprising a Gal4 upstream activator sequence (Gal4 UAS), such that docking of a Gal80 negative regulator of transcription to the Gal4 UAS and binding between SCL and LMO2 is required to repress transcription of the positive reporter gene, thereby preventing cells from proliferating. Conversely, repression of the interaction between the LexA-fusion and VP16 fusion prevents Gal80 expression allowing expression of the positive reporter gene that complements an auxotrophic mutation in the screening cells, particularly in cells that express endogenous Gal4 protein, allowing those cells to grow in the absence of the nutrient which the corresponding auxotrophic mutation had conferred dependence on.

In a preferred embodiment of the present invention, those nucleic acid fragments that encode a polypeptide that binds to a target protein or nucleic acid are exposed to further rounds of selection using, for example, mutagenic PCR or expression of said fragments in "mutator" strains of bacteria. This increases the diversity of the selected nucleic acid. Said selected nucleic acid is again screened for those that encode a peptide having a conformation sufficient for binding a target protein or nucleic acid. Through multiple rounds of screening and selection with lower concentrations of the target protein or nucleic acid, those peptides with the highest affinity for the target protein or nucleic acid are selected.

In a related embodiment, the sequences of those nucleic acid fragments encoding peptides that bind to the target protein or nucleic acid are optimally aligned and the sequences compared to identify those nucleic acids that encode amino acids that are particularly desired for binding the target protein or nucleic acid. Furthermore, this information is used to generate synthetic nucleotide sequences encoding peptides, or synthetic peptides, containing those amino acids that are particularly desirable for binding to a target protein or nucleic acid.

Preferably, those peptides that bind to the target protein or nucleic acid, are recovered and used in further analysis, such as for example, determining the nucleotide sequence of the nucleic acid encoding the identified peptide or protein domain. Initially, the nucleic acid fragment encoding the peptide is isolated using methods known in the art, such as for example, PCR, RT-PCR, and nucleic acid isolation, amongst others. An isolated nucleic acid fragment is then characterized by methods such as nucleic acid sequencing. Such methods are known in the art.

In one embodiment, an insolated nucleic acid fragment is placed into an expression vector using methods known in the art, and described herein. Such a nucleic acid fragment is only expressed in a single reading frame and only in one direction. This method is repeated until all possible open reading frames of the nucleic acid fragment are tested, and that/those that encode a polypeptide having a conformation sufficient for binding a target protein or nucleic acid are identified. As used herein the term "all possible open reading frames" shall include those open reading frames that include the entire nucleic acid fragment, in addition to those open reading frames that are formed within a nucleic acid fragment, such as for example by the inclusion of a second ATG start codon, a Kozak sequence, a Shine-Dalgarno sequence, or an internal ribosome entry sequence (IRES), amongst others. Preferably, such translational start sites are incorporated in order of increasing strength from the 5' end to the 3' end of the ribosome binding region of the expression construct, to compensate for a disproportionately strong initiation from the first Kozak sequence encountered after the cap site of the mRNA. All of the expressed peptides are then screened in an appropriate screening system to determine those that have a conformation sufficient for binding to a target protein or nucleic acid. Accordingly, analysis of the nucleic acid encoding such a peptide is used to determine the amino acid sequence of the peptide. Using such software as the Translate tool available at ExPasy. As used herein, the term "ExPasy" shall be understood to mean, the ExPasy proteomics server provided by the Swiss Institute of Bioinformatics at CMU-Rue Michel - Servet 1 1211 Genève 4 Switzerland

Following isolation of the nucleic acid that encodes a peptide with a conformation sufficient for binding to a target protein or nucleic acid, it is preferred that all homologues of this sequence are isolated from the genomes of the organisms used to generate the expression library. Methods of isolating homologous nucleic acid regions are known in the art and are described, for example, in Dieffenbach (ed) and Dveksler (ed) (In: PCR Primer: A Laboratory Manual, Cold Spring Harbour Laboratories, NY, 1995). Such methods include PCR and degenerate PCR. Such homologues are then screened in all possible reading frames using a suitable screening system, as are known in the art and described herein.

It is a further preferred embodiment that an identified nucleotide sequence or amino acid sequence shall be used as a "reference sequence" for a homology search using a database of all known sequences. Such a reference sequence is a nucleotide or amino acid sequence to which all nucleotides or amino acid sequences in a database are compared. A number of source databases are available that contain either a nucleotide sequence and/or a deduced amino acid sequence that are particularly useful to identify all known sequences that are substantially homologous the sequence of nucleic acid or peptide, polypeptide or protein domain identified as positive in the present invention. Such databases are known in the art and include, for example, Genbank (at NCBI) and SWISS-PROT and TrEMBL (available at ExPasy). A number of different methods of performing such sequence searches are known in the art. The sequence data of the clone is then aligned to the sequences in the database or databases using algorithms designed to measure homology between two or more sequences.

In one embodiment, a nucleic acid identified in a homology search of the known nucleic acids is isolated using one of a variety of methods known in the art, such as for example PCR amplification of the specific region of genomic DNA or cDNA of the organism in which the nucleic acid is naturally found. The sequence of the isolated nucleic acid is determined, used to generate a gene construct as described herein, and screened to determine if it encodes a peptide that has a conformation sufficient for binding the target protein or nucleic acid.

In another embodiment a nucleic acid encoding an amino acid sequence identified in a homology search of known amino acid sequences using techniques known in the art, such as for example degenerate PCR. An isolated nucleic acid is then used to generate a gene construct as described herein, and screened to determine if it encodes a peptide that has a conformation sufficient for binding the target protein or nucleic acid.

It is a particularly preferred embodiment of the present invention that those nucleic acids that encode a polypeptide having a conformation that binds to a target protein or nucleic acid are analyzed to select those nucleic acid fragments that encode polypeptides that do not bind to said target protein or nucleic acid in its native environment. As used herein, the term "native environment" of a polypeptide shall be understood to mean the protein encoded by the gene from which the nucleic acid fragment was isolated. Accordingly, it is the aim of the present invention to identify those polypeptides that display a function of the subdomain of the native protein, for example by binding to a target protein or nucleic acid to which it cannot bind in the context of the protein in which it naturally occurs.

The known function/s of the polypeptides isolated in the screening of the libraries of the present invention are determined using sequence analysis software as is available from, for example NCBI, or Prosite. As used herein the term "Prosite" shall be understood to mean the Prosite protein database which is a part of the ExPasy proteomics server provided by the Swiss Institute of Bioinformatics at CMU-Rue Michel - Servet 1 1211 Genève 4 Switzerland. Accordingly, those polypeptides that are known to bind to the target protein or nucleic acid in their native environment are excluded from any further analysis. Furthermore, analysis of the bioinformatic information available, for example, at NCBI aids in determining the native function of a protein. Such analysis will determine if, for example, the pathway being modified exists in an organism from which a peptide is identified or if a target protein or nucleic acid is found in any of the organisms used to generate an expression library.

It is particularly preferred that an expression library is generated using nucleic acid fragments isolated from organisms that are distinct from the organism in which the target protein or nucleic acid naturally occurs. For example, to identify a nucleic acid that encodes a peptide that has a conformation sufficient for binding the c-Jun protein of *Homo sapiens* an expression library is generated from the organisms *Aeropyrum pernix, Aquifex aeolicus, Archaeoglobus fulgidis, Bacillus subtilis, Bordetella pertussis, Borrelia burgdorferi, Chlamydia trachomatis, Escherichia coli, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis* PCC 6803, *Thermoplasma volcanium* and *Thermotoga maritima.* This will reduce the likelihood of identifying a peptide that interacts with the c-Jun protein in its native environment.

In another embodiment, the expression library is screened using affinity purification. Affinity purification techniques are known in the art and are described in, for example, Scopes (In: Protein purification: principles and practice, Third Edition, Springer Verlag, 1994). Methods of affinity purification typically involve contacting the peptides encoded by the nucleic acid fragment library of the present invention with a specific target protein or nucleic acid, and, following washing, eluting those peptides that remain bound to the target protein or nucleic acid. Said target protein or nucleic acid is bound to another molecule to allow for ease of purification, such as, for example, a molecule selected from the group consisting of protein A, protein G, agarose, biotin, glutathione S-transferase (GST), and FLAG epitope. Accordingly, the target protein or nucleic acid is isolated simply through centrifugation, or through binding to another molecule; eg. streptavidin, or binding of a specific antibody, eg. anti-FLAG anitbodies, or anti-GST antibodies. Methods using target proteins or nucleic acids covalently bound to affinity matrices are particularly preferred.

In another embodiment, the expression library is expressed so as to allow identification of a bound peptide using FACS analysis. The screening of libraries using FACS analysis is described in US Patent No 6,455,63 (Rigel Pharmaceuticals Incorporated). In adapting the protocol to the present invention, it is particularly preferred that the expression libraries are expressed in such that they are displayed, such as for example, using in vitro display, bacterial surface display, yeast display, or mammalian display.

Preferably, an in vitro display library is screened by FACS sorting. In vitro displayed proteins are covalently linked to a particle or bead suitable for FACS sorting, such as, for example, glass, polymers such as for example polystyrene, latex or cross-linked dextrans such as Sepharose, cellulose, nylon, teflon, amongst others.

The displayed library bound to particles or beads is added to a target protein or nucleic acid that has been labelled with a labelling moiety, such as for example a fluorescent molecule, or a molecule which is detected by a second fluorescent molecule. Methods of labelling a target protein or nucleic acid are known in the art, and include methods using direct linkage or methods using a linker. The beads are then washed and subjected to sorting by FACS, which allows the beads with bound fluorescent target proteins or nucleic acids, to be separated from the beads that have not bound to a fluorescent target protein or nucleic acid.

Alternatively the library is screened using a biosensor-based assay, such as, for example, Biacore sensor chip technology (Biacore AB, UK). The Biacore sensor chip is a glass surface coated with a thin layer of gold modified with carboxymethylated dextran, to which the target protein or nucleic acid is covalently attached. The peptides encoded by the expression libraries are then exposed to the Biacore sensor chip comprising the target protein or nucleic acid.

Preferably, the nucleic acid fragment and its encoded polypeptide are linked, such as for example using display technology.

The Biacore sensor chip is further used in the analysis of the kinetics of the interaction of the peptide encoded by the expression library and the target protein or nucleic acid, such as for example through analyzing binding affinity using surface plasmon resonance. Essentially, surface plasmon resonance detects changes in the mass of the aqueous layer close to the chip surface, through measuring changes in the refractive index. Accordingly, when a peptide encoded by the expression library binds to the target protein or nucleic acid the refractive index increases. Such an assay additionally enables determination of the affinity of a peptide for a target protein or target nucleic acid.

As will be apparent ot the skilled artisan another biosensor, such as, for example, an evanescent biosensor, a membrane based biosensor (as described in AU 623,747, US 5,234,566 and USSN 20030143726) or a microcantilever biosensor (as described in USSN 20030010097) is useful for screening the peptides of the present invention.

### Determining the structure of a peptide

In a preferred embodiment, the structure of one or more peptides (and preferably, a plurality of peptides) selected or identified using a screening method described herein is determined. By determining the structure of a plurality of peptides, the present invention enables the identification of a secondary and/or tertiary structure that is conserved between the peptides. Preferably, a peptide having said conserved structure is then selected.

In one embodiment, the conserved structure (or the structure of the selected peptide) is different to that of a protein or fragment thereof that interacts with the target protein or target nucleic acid in nature.

In an alternative embodiment, the conserved structure (or the structure of the selected peptide) is the same as or similar to that of a protein or fragment thereof that interacts with the target protein or target nucleic acid in nature.

Bioinformatics and/or empirical means are preferably employed to determine one or more secondary structure and/or tertiary structures of peptides identified in a screen. It is to be understood and implicit in these processes that, whilst it is not strictly necessary to conduct structural analysis on multiple peptides, the conservation or recurrence of specific structural features in different peptides provides validation of the role of that structure in binding to the target protein or target nucleic acid. This is true even for structural features which have been previously identified or described in protein databases. Accordingly, a comparison of structural features of different peptides selected in the screen process is particularly preferred.

Empirical methods and/or means for determining the structure of a peptide will be apparent to the skilled artisan and include, for example, a technique selected from the group consisting of atomic absorption spectroscopy (AAS), auger electron spectroscopy (AES), coherent anti-Stokes spectroscopy (CARS), circular dichroism (CD), Conversion electron Mossbauer spectroscopy (CEMS), chemical ionization mass spectroscopy, chemically-induced dynamic electron/nuclear polarization (CIDEP/CIDNP), Cross polarization magic angle spinning (CP-MASS), combined rotation and multipulse spectroscopy (CRAMPS), distortionless enhancement by polraisation transfer, 2-Dimensional nuclear magnetic resonance spectroscopy, electron diffraction (ED), energy dispersive X-ray spectroscopy, electron energy-loss spectroscopy, electron-electron double resonance, electronic spectroscopy, electron impact mass spectroscopy, electron-nuclear double resonance (ENDOR), electron paramagnetic resonance spectroscopy, electron spin resonance spectroscopy (ESR), exchange spectroscopy, far infrared laser magnetic resonance, fluorescence spectroscopy, Fourier transform infrared spectroscopy (FTIR), gas-phase electron diffraction (GED), heteronuclear correlation spectroscopy (HETCOR), heteronuclear overhauser effect spectroscopy, Hyper Raman spectroscopy, infrared spectroscopy (IR), laser desorption mass spectroscopy, laser-induced fluorescence, laser magnetic resonance spectroscopy, magnetic circular dichroism, microwave spectroscopy, mass-analyzed ion kinetic energy spectroscopy, microwave optical double resonance spectroscopy, Mossbauer spectroscopy, multiphoton ionization spectroscopy, multistage mass spectroscopy (MS/MS), multiphoton induced fluorescence spectroscopy, nuclear gamma resonance spectroscopy, nuclear overhauser spectroscopy, nuclear quadrupole resonance spectroscopy, optical double resonance spectroscopy, photoelectron spectroscopy, photoionization mass spectroscopy, Raman spectroscopy, Raman-induced Kerr-effect spectroscopy, rotating frame Nuclear Overhauser Effect spectroscopy, rotational Raman spectroscopy, Rotational spectroscopy, resonance Raman spectroscopy, secondary ion mass spectroscopy, total correlation spectroscopy, vibrational spectroscopy, visible spectroscopy, X-ray diffraction, X-ray fluorescence spectroscopy, X-ray photoelectron spectroscopy, correlation spectroscopy (COSY), Coulomb explosion, HPLC, mass spectrometry (for example, MALDI, MALDI-TOF, LC-MS, MS-MS, GC-MS, LC/MS-MS, ES-MS, LC-ES-MS).

### Raman spectroscopy

For example, Raman spectroscopy is useful for the high-throughput screening and/or analysis of multiple samples. The Raman spectrum of a compound provides information both about its chemical nature as well as its physical state. For example, Raman spectra provides information about intra- and inter-molecular interactions, inclusions, salts forms, crystalline forms, and hydration states (or solvation states) of samples to identify suitable or desirable samples, or to classify a large number of samples. Raman spectroscopy is also useful for examining kinetics of changes in the hydration-state of a sample or compound-of-interest. The lack of a strong Raman signal from water, a common solvent or component in preparations allows collection of Raman data in-situ in a manner relevant to many applications. Suitable methods of Raman spectroscopy are described, for example, in Matsousek et al. J. Raman Spectroscopy. 32: 983-988, 2001, and USSN 20050130220.

### Infrared spectroscopy

Infrared (IR) spectroscopy is also a valuable technique for assessing protein secondary structure in solution. One particular form of IR spectroscopy, Fourier transform infrared spectroscopy (FTIR), has become a preferred form of IR spectroscopy for the study of protein secondary structure. FTIR is useful for the rapid determination of secondary structure as it offers accurate, high-resolution spectra with excellent sensitivity and signal-to-noise (S/N) ratios, as compared to other forms of infrared spectroscopy. Fuitable methods of FTIR are described, for example, in Kumosinski & Unruh, (1994) in ACS Symposium Series 576, Molecular Modeling: From Virtual Tools to Real Problems, (Kumosinski & Liebman, eds.) pp. 71-98; Susi & Byler, (1986) Method. Enzymol. 130: 290-311; Susi & Byler, Method Enzymol. 130: 290-311, 1986; Byler & Susi Biopolymers 25: 469-87, 1986; and Miyazawa et al., J. Chem. Phys. 24(2): 408-18., 1956

Proteins are known to have nine characteristic absorption bands in the mid-infrared region (approximately 1250 cm⁻¹ to 1850 cm⁻¹) that yield conformational insight and are known as the amide A, B, and I-VII bands (Susi & Byler, Method. Enzymol. 130: 290-311, 1986). The secondary structure of proteins are primarily been characterized by the frequency of the amide I and II bands.

### Nuclear magnetic resonance spectroscopy

Another preferred class of spectroscopy is nuclear magnetic resonance (NMR). Nuclear magnetic resonance (NMR) spectroscopy uses high magnetic fields and radiofrequency pulses to manipulate the spin statesExamples of nuclei, for example, 1H, 13C, and 15N, that have nonzero-spin angular momentum. For a molecule containing such nuclei, the result is a NMR spectrum with peaks, the positions and intensities of which reflect the chemical environment and nucleic positions within the molecule. As applied to protein-structure analysis, the accuracy now achievable with NMR spectroscopy is comparable to that obtained with X-ray crystallography.

Examples of such methods include, 1D, 2D, and 3D-NMR, including, for example, 1D spectra, such as single pulse, water-peak saturated, spin-echo such as CPMG (i.e., edited on the basis of nuclear spin relaxation times), diffusion-edited; 2D spectra, such as J-resolved (JRES),¹H^{- 1}H correlation methods such as NOESY, COSY, TOCSY and variants thereof, methods which correlated 1H to heteronuclei (including, for example, ^{13C}, ^{15N}, ^{19F}, and ^{31P}), such as direct detection methods such as HETCOR and inverse-detected methods such as ¹H^{- 13}C HMQC, HSQC and HMBO; 3D spectra, including many variants, which are combinations of 2D methods, e.g. HMQC-TOCSY, NOESY-TOCSY, etc. All of these NMR spectroscopic techniques can also be combined with magic-angle-spinning (MAS) to study samples other than isotropic liquids, which are characterized by anisotropic composition.

### Circular dichroism

Circular dichroism spectroscopy is performed by passing plane polarized light through a birefringent plate, which splits the light into two plane-polarized beams oscillating along different axes (e.g., fast and slow). When one of the beams is retarded by 90° (using a quarter-wave retarder) then the two beams which are now 90° out of phase are added together, the result is circularly polarized light of one direction. By inverting the two axes such that the alternate beam is retarded than circularly polarized light of the other direction is generated. The result of adding the right and left circularly polarized that passes through the optically active sample is elliptically polarized light, thus circular dichroism is equivalent to ellipticity. By determining the absorption of a purified peptide in solution at various wavelengths and comparing the absorption to absorptions obtained for proteins and/or peptides of known structure a structure is assigned to the peptide.

### X-ray crystallography

In another embodiment, the structure of a peptide is determined using X-ray crystallography. X-ray crystallography is a method useful for solving the three dimensional structures of a molecule. The structure of a molecule is calculated from X-ray diffraction patterns using a crystal as a diffraction grating. Three dimensional structures of protein molecules arise from crystals grown from a concentrated aqueous solution of that protein. For example, the process of X-ray crystallography includes the following steps:
(a) synthesizing and isolating (or otherwise obtaining) peptide;
(b) growing a crystal from an aqueous solution comprising the peptide; and
(c) collecting X-ray diffraction patterns from the crystals, determining unit cell dimensions and symmetry, determining electron density, fitting the amino acid sequence of the peptide to the electron density, and refining the structure.

Suitable methods for producing a peptide are described hereinabove.

Crystals are then grown from an aqueous solution containing the purified and concentrated peptide by any of a variety of techniques. These techniques include batch, liquid, bridge, dialysis, vapor diffusion, and hanging drop methods (McPherson John Wiley, New York, 1982; McPherson Eur. J. Biochem. 189:1-23, 1990; Webber Adv. Protein Chem. 41:1-36, 1991)

For example, a native crystal of a peptide is, in general, grown by adding precipitants to the concentrated solution of the peptide. The precipitants are added at a concentration just below that necessary to precipitate the protein. Water is removed by controlled evaporation to produce precipitating conditions, which are maintained until crystal growth ceases.

Following crystal growth, the crystal is placed in a glass capillary tube or other mounting device and mounted onto a holding device connected to an X-ray generator and an X-ray detection device. Collection of X-ray diffraction patterns are known in the art (e.g., Ducruix and Geige, (1992), IRL Press, Oxford, England, and references cited therein). A beam of X-rays enters the crystal and then diffracts from the crystal. An X-ray detection device is utilized to record the diffraction patterns emanating from the crystal. Suitable X-ray detection devices include, film or a digital recording device. Suitable X-ray sources are of various types, but advantageously, a high intensity source is used, e.g., a synchrotron beam source.

Methods for obtaining the three dimensional structure of the crystalline form of a peptide molecule or molecule complex are known in the art (e.g., Ducruix and Geige, (1992), IRL Press, Oxford, England, and references cited therein).

For example, after the X-ray diffraction patterns are collected from the crystal, the unit cell dimensions and orientation in the crystal are determined. The unit cell dimensions and orientation are determined from the spacing between the diffraction emissions as well as the patterns made from these emissions. The unit cell dimensions are characterized in three dimensions in units of Angstroms (one angstrom=10⁻¹⁰ meters) and by angles at each vertices. The symmetry of the unit cell in the crystals is also characterized at this stage. The symmetry of the unit cell in the crystal simplifies the complexity of the collected data by identifying repeating patterns.

Each diffraction pattern emission is characterized as a vector and the data collected at this stage of the method determines the amplitude of each vector. The phases of the vectors can be determined using multiple techniques. In one method, heavy atoms are soaked into a crystal (isomorphous replacement), and the phases of the vectors determined by using these heavy atoms as reference points in the X-ray analysis. (Otwinowski, (1991), Daresbury, United Kingdom, 80-86). The isomorphous replacement method usually utilizes more than one heavy atom derivative.

In another method, the amplitudes and phases of vectors from a crystalline polypeptide with an already determined structure is applied to the amplitudes of the vectors from a crystalline peptide of unknown structure and consequently determine the phases of these vectors. This method is known as molecular replacement and the protein structure which is used as a reference must have a closely related structure to the protein of interest (Naraza Proteins 11:281-296, 1994). For example, the structure of c-Jun is useful for the molecular replacement analysis of a peptide that binds to c-Jun.

Following determination of the phases of the vectors describing the unit cell of a crystal, the vector amplitudes and phases, unit cell dimensions, and unit cell symmetry are used as terms in a Fourier transform function. The Fourier transform function calculates the electron density in the unit cell from these measurements. The electron density that describes one of the molecules or one of the molecule complexes in the unit cell can be referred to as an electron density map. The amino acid structures of the sequence or the molecular structures of compounds complexed with the crystalline polypeptide are then fitted to the electron density using any of a variety of computer programs. This step of the process is sometimes referred to as model building and can be accomplished by using computer programs such as Turbo/FRODO or "O". (Jones Methods in Enzymology 115:157-171, 1985).

A theoretical electron density map is then calculated from the amino acid structures and fit to the experimentally determined electron density. The theoretical and experimental electron density maps are compared to one another and the agreement between these two maps described by a parameter (R-factor). A low value for an R-factor describes a high degree of overlapping electron density between a theoretical and experimental electron density map.

The R-factor is then minimized by using a computer program that refine the theoretical electron density map. A computer program such as X-PLOR can be used for model refinement by those skilled in the art (Briinger Nature 355:472-475, 1992). Refinement is achieved in an iterative process. For example, a first step comprises altering the conformation of atoms defined in an electron density map. The conformations of the atoms are altered by simulating a rise in temperature, which will increase the vibrational frequency of the bonds and modify positions of atoms in the structure. At a particular point in the atomic perturbation process, a force field, which typically defines interactions between atoms in terms of allowed bond angles and bond lengths, Van der Waals interactions, hydrogen bonds, ionic interactions, and hydrophobic interactions, are applied to the system of atoms. Favorable interactions are described in terms of free energy and the atoms moved over many iterations until a free energy minimum is achieved. The refinement process can be iterated until the R-factor reaches a minimum value.

The three dimensional structure of the molecule or molecule complex is described by atoms that fit the theoretical electron density characterized by a minimum R-value.

### In silico methods

The present invention also contemplates *an in silico* method for determining the structure of a peptide identified using a method described herein.

For example, structural features are determined using appropriate software available on the website of the National Center for Biotechnology Information (NCBI) at the National Institutes of Health, 8600 Rockville Pike, Bethesda MD 20894 such as, for example, through the NCBI Molecules Modelling Database (MMDB) including three-dimensional biomolecular structures determined using X-ray crystallography and/or NMR spectroscopy. The NCBI conserved domain database (CDD) includes domains from the well-known Smart and Pham collections, with links to a 3D-structure viewer (Cn3D). The NCBI Conserved Domain Architecture Retrieval Tool (CDART) uses precalculated domain assignments to neighbour proteins by their domain architecture.

Additional methods for predicting protein or peptide secondary structure are known in th art and/or described, for example, in Moult, Curr. Opin. Biotechnol. 7:422-27, 1996; Chou et al., Biochemistry 13:222-45, 1974; Chou et al., Biochemistry 113:211-22, 1974; Chou et al., Adv. Enzymo/. Relat. Areas Mol. Biol. 47:45-48, 1978; Chou et al., Ann. Rev. Biochem. 47:251-276, 1978; or Chou et al., Biophys. J. 26:367-84, 1979.

Additionally, computer programs are currently available to assist with predicting secondary structure of a protein or peptide. One such method of predicting secondary structure is based upon homology modeling. For example, two polypeptides or proteins or a peptide and a fragment of a polypeptide or protein that have a sequence identity of greater than 30%, or similarity greater than 40%, often have similar structural topologies. The recent growth of the protein structural database (PDB) has provided enhanced predictability of secondary structure, including the potential number of folds within the structure of a polypeptide or protein (Holm et al., Nucleic Acids Res. 27:244-47, 1999).

For example, methods for determining the structure of a peptide are described, for example, in US Patent Application No 20020150906 (California Institute of Technology), or using a computer program or algorithm, such as, for example, MODELLER, (Sali and Blundell, J. Mol. Biol. 234, 779-815, 1993). These techniques rely upon aligning the sequence of a peptide with the sequences of peptides or proteins that have a characterized structure. Such alignment algorithms are known in the art and are accessed through software packages such as, for example BLAST at NCBI. Structural information, ie. three-dimensional structure, of a query peptide is then be predicted based upon structural information corresponding to the sequence or subsequences aligned in the proteins or peptides that have previously been characterized. In this way it is possible to generate a library of three-dimensional structures of peptides expressed from the expression library. This information is used to determine those sequences that is adopt a conformation sufficient for binding to a target protein or nucleic acid.

Additional methods of predicting secondary structure include, for example, "threading" (Jones, Curr. Opin. Struct. Biol. 7:377-87, 1997; Sippl et al., Structure 4:15-19, 1996), "profile analysis" (Bowie et al., Science, 253:164-70, 1991; Gribskov et al., Methods Enzymol. 183:146-59, 1990; Gribskov et al., Proc. Nat. Acad. Sci. U.S.A. 84:4355-58, 1989), and "evolutionary linkage"

In a preferred embodiment, the secondary structure of a peptide is determined by threading. Conventional threading of protein sequence is used to predict the 3D structure scaffold of a protein. Typically, threading is a process of assigning the folding of the protein by threading (or comparing) its sequence to a library of potential structural templates by using a scoring function that incorporates the sequence as well as the local parameters such as secondary structure and solvent exposure (Rost *et al. 270:* 471-480, 1997; Xu and Xu Proteins: Structure, Function, and Genetics 40: 343-354, 2000); and Panchenko et al. J. Mol. Biol. 296: 1319-1331, 2000). For example, the threading process starts from prediction of the secondary structure of the amino acid sequence and solvent accessibility for each residue of the query sequence. The resulting one-dimensional (1D) profile of the predicted structure is threaded into each member of a library of known 3D structures. The optimal threading for each sequence-structure pair is obtained using dynamic programming. The overall best sequence-structure pair constitutes the predicted 3D structure for the query sequence. Using such a technique, the inventors have determined the structure of a number of peptides using the method of the invention. Additional description of suitable threading methods is provided below in the Examples.

In another embodiment, a peptide is selected that has a secondary and/or tertiary structure that differs to the structure of a protein (or fragment thereof) that binds to the target protein or target nucleic acid in nature. For example, the present inventors have identified a number of peptides that are capable of binding to c-Jun and inhibiting c-Jun dimerization that do not form a similar structure to the region of c-Jun that self-dimerizes.

In an alternative embodiment, the method comprises selecting a peptide that has a secondary and/or tertiary structure that is the same as or similar to the structure of a protein (or fragment thereof) that binds to the target protein or target nucleic acid in nature. For example, the present inventors have identified a number of peptides that are capable of binding to c-Jun and inhibiting c-Jun dimerization that are predicted to form a leucine zipper-like domain (i.e., a similar structure to the region of c-Jun that self-dimerizes).

A preferred embodiment of the invention provides a method of determining a peptide that binds to a target nucleic acid or target protein comprising:
(a) screening an expression library to identify a plurality of peptides expressed by the library that bind to the target protein or target nucleic acid;
(b) selecting a plurality of the peptides from (a) that do not bind to said target protein or nucleic acid in their native environment;
(c) determining the structure of a plurality of the selected peptides;
(d) determining a secondary and/or tertiary structure that is conserved between two or more of the selected peptides; and
(e) selecting one or more peptides from (c) having the conserved secondary structure and/or tertiary structure,
thereby determining a peptide that binds to a target nucleic acid or target protein.

Preferably, the target protein is c-Jun and the peptide that interacts with c-Jun additionally inhibits c-Jun dimerization.

In a preferred embodiment the peptide comprises a leucine zipper-like domain, for example, the leucine zipper-like domain comprises a plurality of amino acid residues spaced at most 6 to 12 residues apart, wherein the amino acid residues are selected from the group consisting of leucine, isoleucine, valine, methionine and mixtures thereof. Preferably, the amino acid residues are spaced 6 to 7 amino acid residues apart.

In a preferred embodiment the plurality of amino acid residues comprises at least 6 amino acid residues selected from the group consisting of leucine, isoleucine, valine, methionine and mixtures thereof.

Preferably, the amino acid residues are interspersed with hydrophobic amino acids. For example, each hydrophobic amino acid is within 3 or 4 amino acids of one or more amino acid residue(s) selected from the group consisting of leucine, isoleucine, valine and methionine.

In a preferred embodiment, the peptide additionally comprises an acidic domain. For example, the acidic domain comprises four or more arginine residues.

As will be apparent to the skilled person from the foregoing, the present invention enables a method of determining a peptide that binds to c-Jun, said method comprising:
(a) screening an expression library to identify a plurality of peptides expressed by the library that bind to c-Jun;
(b) selecting a plurality of the peptides from (a) that do not bind to c-Jun in their native environment;
(c) determining the structure of a plurality of the selected peptides; and
(e) selecting one or more peptides from (c) having a leucine zipper-like domain and optionally, an acidic domain,
thereby determining a peptide that binds to c-Jun.

Preferably, the method additionally comprises:
(f) determining a peptide selected at (e) that inhibits c-Jun dimerization.

In one embodiment, the nucleotide sequence of the nucleic acid encoding the identified peptide or protein domain is determined. Preferably, the sequences of several distinct peptides identified in a specific screen of a library are aligned and compared, and highly conserved primary and/or secondary structures within the peptides or protein domains are determined. Alternatively, or in addition, less conserved structures are also determined. More preferably, the highly conserved structural features are used to design and/or to produce additional peptides having the same or enhanced binding properties as the peptides identified in the initial screening.

### Additional characterization of identified peptides

As exemplified herein, the present inventors have further characterized peptides identified in a primary or secondary screen by introducing the peptide into a cell (e.g., by recombinant expression) and determining the effect of the peptide on the phenotype of a cell.

For example, the present inventors have produced a cell comprising a reporter gene the expression of which is operably under the control of c-Jun dimerization, e.g., by placing the reporter gene operably under the control of an AP-1 enhancer element. A cell in which c-Jun self-dimerizes is determined by detecting the expression of the reporter gene. A peptide identified by a method of the invention is then expressed in the cell and the level of c-Jun dimerization determined by determining the level of reporter gene expression. A peptide that reduces expression of the reporter gene is considered to bind to and inhibit c-Jun dimerization.

Accordingly, in one embodiment, the present invention enables a method for determining a peptide that binds to a target protein or target nucleic acid, the method comprising identifying or determining a peptide using a method described *supra* and additionally comprising characterizing a selected peptide by performing a process comprising:
(a) expressing in a cell comprising or expressing the target nucleic acid or target protein or introducing into a cell comprising or expressing the target nucleic acid or target protein the peptide; and
(b) determining the ability of the peptide to interact with the target nucleic acid or target protein in the cell.

In one embodiment, the ability of the peptide to interact with the target nucleic acid or target protein in the cell is determined by determining the level of expression of a reporter gene the expression of which is placed operably under the control of the interaction of the peptide that the target nucleic acid or target protein.

Preferably, the peptide inhibits the interaction of the target nucleic acid or target protein with another nucleic acid or protein and the ability of the peptide to interact with the target nucleic acid or target protein in the cell is determined by determining a reduced level of interaction between the target nucleic acid or target protein with the other nucleic acid or protein.

For example, the ability of the target nucleic acid or target protein to interact with the other nucleic acid or protein in the cell is determined by determining the level of expression of a reporter gene the expression of which is placed operably under the control of the interaction of the target nucleic acid or target protein and the other nucleic acid or protein.

As exemplified herein, a reporter gene that is placed operably under control of a AP-1 enhancer element is useful, for example, for determining a peptide that binds to and/or inhibits c-Jun dimerization.

In another embodiment, the interaction of a peptide with a target protein or target nucleic acid is determined by detecting or determining the level of a phenotype mediated by the target gene or nucleic acid in a cell that expresses the peptide or into which the peptide has been introduced.

For example, the present inventors have introduced a peptide identified by a screen of the invention into a cell and determined the level of c-Jun mediated cell death. For example, cell death is induced, by the addition of an apoptosis inducing factor (e.g., TNF-α) or by exposing the cell to ultraviolet radiation or by inducing hypoxia in the cell. Accordingly, in a preferred embodiment, a peptide is characterized by (i) introducing the peptide into a cell or expressing the peptide in a cell; (ii) maintaining the cell under conditions sufficient to induce cell death; and (iii) selecting a peptide that prevents cell death.

In a preferred embodiment, a cell is characterized by it's ability to reduce or prevent cell death. Preferably, the cell death is induced by performing a process selected from the group consisting of:
(a) contacting a cell with tumor necrosis factor α (TNFα) for a time and under conditions sufficient to induce cell death;
(b) exposing a cell to ultraviolet radiation for a time and under conditions sufficient to induce cell death; and
(c) contacting a cell with glutamate for a time and under conditions sufficient to induce cell death.

Methods for determining the level of cell death will be apparent to the skilled person. For example; APOPTEST (available from Immunotech) stains cells early in apoptosis, and does not require fixation of the cell sample (Martin *et al.,* 1994). This method utilizes an annexin V antibody to detect cell membrane re-configuration that is characteristic of cells undergoing apoptosis. Apoptotic cells stained in this manner can then sorted either by fluorescence activated cell sorting (FACS), ELISA or by adhesion and panning using immobilized annexin V antibodies.

Alternatively, as exemplified herein, a terminal deoxynucleotidyl transferase-mediated biotinylated UTP nick end-labeling (TUNEL) assay is used to determine the level of cell death. The TUNEL assay uses the enzyme terminal deoxynucleotidyl transferase to label 3'-OH DNA ends, generated during apoptosis, with biotinylated nucleotides. The biotinylated nucleotides are then detected by using streptavidin conjugated to a detectable marker. Kits for TUNEL staining are available from, for example, Intergen Company, Purchase, NY.

Alternatively, or in addition, an activated caspase, such as, for example, Caspase 3 is detected. Several caspases are effectors of apoptosis and, as a consequence, are only activated to significant levels in a cell undergoing programmed cell death. Kits for detection of an activated caspase are available from, for example, Promega Corporation, Madison WI, USA. Such assays are useful for both immunocytochemical or flow cytometric analysis of cell death.

Alternatively, or in addition a marker of cell death, e.g., Annexin V is detected, e.g., using FACS analysis, as exemplified herein.

### Target Validation

As exemplified herein , the nucleic acid fragment expression libraries are screened for encoded peptides that inhibit or antagonize or block dimerization of a protein, such as for example, JUN. Such peptide antagonists ("peptide blockers") are particularly useful for validating c-Jun as a cellular target in the therapeutic treatment of stroke. As exemplified herein, reverse two hybrid screens that assay the interaction between JUN1 and JUNZ (fragments of c-JUN that include the leucine zipper domain), have successfully been used to identify several specific peptide blockers of c-JUN dimerization.

It is therefore apparent that a selected peptide or protein domain and/or nucleic acid encoding same can be recovered and used to validate a therapeutic target (ie. it is used as a target validation reagent). By virtue of its ability to bind to a specific target protein or target nucleic acid, it is well within the ken of a skilled artisan to determine the *in vivo* effect of modulating the activity of the target protein or target nucleic acid by expressing the identified peptide or protein domain in an organism (eg., a bacterium, plant or animal such as, for example, an experimental animal or a human). In accordance with this aspect of the present invention, a phenotype of an organism that expresses the identified peptide or protein domain is compared to a phenotype of an otherwise isogenic organism (ie. an organism of the same species or strain and comprising a substantially identical genotype however does not express the peptide or protein domain). This is performed under conditions sufficient to induce the phenotype that involves the target protein or target nucleic acid. The ability of the peptide or protein domain to specifically prevent expression of the phenotype, preferably without undesirable or pleiotropic side-effects indicates that the target protein or target nucleic acid is a suitable target for development of therapeutic/prophylactic reagents.

Preferably, determining a phenotype of the organism that is modulated by the target protein or target nucleic acid comprises comparing the organism to an otherwise isogenic organism that does not express the selected peptide. For example, animal models of stroke can be assayed in the presence and absence of a peptide or protein domain that blocks c-Jun dimerization and stroke-inducing conditions applied to the animal. Amelioration of stroke damage, or prevention of stroke by the expressed peptide indicates that the c-Jun dimerization is a suitable target for intervention, wherein the peptide is then suitably formulated for therapeutic intervention directly, or alternatively, small molecules are identified that are mimetics of the identified peptide or protein domain.

### Databases of nucleotide sequences and amino acid sequences

The present invention also provides a database of nucleic acids that are selected by screening an expression library, as described herein. As the nucleic acid fragments are derived from organisms with substantially sequenced genomes, it is possible to use this information to generate a database of the nucleotide sequences of nucleic acid fragments that is generated in the construction of an expression library screened as described herein.

The utility of the database lies in the ability for a skilled person to search the database for a nucleotide sequence or amino acid sequence determined by screening the expression library. In this way, it is possible to identify nucleic acid fragments that encode a peptide that is adopt a conformation sufficient for binding to a specific target protein or nucleic acid. Furthermore, the database allows the user to identify a sequence that is homologous to a nucleic acid, in addition to determining from which species it is derived. Once a sequence is identified, the specific nucleic acid is isolated from the expression library using techniques known in the art, eg. PCR and the expressed peptide analyzed.

Nucleotide sequences of the nucleic acid fragments of the expression library are derived from any one of many publicly known databases, such as for example NCBI or TIGR, because the organisms used in the generation of an expression library screened as described herein has a substantially sequenced genome.

Such a database (i.e., comprising the sequences of nucleic acid fragments of the expression library and/or comprising the amino acid sequences of the peptides encoded by each nucleic acid fragment) is used, for example, to direct the synthesis of encoded peptides either by direct chemical synthesis, or alternatively, by producing the encoding nucleic acid and expressing said nucleic acid in a suitable expression system.

Amino acid sequences that are found in the database are derived by conceptual translation of nucleotide sequences that are selected from the screened expression library. The conceptual translation of a nucleotide sequence comprises applying the known codon usage rules to obtain hypothetical peptide sequences by translating a nucleotide sequence in both orientations and in all three reading frames for each possible orientation. Software for translation of nucleotide sequence to amino acid sequence is known in the art, and includes, for example, the Translate tool at ExPasy. Care is taken to translate a nucleotide sequence using the known codon usage of the organism in which a nucleic acid fragment is to be expressed. Such codon usage information is known in the art Amino acid sequences are also derived by sequencing the expressed peptides. Methods of sequencing peptides and proteins are known in the art.

The conceptual translation of the sequences of peptides encoded by the libraries described herein assists the identification and/or isolation of those peptides from complex mixtures.

In a related embodiment, a database of amino acid sequences of peptides is analyzed to generate a database of domain structures, or three-dimensional structures that is formed by a peptide expressed by the expression library. Methods for predicting the 3 dimensional structure of a peptide are known in the art, described *supra.*

### Synthesis ofpeptide inhibitors of c-jun dimerization

As exemplified herein, the present inventors have identified a number of distinct c-Jun inhibitory peptides (Table 4 and 5), the amino acid sequences of which are set forth in the Sequence Listing. These are to be understood to comprise a non-exhaustive list of c-Jun inhibitory peptides. The skilled artisan is readily able to produce additional c-Jun inhibitory peptides following the teaching provided herein, e.g., using different libraries produced according to the methods described, including libraries derived from different genome sources to those exemplified.

In a particularly preferred embodiment, a c-Jun dimerization inhibitory peptide will comprise an amino acid sequence selected from the group consisting of:
A c-Jun dimerization inhibitory peptide of the present invention is readily synthesized by recombinant means using methods known in the art and/or described herein. For example, nucleic acid encoding a peptide is synthesized from the deduced amino acid sequence (e.g., as set forth in Table 5).

Alternatively, a c-Jun dimerization inhibitory peptide of the present invention is readily synthesized from its determined amino acid sequence using standard techniques, e.g., using BOC or FMOC chemistry. Synthetic peptides are prepared using known techniques of solid phase, liquid phase, or peptide condensation, or any combination thereof, and can include natural and/or unnatural amino acids. Amino acids used for peptide synthesis may be standard Boc (Nα-amino protected Nα-t-butyloxycarbonyl) amino acid resin with the deprotecting, neutralization, coupling and wash protocols of the original solid phase procedure of Merrifield, J. Am. Chem. Soc., 85:2149-2154, 1963, or the base-labile Nα-amino protected 9-fluorenylmethoxycarbonyl (Fmoc) amino acids described by Carpino and Han, J. Org. Chem., 37:3403-3409, 1972. Both Fmoc and Boc Nα-amino protected amino acids can be obtained from various commercial sources, such as, for example, Fluka, Bachem, Advanced Chemtech, Sigma, Cambridge Research Biochemical, Bachem, or Peninsula Labs.

The Merrifield method of synthesis (Merrifield, J Am Chem Soc, 85,:2149-2154, 1963) and the myriad of available improvements on that technology are described in the art (see e.g., Synthetic Peptides: A User's Guide, Grant, ed. (1992) W.H. Freeman & Co., New York, pp. 382; Jones (1994) The Chemical Synthesis of Peptides, Clarendon Press, Oxford, pp. 230.); Barany, G. and Merrifield, R.B. (1979) in The Peptides (Gross, E. and Meienhofer, J. eds.), vol. 2, pp. 1-284, Academic Press, New York; Wünsch, E., ed. (1974) Synthese von Peptiden in Houben-Weyls Metoden der Organischen Chemie (Müler, E., ed.), vol. 15, 4th edn., Parts 1 and 2, Thieme, Stuttgart;Bodanszky, M. (1984) Principles of Peptide Synthesis, Springer-Verlag, Heidelberg; Bodanszky, M. & Bodanszky, A. (1984) The Practice of Peptide Synthesis, Springer-Verlag, Heidelberg; Bodanszky, M. (1985) Int. J. Peptide Protein Res. 25, 449-474.

Synthetic peptides may also be produced using techniques known in the art and described, for example, in Stewart and Young (In: Solid Phase Synthesis, Second Edition, Pierce Chemical Co., Rockford, Ill. (1984) and/or Fields and Noble (Int. J. Pept. Protein Res., 35:161-214, 1990), or using automated synthesizers. Accordingly, peptides of the invention may comprise D-amino acids, a combination of D- and L-amino acids, and various unnatural amino acids (e.g., α-methyl amino acids, Cα-methyl amino acids, and Nα-methyl amino acids, etc) to convey special properties. Synthetic amino acids include ornithine for lysine, fluorophenylalanine for phenylalanine, and norleucine for leucine or isoleucine.

### Analogues of c-Jun dimerization inhibitors

The amino acid sequences of the c-Jun dimerization inhibitory peptides described may be modified for particular purposes according to methods well known to those of skill in the art without adversely affecting their c-Jun dimerization inhibitory activity. Such analogues may be produced by chemical means or alternatively, by recombinant expression of nucleic acid encoding an analogue as described herein.

For example, particular peptide residues may be derivatized or chemically modified in order to enhance the stability of the peptide or to permit coupling of the peptide to other agents, particularly lipids. It also is possible to change particular amino acids within the peptides without disturbing the overall structure of the peptide. Such changes are therefore termed "conservative" changes and tend to rely on the hydrophilicity or polarity of the residue. The size and/or charge of the side chains also are relevant factors in determining which substitutions are conservative.

It is well understood by the skilled artisan that, inherent in the definition of a biologically functional equivalent protein or peptide, is the concept that there is a limit to the number of changes that may be made within a defined portion of the molecule and still result in a molecule with an acceptable level of equivalent biological activity. Biologically functional equivalent peptides are thus defined herein as those peptides in which specific amino acids may be substituted. Particular embodiments encompass variants that have one, two, three, four, five or more variations in the amino acid sequence of the peptide. Of course, a plurality of distinct proteins/peptides with different substitutions may easily be made and used in accordance with the invention.

Those skilled in the art are well aware that the following substitutions are permissible conservative substitutions (i) substitutions involving arginine, lysine and histidine; (ii) substitutions involving alanine, glycine and serine; and (iii) substitutions involving phenylalanine, tryptophan and tyrosine. Peptides incorporating such conservative substitutions are defined herein as biologically functional equivalents.

The importance of the hydropathic amino acid index in conferring interactive biological function on a protein is generally understood in the art (Kyte & Doolittle, J. Mol. Biol. 157, 105-132, 1982). It is known that certain amino acids may be substituted for other amino acids having a similar hydropathic index or score and still retain a similar biological activity. The hydropathic index of amino acids also may be considered in determining a conservative substitution that produces a functionally equivalent molecule. Each amino acid has been assigned a hydropathic index on the basis of their hydrophobicity and charge characteristics, as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5). In making changes based upon the hydropathic index, the substitution of amino acids whose hydropathic indices are within .+/- 0.2 is preferred. More preferably, the substitution will involve amino acids having hydropathic indices within .+/- 0.1, and more preferably within about +/-0.05.

It is also understood in the art that the substitution of like amino acids is made effectively on the basis of hydrophilicity, particularly where the biological functional equivalent protein or peptide thereby created is intended for use in immunological embodiments, as in the present case (e.g. US Patent No. 4,554,101), In fact, the greatest local average hydrophilicity of a protein, as governed by the hydrophilicity of its adjacent amino acids, correlates with its immunogenicity and antigenicity. As detailed in US Patent No. 4,554,101, the following hydrophilicity values have been assigned to amino acid residues: arginine (+3.0); lysine (+3.0); aspartate (+3.0 +/- 0.1); glutamate (+3.0 +/- 0.1); serine (+0.3); asparagine (+0.2); glutamine (+0.2); glycine (0); threonine (-0.4); proline (-0.5 +/- 0.1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, it is preferred to substitute amino acids having hydrophilicity values within about +/- 0.2 of each other, more preferably within about +/- 0.1, and even more preferably within about +/- 0.05

It also is contemplated that other sterically similar compounds may be formulated to mimic the key portions of the peptide structure. Such compounds, which may be termed peptidomimetics, may be used in the same manner as the peptides of the invention and hence are also functional equivalents. The generation of a structural functional equivalent may be achieved by the techniques of modeling and chemical design known to those of skill in the art. It will be understood that all such sterically similar constructs fall within the scope of the present invention.

Another method for determining the "equivalence" of modified peptides involves a functional approach. For example, a given peptide analogue is tested fro its ability to inhibit c-Jun dimerization e.g., using any screening method described herein.

Particularly preferred analogues of a peptide of the invention will comprise one or more non-natrually occurring amino acids or amino acid analogues. For example, a c-Jun dimerization inhibitory peptide of the invention may comprise one or more naturally occurring non-genetically encoded L-amino acids, synthetic L-amino acids or D-enantiomers of an amino acid. More particularly, the analogue may comprise one or more residues selected from the group consisting of: hydroxyproline, β-alanine, 2,3-diaminopropionic acid, α-aminoisobutyric acid, N-methylglycine (sarcosine), ornithine, citrulline, t-butylalanine, t-butylglycine, N-methylisoleucine, phenylglycine, cyclohexylalanine, norleucine, naphthylalanine, pyridylananine 3-benzothienyl alanine 4-chlorophenylalanine, 2-fluorophenylalanine, 3-fluorophenylalanine, 4-fluorophenylalanine, penicillamine, 1,2,3,4-tetrahydro-tic isoquinoline-3-carboxylic acid β-2-thienylalanine, methionine sulfoxide, homoarginine, N-acetyl lysine, 2,4-diamino butyric acid, ρ-aminiophenylalanine , N-methylvaline, homocysteine, homoserine, ε-amino hexanoic acid, δ-amino valeric acid, 2,3-diaminobutyric acid and mixtures thereof.

Commonly-encountered amino acids which are not genetically encoded and which can be present, or substituted for an amino acid, in a peptides analogue of the invention include, but are not limited to, β-alanine (b-Ala) and other omega-amino acids such as 3-aminopropionic acid (Dap), 2,3-diaminopropionic acid (Dpr), 4-aminobutyric acid and so forth; α-aminoisobutyric acid (Aib); ε-aminohexanoic acid (Aha); δ-aminovaleric acid (Ava); methylglycine (MeGly); omithine (Om); citrulline (Cit); t-butylalanine (t-BuA); t-butylglycine (t-BuG); N-methylisoleucine (MeIle); phenylglycine (Phg); cyclohexylalanine (Cha); norleucine (Nle); 2-naphthylalanine (2-Nal); 4-chlorophenylalanine (Phe(4-Cl)); 2-fluorophenylalanine (Phe(2-F)); 3-fluorophenylalanine (Phe(3-F)); 4-fluorophenylalanine (Phe(4-F)); penicillamine (Pen); 1,2,3,4-tetrahydroisoquinoline-3-carboxylic acid (Tic); .beta.-2-thienylalanine (Thi); methionine sulfoxide (MSO); homoarginine (hArg); N-acetyl lysine (AcLys); 2,3-diaminobutyric acid (Dab); 2,3-diaminobutyric acid (Dbu); p-aminophenylalanine (Phe(pNH₂)); N-methyl valine (MeVal); homocysteine (hCys) and homoserine (hSer).

Other amino acid residues that are useful for making the peptides and peptide analogues described herein can be found, e.g., in Fasman, 1989, CRC Practical Handbook of Biochemistry and Molecular Biology, CRC Press, Inc., and the references cited therein.

As used herein, "analogues" include "derivatives" or "derivatized peptide compounds", wherein a peptidyl compound is modified to contain one or more-chemical moieties other than an amino acid. The chemicalmoiety may be linked covalently to the peptidyl moiety e.g., via an amino terminal amino acid residue, a carboxy terminal amino acid residue, or at an internal amino acid residue. Such modifications include the addition of a protective or capping group on a reactive moiety in the peptide, addition of a detectable label, and other changes that do not adversely destroy the activity of the peptide compound (e.g., its ability to bind to c-Jun and/or inhibit c-Jun dimerization).

An "amino terminal capping group" of a peptide compound described herein is any chemical compound or moiety that is covalently linked or conjugated to the amino terminal amino acid residue of a peptide compound. An amino terminal capping group may be useful to inhibit or prevent intramolecular cyclization or intermolecular polymerization, to promote transport of the peptide compound across the blood-brain barrier (BBB), to protect the amino terminus from an undesirable reaction with other molecules, to provide additional antioxidative activity, or to provide a combination of these properties. A peptide compound of this invention that possesses an amino terminal capping group may possess other beneficial activities as compared with the uncapped peptide, such as enhanced efficacy or reduced side effects. Examples of amino terminal capping groups that are useful in preparing peptide compounds and compositions according to this invention include, but are not limited to, 1 to 6 naturally occurring L-amino acid residues, preferably, 1-6 lysine residues, 1-6 arginine residues, or a combination of lysine and arginine residues; urethanes; urea compounds; lipoic acid ("Lip"); glucose-3-O-glycolic acid moiety ("Gga"); or an acyl group that is covalently linked to the amino terminal amino acid residue of a peptide, wherein such acyl groups useful in the compositions of the invention may have a carbonyl group and a hydrocarbon chain that ranges from one carbon atom (e.g., as in an acetyl moiety) to up to 25 carbons (e.g., palmitoyl group, "Palm" (16:0) and docosahexaenoyl group, "DHA" (C22:6-3)). Furthermore, the carbon chain of the acyl group may be saturated, as in Palm, or unsaturated, as in DHA. It is understood that when an acid, such as docosahexaenoic acid, palmitic acid, or lipoic acid is designated as an amino terminal capping group, the resultant peptide compound is the condensed product of the uncapped peptide and the acid.

A "carboxy terminal capping group" of a peptide compound described herein is any chemical compound or moiety that is covalently linked or conjugated to the carboxy terminal amino acid residue of the peptide compound. The primary purpose of such a carboxy terminal capping group is to inhibit or prevent intramolecular cyclization or intermolecular polymerization, to promote transport of the peptide compound across the blood-brain barrier, and to provide a combination of these properties. A peptide compound of this invention possessing a carboxy terminal capping group may also possess other beneficial activities as compared with the uncapped peptide, such as enhanced efficacy, reduced side effects, enhanced hydrophilicity, enhanced hydrophobicity. Carboxy terminal capping groups that are particularly useful in the peptide compounds described herein include primary or secondary amines that are linked by an amide bond to the .alpha.-carboxyl group of the carboxy terminal amino acid of the peptide compound. Other carboxy terminal capping groups useful in the invention include aliphatic primary and secondary alcohols and aromatic phenolic derivatives, including flavenoids, with 1 to 26 carbon atoms, which form esters when linked to the carboxylic acid group of the carboxy terminal amino acid residue of a peptide compound described herein.

Other chemical modifications of a peptide or analogue, include, for example, glycosylation, acetylation (including N-terminal acetylation), carboxylation, carbonylation, phosphorylation, PEGylation, amidation, addition of trans olefin, substitution of α-hydrogens with methyl groups, derivatization by known protecting/blocking groups, circularization, inhibition of proteolytic cleavage (e.g., using D amino acids), linkage to an antibody molecule or other cellular ligand, etc. Any of numerous chemical modifications may be carried out by known techniques, including but not limited to specific chemical cleavage by cyanogen bromide, trypsin, chymotrypsin, papain, V8 protease, NaBH₄, acetylation, formylation, oxidation, reduction, etc.

The present invention additionally encompasses an isostere of a peptide described herein. The term "isostere" as used herein is intended to include a chemical structure that can be substituted for a second chemical structure because the steric conformation of the first structure fits a binding site specific for the second structure. The term specifically includes peptide back-bone modifications (i.e., amide bond mimetics) well known to those skilled in the art. Such modifications include modifications of the amide nitrogen, the α-carbon, amide carbonyl, complete replacement of the amide bond, extensions, deletions or backbone crosslinks. Several peptide backbone modifications are known, including ψ[CH₂S], ψ[CH₂NH], ψ[CSNH₂], ψ[NHCO], ψ[COCH₂], and ψ[(E) or (Z) CH=CH]. In the nomenclature used above, ψ indicates the absence of an amide bond. The structure that replaces the amide group is specified within the brackets.

Other possible modifications include an N-alkyl (or aryl) substitution (ψ [CONR]), or backbone crosslinking to construct lactams and other cyclic structures. Other derivatives of the modulator compounds of the invention include C-terminal hydroxymethyl derivatives, O-modified derivatives (e.g., C-terminal hydroxymethyl benzyl ether), N-terminally modified derivatives including substituted amides such as alkylamides and hydrazides and compounds in which a C-terminal phenylalanine residue is replaced with a phenethylamide analogue (e.g., Val-Phe-phenethylamide as an analogue of the tripeptide Val-Phe-Phe).

Particularly preferred analogues of a c-Jun dimerization inhibitory peptide are retro-inverted peptide analogues (also known as retro-inverso peptides). These analogues are isomers of linear peptides in which the direction of the amino acid sequence is reversed (retro) and the chirality, D- or L-, of one or more amino acids therein is inverted (inverso) e.g., using D-amino acids rather than L-amino acids, e.g., Jameson et al., Nature, 368, 744-746 (1994); Brady et al., Nature, 368, 692-693 (1994). The net result of combining D-enantiomers and reverse synthesis is that the positions of carbonyl and amino groups in each amide bond are exchanged, while the position of the side-chain groups at each alpha carbon is preserved.

An advantage of retro-inverso peptides is their enhanced activity *in vivo* due to improved resistance to proteolytic degradation (e.g., Chorev et al., Trends Biotech. 13, 438-445, 1995).

In one embodiment, the retro-inverso peptide is N-terminally modified, for example, with a modifying group comprising an alkyl group such as a C1-C6 lower alkyl group, e.g., a methyl, ethyl, or propyl group; or a cyclic, heterocyclic, polycyclic or branched alkyl group, or one or more an amino acid linker residues.

In another embodiment, the retro-inverso peptide is C-terminally modified, for example with an amide group, an alkyl or aryl amide group (e.g., phenethylamide) or a hydroxy group (i.e., the reduction product of a peptide acid, resulting in a peptide alcohol), or one or more an amino acid linker residuese.g., glycine, cysteine, etc.

It is also within the scope of the present invention for the retro-inverso peptide to be further modified by the inclusion of one or more targeting domains e.g., penetratin, TAT etc added to the N-terminus and/or C-terminus. Such peptide additions may be separated from the retro-inverso peptide moiety by oneo ro more linkers e.g., glycine, cysteine, etc.

Retro-inverso peptide analogues may be complete or partial. Complete retro-inverso peptides are those in which a complete sequence of a c-Jun dimerization inhibitory peptide is reversed and the chirality of each amino acid in a sequence is inverted. Partial retro-inverso peptide analogues are those in which only some of the peptide bonds are reversed and the chirality of only those amino acid residues in the reversed portion is inverted. For example, The present invention clearly encompasses both partial and complete retro-inverso peptide analogues.

For example, the amino acid sequence of a c-Jun dimerization inhibitory peptide of the present invention may be reversed completely and every amino acid residue inverted (i.e., substituted with a corresponding D-amino acid residue) to produce a complete retroinverso analogue of the peptide.

Preferred retro-inverso analogues are partial analogues wherein the complete amino acid sequence of a c-Jun dimerization inhibitory peptide of the present invention is reversed and an amino acid residue in said sequence other than glycine is inverted (i.e., substituted with a corresponding D-amino acid residue). Preferably, all amino acid residues other than glycine are inverted. In accordance with this preferred embodiment, a retro-inverso peptide analogue of the present invention will comprise a protein transduction domain such as penetratin or a TAT sequence, optionally fused to the retro-inverso peptide moiety by means of an amino acid linker, such as glycine.

In a particularly preferred embodiment, the present invention enables an analogue of a peptide that capable of inhibiting c-Jun dimerization, wherein said analogue comprises a complete or partial reverse of an amino acid sequence set forth in SEQ ID NO: 136 and wherein one or more amino acid residues of the reversed amino acid sequence are D-amino acid residues.

More preferably, the present invention enables an analogue of a peptide that capable of inhibiting c-Jun dimerization, wherein said analogue comprises (i) a first peptidyl moiety comprising a sequence that consists of complete or partial reverse of an amino acid sequence set forth in SEQ ID NO: 136 and wherein one or more amino acid residues of the reversed amino acid sequence are D-amino acid residues; and (ii) a protein transduction domain optonally separated from (i) by an amino acid spacer.

Still more preferably, two or three or four or five or six or seven or eight or none or ten or eleven or twelve or thirteen or fourteen or fifteen or sixteen amino acid residues other than glycine are D-amino acids. Even more preferably, the analogue will comprise one or more D-amino acids selected from the group consisting of D-arginine, D-glutamate, D-serine, D-glutamine, D-isoleucine, D-tyrosine, D-alanine, D-lysine, D-proline and D-leucine.

In a particularly preferred embodiment, the analogue will comprise an amino acid sequence set forth in SEQ ID NO: 182.

### Peptidelanalogue isolation

After being produced or synthesized, a peptide compound that is useful in the compositions and methods of the invention may be purified using methods known in the art. Such purification preferably provides a peptide of the invention in a state dissociated from significant or detectable amounts of undesired side reaction products; unattached or unreacted moieties used to modify the peptide compound; and dissociated from other undesirable molecules, including but not limited to other peptides, proteins, nucleic acids, lipids, carbohydrates, and the like.

Standard methods of peptide purification are employed to obtained isolated peptide compounds of the invention, including but not limited to various high-pressure (or performance) liquid chromatography (HPLC) and non-HPLC peptide isolation protocols, such as size exclusion chromatography, ion exchange chromatography, phase separation methods, electrophoretic separations, precipitation methods, salting in/out methods, immunochromatography, and/or other methods.

A preferred method of isolating peptide compounds useful in compositions and methods of the invention employs reversed-phase HPLC using an alkylated silica column such as C₄-, C₈- or C₁₈-silica. A gradient mobile phase of increasing organic content is generally used to achieve purification, for example, acetonitrile in an aqueous buffer, usually containing a small amount of trifluoroacetic acid. Ion-exchange chromatography can also be used to separate peptide compounds based on their charge. The degree of purity of the peptide compound may be determined by various methods, including identification of a major large peak on HPLC. A peptide compound that produces a single peak that is at least 95% of the input material on an HPLC column is preferred. Even more preferable is a polypeptide that produces a single peak that is at least 97%, at least 98%, at least 99% or even 99.5% of the input material on an HPLC column.

To ensure that a peptide compound obtained using any of the techniques described above is the desired peptide compound for use in compositions and methods of the present invention, analysis of the compound's composition determined by any of a variety of analytical methods known in the art. Such composition analysis may be conducted using high resolution mass spectrometry to determine the molecular weight of the peptide. Alternatively, the amino acid content of a peptide can be confirmed by hydrolyzing the peptide in aqueous acid, and separating, identifying and quantifying the components of the mixture using HPLC, or an amino acid analyzer. Protein sequenators, which sequentially degrade the peptide and identify the amino acids in order, may also be used to determine definitely the sequence of the peptide. Since some of the peptide compounds contain amino and/or carboxy terminal capping groups, it may be necessary to remove the capping group or the capped amino acid residue prior to a sequence analysis. Thin-layer chromatographic methods may also be used to authenticate one or more constituent groups or residues of a desired peptide compound. Purity of a peptide compound may also be assessed by electrophoresing the peptide compound in a polyacrylamide gel followed by staining to detect protein components separated in the gel.

### Therapeutic compositions

As will be apparent to the skilled artisan, peptides identified in the method of the present invention are useful as a therapeutic and/or prophylactic treatment of a disease and/or disorder. In addition to producing peptides that inhibit c-Jun dimerization, the present inventors have also produced retro-inverso peptides (i.e., analogues of the exemplified peptides) and shown their efficacy in a cellular model of ischemia, including stroke.

Accordingly, the present invention also provides a method of treatment of a disease or disorder comprising administering an effective amount of a peptide identified by the method of the present invention or an analogue thereof to a subject suffering from the disease and/or disorder or at risk of developing and/or suffering from the disease and/or disorder and/or in need of treatment.

Clearly the present invention encompasses the use of a peptide identified by a method of the present invention or analogue thereof in medicine. Additionally, the present invention encompasses a peptide identified by the present invention when used in medicine.

As will be apparent to the skilled artisan, peptides identified in the method of the present invention and analogues thereof are useful for inhibiting c-Jun dimerization. Such activity renders the peptide(s) and analogues thereof useful for the treatment of ischemia or an ischemic event e.g., stroke.

As will be apparent to the skilled artisan, the use of a peptide identified by the method of the present invention or analogue thereof to treat a disorder may require the peptide or analogue be formulated into a compound for administration.

Preferably, the compound is a pharmaceutical compound.

To prepare pharmaceutical or sterile compositions including a peptide or nucleic acid identified using the method of the invention, the peptide or analogue thereof, or isolated nucleic acid, is mixed with a pharmaceutically acceptable carrier or excipient. Compositions comprising a therapeutic peptide or nucleic acid are prepared, for example, by mixing with physiologically acceptable carriers, excipients, or stabilizers in the form of, e.g., lyophilized powders, slurries, aqueous solutions, lotions, or suspensions (see, e.g., Hardman, et al. (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Formulation of a pharmaceutical compound will vary according to the route of administration selected (e.g., solution, emulsion, capsule). For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils, for instance. Intravenous vehicles can include various additives, preservatives, or fluid, nutrient or electrolyte replenishers and the like (See, generally, Remington's Pharmaceutical Sciences, 17t Edition, Mack Publishing Co., Pa., 1985). For inhalation, the agent can be solubilized and loaded into a suitable dispenser for administration (e.g., an atomizer, nebulizer or pressurized aerosol dispenser).

Furthermore, where the agent is a protein or peptide or analogue thereof, the agent can be administered via in vivo expression of the recombinant protein. In vivo expression can be accomplished via somatic cell expression according to suitable methods (see, e.g. U.S. Pat. No. 5,399,346). In this embodiment, nucleic acid encoding the protein can be incorporated into a retroviral, adenoviral or other suitable vector (preferably, a replication deficient infectious vector) for delivery, or can be introduced into a transfected or transformed host cell capable of expressing the protein for delivery. In the latter embodiment, the cells can be implanted (alone or in a barrier device), injected or otherwise introduced in an amount effective to express the protein in a therapeutically effective amount.

As will be apparent to a skilled artisan, a compound that is active *in vivo* is particular preferred. A compound that is active in a human subject is even more preferred. Accordingly, when manufacturing a compound that is useful for the treatment of a disease it is preferable to ensure that any components added to the peptide does not inhibit or modify the activity of said peptide or analogue.

Selecting an administration regimen for a therapeutic composition depends on several factors, including the serum or tissue turnover rate of the entity, the level of symptoms, the immunogenicity of the entity, and the accessibility of the target cells in the biological matrix. Preferably, an administration regimen maximizes the amount of therapeutic compound delivered to the patient consistent with an acceptable level of side effects. Accordingly, the amount of composition delivered depends in part on the particular entity and the severity of the condition being treated. Guidance in selecting appropriate doses of peptides are available (see, e.g., Milgrom, et al. New Engl. J. Med. 341:1966-1973, 1999; Slamon, et al. New Engl. J. Med. 344:783-792, 2001; Beniaminovitz, et al. New Engl. J. Med. 342:613-619, 2000; Ghosh, et al. New Engl. J. Med. 348:24-32, 2003; or Lipsky, et al. New Engl. J. Med. 343:1594-1602, 2000).

A peptide is provided, for example, by continuous infusion, or by doses at intervals of, e.g., one day, one week, or 1-7 times per week. Doses of a composition may be provided intravenously, subcutaneously, topically, orally, nasally, rectally, intramuscular, intracerebrally, or by inhalation. A preferred dose protocol is one involving the maximal dose or dose frequency that avoids significant undesirable side effects. A total weekly dose depends on the type and activity of the compound being used to deplete B cells. For example, such a dose is at least about 0.05 µg/kg body weight, or at least about 0.2 µg/kg, or at least about 0.5 µg/kg, or at least about 1 µg/kg, or at least about 10 µg/kg, or at least about 100 µg/kg, or at least about 0.2 mg/kg, or at least about 1.0 mg/kg, or at least about 2.0 mg/kg, or at least about 10 mg/kg, or at least about 25 mg/kg, or at least about 50 mg/kg (see, e.g., Yang, et al. New Engl. J. Med. 349:427-434, 2003; or Herold, et al. New Engl. J. Med. 346:1692-1698, 2002.

An effective amount of a peptide for a particular patient may vary depending on factors such as the condition being treated, the overall health of the patient, the method route and dose of administration and the severity of side affects, see, e.g., Maynard, et al. (1996) A Handbook of SOPs for Good Clinical Practice, Interpharm Press, Boca Raton, Fla.; or Dent (2001) Good Laboratory and Good Clinical Practice, Urch Publ., London, UK.

Determination of the appropriate dose is made by a clinician, e.g., using parameters or factors known or suspected in the art to affect treatment or predicted to affect treatment. Generally, the dose begins with an amount somewhat less than the optimum dose and is increased by small increments thereafter until the desired or optimum effect is achieved relative to any negative side effects. Important diagnostic measures include those of symptoms of the disease and/or disorder being treated. Preferably, a compound that will be used is derived from or adapted for use in the same species as the subject targeted for treatment, thereby minimizing a humoral response to the reagent.

An effective amount of therapeutic will decrease disease symptoms, for example, as described *supra,* typically by at least about 10%; usually by at least about 20%; preferably at least about 30%; more preferably at least about 40%, and more preferably by at least about 50%.

The route of administration is preferably by, e.g., topical or cutaneous application, injection or infusion by intravenous, intraperitoneal, intracerebral, intramuscular, intraocular, intraarterial, intracerebrospinal, intralesional, or pulmonary routes, or by sustained release systems or an implant (see, e.g., Sidman et al. Biopolymers 22:547-556, 1983; Langer, et al. J. Biomed Mater. Res. 15:167-277, 1981; Langer Chem. Tech. 12:98-105, 1982; Epstein, et al. Proc. Natl. Acad. Sci. USA 82:3688-3692, 1985; Hwang, et al. Proc. Natl. Acad. Sci. USA 77:4030-4034, 1980; U.S. Pat. Nos. 6,350,466 and 6,316,024).

### Methods of treatment of an ischemic disorder

As exemplified herein, several peptides and peptide analogues isolated by the inventors have been shown to be useful for the treatment of a variety of models of ischemia, an ischemic disorder (e.g., stroke). Accordingly, the present invention provides, a method of treating ischemia, an ischemic disorder, an ischemic event (e.g., stroke), said method comprising administering a peptide according to any embodiment herein or an analogue thereof or a pharmaceutical composition comprising said peptide or analogue to a subject in need of treatment.

Alternatively, the present invention provides a method of treating an ischemic disorder, said method comprising administering a nucleic acid described herein according to any ewmbodiment or a pharmaceutical composition comprising said nucleic acid to a subject in need of treatment.

Methods of administering the peptides, analogues or nucleic acid will be apparent to the skilled person. For example, the peptide, analogue or nucleic acid is administered to a subject by a method selected from the group consisting of intravenous administration, intrathecal administration, intra-arterial administration, local administration following a craniotomy, and mixtures thereof.

Preferred routes of administration of a peptide or functional analogue thereof according to the invention in patients suffering from an ischemic disorder are, for example:
(i) intravenously, for example, in a 0.9% saline solution;
(ii) intrathecally, for example, the peptide composition is given after a lumbar puncture with a 18 G needle or after subsequent insertion of a extralumbal catheter with the tip in the intrathecal space;
(iii) by selective intra-arterial digital subtraction angiography, for example, wherein a microcatheter is inserted in the femoral artery and guided to the cerebral arteries and the peptide of the invention perfused into the area;
(iv) locally after craniotomy;
(v) by intracoronary delivery using catheter-based deliveries of synthesized peptide (or analogue) suspended in a suitable buffer (such as saline) which is injected locally (e.g., by injecting into the myocardium through the vessel wall) in the coronary artery using a suitable local delivery catheter such as a 10 mm InfusaSleeve catheter (Local Med, Palo Alto, Calif.) loaded over a 3.0 mm x 20 mm angioplasty balloon, delivered over a 0.014 inch angioplasty guide wire; or
(vi) by intracoronary bolus infusion of peptide (or derivative) wherein the peptide is manually injected, for example, through an Ultrafuse-X dual lumen catheter (SciMed, Minneapolis, Minn.) or another suitable device into proximal orifices of coronary arteries.
(vii) by intramyocardial delivery of synthesized peptide or analogue e.g., under direct vision following thoracotomy or using thoracoscope or via a catheter.

Pericardial delivery of synthesized peptide or analogue is typically accomplished by installation of the peptide-containing solution into the pericardial sac. The pericardium is accessed via a right atrial puncture, transthoracic puncture or via a direct surgical approach. Once the access is established, the peptide or analogue is infused into the pericardial cavity and the catheter is withdrawn. Alternatively, the delivery is accomplished via the aid of slow-release polymers such as heparinal-alginate or ethylene vinyl acetate (EVAc). In both cases, once the peptide or analogue is integrated into the polymer, the desired amount of peptide/polymer is inserted under the epicardial fat or secured to the myocardial surface using, for example, sutures. In addition, the peptide/polymer composition can be positioned along the adventitial surface of coronary vessels.

In the case of administration of a peptide by a route that does not directly access the central nervous system, the peptide may have to cross the blood brain barrier. Methods and means for enabling a peptide to cross the blood brain barrier are known in the art and/or described, for example, in USSN20050142141. For example, a peptide of the invention is conjugated to an agent that enables the peptide to cross the blood brain barrier (e.g., a Trojan horse). E.g., HIR MAb 83-14 is a murine MAb that binds to the human insulin receptor (HIR). This binding triggers transport across the BBB of MAb 83-14 (Pardridge et al, Pharm., Res. 12: 807-816, 1995), and any drug or gene payload attached to the MAb (Wu et al., J. Clin. Invest., 100: 1804-1812, 1997).

The use of molecular Trojan horses to ferry drugs or genes across the blood brain barrier is described in U.S. Pat. Nos. 4,801,575 and 6,372,250. The linking of drugs to MAb transport vectors is facilitated with use of avidin-biotin technology. In this approach, the drug or protein therapeutic is monobiotinylated and bound to a conjugate of the antibody vector and avidin or streptavidin. The use of avidin-biotin technology to facilitate linking of drugs to antibody-based transport vectors is described in U.S. Pat. No. 6,287,792. Fusion proteins have also been used where a drug is genetically fused to the MAb transport vector.

In a preferred embodiment, a therapeutic peptide described herein is administered to a subject when the subject is suffering from or has suffered from an ischemic event (e.g., a stroke). Such timing of administration is useful for, for example, reducing the effect of reperfusion following the ischemic event.

In another embodiment, a therapeutic peptide described herein is administered to a subject when the subject is at risk of experiencing a reperfusion injury following an ischemic event.

The present invention is further described with reference to the following examples.

### EXAMPLE 1

### The construction of a biodiverse nucleic acid fragment expression library in the vector pDEATH-Trp

Nucleic acid was isolated from the following bacterial species:

| | |
|---|---|
| *1* | *Archaeoglobus fulgidis* |
| *2* | *Aquifex aeliticus* |
| *3* | *Aeropyrum pernix* |
| *4* | *Bacillus subtilis* |
| *5* | *Bordetella pertussis TOX6* |
| *6* | *Borrelia burgdorferi* |
| *7* | *Chlamydia trachomatis* |
| *8* | *Escherichia coli Kl2* |
| *9* | *Haemophilus influenzae. (rd)* |
| *10* | *Helicobacter pylori* |
| *11* | *Methanobacterium thermoautotrophicum* |
| *12* | *Methanococcus jannaschii* |
| *13* | *Mycoplasma pneumoniae* |
| *14* | *Neisseria meningitidis* |
| *15* | *Pseudomonas aeruginosa* |
| *16* | *Pyrococcus horikoshii* |
| *17* | *S nechosistis PCC 6803* |
| *18* | *Thermoplasma volcanium* |
| *19* | *Thermotoga maritima* |

Nucleic acid fragments were generated from the genomic DNA of each genome using 2 consecutive rounds of primer extension amplification using tagged random oligonucleotides with the sequence:
5'-GACTACAAGGACGACGACGACAAGGCTTATCAATCAATCAN₆-3' (SEQ ID NO: 38). The PCR amplification was completed using the Klenow fragment of *E. coli* DNA polymerase I in the following primer extension reaction:

| Reagent | Volume |
|---|---|
| DNA (100-200ng) | |
| Oligonucleotide comprising SEQ ID NO: 38 (25µM) | 4µl |
| H₂O | to 17.4µl. |

Samples were then boiled for 3-5 minutes to denature the nucleic acid isolated from the bacteria, before being snap cooled, to allow the tagged random oligonucleotides to anneal to said nucleic acid. These samples were then added to the following reagents:

| | |
|---|---|
| Klenow buffer | 3µl |
| dNTP (2mM) | 3µl |
| Klenow | 0.6µl |
| Polyethylene Glycol (8,500) | 6µl |

Primer extension reactions were then incubated at 15°C for 30 minutes, then at room temperature for 2 hours, before being heated to 37°C for 15 minutes.

Samples were boiled for 5 minutes to again denature the nucleic acid, before being snap cooled to allow renaturation of said nucleic acid. Another 0.5µl of the Klenow fragment of *E*. *coli* DNA polymerase I was added to each reaction and the samples incubated at 15°C for 30 minutes, then at room temperature for 2 hours, before being heated to 37°C for 15 minutes.

Following boiling the samples, following snap cooling another 2 rounds of primer extension were completed using the tagged random oligonucleotide:
5'-GACTACAAGGACGACGACGACAAGGCTTATCAATCAATCAN₉-3' (SEQ ID NO: 39)

To complete this the following reagents were added to the samples of the previous step:

| | |
|---|---|
| Oligonucleotide comprising SEQ ID NO 39 (25µM) | 4µl |
| Klenow Buffer | 1µl |
| dNTP(2mM) | 3µl |
| Klenow | 0.5µl |
| H₂O | to 40µl |

Samples were then incubated at 15°C for 30 minutes, then at room temperature for 2 hours, before being heated to 37°C for 15 minutes.

Samples were boiled for 5 minutes to again denature the nucleic acid, before being snap cooled to allow renaturation of said nucleic acid. Another 0.5µl of the Klenow fragment of *E. coli* DNA polymerase I was added to each reaction and the samples incubated at 15°C for 30 minutes, then at room temperature for 2 hours, before being heated to 37°C for 15 minutes.

Following completion of the primer extension amplification all sample volumes were increased to 500µl with TE buffer and added to an Amicon spin column. These columns were then centrifuged for 15 minutes at 3,800 rpm in a microcentrifuge. Columns were then inverted and 30µl of TE buffer was added before the columns were centrifuged for 2 minutes at 3,800rpm, with this fraction collected for later use. The Klenow amplified DNA was then used in subsequent DNA manipulations.

The now purified primer extension products were then used in a PCR reaction with an oligonucleotide comprising the following sequence:
5'-GAGA**GAAT****T**CAGGTCAGACTACAAGGACGACGACGACAAG-3' (SEQ ID NO: 40), wherein an *Eco*RI restriction endonuclease site is shown in bold text, and three stop codons are underlined. Note that each of the stop codons is in a different reading frame.

Thus, the following PCR reaction was used:

| | |
|---|---|
| Oligonucleotide comprising SEQ ID NO: 40 (10µM) | 12µl |
| PCR buffer | 5µl |
| dNTP (2mM) | 5µl |
| Taq polymerase (Boehringer) 5.5U/µl) | 0.4µl |
| H₂O | 26.6µl |
| Klenow amplified DNA | 2µl |

Reactions were then cycled in a thermocycler using the following program:
95°C for 2 min; 60°C for 30sec; 72°C for 1 min;
95°C for 20 sec; 60°C for 30sec; 72°C for 1 min (repeated 29 times); and
72°C for 5 min.

PCR products were then purified using Amicon spins columns which fractionate on the basis of size.

The PCR products were then analyzed by electrophoresis on standard TAE-agarose gels to determine the approximate size of the nucleic acid fragments generated as shown in Figure 2. The nucleic acid concentration of the samples was also determined.

PCR products from each of the 19 bacterial species were then pooled to generate a biodiverse nucleic acid library. To do so, DNA from each organism was added in an equimolar amount when compared to the amount of nucleic acid added to the pool from the organism with the smallest genome. Between 1µg and 10µg of DNA from each organism was used, depending on the genome size of the organism from which the DNA was obtained.

In order to allow efficient cloning of the nucleic acid fragments into the pDEATH-Trp vector (SEQ ID NO: 41; Figure 3), both the fragments and the vector were digested with the EcoRI restriction endonuclease. Restriction digests were completed in the following reactions:
Digestion of PCR products used the following reaction conditions:

| | |
|---|---|
| PCR products (1µg) | |
| EcoR I Buffer (Promega) | 17µl |
| BSA (10x) | 17µl |
| EcoR I enzyme (20U/µL) (Promega) | 0.9ul |
| H₂O | to 170 µl |

Restriction digests were allowed to proceed for 40 minutes at 37°C. Samples were then purified using QIAquick PCR purification columns as per manufacturer's instructions.
Nucleic acid was eluted into 50µl of H₂O.

Digestion of pDEATH-Trp vector used the following reaction conditions:

| | |
|---|---|
| pDEATH-Trp (25µg) | |
| EcoR I Buffer (Promega) | 100µl |
| BSA (10X) | 100µl |
| EcoR I enzyme (20U/µL) | 4µl |
| H₂O | to 1000µl |

Restriction digests were allowed to proceed for 5 minutes at 37°C. Samples were then purified using 3 QIAquick PCR purification columns as per manufacturer's instructions. Nucleic acid was eluted into 150µl of H₂O.

The fragments generated from the PCR products were then ligated into the pDEATH-Trp vector (SEQ ID NO 41) using the following reaction:

| | |
|---|---|
| pDEATH-Trp (2 µg) | |
| BGF-PCR Fragments (1µg) | |
| Ligation Buffer (10x) (NEB) | 20µl |
| T4 DNA Ligase (NEB) | 10µl |
| H₂O. | to 200µl |

Ligation reactions were allowed to proceed overnight at 16°C. The ligase was then heat inactivated by incubating the samples at 65°C for 30 minutes. Following completion of the ligation reaction sample volumes were increased to 500µl with TE buffer and added to an Amicon spin column. These columns were then centrifuged for 15 minutes at 3,800 rpm in a microcentrifuge. Columns were then inverted and 30µl of TE buffer was added before the columns were centrifuged for 2 minutes at 3,800rpm, with this fraction collected for later use.

The pDEATH-Trp vector containing the biodiverse nucleic acid fragment was then transformed into *E. coli* TOP10 cells. Expression vectors were then isolated from bacteria using standard procedures. Restriction enzyme digestion of the isolated vectors using EcoRI was then used to characterise the size of the inserts contained in the library, as shown in Figure 4.

Vectors were then pooled and transformed into the yeast strain PRT 51. Yeast strain PRT-51 is characterized by the following genotype: *MATα his3, trp1, ura3, 6 LexA-LEU2, lys2:3 cIop-LYS2, CYH2^{R}, ade2:G418-pZero-ade2, met15:Zeo-pBLUE-met15, his5: : hygro.*

The result of this transformation was a library of 61 million clones. The recombinant clones each express a peptide that is fused to another polynucleotide sequence encoding the FLAG epitope or other marker.

### EXAMPLE 2

### Characterization of a biodiverse nucleic acid fragment expression library in the pDEATH-Trp vector

Sequence analysis of nucleic acids cloned into pDEATH-Trp vector show that the fragments are derived from a variety of organisms, and encode a variety of proteins, as shown in Table 2.

**TABLE 2.**

| Characterization of nucleic acid fragment cloned into pDEATH-Trp | | | | |
|---|---|---|---|---|
| No. | Insert size (bp) | Organism | Genbank ID | Function |
| 1 | 114 | *P. aeruginosa* | AAG05339.1 | Hypothetical Protein |
| 2 | 143 | *Synechocystis PCC6803* | BAA10184.1 | Fructose |
| 3 | 166 | *E. coli* | AAC73742.1 | Lipoprotein |
| 4 | 180 | *B. subtilis* | CAB12555.1 | methyl-accepting chemotaxis protein |
| 5 | 150 | *N. meningitis* | AAF41991.1 | N utilization substance protein A |
| 6 | 240 | *E. coli* | AAC75637.1 | Hypothetical protein |
| 7 | 357 | *H. pylori* | AAD08555.1 | transcription termination factor NusA |
| 8 | 83 | *Z. maritima* | AAD36283.1 | Hypothetical protein |

### EXAMPLE 3

### The construction of a biodiverse nucleic acid fragment expression library in the vector T7Select415-1

Nucleic acid was isolated from the following bacterial species:

| | |
|---|---|
| *1* | *Archaeoglobus fulgidis* |
| *2* | *Aquifex aeliticus* |
| *3* | *Aeropyrum pernix* |
| *4* | *Bacillus subtilis* |
| *5* | *Bordetella pertussis TOX6* |
| *6* | *Borrelia burgdorferi* |
| *7* | *Chlamydia trachomatis* |
| *8* | *Escherichia coli Kl2* |
| *9* | *Haemophilus influenzae (rd)* |
| *10* | *Helicobacter pylori* |
| *11* | *Methanobacterium thermoautotrophicum* |
| *12* | *Methanococcus jannaschii* |
| *13* | *Mycoplasma pneumoniae* |
| *14* | *Neisseria meningitidis* |
| *15* | *Pseudomonas aeruginosa* |
| *16* | *Pyrococcus horikoshii* |
| *17* | *Synechosistis PCC 6803* |
| *18* | *Thermoplasma volcanium* |
| *19* | *Thermotoga maritima* |

Nucleic acid fragments were generated from each of these genomes using multiple consecutive rounds of Klenow primer extension using tagged random oligonucleotides.

In the final round of PCR, the sequence of the oligonucleotide primer comprised the sequence:
5'-AGAGGAATTCAGGTCAGACTACAAGGACGACGACGACAAG-3' (SEQ ID NO: 42).

The primer extension products generated were then used as a template for PCR reactions using the following oligonucleotides:
5'-CAG**AAGCTT**AAGGACGACGACGACAAG-3' (SEQ ID NO: 43);
5'-CAG**GAATTC**AAGGACGACGACGACAAG-3' (SEQ ID NO: 44);
5'-CAG**GAATTC***C*AAGGACGACGACGACAAG-3' (SEQ ID NO: 45); and
5'-CAG**GAATTC***A*CAAGGACGACGACGACAAG-3' (SEQ ID NO: 46),
wherein the underlined sequence in SEQ ID Nos: 42-46 permits amplification of the PCR products. Furthermore, the sequence shown in bold highlights a *Hin*dIII restriction endonuclease recognition site or *Eco*RI recognition site. Furthermore, note the addition of one or two nucleotides after the *Eco*RI restriction site in SEQ ID Nos: 45 and 46, respectively (shown in italics). These nucleotides allow expression of amplified nucleic acid in multiple forward reading frames.

Each DNA template was amplified by "one armed" (ie. using only 1 oligonucleotide primer) PCR, with each of the oligonucleotides (ie., SEQ ID Nos: 43-46) in separate reactions (ie. 76 reactions).

Each PCR reaction contained:

| | |
|---|---|
| Template DNA | 1 µl |
| Taq buffer (10x) (Promega) | 5µl |
| MgCl₂ (25mM) | 4µl |
| dNTP (2mM) | 5µl |
| a primer selected from the group consisting of SEQ ID Nos: 43-46 (10pmol/µl) | 10µl |
| Taq DNA polymerase (Promega 5U/µl) | 0.4µl |
| H₂O | to 50µl |

Reactions were then cycled in a Perkin Elmer thermocycler PE 9700 or PE 2400 using the following program:
5 min at 94°C, followed by 30 cycles wherein each cycle consists of 30 sec at 94°C, followed by 30 sec at 55°C, and followed by 1 min at 72°C], followed by 5 min at 72°C.

A sample of the resulting PCR products was analyzed by electrophoresis using a 2% agarose/TAE gel. The amount of nucleic acid in each of the PCR products was also determined using the picogreen method following instructions provided by the manufacturer.

PCR products generated with each of the oligonucleotides SEQ ID Nos: 43-46 were pooled. DNA from each organism was added in an equimolar amount when compared to the amount of nucleic acid added to the pool from the organism with the smallest genome.

Subsequently, the pools generated from PCR products amplified using the oligonucleotides SEQ ID NO: 44, SEQ ID NO: 45 or SEQ ID NO: 46 were combined in equal ratios (ie. equal amounts of nucleic acid) to form one pool.

The pooled PCR products were then purified using QIAquick PCR purification columns (QIAGEN) as per manufacturer's instructions. This step removes any unincorporated oligonucleotides, dNTPs and contaminating proteins.

Each of the pools of PCR products (6µg) was then divided into 3 equal parts and each part digested with a different one of the restriction enzymes AluI, HaeIII or RsaI (NEB) in the following reaction:

| | |
|---|---|
| PCR product (2µg) | |
| Restriction endonuclease buffer (10x) (NEB) | 4µl |
| Restriction endonuclease | 1µl |
| H₂O | to 40µl |

Reactions were allowed to proceed for 2 hours at 37°C, before being heat inactivated by incubating at 65°C for 20 minutes. Restriction digests were then re-pooled and purified using QIAquick PCR purification columns (QIAGEN) as per manufacturer's instructions.

Each of the enzymes *Alu*I, *Hae*III and *Rsa*I produce blunt ends. Accordingly, it is possible to ligate blunt end adaptors to the restriction digested PCR products to allow directional cloning into the T7Select415-1 vector. Oligonucleotides encoding the blunt-end adaptors were generated comprising the following sequences:
5'-AATTCGAACCCCTTCG-3' (SEQ ID NO: 47)
5'-CGAAGGGGTTCG-3' (SEQ ID NO: 48)
5'-AATTCGAACCCCTTCGC-3' (SEQ ID NO: 49)
5'-GCGAAGGGGTTCG-3' (SEQ ID NO: 50)
5'-AATTCGAACCCCTTCGCG-3' (SEQ ID NO: 51)
5'- CGCGAAGGGGTTCG-3' (SEQ ID NO: 52)
5'-AGCTCGAAGGGGTTCG-3' (SEQ ID NO: 53)
5'-CGAACCCCTTCG-3' (SEQ ID NO: 54).

The adaptor pairs SEQ ID Nos: 47 and 48; SEQ ID Nos: 49 and 50; SEQ ID NOs: 51 and 52; SEQ ID NOs: 53 and 54 were then annealed to one another. This process was completed in H₂O with each of the oligonucleotides at a concentration of 50uM. Pairs of adaptors were incubated at 94°C for 10 minutes and then allowed to cool to room temperature slowly.

The annealed adaptors were then ligated to the pool of amplified PCR products in separate ligation reactions. The adaptor formed through annealing of SEQ ID NOs: 53 and 54 was ligated to the pool of PCR products amplified using the oligonucleotides set forth in SEQ ID NO: 44, SEQ ID NO: 45 and SEQ ID NO: 46.

Ligations were carried out in the following reactions:

| | |
|---|---|
| Pooled PCR product (average length of 200bp) | 2 pmol |
| Annealed adaptor | 150 pmol |
| Ligation buffer (10x) (Promega) | 1µl |
| T4 DNA ligase (3U/µl) (Promega) | 1µl |
| H₂O | to 10µl |

Samples were then incubated at 4°C overnight before being heat inactivated through incubation at 65°C for 20 minutes.

Samples were then phosphorylated using T4 polynucleotide kinase (Promega) in the following reaction:

| | |
|---|---|
| Ligation buffer (10x) (Promega) | 1µl |
| rATP (10mM) | 2µl |
| T4 polynucleotide kinase (5U/µl) | 1µl |
| H₂O | 20µl |

Samples were incubated at 37°C for 30 minutes followed by incubation at 65°C for 20 minutes to heat inactivate the T4 polynucleotide kinase.

Following ligation and phosphorylation each of the three reactions comprising nucleic acid amplified using the oligonucleotide SEQ ID NO: 43 were combined in equal ratios, ie. equal amounts of nucleic acid to form one pool.

The nucleic acids originally amplified with SEQ ID NO: 43 were then digested with the restriction endonuclease *Hin*dIII in the following reaction:

| | |
|---|---|
| PCR product (2 µg) | |
| HindIII buffer (10x) (Promega) | 8µl |
| HindIII (10U/µl) (Promega) | 1µl |
| H₂O | to 80µl |

The nucleic acids in the pool originally amplified by one of SEQ ID Nos: 44-46 were digested with the restriction endonuclease *Eco*RI in the following reaction:

| | |
|---|---|
| PCR product (2 µg) | |
| EcoRI buffer (10x) (Promega) | 8µl |
| EcoRI (10U/µl) (Promega) | 1µl |
| H₂O | to 80µl |

Samples were then purified using a QIAquick PCR purification column (QIAGEN) as per manufacturer's instructions. Nucleic acid concentration was then determined by spectrophotometry measuring UV absorption at 260nm.

Both pools of nucleic acid fragments (ie. those digested with EcoRI and those digested with HindIII) were then combined in equal ratios, ie. equal amounts of nucleic acid, to form one pool. This pool of nucleic acid fragments was then suitable for cloning into the peptide display vector T7Select415-1 (Novagen). The T7415-1 vector is provided in a form for nucleic acids to be ligated into EcoRI and HindIII. restriction endonuclease sites.

The nucleic acid fragments were then ligated into the T7Select415-1 vector using the following reaction:

| | |
|---|---|
| Ligation buffer (lOx) (Novagen) | 0.5µl |
| rATP (10mM) | 0.5µl |
| DTT (10mM) | 0.5µl |
| T7Select415-1 EcoRI/HindIII vector arms (0.02pmol) | 1µl |
| Nucleic acid fragments (0; 0.02; and 0.06 pmol in independent reactions) | |
| H₂O | to 5µl |

Reactions were incubated at 16°C overnight.

### EXAMPLE 5

### Packaging and amplification of a biodiverse nucleic acid fragment expression library

The ligation reactions of Example 4 were packaged using commercial packaging extract available from Novagen. These reactions were then titered according to manufacturer's instructions by infection of *E. coli* BL21 cells. By using 1µl from each of three independent ligations, titers between 1.3 x 10⁷ and 7 x 10⁷ plaque forming units (pfu)/ml were obtained.

Pooling of three ligation reactions containing a total of 1 µg of T7Select415-1 vector, and packaging, resulted in a library with 2.75 x 10⁷ pfu, ie 2.75 x 10⁷ initial recombination events. The library was immediately amplified by "plate lysate amplification" (as per manufacturer's instructions) on 180 LB Petri dishes (14 cm diameter). Titers of the amplified lysates varied between 1 and 5 x 10¹⁰ pfu/ml. Two liters of lysate were harvested, pooled and the titer determined at 1.5 x 10¹⁰ pfu/ml, ie 3 x 10¹³ pfu in total. The lysate was stored at 4°C over CHCl₃ (as per manufacturer's instructions) and glycerol stocks containing 10% glycerol were stored at -80°C.

### EXAMPLE 6

### Characterization of a T7-displayed biodiverse nucleic acid fragment library

During the amplification of the library described in Example 5, individual plaques from low-density plates were collected and analyzed by PCR with primers specific to T7Select415-1 of the nucleotide sequence.

Thirty nine plaques with insert sizes larger than 70bp were analyzed by DNA sequence analysis. The resulting sequences are summarised in the Table 3

DNA from 13 of the 19 bacterial genomes could be identified in the recombinant phage analyzed,. In most cases, the homology was between 96 and 100% in the regions that were derived from the genomic starting material. In addition, primers and adapters were identified, however, there were also many cases of strings of adapters and multiple PCR primers in the insert regions. The inserted DNA of the analyzed phage clones was up to 250bp long.

**TABLE 3**

| Characterization of nucleic acid fragments in T7Select-415-1 | | | | | |
|---|---|---|---|---|---|
| BGF clone | T7for/ rev PCR fragment (bp) | Insert homology to organism (% homology in the matching region) | Size of homologous region (bp) | Extra amino Acids after Asn (T7) | Natural reading frame |
| SP8 | 255 | *B. pertussis (98%)* | 112 | 16 | |
| SP14 | 212 | *M. thermoautotrophicum (98%)* | 73 | 12 | |
| SP15 | 350 | *B. pertussis (98%)* | 171 | 0 | |
| SP16 | 263 | *A. fulgidus (100%)* | 125 | 20 | |
| SP18 | 260 | *A. fulgidus (100%)* | 112 | 0 | |
| SP31 | 260 | *A. fulgidus (96%)* | 118 | 65 | yes |
| SP52 | 240 | *T. volcanicum (100%)* | 39 | 0 | |
| SP61 | 272 | *M. jannashii (100%)* | 90 | 12 | |
| SP65 | 230 | *N. meningiditis (100%)* | 107 | 0 | |
| SP73 | 230 | *C. trachomatis (98%)* | 62 | 10 | |
| SP83 | 200 | *B. burgdorferi (100%)* | 46 | 8 | |
| SP89 | 411 | *B. subtilis(98%)* | 170 | 15 | |
| SP100 | 268 | *P. aeruginosa* | 159 | 11 | |
| SP104 | 174 | no match | - | 12 | |
| SP125 | 250 | *E. coli K12 (98%)* | 109 | 4 | |
| SP126 | 220 | E. *coli K12* | 91 | 6 | |
| SP139 | 240 | *Synechocystis PCC 6803* (100%) | 109 | 26 | yes |
| SP141 | 250 | *E. coli Kl2* | 126 | 6 | |
| SP144 | 170 | no match | - | 15 | |
| SP152 | 160 | *E. coli K12 (100%)* | 39 | 13 | |
| SP153 | 290 | C. *trachomatis (100%)* | 131 | 7 | |
| SP163 | 260 | *C. trachomatis (100%)* | 90 | 5 | |
| SP166 | 270 | E. *coli Kl2 (100%)* | 112 | 20 | |
| SP169 | 240 | *M thermoautotrophicum (100%)* | 112 | 6 | |
| SP10 | 180 | no match | - | 7 | |
| SP17 | 190 | *M. jannashii* | 68 | 13 | |
| SP20 | 190 | *E. coli K12* | 58 | 22 | |
| SP25 | 170 | *P. horikoshii* | 40 | 10 | |
| SP30 | 200 | *P. aeruginosa* | 54 | 13 | |
| SP40 | 190 | no match | - | 24 | |
| 42 | 190 | *B. sublilis* | 44 | 0 | |
| SP44 | 250 | *B. burgdorferi* | 130 | 6 | |
| SP47 | 210 | *C. trachomatis* | 95 | 13 | |
| SP48 | 200 | *Synechocystis PCC 6803* | 82 | 20 | |
| SP55 | 180 | no match | - | 11 | |
| SP64 | 190 | *Synechocystis PCC 6803* | 46 | 16 | |
| SP82 | 180 | *M thermoautotrophicum* | 39 | 8 | |
| SP87 | 250 | No match | - | 51 | |
| SP134 | 280 | *M thermoautotrophicum* | | | |

### EXAMPLE 7

### Production and screening of a biodiverse nucleic acid fragment library from Takifigu rubripes

Nucleic acid fragments are generated from genomic DNA from the Japanese puffer fish *T. rubripes* using a restriction enzyme digestion with the enzymes AluI and HaeIII, in the following reaction:

| | |
|---|---|
| Genomic DNA (20µg) | |
| Restriction enzyme buffer (10x) | 5µl |
| AluI(10U/µg) | 4µl |
| HaeIII(10U/µg) | 4µl |
| H₂O | to50µl |

The DNA fragments are then separated by electrophoresis using a 2% agarose/TAE gel. Fragments in the 90-120bp range are isolated using the QIAquick Gel Extraction Kit (QIAGEN) following manufacturer's instructions.

The concentration of DNA is determined using spectrophotometry at 260nm.

The adaptor pairs SEQ ID Nos: 47 and 48; SEQ ID Nos: 49 and 50; SEQ ID NOs: 51 and 52; SEQ ID NOs: 53 and 54 are then annealed to one another. This process is completed in H₂O with each of the oligonucleotides at a concentration of 50µM. Pairs of adaptors are incubated at 94°C for 10 minutes and then allowed to cool to room temperature slowly.

The annealed adaptors are then ligated to the isolated nucleic acid fragments in separate ligation reactions.

Ligations are carried out in the following reactions:

| | |
|---|---|
| Pooled genomic DNA fragments (ave. fragment length 100bp) | 2 pmol |
| Annealed adaptor | 150 pmol |
| Ligation buffer (10x) (Promega) | 1µl |
| T4 DNA ligase (3U/µl) (Promega) | 1µl |
| H₂O | to 10µl |

Samples are then incubated at 4°C overnight before being heat-inactivated through incubation at 65°C for 20 minutes.

Samples are phosphorylated using T4 polynucleotide kinase (Promega) in the following reaction:

| | |
|---|---|
| Ligation buffer (10x) (Promega) | 1µl |
| rATP (10mM) | 2µl |
| T4 polynucleotide kinase (5U/µl) | 1µl |
| H₂O | 20µl |

Samples are incubated at 37°C for 30 minutes followed by incubation at 65°C for 20 minutes to heat inactivate the enzyme.

Nucleic acid fragments from each of the ligation reactions are then combined in equal ratios, ie. equal amounts of nucleic acid, to form one pool. This pool of nucleic acid fragments is then suitable for cloning into the peptide display vector T7Select415-1 (Novagen). However, it is first necessary to digest the T7Select415-1 vector with EcoRI in the following reaction:

| | |
|---|---|
| T7Select415-1 vector(1µg) | |
| EcoRI buffer (10x) (Promega) | 3µl |
| BSA (10x) | 3µl |
| EcoRI (20U/µl) (Promega) | 2µl |
| H₂O | to 30µl |

Reactions proceed at 37°C for 2 hours, before enzymes are heat inactivated by incubating the reactions at 65°C for 20 minutes. Samples are then purified using a QIAquick PCR purification column using manufacturer's instructions. Nucleic acid concentration are then determined by spectrophotometry measuring UV absorption at 260nm, before diluting the DNA to a final concentration of 0.02µM.

The nucleic acid fragments are then ligated into the T7Select415-1 vector using the following reaction:

| | |
|---|---|
| Ligation buffer (10x) (Novagen) | 0.5µl |
| rATP (10mM) | 0.5µl |
| DTT (1.0mM) | 0.5µl |
| T7Select415-1 (0.02pmol) | 1µl |
| Nucleic acid fragments (0; 0.02; and 0.06 pmol in independent reactions) | |
| H₂O | to 5µl |

Reactions are incubated at 16°C overnight. Samples are then purified using a QIAquick PCR purification column (QIAGEN), before being diluted in 1 ml of phosphate buffered saline.

The library generated from *T*. *rubripes* is then screened for mimotopes of epitopes of the D 15 protein. The D 15 protein is a 80 kDa outer membrane protein of *Haemophil us influenzae,* which are shown to elicit an immune response in rabbits. The antibodies isolated from these rabbits, in turn, are shown to confer resistance to *H. influenzae* to infant rats. Affinity-purified antibodies isolated from rabbits have also been shown to be protective in screens using infant rats *(*Thomas et al, Infect Immunol, 58(6), 1909-1915, 1990).

In an attempt to identify mimotopes of epitopes of the D 15 protein, the phage displayed library generated from *T*. *rubripes,* is screened for those peptides that have a conformation sufficient for binding the affinity purified antibody described in Thomas *et al* (1990).

The phage display library is added to the affinity purified antibody, which is linked to an antibody coated goat anti-rabbit coupled magnetic beads. These beads are generated by incubating 10 µg of the antibody with 5 mg Dynal beads and incubating at 25°C for 1 hour, followed by 6 washes with HEG buffer (35mM HEPES-KOH, pH 7.5/0.1mM EDTA/100mM sodium glutamate).

Phage are incubated with these beads at 0°C for 1 hour, before being washing three times with 5 ml cold HEG buffer/0.1% BSA. Beads are then washed a further three times with HEG buffer using a magnet, such as a tesla magnet (Miltenyi Biotec, Bergish Gladbach, Germany) to immobilise the beads. Bound phage are then eluted with 0.5 ml of 1% SDS. Phage isolated by this method are re-screened, or, alternatively, the nucleic acid fragments encoding the binding peptide are isolated from the phage and analyzed. For example, the amino acid sequences of the peptides are determined.

### EXAMPLE 8

Construction of a biodiverse nucleic acid fragment for ribosome display Nucleic acid is isolated from the following bacterial species:

| | |
|---|---|
| *1* | *Archaeoglobus fulgidis* |
| 2 | *Aquifex aeliticus* |
| 3 | *Aeropyrum pernix* |
| 4 | *Bacillus subtilis* |
| 5 | *Bordetella pertussis TOX6* |
| 6 | *Borrelia burgdorferi* |
| 7 | *Chlamydia trachomatis* |
| 8 | *Escherichia coli K12* |
| *9* | *Haemophilus influenzae (rd)* |
| *10* | *Helicobacter pylori* |
| *11* | *Methanobacterium thermoautotrophicum* |
| 12 | *Methanococcus jannaschii* |
| 13 | *Mycoplasma pneumoniae* |
| 14 | *Neisseria meningitidis* |
| *15* | *Pseudomonas aeruginosa* |
| 16 | *Pyrococcus horikoshii* |
| *17* | *Synechosistis PCC 6803* |
| *18* | *Thermoplasma volcanium* |
| 19 | *Thermotoga maritima* |

Nucleic acid fragments are generated from each of these genomes using 4 consecutive rounds of PCR using tagged random oligonucleotides with the sequence: This oligonucleotide introduces a ribosome binding site.

In order to complete this the following reagents are added to the samples:

| | |
|---|---|
| Genomic DNA (100-200ng) | |
| Oligonucleotide comprising SEQ ID NO: 55 (25µM) | 4µl |
| Klenow Buffer | 1µl |
| dNTP(2mM) | 3µl |
| Klenow | 0.5µl |
| H₂O | to 40µl |

Samples are incubated at 15°C for 30 minutes, then at room temperature for 2 hours, before being heated to 37°C for 15 minutes.

Samples are boiled for 5 minutes to again denature the nucleic acid in said sample, before being snap cooled to allow renaturation of said nucleic acid. Another 0.5µl of the Klenow fragment of E. *coli* DNA polymerase I is added to each reaction, and the samples incubated at 15°C for 30 minutes, then at room temperature for 2 hours, before being heated to 37°C for 15 minutes.

The PCR products generated are then used as a template for PCR reactions using the following oligonucleotide:

This oligonucleotide comprises a T7 promoter and a region that is homologous a region of to SEQ ID NO: 53).

Each DNA template is amplified by "one armed" PCR, with the oligonucleotide SEQ ID NO: 54 in separate reactions (ie. 19 reactions). Each PCR reaction contains the following:

| | |
|---|---|
| Template DNA | 1µl |
| Taq buffer (10x) (Promega) | 5µl |
| MgCl₂ (25mM) | 4µl |
| dNTP (2mM) | 5µl. |
| Oligonucleotide comprising SEQ ID NO: 56 (10pmol/µl) | 10µl |
| Taq DNA polymerase (Promega 5U/µl) | 0.4µl |
| H₂O | to 50µl |

Reactions are then cycled in a Perkin Elmer thermocycler PE 9700 or PE 2400 using the following program:
5 min 94°C + 30x [30sec 94°C, 30 sec. 55°C, 1 min 72°C] + 5 min 72°C.

The resulting PCR products are electrophoresed using a 2% agarose/TAE gel, and the nucleic acid fragments between 50bp to 250bp extracted using a QIAquick gel extraction kit (QIAGEN) using manufacturer's instructions. Nucleic acid concentration is determined by spectrophotometry measuring UV absorption at 260nm.

Pools of PCR products derived from each of the 19 bacterial species are produced. To do so, DNA from each organism is added in an equimolar amount when compared to the amount of nucleic acid added to the pool from the organism with the smallest genome.

Nucleic acid fragments are then blunt ended using Mung Bean Nuclease (NEB) in the following reaction:

| | |
|---|---|
| Nucleic acid fragments (2µg) | |
| Mung bean nuclease buffer (10x) | 3µl |
| Mung bean nuclease (10U/µl)(NEB) | 2µl |
| H₂O | to 30µl |

The reaction proceeds at 30°C for 1 hour. The sample is then purified using a QIAquick PCR purification column (QIAGEN) as per manufacturer's instructions.

Oligonucleotides encoding a blunt-end adaptor are generated comprising the following sequences:
5'-TTTAAGCAGCTCGATAGCAGCAC-3' (SEQ ID NO: 57); and
5'-GTGCTGCTATCGAGCTGCTTAAA-3' (SEQ ID NO: 58).

The adaptors are annealed to one another. This process is completed in H₂O with each of the oligonucleotides at a concentration of 50µM. Pairs of adaptors are incubated at 94°C for 10 minutes and then allowed to cool to room temperature slowly. Annealed adaptors are ligated to the nucleic acid fragments in the following reactions:

| | |
|---|---|
| Pooled PCR product (average length of 150bp) | 2 pmol |
| Annealed adaptor | 150 pmol |
| Ligation buffer (10x) (Promega) | 1µl |
| T4 DNA ligase (3U/µl) (Promega) | 1 µl |
| H₂O | to 10µl |

Samples are then incubated at 4°C overnight before being heat inactivated through incubation at 65°C for 20 minutes. The ligation reaction is then purified using a QIAquick PCR purification kit (QIAGEN)

The modified nucleic acid fragments are then amplified in a PCR reaction with oligonucleotides of the sequence SEQ ID NO: 56 and the following sequence:
5'AGACCCGTTTAGAGGCCCCAAGGGGTTATGGAATTCACCTTTAAGCAGCT C-3' (SEQ ID NO: 59). The oligonucleotide of SEQ ID NO: 59 introduces a modified lipoprotein terminator with the stop codon removed.

The PCR reactions are completed in the following reaction:

| | |
|---|---|
| Template DNA | 1µl |
| pfu buffer (10x) (Promega) | 5µl |
| MgCl₂ (25mM) | 4µl |
| dNTP (2mM) | 5µl |
| oligonucleotide SEQ ID NO: 54 (10pmol/µl) | 10µl |
| oligonucleotide SEQ ID NO: 57 (120pmol/µl) | 10µl |
| pfu DNA polymerase (Promega 5U/µl) | 0.4µl |
| H₂O | to 50µl |

The PCR reactions are completed with the following cycling conditions:
5 min 94°C + 30x [30sec 94°C, 30 sec. 55°C, 1 min 72°C] + 5 min 72°C

PCR products are then purified using a QIAquick PCR purification column (QIAGEN).

In a separate reaction the amino acids 211-299 of gene III of filamentous phage M13 are amplified using the following oligonucleotides:
5'-CGTGAAAAAATTATTATTCGCAATTC-3' (SEQ ID NO: 60)
5'-TTAAGACTCCTTATTACGCAGTATGTTAGC-3' (SEQ ID NO: 61)
The oligonucleotide SEQ ID NO: 60 is phosphorylated using T4 polynucleotide kinase (Promega), to allow for later directional cloning of the PCR product. The phosphorylation proceeds in the following reaction:

| | |
|---|---|
| Oligonucleotide (SEQ ID NO: 60) | |
| Ligation buffer (10x) (Promega) | 1µl |
| rATP (10mM) | 2µl |
| T4 polynucleotide kinase (5U/µl) | 1µl |
| H₂O | 20µl |

Samples are incubated at 37°C for 30 minutes followed by incubation at 65°C for 20 minutes to heat inactivate the T4 polynucleotide kinase.

The oligonucleotides are then used in the following PCR reaction:

| | |
|---|---|
| Template DNA | 1µl |
| pfu buffer (10x) (Promega) | 5µl |
| MgCl₂ (25mM) | 4µl |
| dNTP (2mM) | 5µl |
| oligonucleotide SEQ ID NO: 60 (10pmol/µl) | 10µl |
| oligonucleotide SEQ ID NO: 61 (10pmol/µl) | 10µl |
| pfu DNA polymerase (Promega 5U/µl) | 0.4µl |
| H₂O | to 50µl |

Reactions are then cycled in a Perkin Elmer thermocycler PE 9700 or PE 2400 using the following program:
5 min 94°C + 30x [30sec 94°C, 30 sec. 59°C, 1 min 72°C] + 5 min 72°C

Reactions are electrophoresed in a 2% TAE/agarose gel and the 1276bp fragment isolated using a QIAquick gel purification kit (QIAGEN).

The modified nucleic acid fragments and the spacer sequence isolated from M13 phage are then ligated in the following reaction:

| | |
|---|---|
| Modified nucleic acid fragment (2µg) | |
| Spacer (2µg) | |
| Ligation buffer (10x) (Promega) | 2µl |
| T4 DNA ligase (3U/µl) (Promega) | 1µl |
| H₂O | to 20µl |

Samples are then incubated at 4°C overnight before being heat inactivated through incubation at 65°C for 20 minutes. The ligation reaction is then purified using a QIAquick PCR purification kit (Qiagen)

The resulting gene constructs are transcribed and translated *in vitro* using the Promega *E. coli* S 30 Extract system for linear templates as per manufacturer's instructions, which are a modification of the protocol of Leslie et al, J Biol. Chem. 266, 2632 - 1991.

The translation reaction is stopped by adding magnesium acetate [Mg(OAc)₂] to a final concentration of 50mM, chloroamphenicol to a final concentration of 50µM and cooling the samples on ice. The samples are then diluted 8 fold with ice-cold wash buffer (50mM Tris-HOAc, pH7.5/150mM NaCl/50mM Mg(Oac)₂/0.1% Tween 20) and centrifuged for 5 minutes at 4°C at 100,000g to remove any insoluble components.

The *in vitro* displayed library is then screened to isolate peptides that bind to α-FLAG monoclonal antibody. The monoclonal antibody is first adsorbed to a microtiter plate. Each well of a microtiter plate is rinsed twice with distilled water. The α-FLAG monoclonal antibody (α-FLAG M2, Sigma Aldrich) is diluted in TBS buffer to 20 µg/ml and 100µl added per well. The antibody is allowed to adsorb at 4°C overnight. The microtiter plate is then rinsed three times with TBS buffer and filled with 5% skim milk in distilled water. For blocking the skim milk solution is allowed to bind with gentle rocking for 1 hour at room temperature. The dish is then rinsed five times with double distilled water (ddH₂O) and filled with ddH₂O until use.

Prior to use, each well of the microtiter plate is washed with ice-cold wash buffer, and the supernatant from the centrifuged translation mixture applied (200µl per well). The plate is then gently rocked for 1 hour at room temperature. Each well of the microtiter plate is then washed with ice-cold wash buffer five times, and the bound ribosome displayed peptides eluted using ice cold elution buffer (50mM Tris-HOAc, pH7.5/150mM NaCl/10mM EDTA/50µg/ml E. *coli* tRNA). Elution buffer (100µl) is added per well, and the plates gently rocked for 10 minutes at 4°C. The released mRNA is recovered using the RNeasy kit (QIAGEN) using manufacturer's instructions.

Recovered mRNAs are then reverse transcribed using Superscript reverse transcriptase (Invitrogen) according to manufacturer's instructions. The positive nucleic acid fragments are then amplified using PCR with the oligonucleotides (very first ones without random bases). PCR products are electrophoresed in a 2% TAE/agarose gel and the PCR products recovered using QIAquick gel extraction kit. Recovered nucleic acids are then sequenced using a Big Dye Terminator system (Perkin Elmer).

### EXAMPLE 9

### Identification of a peptide capable of inhibiting the dimerization of c-Jun.

A biodiverse nucleic acid fragment library was produced in the vector pMF4-5 (Phylogica Ltd, Australia) (SEQ ID NO: 62) essentially as described in Example 1. Amplified fragments were digested with *EcoR*I and *Acc*651. The resulting fragments were then purified using a QIAQuick PCR purification column (Qiagen) essentially according to manufacturer's instructions. The expression vector pMF4-5 was also digested with *EcoR*I and *Acc*651, treated with shrimp alkaline phosphatase and then purified using a QIAQuick PCR purification column (Qiagen) essentially according to manufacturer's instructions. Ligations were then performed at a molar ratio of 10:1 insert:vector, and transformed into TOP10 electrocompetent cells (Invitrogen).

These vectors were then isolated from bacteria using standard methods and transformed into the PRT51 yeast strain (with the genotype MATα, his3, trp1, ura3, 6 LexA-LEU2, lys2::3 cIop-LYS2, CYH2R, ade2::G418-pZero-ade2, met15::Zeo-pBLUE-met15, his5::hygroR). Transformants were then aliquoted and snap frozen in 15% glycerol.

The bait and prey used in the present screen were JUN1 and JUNZ (these regions of c-Jun are shown in Figure 8). Briefly, nucleic acid encoding the JUN1 protein was cloned into the prey vector pJFK (SEQ ID NO: 63; Figure 5) in operable connection with a nuclear localisation signal, and a B42 activation domain. The nucleic acid encoding the JUNZ protein was cloned into the bait vector pDD (SEQ ID NO: 64; Figure 6) in operable connection with the LexA DNA binding domain. The pDD vector also contains a nucleic acid encoding the HIS3 gene (Figure 6). These vectors were then transformed into the yeast strain PRT480 (with the genotype MATα, his3, trp1, ura3, 4 LexA-LEU2, lys2::3 cIop-LYS2, CANR , CYH2R , ade2::2 LexA-CYH2-ZEO, his5::1 LexA-URA3-G418).

The yeast that carry the bait and prey proteins and the potential blocking peptides were then mass mated, and from approximately 300,000 clones, 95 positives were identified (ie, approximately 1/3000).

Two methods of analysis were used to identify interaction-blocking activity:
The first of these comprised plating approximately 500 cells per half plate onto HTU media containing plates and counting the number of colonies growing after 3 days. In these conditions, an interaction of JUN1 and JUNZ enables the cells to grow. Accordingly, a reduction in the number of colonies indicates that the library being screened comprises peptide inhibitors of the JUN1/JUNZ interaction.

The second screening method involved isolation and streaking of 10 individual colonies to new HTU media containing plates and analysing for growth of new single colonies. After 3 days, those that express a peptide inhibitor generally have very little or no new growth, while those that do not express a peptide inhibitor have re-grown a streak of single colonies. As a positive control a known inhibitor of JUN1/JUNZ interaction, FosZ was used. As a negative control empty pYTB3 vector (Phylogica Ltd, Perth, Australia) with no peptide insert was used. A score of 1-10 given depending on growth of 10 individual clones of each peptide copared to the two control samples.

The score from method 1 and method 2 was then combined to determine if a specific colony expressed a peptide inhibotor of JUN1/JUNZ interaction. In the present case a cell expressing a peptide inhibitor was one that showed >50% reduction of growth compared to negative control in both tests.

All scoring was performed by two independent individuals and scores of both individuals were combined.

Following screening it was found that 60 of the clones were capable of inhibiting the interaction of JUN1 and JUNZ.

Of the 60 clones identified, 27 were sequenced and analyzed to determine their most likely source using BLAST-P. Results of this analysis are set forth in Table 4.

**Table 4**

| Characterization of peptides capable of blocking the interaction of JUNZ and JUN1. | | | |
|---|---|---|---|
| Peptide# | Length (aa) | Native ORF (Yes/No) | Species |
| SP4 | 75 | No | *Bacillus subtilis* |
| SP6 | 12 | No | *Aquifex aeolicus* |
| SP8 | 39 | Yes | *Helicobacter pylorii* |
| SP12 | 27 | Yes | *Escherichia coli* |
| SP15 | 86 | Yes | *Escherichia coli* |
| SP20 | 20 | No | *Helicobacter pylorii* |
| SP21 | 25 | No | *Borrelia burgdorferi* |
| SP22 | 40 | Yes | *Bordatella pertussis* |
| SP24 | 26 | No | *Haemophilus influenza* |
| SP30 | 53 | No | *Pseudomonas aeruginosa* |
| SP32 | 13 | No | *Plasmodium falciparum* |
| SP33 | 11 | No | *Haemophilus influenzae* |
| SP34 | 29 | No | *Aquifex aeolicus* |
| SP35 | 62 | Yes | *Pyrococcus horikoshii* |
| SP36 | 16 | Yes | *Bacillus subtilis* |
| SP39 | 12 | No | *Bordatella pertussis* |
| SP43 | 12 | No | *Neisseria meningitidis* |
| SP54 | 32 | Yes | *Escherichia coli* |
| SP58 | 45 | No | *Bacillus subtilis* |
| SP60 | 20 | No | *Bacillus subtilis* |
| SP66 | 39 | Yes | *Bacillus subtilis* |
| SP72 | 38 | No | *Haemophilus influenza* |
| SP73 | 33 | No | *Pyrococcus horikoshii* |
| SP76 | 24 | No | *Thermoplasma volcanium* |
| SP77 | 18 | No | *Thermoplasma volcanium* |
| SP79 | 12 | No | *Haemophilus influenza* |
| SP80 | 26 | Yes | *Bacillus sublilis* |

Note that 30% of the identified peptides are expressed in their native reading frame (i.e. they are identical to a region of a protein found in nature). This represents a significantly greater (p<0.009) number than would be expected by chance (as only 1 in 6 fragments would be expected to be in their native reading frame).

The sequence of the peptides identified in this screen are set forth in Table 5.

| Peptide number | SEQ ID NO: of nucleotide sequence with flanking vector sequence | SEQ ID NO: of nucleotide sequence without flanking vector sequence | SEQ ID NO: of amino sequence of peptide encoded by 1st ORF with flanking vector encoded sequence | SEQ ID NO: of sequence of peptide encoded by 1st ORF without flanking vector encoded sequence |
|---|---|---|---|---|
| SP4 | 65 | 67 | 66 | 68 |
| SP5 | 69 | 71 | 70 | 72 |
| SP6 | 73 | 75 | 74 | 76 |
| SP8 | 77 | 79 | 78 | 80 |
| SP12 | 81 | 83 | 82 | 84 |
| SP15 | 85 | 87 | 86 | 88 |
| SP18 | 89 | 91 | 90 | 92 |
| SP20 | 93 | 95 | 94 | 96 |
| SP21 | 97 | 99 | 98 | 100 |
| SP22 | 101 | 103 | 102 | 104 |
| SP24 | 105 | 107 | 106 | 108 |
| SP29 | 109 | 111 | 110 | 112 |
| SP30 | 113 | 115 | 114 | 116 |
| SP32 | 117 | 119 | 118 | 120 |
| SP33 | 121 | 123 | 122 | 124 |
| SP34 | 125 | 127 | 126 | 128 |
| SP35 | 129 | 131 | 130 | 132 |
| SP36 | 133 | 135 | 134 | 136 |
| SP45 | 137 | 139 | 138 | 140 |
| 54 | 141 | 143 | 142 | 144 |
| SP58 | 145 | 147 | 146 | 148 |
| SP60 | 149 | 151 | 150 | 152 |
| SP66 | 153 | 155 | 154 | 156 |
| SP71 | 157 | 159 | 158 | 160 |
| SP72, SP73, SP76, SP77 | 161 | 163 | 162 | 164 |
| SP79 | 165 | 167 | 166 | 168 |
| SP80 | 169 | 171 | 170 | 172 |
| SP54-1 | 173 | 175 | 174 | 176 |
| SP66-1 | 177 | 179 | 178 | 180 |

The ability of the peptides to interact with JUN1 was then confirmed with a forward two-hybrid assay. Each of the identified peptides capable of inhibiting the interaction of JUN1 and JUNZ was cloned into the bait vector pDD (SEQ ID NO: 61; Figure 6). Additionally nucleic acid encoding a peptide known not to inhibit the interaction between JUN1 and JUNZ was also cloned into pDD. The pDD vector and the JUN1 prey vector was transformed into the yeast strain PRT480 and the interaction of the encoded peptide and JUN1 assessed by determining the amount of growth in the absence of uracil. An example of such a screen is shown in Figure 9.

### EXAMPLE 10

### The structure of a Jun dimerization inhibitory peptide mimics the structure of the leucine zipper of Jun

The structure of peptide 22 was determined using threading. Threading is useful for determining or predicting the structure of a particular protein based on the structure of a related protein, for example, where only sparse information on the sequence identity of a target protein is known. This method uses a library of unique protein folds that are derived from structures deposited in the PDB. The sequence of the target protein is optimally threaded onto each protein fold in turn, allowing for relative insertions and deletions in the loop regions. The different trial threadings are each assigned an "energy" score based on summing the pairwise interactions between the residues in the given threading. The library of folds is ranked in ascending order of energy, with the lowest energy being taken as the most probable match.

The sequence of Peptide 22 was threaded onto a Jun-Jun dimer to determine the secondary structure of the peptide, using Swiss-PDB Viewer software (Geneva Biomedical Research Institute). The threaded structure of peptide 22 is depicted in Figure 10. Using this method it was determined that the peptide contained a number of leucine residues (or leucine like residues, e.g., methionine, valine or isoleucine) and hydrophobic molecules located approximately 3 to 4 amino acids after a leucine or leucine like amino acid to form a leucine zipper like structure. The structure of peptide 22 with the hydrophobic core is depicted in Figure 11). The leucine zipper like structure is capable of binding to the leucine zipper of Jun.

Furthermore, the acidic amino acids in the FLAG epitope expressed as a fusion with the peptide formed a structure capable of binding to the basic region of Jun. This region of Jun normally binds to DNA. The structure of the amino acids of the FLAG epitope bound to residues Arg 276, Lys-273 and Arg-270 of Jun is shown in Figures 12 and 13).

By analyzing the other peptides isolated in the screen described *supra* it was determined that a number of these peptides also contained a number of leucine residues and hydrophobic amino acids positioned to facilitate formation of a leucine zipper-like structure. Furthermore, several of these peptides also comprised acidic regions either formed by the FLAG epitope or a region of the peptide suitable for binding to the DNA binding region of Jun. The position of each of these regions and residues is shown in Table 6. Furthermore, the alignment of peptides is depicted in Figure 14.

**Table 6: Characteristics of C-Jun dimerization inhibitory peptides**

| **Peptide sequence** | **SEQ ID NO:** | **Leucine zipper-like subdomain** | **Leucine like residues forming leucine like subdomain** | **Hydrophobic residues within 3-4 residues of Leu** | **Acidic region** | **Acidic residues** |
|---|---|---|---|---|---|---|
| | | 5-73 | M(5), L(12), M(20), V(31), . L(32), L(35), I(42), L(43), I(46), L(51), I(62), M(68), M(69), M(71),1(72) | A(15), A(23), L(35), T(36), I(46), T(47), T(54), M(71), I(72) | | E(8), E(28), E(29), D(49), D(50), D(73) |
| | | 14-82 | M(15), L(22), M(30), V(41), L(42), L(45), I(52), L(53), I(56), I(61), I(72), M(78), M(79), I(82) | A(25), A(33), L(45), T(46), I(56), T(57), T(64), M(81), I(82) | 3-9 | D(3), D(6), D(7), D(8), D(9), E(18), E(38), E(39), D(59), D(60), D(83) |
| | | 14-85 | M(14), L(17), L(20), I(38), I(45), I(50), L(52), V(56), V(57), I(60), I(61),L(66), L(70), L(79), I(80), M(85) | L(17), Y(42), V(48), V(56), I(60), 1(61), F(69), F(83), P(84), P(88) | | E(9), D(19), D(26), D(34), D(37), D(41), E(43), E(74) |
| | | 24-95 | M(24), L(27), L(30), I(48), I(55), I(60), L(62), V(66), V(67), I(70), I(71), L(76), L(80), L(89), I(90), M(95) | L(27), Y(52), V(58), V(66), 1(70), I(71), F(79), F(93), P(94), P(98) | 3-9 | D(3), D(6), D(7), D(8), D(9), E(19), D(29), D(36), D(44), D(47), D(51), E(53), E(84) |
| RSDYKDDDDKKDSIRRXPENISSQEVEAVLMSHPEVVNAAVYPVRGDLPGD | | 14-48 | I(14), I(21), V(26), V(29), L(30), M(31), V(36), V(37), V(41), V(44), L(48) | P(18), V(29), P(34), A(39), A(40), V(44), L(48) | 3-9 | D(3), D(6), D(7), D(8), D(9), E(19). D(47), D(51) |
| VYAYFGXTGDVVEVGVDLVGIAGVAHAQAADPQGQQQQGQQAGQEEQADTD | | 1-24 | V(1), V(11), V(12), V(14), V(16), L(18), V(19), I(21), V(24) | Y(4), V(14), V(16), V(19), I(21), A(22), V(24), A(27) | | D(10), E(13), D(17), D(31), E(45), E(46), D(49), D(51) |
| SIRSGGIESSSKREKVRVGMTLRTYNPNETFFSILHEFVKFLKRRRLLQEAIDLSSSSL | | 2-58 | I(2), I(7), V(16), V(18), M(20), L(22), I(34), L(35), L(42), L(47), L(48), I(52), L(54), L(58) | M(20), T(21), Y(25), F(38), V(39), A(51), I(52), | | E(8), E(14), E(29), E(37), E(50), D(53) |
| | | 12-68 | I(12), I(17), V(26), V(28), M(30), L(32), I(44), L(45), L(52), L(57), L(58), 1(62), L(64), L(68) | M(30), T(31), Y(35), F(48), V(49), A(61), I(62), | 3-9 | D(3), D(6), D(7), D(8), D(9), E(18), E(24), E(39), E(47), E(60), D(63) |
| SFXXAGYHGXTSRTFLVGSVSATARKLVEATQETMIDYTCRRRPCSLTWYQLMHRYRY | | 16-53 | L(16), V(17), V(20), L(27), V(28), M(35), I(36), L(47), L(52), M(53) | V(20), T(23), A(30, T(31), Y(50), Y(56) | | E(29), E(33), D(37) |
| | | 26-63 | L(26), V(27), V(30), L(37), V(38), M(45), I(46), L(57), L(62), M(63) | V(30), T(33), A(40), T(41), Y(60), Y(66) | 3-9 | D(3), D(6), D(7), D(8), D(9), E(29), E(33), D(37) |
| | | 2-55 | I(2), M(3), V(5), L(14), V(15), V(24), I(26), L(33), L(41), L(49), L(54), L(55) | A(4), V(5), P(36), L(41), Y(52), Y(58) | | D(27), D(30), E(40) |
| | | 12-65 | I(12), M(13), V(15), L(24), V(25), V(34), I(36), L(43), L(51), L(59), L(64), L(65) | A(14), V(15), P(46), L(51), Y(62), Y(68) | 3-9 | D(3), D(6), D(7), D(8), D(9), D(37), D(40), E(50) |
| | | 5-59 | 1(5), I(6), L(13), I(14), L(17), V(20), I(28), L(53), L(58), M(59) | A(8), A(16), L(17), V(20), A(23), L(30), Y(56), Y(62) | | D(24), D(31) |
| | | 15-69 | I(15), I(16), L(23), 1(24), L(27), V(30), I(38), L(63), L(68), M(69) | A(18), A(26), L(27), V(30), A(33), L(40), Y(66), Y(72) | 3-9 | D(3), D(6), D(7), D(8), D(9), D(34), D(41) |
| ELRSQLGPVPLIDASIPVLVGPHMPGRTAAARGMHLEGRIM | | 2-41 | L(2), L(6), V(9), L(1), I(12), I(16), V(18), L(19), V(20), M(24), L(36), I(40), M(41) | L(6), V(9), I(12), A(14), I(16), L(20), P(22), M(24), T(28), I(40) | | E(1), D(13), E(37) |
| | | 25-74 | M(25), V(32), M(43), L(53), L(63), L(68), M(69) | A(30), Y(36), P(45), P(60), Y(66) | 3-9 | D(3), D(6), D(7), D(8). D(9) |
| | | 15-64 | M(15), V(22), M(33), L(43), L(53), L(58), M(59) | A(20), Y(26), P(35), P(50), Y(56) | | |
| RSDYKDDDDKAYQSFXLAGYHGDTSRTFLVGSVSATARKLVEATQETMIDY | | 17-51 | L(17), L(29), V(30), V(33), L(40), V(41), M(48) | Y(20), T(24), T(27), A(35), T(36), A(37), A(43), T(44), Y(41) | 3-9 | D(3), D(6), D(7), D(8), D(9) |
| AYQSFXLAGYHGDTSRTFLVGSVSATARKLVEATQETMIDY | | 7-41 | L(7), L(19), V(20), V(23), L(30), V(31), M(38) | Y(10), T(14), T(17), A(25), T(26), A(27), A(33), T(34), Y(41) | | |
| | | 16-71 | M(16), V(18), V(24), L(27), V(28), V(37), L(46), L(50), L(54), L(62), L(67), M(68) | A(17), A(19), A(20), P(23), A(25), T(48), P(49), Y(65), Y(71) | 3-9 | D(3), D(6), D(7), D(8), D(9) |
| | | 6-61 | M(6), V(8), V(14), L(17), V(18), V(27), L(36), L(40), L(44), L(52), L(57), M(58) | A(7), A(9), A(10), P(13), A(15), T(38), P(39), Y(55), Y(61) | | |
| | | 18-44 | L(23), L(27), V(30), L(40) | A(18), T(22), A(26), T(28), Y(31), A(33), T(37), A(44) | 3-9 | D(3), D(6), D(7), D(8), D(9) |
| | | 8-34 | L(13), L(17), V(20), L(30) | A(8), T(12), A(16), T(18), Y(21), A(23), T(27), A(34) | | |

### EXAMPLE 11

### c-Jun dimerization inhibitors reduce c-Jun mediated gene expression

The K562 cell line was stably-transfected with the AP-1 luciferase reporter of the Mercury Profiling kit (Clontech, U.S.A.), and clonal cell line 26 established. In 6-well tissue culture plate format, K562-AP1 cells were transfected with either pcDNA3 control, pcDNA3-Jun or pcDNA3-peptide using Lipofectamine2000 (Life Technologies), according to manufacturer's instructions. Transfections were incubated for 48 hours, cells collected and protein lysates extracted for luciferase assay according to Mercury Profiling kit and associated protocols. Luciferase assays were performed in independent triplicates, and results for each peptide subjected to statistical analysis (SPSS software package) to determine if they were different to Jun (positive control for AP-1 activation) or pcDNA-3 (negative control for AP-1 activation).

As shown in Figure 15 peptides SP36 (SEQ ID NO: 134), SP35 (SEQ ID NO: 130), SP71 (SEQ ID NO: 158) and SP34 (SEQ ID NO: 126) are capable of significantly reducing expression of a reporter gene placed in operable connection with an AP-1 regulatory region compared to control cells. As AP-1 mediated transcription is mediated by, for example, c-Jun dimerization, these results indicate that each of these peptides inhibit or reduce c-Jun dimerization.

Results from these studies indicate that a significant proportion of peptides identified using the reverse hybrid screen (p<0.05) are capable of reducing AP-1 mediated gene expression.

### EXAMPLE 12

### c-Jun dimerization inhibitors bind to c-Jun

HEK293 cells were cultured in DMEM + 10% FCS, 2mM L-glutamine. On the day prior to transfection, cells were trypsinised and split into 6-well tissue culture plates so that they reached 80-90% confluency for transfection. Cells were co-transfected with pcDNA3-Jun (1.3µg) and pcDNA3-peptide (2.6µg) using Lipofectamine2000 reagent (Life Technologies, U.S.A.) as per manufacturer's instructions. Forty-eight hours post-transfection, transfected cells were scraped from the plates, collected by centrifugation and proteins extracted in hypotonic lysis buffer (10mM Tris, 10mM NaCl, 2mM EDTA, pH 7.5 + protease inhibitors (Roche, U.S.A.)). Salt concentration was adjusted to 150mM by addition of NaCl, debris pelleted and proteins in supernatant collected in fresh tubes.

A small aliquot (40µl) of protein was set aside for western analysis. The remainder was incubated by rotation at at 4°C for two hours, with either anti-Flag conjugated agarose beads (Sigma-Aldrich, U.S.A.) anti-Flag antibody (Sigma-Aldrich), preconjugated to anti-mouse magnetic Dynabeads (Dynal Biotech, Norway) according to manufacturer's directions. Protein complexes bound to conjugated beads were collected by centrifugation or over a Dynal magnet, washed eight times for five minutes with NET-2 buffer (50mM Tris-Cl pH7.5, 150mM NaCl, 0.05% Nonidet P-40). Beads and associated complexes were resuspended in 3.3X Laemmli SDS loading buffer, incubated for 5 minutes at 100°C, and stored at -20°C.

Co-immunoprecipitations and protein extracts were separated on 12% Tris-glycine gels, transferred to membrane (Hybond C-super, Amersham), and probed with anti-Jun primary antibody, anti-rabbit secondary (Amersham) and visualized with autoradiograph exposure and an ECL detection kit (Amersham).

Anti-FLAG antibodies to capture FLAG tagged c-Jun inhibitory peptides from mammalian cells in which they were expressed. Following separation of proteins by SDS-PAGE and transfer to a membrane, membranes were probed with anti-c-Jun antibodies. As shown in Figure 16, peptides SP15 (SEQ ID NO: 86), SP20 (SEQ ID NO: 94), SP30 (SEQ ID NO: 114), and SP35 (SEQ ID NO: 130) were capable of binding c-Jun to a level detectable in a co-immunoprecipitation. These results are representative of assays in which it was found that the majority of peptides tested were capable of co-immunoprecipitating c-Jun.

Furthermore, by comparing the total level of c-Jun in the cells to that obtained in a co-iimunoprecipitation, it is seen that several of the peptides bind a significant portion of the c-Jun expressed in the cell.

### EXAMPLE 13

### c-Jun dimerization inhibitors reduce TNF-α mediated cell death

Neuronal PC 12 cells were transfected with an expression construct encoding a c-Jun dimerization inhibitor (e.g., peptide SP34 (SEQ ID NO: 126), SP36 (SEQ ID NO: 134) or SP71 (SEQ ID NO: 158)). The cells were then exposed to an TNF-α, which has been shown to induce cell death in this cell line.

The PC12 cell line is derived from a transplantable rat pheochromocytoma (ATCC Accession Number: CRL-1721). Cells were maintained in DMEM +10% foetal calf serum (FCS), 15% horse serum, and 2mM L-glutamine, and were fed every three days and split no more than once before transfection and TNF exposure.

On day 1, PC12 cells were trypsinised to separate multicell aggregates, counted, and in duplicate for each peptide and control, 8x10⁵ cells in 0.5ml were seeded per well in 24-well tissue culture plates. In each well, cells were transfected using Lipofectamine2000 reagent (Life Technologies, U.S.A.), with 4µl Lipofectamine2000 reagent diluted in 100µl DMEM complexed with 1.6µg plasmid diluted in 100µl DMEM. Transfections were incubated at 37°C/5% CO₂ overnight.

On day 2, tranfected PC12 cells were collected by centrifugation, then resuspended in DMEM + 2mM L-glutamine and transferred to fresh 24-well tissue culture plates. TNFa (Roche, U.S.A.) diluted in DMEM + 2mM L-glutamine was added to the cells in each well to a final volume of 1ml and final concentration of 100ng/ml TNFa, and cells were returned to the incubator for 48 hours.

On day 4, duplicate transfections were combined and the total cells were collected by centrifugation, fixed on charged slides and stained with a TUNEL assay kit (Promega, U.S.A.) as per manufacturer's protocol. For each slide, six different sections of 150 cells were counted for apoptosing (stained brown or with punctate brown staining) and non-apoptosing cells (counterstained green) and the percentage of apoptosing cells was calculated and then averaged. Peptide protection against TNFa-induced apoptosis was assessed by comparing the percentage of apoptosed cells to that of the pcDNA3 positive control (maximum apoptosis induction).

As shown in Figure 17a-d, TNFα induced apoptosis in control cells. However, each of the peptides tested were capable of inhibiting TNFα induced apoptosis.

Figure 17e shows the percentage of cells undergoing apoptosis (detected using a TUNEL assay). Clearly, each of the tested peptides significantly reduce the level of apoptosis compared to control samples.

### EXAMPLE 14

### c-Jun dimerization inhibitors reduce UV mediated cell death

Cells were exposed to UV B radiation and the level of cell death determined. Briefly, corneal keratinocytes in culture were exposed to 10mins UV irradiation. Post-exposure, media was replaced with either normal media or media containing 10 micromolar peptide. Subsequently, cells were prepared for FACS analysis. FACS analysis was used to detect propidium iodide and the level of Annexin V in a cell to determine the number of cells undergoing necrosis, early apoptosis or late apoptosis.

As shown in Figures 18a-c, control a portion of SIRC cells (not exposed to UVB) are necrotic and a portion are alive. Following exposure to UV B an increased number of SIRC cells are observed undergoing apoptosis. However, as shown in Figure 18c, peptide SP36 (SEQ ID NO: 134 or 136) is capable of reducing the number of cells undergoing apoptosis.

### EXAMPLE 15

### c-Jun dimerization inhibitors reduce cell death in an in vitro ischemia cell model

Primary neuronal cells were isolated and cultured in the presence of glutamate (250µM) for 25 minutes to induce cell death as a model of ischemia induced cell death.

Primary rat neurons were isolated from embryos (standartd protocols), plated in cell culture dishes and maintained for 11 days in culture before experiment. Peptide was added 15 minutes to media before glutamate addition. Glutamate was added to final concentrations of 250 micromolar, for 5 mins at 37 degrees. Glutamate media removed, fresh media added. Assays for live cells done 24 hours later. Live cells were assayed using MTS assay.

As shown in Figure 19, glutamate caused a significant proportion of cells to die compared to control cells.

Peptides SP35 (SEQ ID NO: 130), SP36 (SEQ ID NO: 134) and SP71 (SEQ ID NO: 158) were capable of rescuing a significant proportion of cells from cell death. In fact, peptide SP36 was capable of rescuing almost all cells from cell death. The number of cells expressing these peptides that survived exposure to glutamate was considerably greater than the number of cells expressing the known c-Jun dimerization inhibitory peptide TI-JIP (Barr et al., J Biol Chem. 279:36327-38, 2004).

Furthermore, as shown in Figure 20, peptide SP36 rescued cells from glutamate induced cell death in a dose dependent manner with about 5µM of peptide rescuing about 100% of cells.

### EXAMPLE 16

### Analogue of c-Jun dimerization inhibitory peptides reduce cell death in an in vitro ischemia cell model

Experiments were performed to determine the efficacy of D-amino acid forms of c-Jun inhibitory peptides in the treatment of ischemia. Peptides comprising D amino aicds are protease resistant and, as a consequence, have a longer half-life when administered to a subject.

D amino acid forms of peptides SP35 (designated D35) (SEQ ID NO: 132) and SP36 (designated D36) (SEQ ID NO: 136) comprising D amino acids other than glycine were produced synthetically, as were peptides SP35, SP36 and TIJIP comprising L- amino acids. The retro-inverted peptides further comprised a TAT protein targeting domain fused to the C-terminus of the inverted peptide moiety and separated therefrom by a single L-glycine residue in each case. The amino acid sequences of the retro-inverted peptide analogues of SEQ ID NOs: 132 and 136 are set forth in SEQ ID NOs: 181 and 182, respectively.

Primary rat neuronal cells were isolated and cultured using methods known in the art. Cells were then incubated in the presence or absence of a test peptide, a positive control peptide (Ti JIP) or a combination of known small-molecule glutamate inhibitors (MK801 and CNQX). Cells were incubated in the presence of 250 µM glutamate for 5 minutes to induce cell death representative of ischemia induced cell death.

As shown in Figure 21 in presence of glutamate approximately 3% of control cells survive (relative to the number of cells suriviving in the absence of glutamate). Addition of either D or L form of each peptide protects a considerable proportion of neurons from glutamate induced cell death (approximately equivalent to the level of protection conferred by known glutamate receptor inhibtitors). When used at the same concentrations the protection offered by the D form of each peptide is either equivalent (SP36 and D36) or superior (SP35 and D35) to the L form of the peptide.

### EXAMPLE 17

### c-Jun dimerization inhibitory peptides protect cells from acute ischemia

Cells at the core of an ischemic event (e.g., a stroke) are subject to anaerobic conditions leading to severe energy depletion and glutamate release, which causes necrotic cell death. Such a condition is mimicked by incubating cell cultures in anaerobic conditions.

To determine the effect of peptides 35 (SEQ ID NO: 130) and 36 (SEQ ID NO: 134) comprising either D- or L- amino acids on an acute ischemic effect, primary rat neuronal cells were isolated and cultured. Synthetic peptides were added to cultures and the cells maintained in an anaerobic chamber for approximately 35 minutes. Cell survival was then measured.

Briefly, isolated rat neurons were treated with peptide for 15 mins pre-insult. After addition of peptide or control, Cells were washed in glucose free balanced salt solution containing deoxy glucose to prevent glycolysis. Cells were then incubated in anaerobic incubator for 35 minutes. Post insult, solution was removed, fresh media added to cells and MTS assayed for live cells 24 hours later.

As shown in Figure 22 the peptides 35 and 36 comprising D-amino acids a considerable proportion of cells from cell death caused by acute ischemia. Peptides comprising D-amino aicds rescued more cells from cell death than corresponding cells with L-amino aicds.

### EXAMPLE 18

### Identifying those peptides capable of inhibiting stroke

High affinity peptide inhibitors of c-Jun dimerization identified as described in the preceding examples are cloned into an adenoviral expression vector. Primary neuronal cell cultures are then infected with the peptides and subjected to *an in vitro* stroke simulation using an anaerobic incubation period of 10 minutes. The viability of the neurons is ascertained at a number of time points subsequent to the ischemic event to determine the level of protection each peptide provides against apoptosis.

Purified synthesized TAT-peptide fusions are used. There is significant *in vivo* evidence that TAT-peptides can be successfully delivered to the brain using IV delivery. To determine those peptides that exhibit the greatest *in vivo* stability and deliverability, IV injections of TAT-peptide fusions into rat and subsequent analysis of brain tissue at a number of time points and doses is performed to determine those peptides that undergo *in vivo* analysis.

TAT-peptide fusions are delivered intravenously at 1 hour pre-ischemia, and 3, 6, and 9 hours post-ischemia. The rat temporary occlusion of the MCA model is used to induce transient focal ischemia. Induction of focal ischemia involves placing a monofilament nylon suture to occlude the middle cerebral artery (MCA) for 45 minutes and maintaining blood pressure at 90 mmHg, followed by reperfusion. MCA occlusion and re-establishment of blood flow is monitored using Laser Doppler. Animals are anesthetized during MCA occlusion to allow Laser Doppler and blood pressure monitoring. The animals are sacrificed at 72 hours following reperfusion and the area of infarction is determined, by incubating coronal brain sections in a 2% solution of triphenyltetrazolium chloride, which stains mitochondrial dehydrogenase activity. Stained serial 1mm brain slices are scanned and analyzed using the NIH image system to calculate infarct volume. Total infarct volume is calculated by multiplying the area of infarct in each slice by the slice thickness and is expressed as a percentage of the contralateral unaffected hemisphere volume. For long term protection studies infarct volume is assessed at 3 weeks post-ischemia. The extent of infarct are expressed as a percentage of the whole brain volume and data analyzed by ANOVA followed by post-hoc Bonferroni/Dunn test.

Behavioral testing following focal ischemia is performed 24, 48 and 72 hours following ischemia. Two tests are used. A cumulative 5-point scale of deficit in which a given score encompasses all deficits lower on the scale. The scale consists of: 0 = no apparent deficit; 1 = asymmetrical paw extension, torsion to paretic side (minor deficit), 2 = non-responsive to touch on left face and shoulder (mild deficit), 3 = spontaneous circling to the paretic side (considerable deficit), 4 = seizures or no spontaneous movement(severe deficit).

In addition to these tests, a bilateral asymmetry paw test which assesses both motor and sensory impairment is employed. For this test, a single 20 x 14 mm rectangular piece of masking tape is applied with equal pressure to the pad of each forepaw. The time required by the animal to remove the tape is recorded (maximum time allowable for task 2 minutes).

TAT-peptide fusions are delivered intravenously at 1 hour pre-ischemia, and 3, 6, and 9 hours post ischemia. A rat two-vessel occlusion with hypotension model is used to induce transient global cerebral ischemia. This involves occluding both carotid arteries and lowering blood pressure to 45mmHg (by removing arterial blood) for 8 minutes, followed by reperfusion and restoration of blood pressure. Parameters such as blood pH, pressure, gases and glucose, EEG, body and cranial temperature are monitored during the procedure. Following 8 minutes of global ischemia in this model there is no or little hippocampal CAI neuronal death for up to 24 hours post ischemia, but significant CAI neuronal death by 48-72 hours. At seven days post-ischemia there is <5-6% CAI neuronal survival. Hippocampal neuronal viability is assessed at day 7 post-ischemia, by counting the number of viable CAI neurons in a 1000pM region at bregma section 3.8 in hippocampi from control and treated rats. For long term survival studies CAI neuronal counts are performed at 3 months. Data are analyzed by ANOVA, followed by post-hoc Bonferroni/Dunn.

The 8 arm radial-maze test, developed by Olton & Samuelson in 1976, has become one of the standard approaches to testing reference and working memory and spatial cognition in studies of hippocampal function in rats. The protocol requires animals to learn to enter only the baited arms of a maze in which alternate arms are baited, the numbers of the different types of erroneous arm (never-baited or already-rewarded) entries made providing the measures of reference and spatial working memory. Maze training begins within three days of maze familiarization. After maze training, the following 7-8 days form the test phase of the experiment. Each day each animal is placed once on a central platform of the maze and left in the maze until they have retrieved the rewards from all four baited arms, or until 10 minutes have elapsed. Records are kept of the total time elapsed until completion of the task, the path taken around the maze and general demeanor (episodes of grooming, defecation, miction). This combination of measures allows estimation of levels of locomotor activity, the number of each type of error, and the spatial strategy employed (learned sequence of movements versus use of a spatial map). Comparisons of the performance of animals subject to the various experimental treatments are made using the ANOVA, Chi-square and time series functions of the SPSS statistical program.

## Claims

1. An isolated or recombinant peptide or peptide analogue for use in a method for the treatment of ischemia comprising an amino acid sequence selected from the group consisting of:
(i) the sequence set forth in SEQ ID NO: 134, or SEQ ID NO: 136, and
(ii) a retro-inverted peptide analogue of (i), wherein said analogue comprises the amino acid sequence set forth in SEQ ID NO: 182.

2. The isolated or recombinant peptide or peptide analogue for use in a method for the treatment of ischemia according to claim 1, wherein the peptide comprises the sequence set forth in SEQ ID NO: 134.

3. The isolated or recombinant peptide or peptide analogue according to claim 1 or 2, wherein the peptide consists of the sequence set forth in SEQ ID NO: 134.

4. The isolated or recombinant peptide or peptide analogue according to claim 1 or 2, wherein the peptide consists of the sequence set forth in SEQ ID NO: 136.

5. The isolated or recombinant peptide or peptide analogue according to any of claims 1 to 4, wherein the peptide is expressed as a fusion with a protein transduction domain.

6. The isolated or recombinant peptide or peptide analogue according to any of claims 1 to 5, wherein the protein transduction domain is a *tat* sequence of HIV.

7. The isolated or recombinant peptide or peptide analogue for use in a method for the treatment of ischemia according to claim 1, wherein the peptide analogue consists of the sequence set forth in SEQ ID NO: 182.

8. A pharmaceutical composition for use in a method for the treatment of ischemia comprising the isolated or recombinant peptide or peptide analogue according to any one of claims 1 to 7 and a pharmaceutically acceptable carrier or excipient.

9. The isolated or recombinant peptide or peptide analogue according to any one of claims 1 to 7 or the pharmaceutical composition according to claim 8 for use in a method for the treatment of ischemia in a subject suffering from ischemia or is a subject that has suffered from ischemia or in a subject at risk of experiencing a reperfusion injury following an ischemic event.

10. A pharmaceutical composition for use in a method for the treatment of ischemia comprising nucleic acid that encodes the isolated or recombinant peptide according to any one of claims 1 to 7 and a pharmaceutically acceptable carrier or excipient.

11. The isolated or recombinant peptide or peptide analogue or pharmaceutical composition according to claim 9 for administration to a subject by a method selected from the group consisting of intravenous administration, intrathecal administration, intra-arterial administration, local administration following a craniotomy, and mixtures thereof.

## Patentansprüche

1. Isoliertes oder rekombinantes Peptid oder Peptidanalogon zur Verwendung in einem Verfahren zur Behandlung von Ischämie, umfassend eine Aminosäuresequenz, ausgewählt aus der Gruppe, bestehend aus:
(i) der in SEQ ID NR.: 134 oder SEQ ID NR.: 136 angeführten Sequenz und
(ii) einem retroinvertierten Peptidanalogon von (i), wobei das Analogon die in SEQ ID NR.: 182 angeführte Aminosäuresequenz umfasst.

2. Isoliertes oder rekombinantes Peptid oder Peptidanalogon zur Verwendung in einem Verfahren zur Behandlung von Ischämie nach Anspruch 1, wobei das Peptid die in SEQ ID NR.: 134 angeführte Sequenz umfasst.

3. Isoliertes oder rekombinantes Peptid oder Peptidanalogon nach Anspruch 1 oder 2, wobei das Peptid aus der in SEQ ID NR.: 134 angeführten Sequenz besteht.

4. Isoliertes oder rekombinantes Peptid oder Peptidanalogon nach Anspruch 1 oder 2, wobei das Peptid aus der in SEQ ID NR.: 136 angeführten Sequenz besteht.

5. Isoliertes oder rekombinantes Peptid oder Peptidanalogon nach einem der Ansprüche 1 bis 4, wobei das Peptid als eine Fusion mit einer Proteintransduktionsdomäne exprimiert wird.

6. Isoliertes oder rekombinantes Peptid oder Peptidanalogon nach einem der Ansprüche 1 bis 5, wobei die Proteintransduktionsdomäne eine *tat*-Sequenz von HIV ist.

7. Isoliertes oder rekombinantes Peptid oder Peptidanalogon zur Verwendung in einem Verfahren zur Behandlung von Ischämie nach Anspruch 1, wobei das Peptidanalogon aus der in SEQ ID NR.: 182 angeführte Sequenz besteht.

8. Pharmazeutische Zusammensetzung zur Verwendung in einem Verfahren zur Behandlung von Ischämie, umfassend das isolierte oder rekombinante Peptid oder Peptidanalogon nach einem der Ansprüche 1 bis 7 und einen pharmazeutisch unbedenklichen Träger oder Hilfsstoff.

9. Isoliertes oder rekombinantes Peptid oder Peptidanalogon nach einem der Ansprüche 1 bis 7 oder pharmazeutische Zusammensetzung nach Anspruch 8 zur Verwendung in einem Verfahren zur Behandlung von Ischämie bei einem Patienten, der an Ischämie leidet, oder bei einem Patienten, der an Ischämie gelitten hat, oder bei einem Patienten, bei dem das Risiko eines Reperfusionsschadens nach einem ischämischen Ereignis besteht.

10. Pharmazeutische Zusammensetzung zur Verwendung in einem Verfahren zur Behandlung von Ischämie, umfassend eine Nukleinsäure, die für das isolierte oder rekombinante Peptid oder Peptidanalogon nach einem der Ansprüche 1 bis 7 codiert, und einen pharmazeutisch unbedenklichen Träger oder Hilfsstoff.

11. Isoliertes oder rekombinantes Peptid oder Peptidanalogon oder pharmazeutische Zusammensetzung nach Anspruch 9 zur Verabreichung an einen Patienten mittels eines Verfahrens, ausgewählt aus der Gruppe, bestehend aus intravenöser Verabreichung, intrathekaler Verabreichung, intraarterieller Verabreichung, lokaler Verabreichung nach Kraniotomie und Mischungen davon.

## Revendications

1. Peptide ou peptide analogue isolé ou recombinant destiné à être utilisé dans une méthode de traitement de l'ischémie comprenant une séquence d'acides aminés choisie dans le groupe consistant en :
(i) la séquence SEQ ID NO: 134, ou SEQ ID NO: 136, et
(ii) un peptide analogue rétro-inversé de (i), dans lequel ledit analogue comprend la séquence d'acides aminés SEQ ID NO: 182.

2. Le peptide ou peptide analogue isolé ou recombinant destiné à être utilisé dans une méthode de traitement de l'ischémie selon la revendication 1, dans lequel le peptide comprend la séquence SEQ ID NO: 134.

3. Le peptide ou peptide analogue isolé ou recombinant selon la revendication 1 ou 2, dans lequel le peptide se compose de la séquence SEQ ID NO: 134.

4. Le peptide ou peptide analogue isolé ou recombinant selon la revendication 1 ou 2, dans lequel le peptide se compose de la séquence SEQ ID NO: 136.

5. Le peptide ou peptide analogue isolé ou recombinant selon l'une quelconque des revendications 1 à 4, dans lequel le peptide est exprimé sous la forme d'une fusion avec un domaine de transduction de protéine.

6. Le peptide ou peptide analogue isolé ou recombinant selon l'une quelconque des revendications 1 à 5, dans lequel le domaine de transduction de protéine est une séquence *tat* du VIH.

7. Le peptide ou peptide analogue isolé ou recombinant destiné à être utilisé dans une méthode de traitement de l'ischémie selon la revendication 1, dans lequel le peptide analogue se compose de la séquence SEQ ID NO: 182.

8. Composition pharmaceutique destinée à être utilisé dans une méthode de traitement de l'ischémie comprenant le peptide ou peptide analogue isolé ou recombinant selon l'une quelconque des revendications 1 à 7 et un véhicule ou un excipient pharmaceutiquement acceptable.

9. Le peptide ou peptide analogue isolé ou recombinant selon l'une quelconque des revendications 1 à 7 ou la composition pharmaceutique selon la revendication 8 destiné(e) à être utilisé(e) dans une méthode de traitement de l'ischémie chez un sujet souffrant d'ischémie ou chez un sujet ayant souffert d'ischémie ou chez un sujet présentant un risque de lésion de reperfusion à la suite d'un épisode d'ischémie.

10. Composition pharmaceutique destinée à être utilisée dans une méthode de traitement de l'ischémie comprenant un acide nucléique qui code pour le peptide isolé ou recombinant selon l'une quelconque des revendications 1 à 7 et un véhicule ou un excipient pharmaceutiquement acceptable.

11. Le peptide ou peptide analogue isolé ou recombinant ou la composition pharmaceutique selon la revendication 9 destiné(e) à être administré(e) à un sujet à l'aide d'une méthode choisie dans le groupe consistant en administration intraveineuse, administration intrathécale, administration intra-artérielle, administration locale après craniotomie, et des combinaisons de celles-ci.


### SEQUENCE LISTING

<110> Phylogica Limited
<120> Methods of screening biodiverse gene fragment libraries and uses of biological modulators isolated therefrom
<130> 123266
<150> US60/603,525
   <151> 2004-08-20
<160> 182
<170> PatentIn version 3.3
<210> 1
   <211> 6
   <212> DNA
   <213> artificial sequence
<220>
   <223> Kozak sequence
<220>
   <221> misc_feature
   <222> (2)..(3)
   <223> n is a, c, g, or t
<400> 1
   rnnatg 6
<210> 2
   <211> 8
   <212> DNA
   <213> artificial sequence
<220>
   <223> Kozak sequence
<400> 2
   ccrccatg 8
<210> 3
   <211> 11
   <212> DNA
   <213> artificial sequence
<220>
   <223> Kozak sequence
<400> 3
   gccagccatg g 11
<210> 4
   <211> 8
   <212> DNA
   <213> artificial sequence
<220>
   <223> Kozak sequence
<400> 4
   ctaccatg 8
<210> 5
   <211> 10
   <212> DNA
   <213> artificial sequence
<220>
   <223> Shine Dalgarno sequence
<400> 5
   gaagaagata 10
<210> 6
   <211> 7
   <212> DNA
   <213> artificial sequence
<220>
   <223> Translational slippage sequence
<400> 6
   aaaaaac 7
<210> 7
   <211> 7
   <212> DNA
   <213> artificial sequence
<220>
   <223> Translational slippage sequence
<400> 7
   aaattta 7
<210> 8
   <211> 7
   <212> DNA
   <213> artificial sequence
<220>
   <223> Translational slippage sequence
<400> 8
   aaatttt 7
<210> 9
   <211> 7
   <212> DNA
   <213> artificial sequence
<220>
   <223> Translational slippage sequence
<400> 9
   gggaaac 7
<210> 10
   <211> 7
   <212> DNA
   <213> artificial sequence
<220>
   <223> Translational slippage sequence
<400> 10
   gggcccc 7
<210> 11
   <211> 7
   <212> DNA
   <213> artificial sequence
<220>
   <223> Translational slippage sequence
<400> 11
   gggttta 7
<210> 12
   <211> 7
   <212> DNA
   <213> artificial sequence
<220>
   <223> Translational slippage sequence
<400> 12
   gggtttt 7
<210> 13
   <211> 7
   <212> DNA
   <213> artificial sequence
<220>
   <223> Translational slippage sequence
<400> 13
   tttaaac 7
<210> 14
   <211> 7
   <212> DNA
   <213> artificial sequence
<220>
   <223> Translational slippage sequence
<400> 14
   tttaaat 7
<210> 15
   <211> 6
   <212> DNA
   <213> artificial sequence
<220>
   <223> Translational slippage sequence
<400> 15
   ttttta 6
<210> 16
   <211> 7
   <212> DNA
   <213> artificial sequence
<220>
   <223> Translational slippage sequence
<400> 16
   ggattta 7
<210> 17
   <211> 7
   <212> DNA
   <213> artificial sequence
<220>
   <223> Translational slippage sequence
<400> 17
   cttaggc 7
<210> 18
   <211> 7
   <212> DNA
   <213> artificial sequence
<220>
   <223> Translational slippage sequence
<400> 18
   gcgagtt 7
<210> 19
   <211> 7
   <212> DNA
   <213> artificial sequence
<220>
   <223> Translational slippage sequence
<400> 19
   tcctgat 7
<210> 20
   <211> 7
   <212> DNA
   <213> artificial sequence
<220>
   <223> Translational slippage sequence
<400> 20
   aaaaaag 7
<210> 21
   <211> 7
   <212> DNA
   <213> artificial sequence
<220>
   <223> Translational slippage sequence
<400> 21
   aaaaaaa 7
<210> 22
   <211> 7
   <212> DNA
   <213> artificial sequence
<220>
   <223> Translational slippage sequence
<400> 22
   aaaaaac 7
<210> 23
   <211> 7
   <212> DNA
   <213> artificial sequence
<220>
   <223> Translational slippage sequence
<400> 23
   gggaaag 7
<210> 24
   <211> 7
   <212> DNA
   <213> artificial sequence
<220>
   <223> Translational slippage sequence
<400> 24
   aaaaggg 7
<210> 25
   <211> 7
   <212> DNA
   <213> artificial sequence
<220>
   <223> Translational slippage sequence
<400> 25
   gggaaaa 7
<210> 26
   <211> 7
   <212> DNA
   <213> artificial sequence
<220>
   <223> Translational slippage sequence
<400> 26
   tttaaag 7
<210> 27
   <211> 7
   <212> DNA
   <213> artificial sequence
<220>
   <223> Translational slippage sequence
<400> 27
   aaagggg 7
<210> 28
   <211> 3
   <212> DNA
   <213> artificial sequence
<220>
   <223> Translational slippage sequence
<400> 28
   ctt 3
<210> 29
   <211> 17
   <212> PRT
   <213> artificial sequence
<220>
   <223> Penetratin protein transduction domain peptide
<400> 29
   Cys Arg Gln Ile Lys Ile Trp Phe Gln Asn Arg Arg Met Lys Trp Lys
   1               5                   10                  15
   Lys
<210> 30
   <211> 21
   <212> PRT
   <213> artificial sequence
<220>
   <223> Pep 1 protein transduction domain peptide
<400> 30
   Lys Glu Thr Trp Trp Glu Thr Trp Trp Thr Glu Trp Ser Gln Lys Lys
   1               5                   10                  15
   Lys Lys Arg Lys Val
               20
<210> 31
   <211> 13
   <212> PRT
   <213> artificial sequence
<220>
   <223> HIV-1 TAT protein transduction domain peptide
<400> 31
   Gly Arg Lys Lys Arg Arg Gln Arg Arg Arg Pro Pro Gln
   1               5                   10
<210> 32
   <211> 27
   <212> PRT
   <213> artificial sequence
<220>
   <223> signal sequence based peptide 1 protein transduction domain peptide
<400> 32
   Gly Ala Leu Phe Leu Gly Trp Leu Gly Ala Ala Gly Ser Thr Met Gly
   1               5                   10                  15
   Ala Trp Ser Gln Pro Lys Lys Lys Arg Lys Val
               20                  25
<210> 33
   <211> 16
   <212> PRT
   <213> artificial sequence
<220>
   <223> Signal sequence based peptide 2 protein transduction domain peptide
<400> 33
   Ala Ala Val Ala Leu Leu Pro Ala Val Leu Leu Ala Leu Leu Ala Pro
   1               5                   10                  15
<210> 34
   <211> 26
   <212> PRT
   <213> artificial sequence
<220>
   <223> Transportan protein transduction domain peptide
<400> 34
   Gly Trp Thr Leu Asn Ser Ala Gly Tyr Leu Leu Lys Ile Asn Leu Lys
   1               5                   10                  15
   Ala Leu Ala Ala Leu Ala Lys Lys Ile Leu
               20                  25
<210> 35
   <211> 18
   <212> PRT
   <213> artificial sequence
<220>
   <223> amphiphilic model peptide protein transduction domain peptide
<400> 35
   Lys Leu Ala Leu Lys Leu Ala Leu Lys Ala Leu Lys Ala Ala Leu Lys
   1               5                   10                  15
   Leu Ala
<210> 36
   <211> 11
   <212> PRT
   <213> artificial sequence
<220>
   <223> Polyarginine protein transduction domain peptide
<400> 36
   Arg Arg Arg Arg Arg Arg Arg Arg Arg Arg Arg
   1               5                   10
<210> 37
   <211> 5
   <212> DNA
   <213> artificial sequence
<220>
   <223> P2A initiator sequence
<400> 37
   tcgga 5
<210> 38
   <211> 46
   <212> DNA
   <213> artificial sequence
<220>
   <223> Tagged random primer
<220>
   <221> misc feature
   <222> (41)..(46)
   <223> n is a, c, g, or t
<400> 38
   gactacaagg acgacgacga caaggcttat caatcaatca nnnnnn 46
<210> 39
   <211> 49
   <212> DNA
   <213> artificial sequence
<220>
   <223> Tagged random primer
<220>
   <221> misc feature
   <222> (41)..(49)
   <223> n is a, c, g, or t.
<400> 39
   gactacaagg acgacgacga caaggcttat caatcaatca nnnnnnnnn 49
<210> 40
   <211> 40
   <212> DNA
   <213> artificial sequence
<220>
   <223> Oligonucleotide for preparing expression library
<400> 40
   gagagaattc aggtcagact acaaggacga cgacgacaag 40
<210> 41
   <211> 5562
   <212> DNA
   <213> artificial sequence
<220>
   <223> Vector - pDEATH-Trp
<400> 41
   ctagcgattt tggtcatgag atcagatcaa cttcttttct ttttttttct tttctctctc 60
   ccccgttgtt gtctcaccat atccgcaatg acaaaaaaat gatggaagac actaaaggaa 120
   aaaattaacg acaaagacag caccaacaga tgtcgttgtt ccagagctga tgaggggtat 180
   ctcgaagcac acgaaacttt ttccttcctt cattcacgca cactactctc taatgagcaa 240
   cggtatacgg ccttccttcc agttacttga atttgaaata aaaaaaagtt tgctgtcttg 300
   ctatcaagta taaatagacc tgcaattatt aatcttttgt ttcctcgtca ttgttctcgt 360
   tccctttctt ccttgtttct ttttctgcac aatatttcaa gctataccaa gcatacaatc 420
   aactccaagc ttccccggat cggactacta gcagctgtaa tacgactcac tatagggaat 480
   attaagctca ccatgggtaa gcctatccct aaccctctcc tcggtctcga ttctacacaa 540
   gctatgggtg ctcctccaaa aaagaagaga aaggtagctg aattcgagct cagatctcag 600
   ctgggcccgg taccaattga tgcatcgata ccggtactag tcggaccgca tatgcccggg 660
   cgtaccgcgg ccgctcgagg catgcatcta gagggccgca tcatgtaatt agttatgtca 720
   cgcttacatt cacgccctcc ccccacatcc gctctaaccg aaaaggaagg agttagacaa 780
   cctgaagtct aggtccctat ttattttttt atagttatgt tagtattaag aacgttattt 840
   atatttcaaa tttttctttt ttttctgtac agacgcgtgt acgcatgtaa cattatactg 900
   aaaaccttgc ttgagaaggt tttgggacgc tcgaaggctt taatttgcgg ccctgcatta 960
   atgaatcggc caacgcgcgg ggagaggcgg tttgcgtatt gggcgctctt ccgcttcctc 1020
   gctcactgac tcgctgcgct cggtcgttcg gctgcggcga gcggtatcag ctcactcaaa 1080
   ggcggtaata cggttatcca cagaatcagg ggataacgca ggaaagaaca tgtgagcaaa 1140
   aggccagcaa aagcccagga accgtaaaaa ggccgcgttg ctggcgtttt tccataggct 1200
   ccgcccccct gacgagcatc acaaaaatcg acgctcaagt cagaggtggc gaaacccgac 1260
   aggactataa agataccagg cgtttccccc tggaagctcc ctcgtgcgct ctcctgttcc 1320
   gaccctgccg cttaccggat acctgtccgc ctttctccct tcgggaagcg tggcgctttc 1380
   tcatagctca cgctgtaggt atctcagttc ggtgtaggtc gttcgctcca agctgggctg 1440
   tgtgcacgaa ccccccgttc agcccgaccg ctgcgcctta tccggtaact atcgtcttga 1500
   gtccaacccg gtaagacacg acttatcgcc actggcagca gccactggta acaggattag 1560
   cagagcgagg tatgtaggcg gtgctacaga gttcttgaag tggtggccta actacggcta 1620
   cactagaagg acagtatttg gtatctgcgc tctgctgaag ccagttacct tcggaaaaag 1680
   agttggtagc tcttgatccg gcaaacaaac caccgctggt agcggtggtt tttttgtttg 1740
   caagcagcag attacgcgca gaaaaaaagg atctcaagaa gatcctttga tcttttctac 1800
   ggggtctgac gctcagtgga acgaaaactc acgttaaggg attttggtca tgagattatc 1860
   aaaaaggatc ttcacctaga tccttttaaa ttaaaaatga agttttaaat caatctaaag 1920
   tatatatgag taaacttggt ctgacagtta ccaatgctta atcagtgagg cacctatctc 1980
   agcgatctgt ctatttcgtt catccatagt tgcctgactc cccgtcgtgt agataactac 2040
   gatacgggag cgcttaccat ctggccccag tgctgcaatg ataccgcgag acccacgctc 2100
   accggctcca gatttatcag caataaacca gccagccgga agggccgagc gcagaagtgg 2160
   tcctgcaact ttatccgcct ccatccagtc tattaattgt tgccgggaag ctagagtaag 2220
   tagttcgcca gttaatagtt tgcgcaacgt tgttgccatt gctacaggca tcgtggtgtc 2280
   acgctcgtcg tttggtatgg cttcattcag ctccggttcc caacgatcaa ggcgagttac 2340
   atgatccccc atgttgtgca aaaaagcggt tagctccttc ggtcctccga tcgttgtcag 2400
   aagtaagttg gccgcagtgt tatcactcat ggttatggca gcactgcata attctcttac 2460
   tgtcatgcca tccgtaagat gcttttctgt gactggtgag tactcaacca agtcattctg 2520
   agaatagtgt atgcggcgac cgagttgctc ttgcccggcg tcaacacggg ataataccgc 2580
   gccacatagc agaactttaa aagtgctcat cattggaaaa cgttcttcgg ggcgaaaact 2640
   ctcaaggatc ttaccgctgt tgagatccag ttcgatgtaa cccactcgtg cacccaactg 2700
   atcttcagca tcttttactt tcaccagcgt ttctgggtga gcaaaaacag gaaggcaaaa 2760
   tgccgcaaaa aagggaataa gggcgacacg gaaatgttga atactcatac tcttcctttt 2820
   tcaatattat tgaagcattt atcagggtta ttgtctcatg agcggataca tatttgaatg 2880
   tatttagaaa aataaacaaa taggggttcc gcgcacattt ccccgaaaag tgccacctga 2940
   cgtctaagaa accattatta tcatgacatt aacctataaa aataggcgta tcacgaggcc 3000
   ctttcgtctt caagaaattc ggtcgaaaaa agaaaaggag agggccaaga gggagggcat 3060
   tggtgactat tgagcacgtg agtatacgtg attaagcaca caaaggcagc ttggagtatg 3120
   tctgttatta atttcacagg tagttctggt ccattggtga aagtttgcgg cttgcagagc 3180
   acagaggccg cagaatgtgc tctagattcc gatgctgact tgctgggtat tatatgtgtg 3240
   cccaatagaa agagaacaat tgacccggtt attgcaagga aaatttcaag tcttgtaaaa 3300
   gcatataaaa atagttcagg cactccgaaa tacttggttg gcgtgtttcg taatcaacct 3360
   aaggaggatg ttttggctct ggtcaatgat tacggcattg atatcgtcca actgcacgga 3420
   gatgagtcgt ggcaagaata ccaagagttc ctcggtttgc cagttattaa aagactcgta 3480
   tttccaaaag actgcaacat actactcagt gcagcttcac agaaacctca ttcgtttatt 3540
   cccttgtttg attcagaagc aggtgggaca ggtgaacttt tggattggaa ctcgatttct 3600
   gactgggttg gaaggcaaga gagccccgag agcttacatt ttatgttagc tggtggactg 3660
   acgccagaaa atgttggtga tgcgcttaga ttaaatggcg ttattggtgt tgatgtaagc 3720
   ggaggtgtgg agacaaatgg tgtaaaagac tctaacaaaa tagcaaattt cgtcaaaaat 3780
   gctaagaaat aggttattac tgagtagtat ttatttaagt attgtttgtg cacttgcctg 3840
   cagcttctca atgatattcg aatacgcttt gaggagatac agcctaatat ccgacaaact 3900
   gttttacaga tttacgatcg tacttgttac ccatcattga attttgaaca tccgaacctg 3960
   ggagttttcc ctgaaacaga tagtatattt gaacctgtat aataatatat agtctagcgc 4020
   tttacggaag acaatgtatg tatttcggtt cctggagaaa ctattgcatc tattgcatag 4080
   gtaatcttgc acgtcgcatc cccggttcat tttctgcgtt tccatcttgc acttcaatag 4140
   catatctttg ttaacgaagc atctgtgctt cattttgtag aacaaaaatg caacgcgaga 4200
   gcgctaattt ttcaaacaaa gaatctgagc tgcattttta cagaacagaa atgcaacgcg 4260
   aaagcgctat tttaccaacg aagaatctgt gcttcatttt tgtaaaacaa aaatgcaacg 4320
   cgagagcgct aatttttcaa acaaagaatc tgagctgcat ttttacagaa cagaaatgca 4380
   acgcgagagc gctattttac caacaaagaa tctatacttc ttttttgttc tacaaaaatg 4440
   catcccgaga gcgctatttt tctaacaaag catcttagat tacttttttt ctcctttgtg 4500
   cgctctataa tgcagtctct tgataacttt ttgcactgta ggtccgttaa ggttagaaga 4560
   aggctacttt ggtgtctatt ttctcttcca taaaaaaagc ctgactccac ttcccgcgtt 4620
   tactgattac tagcgaagct gcgggtgcat tttttcaaga taaaggcatc cccgattata 4680
   ttctataccg atgtggattg cgcatacttt gtgaacagaa agtgatagcg ttgatgattc 4740
   ttcattggtc agaaaattat gaacggtttc ttctattttg tctctatata ctacgtatag 4800
   gaaatgttta cattttcgta ttgttttcga ttcactctat gaatagttct tactacaatt 4860
   tttttgtcta aagagtaata ctagagataa acataaaaaa tgtagaggtc gagtttagat 4920
   gcaagttcaa ggagcgaaag gtggatgggt aggttatata gggatatagc acagagatat 4980
   atagcaaaga gatacttttg agcaatgttt gtggaagcgg tattcgcaat gggaagctcc 5040
   accccggttg ataatcagaa aagccccaaa aacaggaaga ttgtataagc aaatatttaa 5100
   attgtaaacg ttaatatttt gttaaaattc gcgttaaatt tttgttaaat cagctcattt 5160
   tttaacgaat agcccgaaat cggcaaaatc ccttataaat caaaagaata gaccgagata 5220
   gggttgagtg ttgttccagt ttccaacaag agtccactat taaagaacgt ggactccaac 5280
   gtcaaagggc gaaaaagggt ctatcagggc gatggcccac tacgtgaacc atcaccctaa 5340
   tcaagttttt tggggtcgag gtgccgtaaa gcagtaaatc ggaagggtaa acggatgccc 5400
   ccatttagag cttgacgggg aaagccggcg aacgtggcga gaaaggaagg gaagaaagcg 5460
   aaaggagcgg gggctagggc ggtgggaagt gtaggggtca cgctgggcgt aaccaccaca 5520
   cccgccgcgc ttaatggggc gctacagggc gcgtggggat ga 5562
<210> 42
   <211> 40
   <212> DNA
   <213> artificial sequence
<220>
   <223> Oligonucleotide for library preparation
<400> 42
   agaggaattc aggtcagact acaaggacga cgacgacaag 40
<210> 43
   <211> 27
   <212> DNA
   <213> artificial sequence
<220>
   <223> Oligonucleotide for library preparation
<400> 43
   cagaagctta aggacgacga cgacaag 27
<210> 44
   <211> 27
   <212> DNA
   <213> artificial sequence
<220>
   <223> Oligonucleotide for library preparation
<400> 44
   caggaattca aggacgacga cgacaag 27
<210> 45
   <211> 28
   <212> DNA
   <213> artificial sequence
<220>
   <223> Oligonucleotide for library preparation
<400> 45
   caggaattcc aaggacgacg acgacaag 28
<210> 46
   <211> 29
   <212> DNA
   <213> artificial sequence
<220>
   <223> Oligonucleotide for library preparation
<400> 46
   caggaattca caaggacgac gacgacaag 29
<210> 47
   <211> 16
   <212> DNA
   <213> artificial sequence
<220>
   <223> Adaptor oligonucleotide
<400> 47
   aattcgaacc ccttcg 16
<210> 48
   <211> 12
   <212> DNA
   <213> artificial sequence
<220>
   <223> Adaptor oligonucleotide
<400> 48
   cgaaggggtt cg 12
<210> 49
   <211> 17
   <212> DNA
   <213> artificial sequence
<220>
   <223> Adaptor oligonucleotide
<400> 49
   aattcgaacc ccttcgc 17
<210> 50
   <211> 13
   <212> DNA
   <213> artificial sequence
<220>
   <223> Adaptor oligonucleotide
<400> 50
   gcgaaggggt tcg 13
<210> 51
   <211> 18
   <212> DNA
   <213> artificial sequence
<220>
   <223> Adaptor oligonucleotide
<400> 51
   aattcgaacc ccttcgcg 18
<210> 52
   <211> 14
   <212> DNA
   <213> artificial sequence
<220>
   <223> Adaptor oligonucleotide
<400> 52
   cgcgaagggg ttcg 14
<210> 53
   <211> 16
   <212> DNA
   <213> artificial sequence
<220>
   <223> Adaptor oligonucleotide
<400> 53
   agctcgaagg ggttcg 16
<210> 54
   <211> 12
   <212> DNA
   <213> artificial sequence
<220>
   <223> Adaptor oligonucleotide
<400> 54
   cgaacccctt cg 12
<210> 55
   <211> 89
   <212> DNA
   <213> artificial sequence
<220>
   <223> Tagged random oligonucleotide
<220>
   <221> misc_feature
   <222> (81)..(89)
   <223> n is a, c, g, or t
<400> 55
   tttcccgaat tgtgagcgga taacaataga aataattttg tttaacttta agaaggagat 60
   atatccatgg actacaaaga nnnnnnnnn 89
<210> 56
   <211> 61
   <212> DNA
   <213> artificial sequence
<220>
   <223> Oligonucleotide for library preparation
<400> 56
   ggggccaagc agtaataata cgagtcacta tagggagacc acaacggttt cccgaattgt 60
   g 61
<210> 57
   <211> 23
   <212> DNA
   <213> artificial sequence
<220>
   <223> Adaptor oligonucleotide
<400> 57
   tttaagcagc tcgatagcag cac 23
<210> 58
   <211> 23
   <212> DNA
   <213> artificial sequence
<220>
   <223> Adaptor oligonucleotide
<400> 58
   gtgctgctat cgagctgctt aaa 23
<210> 59
   <211> 51
   <212> DNA
   <213> artificial sequence
<220>
   <223> Oligonucleotide for library preparation
<400> 59
   agacccgttt agaggcccca aggggttatg gaattcacct ttaagcagct c 51
<210> 60
   <211> 26
   <212> DNA
   <213> artificial sequence
<220>
   <223> Oligonucleotide for amplifying fragment of gene III of M13
<400> 60
   cgtgaaaaaa ttattattcg caattc 26
<210> 61
   <211> 30
   <212> DNA
   <213> artificial sequence
<220>
   <223> Oligonucleotide for amplifying fragment of gene III of M13
<400> 61
   ttaagactcc ttattacgca gtatgttagc 30
<210> 62
   <211> 6482
   <212> DNA
   <213> artificial sequence
<220>
   <223> Vector - pMF4-5

<400> 62
   cgtacccatt atcttagcct aaaaaaacct tctctttgga actttcagta atacgcttaa      60
   ctgctcattg ctatattgaa gtacggatta gaagccgccg agcgggtgac agccctccga     120
   aggaagactc tcctccgtgc gtcctcgtct tcaccggtcg cgttcctgaa acgcagatgt     180
   gcctcgcgcc gcactgctcc gaacaataaa gattctacaa tactagcttt tatggttatg     240
   aagaggaaaa attggcagta acctggcccc acaaaccttc aaatgaacga atcaaattaa     300
   caaccatagg atgataatgc gattagtttt ttagccttat ttctggggta attaatcagc     360
   gaagcgatga tttttgatct attaacagat atataaatgc aaaaactgca taaccacttt     420
   aactaatact ttcaacattt tcggtttgta ttacttctta ttcaaatgta ataaaagtat     480
   caacaaaaaa ttgttaatat acctctatac tttaacgtca aggaggaatt aagcttatgg 540
   gtgctcctcc aaaaaagaag agaaaggtag ctggtatcaa taaagatatc gaggagtgca 600
   atgccatcat tgagcagttt atcgactacc tgcgcaccgg acaggagatg ccgatggaaa 660
   tggcggatca ggcgattaac gtggtgccgg gcatgacgcc gaaaaccatt cttcacgccg 720
   ggccgccgat ccagcctgac tggctgaaat cgaatggttt tcatgaaatt gaagcggatg 780
   ttaacgatac cagcctcttg ctgagtggag atgcctccta cccttatgat gtgccagatt 840
   atgcctctcc cgaattccgt tgtgcaggta ccagagtact gagcggccgc aatctcgaga 900
   agctttggac ttcttcgcca gaggtttggt caagtctcca atcaaggttg tcggcttgtc 960
   taccttgcca gaaatttacg aaaagatgga aaagggtcaa atcgttggta gatacgttgt 1020
   tgacacttct aaataagcga atttcttatg atttatgatt tttattatta aataagttat 1080
   aaaaaaaata agtgtataca aattttaaag tgactcttag gttttaaaac gaaaattctt 1140
   gttcttgagt aactctttcc tgtaggtcag gttgctttct caggtatagc atgaggtcgc 1200
   tcttattgac cacacctcta ccggcatgcc gagcaaatgc ctgcaaatcg ctccccattt 1260
   cacccaattg tagatatgct aactccagca atgagttgat gaatctcggt gtgtatttta 1320
   tgtcctcaga ggacaacacc tgttgtaatc gttcttccac acggatcctc tagagtcgac 1380
   tagcggccgc ttcgacctgc agcaattctg aaccagtcct aaaacgagta aataggaccg 1440
   gcaattcttc aagcaataaa caggaatacc aattattaaa agataactta gtcagatcgt 1500
   acaataaagc tttgaagaaa aatgcgcctt attcaatctt tgctataaaa aatggcccaa 1560
   aatctcacat tggaagacat ttgatgacct catttctttc aatgaagggc ctaacggagt 1620
   tgactaatgt tgtgggaaat tggagcgata agcgtgcttc tgccgtggcc aggacaacgt 1680
   atactcatca gataacagca atacctgatc actacttcgc actagtttct cggtactatg 1740
   catatgatcc aatatcaaag gaaatgatag cattgaagga tgagactaat ccaattgagg 1800
   agtggcagca tatagaacag ctaaagggta gtgctgaagg aagcatacga taccccgcat 1860
   ggaatgggat aatatcacag gaggtactag actacctttc atcctacata aatagacgca 1920
   tataagtacg catttaagca taaacacgca ctatgccgtt cttctcatgt atatatatat 1980
   acaggcaaca cgcagatata ggtgcgacgt gaacagtgag ctgtatgtgc gcagctcgcg 2040
   ttgcattttc ggaagcgctc gttttcggaa acgctttgaa gttcctattc cgaagttcct 2100
   attctctaga aagtatagga acttcagagc gcttttgaaa accaaaagcg ctctgaagac 2160
   gcactttcaa aaaaccaaaa acgcaccgga ctgtaacgag ctactaaaat attgcgaata 2220
   ccgcttccac aaacattgct caaaagtatc tctttgctat atatctctgt gctatatccc 2280
   tatataacct acccatccac ctttcgctcc ttgaacttgc atctaaactc gacctctaca 2340
   ttttttatgt ttatctctag tattactctt tagacaaaaa aattgtagta agaactattc 2400
   atagagtgaa tcgaaaacaa tacgaaaatg taaacatttc ctatacgtag tatatagaga 2460
   caaaatagaa gaaaccgttc ataattttct gaccaatgaa gaatcatcaa cgctatcact 2520
   ttctgttcac aaagtatgcg caatccacat cggtatagaa tataatcggg gatgccttta 2580
   tcttgaaaaa atgcacccgc agcttcgcta gtaatcagta aacgcgggaa gtggagtcag 2640
   gcttttttta tggaagagaa aatagacacc aaagtagcct tcttctaacc ttaacggacc 2700
   tacagtgcaa aaagttatca agagactgca ttatagagcg cacaaaggag aaaaaaagta 2760
   atctaagatg ctttgttaga aaaatagcgc tctcgggatg catttttgta gaacaaaaaa 2820
   gaagtataga ttctttgttg gtaaaatagc gctctcgcgt tgcatttctg ttctgtaaaa 2880
   atgcagctca gattctttgt ttgaaaaatt agcgctctcg cgttgcattt ttgttttaca 2940
   aaaatgaagc acagattctt cgttggtaaa atagcgcttt cgcgttgcat ttctgttctg 3000
   taaaaatgca gctcagattc tttgtttgaa aaattagcgc tctcgcgttg catttttgtt 3060
   ctacaaaatg aagcacagat gcttcgttaa caaagatatg ctattgaagt gcaagatgga 3120
   aacgcagaaa atgaaccggg gatgcgacgt gcaagattac ctatgcaata gatgcaatag 3180
   tttctccagg aaccgaaata catacattgt cttccgtaaa gcgctagact atatattatt 3240
   atacaggttc aaatatacta tctgtttcag ggaaaactcc caggttcgga tgttcaaaat 3300
   tcaatgatgg gtaacaagta cgatcgtaaa tctgtaaaac agtttgtcgg atattaggct 3360
   gtatctcctc aaagcgtatt cgaatatcat tgagaagctg caggcaagtg cacaaacaat 3420
   acttaaataa atactactca gtaataacct atttcttagc atttttgacg aaatttgcta 3480
   ttttgttaga gtcttttaca ccatttgtct ccacacctcc gcttacatca acaccaataa 3540
   cgccatttaa tctaagcgca tcaccaacat tttctggcgt cagtccacca gctaacataa 3600
   aatgtaagct ttcggggctc tcttgccttc caacccagtc agaaatcgag ttccaatcca 3660
   aaagttcacc tgtcccacct gcttctgaat caaacaaggg aataaacgaa tgaggtttct 3720
   gtgaagctgc actgagtagt atgttgcagt cttttggaaa tacgagtctt ttaataactg 3780
   gcaaaccgag gaactcttgg tattcttgcc acgactcatc tccatgcagt tggacgatat 3840
   caatgccgta atcattgacc agagccaaaa catcctcctt aggttgatta cgaaacacgc 3900
   caaccaagta tttcggagtg cctgaactat ttttatatgc ttttacaaga cttgaaattt 3960
   tccttgcaat aaccgggtca attgttctct ttctattggg cacacatata atacccagca 4020
   agtcagcatc ggaatctaga gcacattctg cggcctctgt gctctgcaag ccgcaaactt 4080
   tcaccaatgg accagaacta cctgtgaaat taataacaga catactccaa gctgcctttg 4140
   tgtgcttaat cacgtatact cacgtgctca atagtcacca atgccctccc tcttggccct 4200
   ctccttttct tttttcgacc gaatttcttg aagacgaaag ggcctcgtga tacgcctatt 4260
   tttataggtt aatgtcatga taataatggt ttcttagacg tcaggtggca cttttcgggg 4320
   aaatgtgcgc ggaaccccta tttgtttatt tttctaaata cattcaaata tgtatccgct 4380
   catgagacaa taaccctgat aaatgcttca ataatattga aaaaggaaga gtatgagtat 4440
   tcaacatttc cgtgtcgccc ttattccctt ttttgcggca ttttgccttc ctgtttttgc 4500
   tcacccagaa acgctggtga aagtaaaaga tgctgaagat cagttgggtg cacgagtggg 4560
   ttacatcgaa ctggatctca acagcggtaa gatccttgag agttttcgcc ccgaagaacg 4620
   ttttccaatg atgagcactt ttaaagttct gctatgtggc gcggtattat cccgtgttga 4680
   cgccgggcaa gagcaactcg gtcgccgcat acactattct cagaatgact tggttgagta 4740
   ctcaccagtc acagaaaagc atcttacgga tggcatgaca gtaagagaat tatgcagtgc 4800
   tgccataacc atgagtgata acactgcggc caacttactt ctgacaacga tcggaggacc 4860
   gaaggagcta accgcttttt tgcacaacat gggggatcat gtaactcgcc ttgatcgttg 4920
   ggaaccggag ctgaatgaag ccataccaaa cgacgagcgt gacaccacga tgcctgtagc 4980
   aatggcaaca acgttgcgca aactattaac tggcgaacta cttactctag cttcccggca 5040
   acaattaata gactggatgg aggcggataa agttgcagga ccacttctgc gctcggccct 5100
   tccggctggc tggtttattg ctgataaatc tggagccggt gagcgtgggt ctcgcggtat 5160
   cattgcagca ctggggccag atggtaagcc ctcccgtatc gtagttatct acacgacggg 5220
   gagtcaggca actatggatg aacgaaatag acagatcgct gagataggtg cctcactgat 5280
   taagcattgg taactgtcag accaagttta ctcatatata ctttagattg atttaaaact 5340
   tcatttttaa tttaaaagga tctaggtgaa gatccttttt gataatctca tgaccaaaat 5400
   cccttaacgt gagttttcgt tccactgagc gtcagacccc gtagaaaaga tcaaaggatc 5460
   ttcttgagat cctttttttc tgcgcgtaat ctgctgcttg caaacaaaaa aaccaccgct 5520
   accagcggtg gtttgtttgc cggatcaaga gctaccaact ctttttccga aggtaactgg 5580
   cttcagcaga gcgcagatac caaatactgt ccttctagtg tagccgtagt taggccacca 5640
   cttcaagaac tctgtagcac cgcctacata cctcgctctg ctaatcctgt taccagtggc 5700
   tgctgccagt ggcgataagt cgtgtcttac cgggttggac tcaagacgat agttaccgga 5760
   taaggcgcag cggtcgggct gaacgggggg ttcgtgcaca cagcccagct tggagcgaac 5820
   gacctacacc gaactgagat acctacagcg tgagctatga gaaagcgcca cgcttcccga 5880
   agggagaaag gcggacaggt atccggtaag cggcagggtc ggaacaggag agcgcacgag 5940
   ggagcttcca gggggaaacg cctggtatct ttatagtcct gtcgggtttc gccacctctg 6000
   acttgagcgt cgatttttgt gatgctcgtc aggggggcgg agcctatgga aaaacgccag 6060
   caacgcggcc tttttacggt tcctggcctt ttgctggcct tttgctcaca tgttctttcc 6120
   tgcgttatcc cctgattctg tggataaccg tattaccgcc tttgagtgag ctgataccgc 6180
   tcgccgcagc cgaacgaccg agcgcagcga gtcagtgagc gaggaagcgg aagagcgccc 6240
   aatacgcaaa ccgcctctcc ccgcgcgttg gccgattcat taatgcagct ggcacgacag 6300
   gtttcccgac tggaaagcgg gcagtgagcg caacgcaatt aatgtgagtt agctcactca 6360
   ttaggcaccc caggctttac actttatgct tccggctcgt atgttgtgtg gaattgtgag 6420
   cggataacaa tttcacacag gaaacagcta tgacatgatt acgaattaat tcgagctcgg 6480
   ta 6482
<210> 63
   <211> 7551
   <212> DNA
   <213> artificial sequence
<220>
   <223> Vector - pJFK
<400> 63
<210> 64
   <211> 7308
   <212> DNA
   <213> artificial sequence
<220>
   <223> Vector - pDD
<400> 64
   cttgaatttt caaaaattct tacttttttt ttggatggac gcaaagaagt ttaataatca 60
   tattacatgg cattaccacc atatacatat ccatatacat atccatatct aatcttactt 120
   atatgttgtg gaaatgtaaa gagccccatt atcttagcct aaaaaaacct tctctttgga 180
   actttcagta atacgcttaa ctgctcattg ctatattgaa gtacggatta gaagccgccg 240
   agcgggtgac agccctccga aggaagactc tcctccgtgc gtcctcgtct tcaccggtcg 300
   cgttcctgaa acgcagatgt gcctcgcgcc gcactgctcc gaacaataaa gattctacaa 360
   tactagcttt tatggttatg aagaggaaaa attggcagta acctggcccc acaaaccttc 420
   aaatgaacga atcaaattaa caaccatagg atgataatgc gattagtttt ttagccttat 480
   ttctggggta attaatcagc gaagcgatga tttttgatct attaacagat atataaatgc 540
   aaaaactgca taaccacttt aactaatact ttcaacattt tcggtttgta ttacttctta 600
   ttcaaatgta ataaaagtat caacaaaaaa ttgttaatat acctctatac tttaacgtca 660
   aggagaaaaa accccggatc aagggtgcga tatgaaagcg ttaacggcca ggcaacaaga 720
   ggtgtttgat ctcatccgtg atcacatcag ccagacaggt atgccgccga cgcgtgcgga 780
   aatcgcgcag cgtttggggt tccgttcccc aaacgcggct gaagaacatc tgaaggcgct 840
   ggcacgcaaa ggcgttattg aaattgtttc cggcgcatca cgcgggattc gtctgttgca 900
   ggaagaggaa gaagggttgc cgctggtagg tcgtgtggct gccggtgaac cacttctggc 960
   gcaacagcat attgaaggtc attatcaggt cgatccttcc ttattcaagc cgaatgctga 1020
   tttcctgctg cgcgtcagcg ggatgtcgat gaaagatatc ggcattatgg atggtgactt 1080
   gctggcagtg cataaaactc aggatgtacg taacggtcag gtcgttgtcg cacgtattga 1140
   tgacgaagtt accgttaagc gcctgaaaaa acagggcaat aaagtcgaac tgttgccaga 1200
   aaatagcgag tttaaaccaa ttgtcgtaga tcttcgtcag cagagcttca ccattgaagg 1260
   gctggcggtt ggggttattc gcaacggcga ctggctggaa ttcccgggga tccgtcgacc 1320
   atggcggccg ctcgagtcga cctgcagcca agctaattcc gggcgaattt cttatgattt 1380
   atgattttta ttattaaata agttataaaa aaaataagtg tatacaaatt ttaaagtgac 1440
   tcttaggttt taaaacgaaa attcttgttc ttgagtaact ctttcctgta ggtcaggttg 1500
   ctttctcagg tatagcatga ggtcgctctt attgaccaca cctctaccgg catgccgagc 1560
   aaatgcctgc aaatcgctcc ccatttcacc caattgtaga tatgctaact ccagcaatga 1620
   gttgatgaat ctcggtgtgt attttatgtc ctcagaggac aacacctgtt gtaatccgtc 1680
   cgagctccaa ttcgccctat agtgagtcgt attacaattc actggccgtc gttttacaac 1740
   gtcgtgactg ggaaaaccct ggcgttaccc aacttaatcg ccttgcagca catccccctt 1800
   tcgccagctg gcgtaatagc gaagaggccc gcaccgatcg cccttcccaa cagttgcgca 1860
   gcctgaatgg cgaatggcgc gacgcgccct gtagcggcgc attaagcgcg gcgggtgtgg 1920
   tggttacgcg cagcgtgacc gctacacttg ccagcgccct agcgcccgct cctttcgctt 1980
   tcttcccttc ctttctcgcc acgttcgccg gctttccccg tcaagctcta aatcgggggc 2040
   tccctttagg gttccgattt agtgctttac ggcacctcga ccccaaaaaa cttgattagg 2100
   gtgatggttc acgtagtggg ccatcgccct gatagacggt ttttcgccct ttgacgttgg 2160
   agtccacgtt ctttaatagt ggactcttgt tccaaactgg aacaacactc aaccctatct 2220
   cggtctattc ttttgattta taagggattt tgccgatttc ggcctattgg ttaaaaaatg 2280
   agctgattta acaaaaattt aacgcgaatt ttaacaaaat attaacgttt acaatttcct 2340
   gatgcggtat tttctcctta cgcatctgtg cggtatttca caccgcatat gatccgtcga 2400
   gttcaagaga aaaaaaaaga aaaagcaaaa agaaaaaagg aaagcgcgcc tcgttcagaa 2460
   tgacacgtat agaatgatgc attaccttgt catcttcagt atcatactgt tcgtatacat 2520
   acttactgac attcataggt atacatatat acacatgtat atatatcgta tgctgcagct 2580
   ttaaataatc ggtgtcacta cataagaaca cctttggtgg agggaacatc gttggtacca 2640
   ttgggcgagg tggcttctct tatggcaacc gcaagagcct tgaacgcact ctcactacgg 2700
   tgatgatcat tcttgcctcg cagacaatca acgtggaggg taattctgct agcctctgca 2760
   aagctttcaa gaaaatgcgg gatcatctcg caagagagat ctcctacttt ctccctttgc 2820
   aaaccaagtt cgacaactgc gtacggcctg ttcgaaagat ctaccaccgc tctggaaagt 2880
   gcctcatcca aaggcgcaaa tcctgatcca aaccttttta ctccacgcgc cagtagggcc 2940
   tctttaaaag cttgaccgag agcaatcccg cagtcttcag tggtgtgatg gtcgtctatg 3000
   tgtaagtcac caatgcactc aacgattagc gaccagccgg aatgcttggc cagagcatgt 3060
   atcatatggt ccagaaaccc tatacctgtg tggacgttaa tcacttgcga ttgtgtggcc 3120
   tgttctgcta ctgcttctgc ctctttttct gggaagatcg agtgctctat cgctagggga 3180
   ccacccttta aagagatcgc aatctgaatc ttggtttcat ttgtaatacg ctttactagg 3240
   gctttctgct ctgtcatctt tgccttcgtt tatcttgcct gctcattttt tagtatattc 3300
   ttcgaagaaa tcacattact ttatataatg tataattcat tatgtgataa tgccaatcgc 3360
   taagaaaaaa aaagagtcat ccgctaggtg gaaaaaaaaa aatgaaaatc attaccgagg 3420
   cataaaaaaa tatagagtgt actagaggag gccaagagta atagaaaaag aaaattgcgg 3480
   gaaaggactg tgttatgact tccctgacta atgccgtgtt caaacgatac ctggcagtga 3540
   ctcctagcgc tcaccaagct cttaaaacgg aattatggtg cactctcagt acaatctgct 3600
   ctgatgccgc atagttaagc cagccccgac acccgccaac acccgctgac gcgccctgac 3660
   gggcttgtct gctcccggca tccgcttaca gacaagctgt gaccgtctcc gggagctgca 3720
   tgtgtcagag gttttcaccg tcatcaccga aacgcgcgag acgaaagggc ctcgtgatac 3780
   gcctattttt ataggttaat gtcatgataa taatggtttc ttaggacgga tcgcttgcct 3840
   gtaacttaca cgcgcctcgt atcttttaat gatggaataa tttgggaatt tactctgtgt 3900
   ttatttattt ttatgttttg tatttggatt ttagaaagta aataaagaag gtagaagagt 3960
   tacggaatga agaaaaaaaa ataaacaaag gtttaaaaaa tttcaacaaa aagcgtactt 4020
   tacatatata tttattagac aagaaaagca gattaaatag atatacattc gattaacgat 4080
   aagtaaaatg taaaatcaca ggattttcgt gtgtggtctt ctacacagac aagatgaaac 4140
   aattcggcat taatacctga gagcaggaag agcaagataa aaggtagtat ttgttggcga 4200
   tccccctaga gtcttttaca tcttcggaaa acaaaaacta ttttttcttt aatttctttt 4260
   tttactttct atttttaatt tatatattta tattaaaaaa tttaaattat aattattttt 4320
   atagcacgtg atgaaaagga cccaggtggc acttttcggg gaaatgtgcg cggaacccct 4380
   atttgtttat ttttctaaat acattcaaat atgtatccgc tcatgagaca ataaccctga 4440
   taaatgcttc aataaattgg tcacccggcc agcgacatgg aggcccagaa taccctcctt 4500
   gacagtcttg acgtgcgcag ctcaggggca tgatgtgact gtcgcccgta catttagccc 4560
   atacatcccc atgtataatc atttgcatcc atacattttg atggccgcac ggcgcgaagc 4620
   aaaaattacg gctcctcgct gcagacctgc gagcagggaa acgctcccct cacagacgcg 4680
   ttgaattgtc cccacgccgc gcccctgtag agaaatataa aaggttagga tttgccactg 4740
   aggttcttct ttcatatact tccttttaaa atcttgctag gatacagttc tcacatcaca 4800
   tccgaacata aacaaccatg ggtaaggaaa agactcacgt ttcgaggccg cgattaaatt 4860
   ccaacatgga tgctgattta tatgggtata aatgggctcg cgataatgtc gggcaatcag 4920
   gtgcgacaat ctatcgattg tatgggaagc ccgatgcgcc agagttgttt ctgaaacatg 4980
   gcaaaggtag cgttgccaat gatgttacag atgagatggt cagactaaac tggctgacgg 5040
   aatttatgcc tcttccgacc atcaagcatt ttatccgtac tcctgatgat gcatggttac 5100
   tcaccactgc gatccccggc aaaacagcat tccaggtatt agaagaatat cctgattcag 5160
   gtgaaaatat tgttgatgcg ctggcagtgt tcctgcgccg gttgcattcg attcctgttt 5220
   gtaattgtcc ttttaacagc gatcgcgtat ttcgtctcgc tcaggcgcaa tcacgaatga 5280
   ataacggttt ggttgatgcg agtgattttg atgacgagcg taatggctgg cctgttgaac 5340
   aagtctggaa agaaatgcat aagcttttgc cattctcacc ggattcagtc gtcactcatg 5400
   gtgatttctc acttgataac cttatttttg acgaggggaa attaataggt tgtattgatg 5460
   ttggacgagt cggaatcgca gaccgatacc aggatcttgc catcctatgg aactgcctcg 5520
   gtgagttttc tccttcatta cagaaacggc tttttcaaaa atatggtatt gataatcctg 5580
   atatgaataa attgcagttt catttgatgc tcgatgagtt tttctaatca gtcctcggag 5640
   atccgtcccc cttttccttt gtcgatatca tgtaattagt tatgtcacgc ttacattcac 5700
   gccctccccc cacatccgct ctaaccgaaa aggaaggagt tagacaacct gaagtctagg 5760
   tccctattta tttttttata gttatgttag tattaagaac gttatttata tttcaaattt 5820
   ttcttttttt tctgtacaga cgcgtgtacg catgtaacat tatactgaaa accttgcttg 5880
   agaaggtttt gggacgctcg aaggctttaa tttgcaagct ggggtctcgc ggtcggtatc 5940
   attgcagcac tggggccaga tggtaagccc tcccgtatcg tagttatcta cacgacgggc 6000
   agtcaggcaa ctatggatga acgaaataga cagatcgctg agataggtgc ctcactgatt 6060
   aagcattggt aactgtcaga ccaagtttac tcatatatac tttagattga tttaaaactt 6120
   catttttaat ttaaaaggat ctaggtgaag atcctttttg ataatctcat gaccaaaatc 6180
   ccttaacgtg agttttcgtt ccactgagcg tcagaccccg tagaaaagat caaaggatct 6240
   tcttgagatc ctttttttct gcgcgtaatc tgctgcttgc aaacaaaaaa accaccgcta 6300
   ccagcggtgg tttgtttgcc ggatcaagag ctaccaactc tttttccgaa ggtaactggc 6360
   ttcagcagag cgcagatacc aaatactgtc cttctagtgt agccgtagtt aggccaccac 6420
   ttcaagaact ctgtagcacc gcctacatac ctcgctctgc taatcctgtt accagtggct 6480
   gctgccagtg gcgataagtc gtgtcttacc gggttggact caagacgata gttaccggat 6540
   aaggcgcagc ggtcgggctg aacggggggt tcgtgcacac agcccagctt ggagcgaacg 6600
   acctacaccg aactgagata cctacagcgt gagcattgag aaagcgccac gcttcccgaa 6660
   gggagaaagg cggacaggta tccggtaagc ggcagggtcg gaacaggaga gcgcacgagg 6720
   gagcttccag gggggaacgc ctggtatctt tatagtcctg tcgggtttcg ccacctctga 6780
   cttgagcgtc gatttttgtg atgctcgtca ggggggccga gcctatggaa aaacgccagc 6840
   aacgcggcct ttttacggtt cctggccttt tgctggcctt ttgctcacat gttctttcct 6900
   gcgttatccc ctgattctgt ggataaccgt attaccgcct ttgagtgagc tgataccgct   6960
   cgccgcagcc gaacgaccga gcgcagcgag tcagtgagcg aggaagcgga agagcgccca   7020
   atacgcaaac cgcctctccc cgcgcgttgg ccgattcatt aatgcagctg gcacgacagg   7080
   tttcccgact ggaaagcggg cagtgagcgc aacgcaatta atgtgagtta gctcactcat   7140
   taggcacccc aggctttaca ctttatgctt ccggctcgta tgttgtgtgg aattgtgagc   7200
   ggataacaat ttcacacagg aaacagctat gaccatgatt accccaagct cgaaattaac   7260
   cctcactaaa gggaacaaaa gctggtaccg ggccccccct cgaaattc                 7308

<210> 65
   <211> 254
   <212> DNA
   <213> artificial sequence
<220>
   <223> c-Jun self dimerization inhibitory peptide (SP4) with vector sequence
<220>
   <221> CDS
   <222> (1)..(249)
<400> 65
   agg tca gac tac aag gac gac gac gac aag gct tat caa tcc atg ttc      48
   Arg Ser Asp Tyr Lys Asp Asp Asp Asp Lys Ala Tyr Gln Ser Met Phe
   1               5                   10                  15
   tgt gaa agc cgc ttc ctg gac aat gca tct gcc cct gcc atg agg aat      96
   Cys Glu Ser Arg Phe Leu Asp Asn Ala Ser Ala Pro Ala Met Arg Asn
               20                  25                  30
   gca aag agg cgt tcc gaa gag cgg gtc ctg tgt aac ctg aca gtt cat     144
   Ala Lys Arg Arg Ser Glu Glu Arg Val Leu Cys Asn Leu Thr Val His
           35                  40                  45
   aga aaa cac att ttg cac aag atc aca agt gat gac ctc ttc cgg acg     192
   Arg Lys His Ile Leu His Lys Ile Thr Ser Asp Asp Leu Phe Arg Thr
       50                  55                  60
   gcc ttc tgc aga aat ccg ttt atc ttt tat ggc cac aag atg atg cgc     240
   Ala Phe Cys Arg Asn Pro Phe Ile Phe Tyr Gly His Lys Met Met Arg
   65                  70                  75                  80
   atg att gat tgata                                                   254
   Met Ile Asp
<210> 66
   <211> 83
   <212> PRT
   <213> artificial sequence
<220>
   <223> Synthetic Construct
<400> 66
   Arg Ser Asp Tyr Lys Asp Asp Asp Asp Lys Ala Tyr Gln Ser Met Phe
   1               5                   10                  15
   Cys Glu Ser Arg Phe Leu Asp Asn Ala Ser Ala Pro Ala Met Arg Asn
               20                  25                  30
   Ala Lys Arg Arg Ser Glu Glu Arg Val Leu Cys Asn Leu Thr Val His
           35                  40                  45
   Arg Lys His Ile Leu His Lys Ile Thr Ser Asp Asp Leu Phe Arg Thr
       50                  55                  60
   Ala Phe Cys Arg Asn Pro Phe Ile Phe Tyr Gly His Lys Met Met Arg
   65                  70                  75                  80
   Met Ile Asp
<210> 67
   <211> 224
   <212> DNA
   <213> artificial sequence
<220>
   <223> c-Jun self dimerization inhibitory peptide (SP4)
<220>
   <221> CDS
   <222> (1)..(222)
<400> 67
   gct tat caa tcc atg ttc tgt gaa agc cgc ttc ctg gac aat gca tct        48
   Ala Tyr Gln Ser Met Phe Cys Glu Ser Arg Phe Leu Asp Asn Ala Ser
   1               5                   10                  15
   gcc cct gcc atg agg aat gca aag agg cgt tcc gaa gag cgg gtc ctg        96
   Ala Pro Ala Met Arg Asn Ala Lys Arg Arg Ser Glu Glu Arg Val Leu
               20                  25                  30
   tgt aac ctg aca gtt cat aga aaa cac att ttg cac aag atc aca agt       144
   Cys Asn Leu Thr Val His Arg Lys His Ile Leu His Lys Ile Thr Ser
           35                  40                  45
   gat gac ctc ttc cgg acg gcc ttc tgc aga aat ccg ttt atc ttt tat       192
   Asp Asp Leu Phe Arg Thr Ala Phe Cys Arg Asn Pro Phe Ile Phe Tyr
       50                  55                  60
   ggc cac aag atg atg cgc atg att gat tga ta                            224
   Gly His Lys Met Met Arg Met Ile Asp
   65                  70
<210> 68
   <211> 73
   <212> PRT
   <213> artificial sequence
<220>
   <223> Synthetic Construct
<400> 68
   Ala Tyr Gln Ser Met Phe Cys Glu Ser Arg Phe Leu Asp Asn Ala Ser
   1               5                   10                  15
   Ala Pro Ala Met Arg Asn Ala Lys Arg Arg Ser Glu Glu Arg Val Leu
               20                  25                  30
   Cys Asn Leu Thr Val His Arg Lys His Ile Leu His Lys Ile Thr Ser
           35                  40                  45
   Asp Asp Leu Phe Arg Thr Ala Phe Cys Arg Asn Pro Phe Ile Phe Tyr
       50                  55                  60
   Gly His Lys Met Met Arg Met Ile Asp
   65                  70
<210> 69
   <211> 38
   <212> DNA
   <213> artificial sequence
<220>
   <223> c-Jun self dimerization inhibitory peptide (SP5) with vector sequence
<220>
   <221> CDS
   <222> (1)..(36)
<400> 69
   agg tca gac tac aag gac gac gac gac aag gct tat ca                   38
   Arg Ser Asp Tyr Lys Asp Asp Asp Asp Lys Ala Tyr
   1               5                   10
<210> 70
   <211> 12
   <212> PRT
   <213> artificial sequence
<220>
   <223> Synthetic Construct
<400> 70
   Arg Ser Asp Tyr Lys Asp Asp Asp Asp Lys Ala Tyr
   1               5                   10
<210> 71
   <211> 8
   <212> DNA
   <213> artificial sequence
<220>
   <223> c-Jun self dimerization inhibitory peptide (SP5)
<220>
   <221> CDS
   <222> (1)..(6)
<400> 71
   gct tat ca                                                                8
   Ala Tyr
   1
<210> 72
   <211> 2
   <212> PRT
   <213> artificial sequence
<220>
   <223> c-Jun self dimerization inhibitory peptide (SP24)
<400> 72
   Ala Tyr
   1
<210> 73
   <211> 240
   <212> DNA
   <213> artificial sequence
<220>
   <223> c-Jun self dimerization inhibitory peptide (SP6) with vector sequence
<220>
   <221> CDS
   <222> (1)..(54)
<220>
   <221> misc_feature
   <222> (56)..(56)
   <223> n is a, c, g, or t
<220>
   <221> misc_feature
   <222> (84)..(84)
   <223> n is a, c, g, or t
<220>
   <221> misc_feature
   <222> (130)..(130)
   <223> n is a, c, g, or t
<220>
   <221> misc_feature
   <222> (163)..(163)
   <223> n is a, c, g, or t
<220>
   <221> misc_feature
   <222> (178)..(178)
   <223> n is a, c, g, or t
<220>
   <221> misc_feature
   <222> (184)..(184)
   <223> n is a, c, g, or t
<220>
   <221> misc_feature
   <222> (205)..(205)
   <223> n is a, c, g, or t
<220>
   <221> misc_feature
   <222> (207)..(208)
   <223> n is a, c, g, or t
<220>
   <221> misc_feature
   <222> (234)..(234)
   <223> n is a, c, g, or t
<220>
   <221> misc_feature
   <222> (239)..(239)
   <223> n is a, c, g, or t
<400> 73
   agg tca gac tac aag gac gac gac gac aag gct tat caa tct aag aga       48
   Arg Ser Asp Tyr Lys Asp Asp Asp Asp Lys Ala Tyr Gln Ser Lys Arg
   1               5                   10                  15
   ctt tga anatagtgtc caattggcat gtgccacagn taaccaactt actgcaatca       104
   Leu
   ttacccgtga caccaaagac tttgtntgag agtcctttgc ctatttactc ccctgcggna    164
   cgaagtgatt gatnataagn ttgtcgcgcg tcgtccctgt ngnnctgacc tgcctaccaa    224
   gttgatggcn tcgcnt                                                    240
<210> 74
   <211> 17
   <212> PRT
   <213> artificial sequence
<220>
   <223> Synthetic Construct
<400> 74
   Arg Ser Asp Tyr Lys Asp Asp Asp Asp Lys Ala Tyr Gln Ser Lys Arg
   1 5 10 15
   Leu
<210> 75
   <211> 210
   <212> DNA
   <213> artificial sequence
<220>
   <223> c-Jun self dimerization inhibitory peptide (SP6)
<220>
   <221> CDS
   <222> (1)..(24)
<220>
   <221> misc_feature
   <222> (26)..(26)
   <223> n is a, c, g, or t
<220>
   <221> misc_feature
   <222> (54)..(54)
   <223> n is a, c, g, or t
<220>
   <221> misc_feature
   <222> (100)..(100)
   <223> n is a, c, g, or t
<220>
   <221> misc_feature
   <222> (133)..(133)
   <223> n is a, c, g, or t
<220>
   <221> misc_feature
   <222> (148)..(148)
   <223> n is a, c, g, or t
<220>
   <221> misc_feature
   <222> (154)..(154)
   <223> n is a, c, g, or t
<220>
   <221> misc_feature
   <222> (175)..(175)
   <223> n is a, c, g, or t
<220>
   <221> misc feature
   <222> (177)..(178)
   <223> n is a, c, g, or t
<220>
   <221> misc_feature
   <222> (204)..(204)
   <223> n is a, c, g, or t
<220>
   <221> misc_feature
   <222> (209)..(209)
   <223> n is a, c, g, or t
<400> 75
   gct tat caa tct aag aga ctt tga anatagtgtc caattggcat gtgccacagn      54
   Ala Tyr Gln Ser Lys Arg Leu
   1               5
   taaccaactt actgcaatca ttacccgtga caccaaagac tttgtntgag agtcctttgc    114
   ctatttactc ccctgcggna cgaagtgatt gatnataagn ttgtcgcgcg tcgtccctgt    174
   ngnnctgacc tgcctaccaa gttgatggcn tcgcnt                              210
<210> 76
   <211> 7
   <212> PRT
   <213> artificial sequence
<220>
   <223> Synthetic Construct
<400> 76
   Ala Tyr Gln Ser Lys Arg Leu
   1               5
<210> 77
   <211> 387
   <212> DNA
   <213> artificial sequence
<220>
   <223> c-Jun self dimerization inhibitory peptide (SP8) with vector sequence
<220>
   <221> CDS
   <222> (1)..(120)
<400> 77
   agg tca gac tac aag gac gac gac gac aag gct tat caa tca atc ata       48
   Arg Ser Asp Tyr Lys Asp Asp Asp Asp Lys Ala Tyr Gln Ser Ile Ile
   1               5                   10                  15
   gct aat gaa gag gag agg gag aaa aat ttt gca tcc agc aaa aag gac       96
   Ala Asn Glu Glu Glu Arg Glu Lys Asn Phe Ala Ser Ser Lys Lys Asp
               20                  25                  30
   gga tcc tat acc gat ctc ttg tga aacgaatgaa aaatagctct taaatccaga     150
   Gly Ser Tyr Thr Asp Leu Leu
           35
   tatgtgtaag aatgcctcca tgattcgtgg atcagaggat tgatagacca gagcttgtcg    210
   tcgtcgtcct tgtagtctga cctggtacca attgatgcat cgataccggt actagtcgga    270
   ccgcatatgc ccgggcgtac cgcggccgct cgaggcatgc atctagaggg ccgcatcatg    330
   taattagtta tgtcacgctt acattcacgc cctcccccca catccgctct aaccgaa       387
<210> 78
   <211> 39
   <212> PRT
   <213> artificial sequence
<220>
   <223> Synthetic Construct
<400> 78
   Arg Ser Asp Tyr Lys Asp Asp Asp Asp Lys Ala Tyr Gln Ser Ile Ile
   1               5                   10                  15
   Ala Asn Glu Glu Glu Arg Glu Lys Asn Phe Ala Ser Ser Lys Lys Asp
               20                  25                  30
   Gly Ser Tyr Thr Asp Leu Leu
           35
<210> 79
   <211> 357
   <212> DNA
   <213> artificial sequence
<220>
   <223> c-Jun self dimerization inhibitory peptide (SP8)
<220>
   <221> CDS
   <222> (1)..(90)
<400> 79
   gct tat caa tca atc ata gct aat gaa gag gag agg gag aaa aat ttt      48
   Ala Tyr Gln Ser Ile Ile Ala Asn Glu Glu Glu Arg Glu Lys Asn Phe
   1               5                   10                  15
   gca tcc agc aaa aag gac gga tcc tat acc gat ctc ttg tga              90
   Ala Ser Ser Lys Lys Asp Gly Ser Tyr Thr Asp Leu Leu
               20                  25
   aacgaatgaa aaatagctct taaatccaga tatgtgtaag aatgcctcca tgattcgtgg   150
   atcagaggat tgatagacca gagcttgtcg tcgtcgtcct tgtagtctga cctggtacca   210
   attgatgcat cgataccggt actagtcgga ccgcatatgc ccgggcgtac cgcggccgct   270
   cgaggcatgc atctagaggg ccgcatcatg taattagtta tgtcacgctt acattcacgc   330
   cctcccccca catccgctct aaccgaa                                       357
<210> 80
   <211> 29
   <212> PRT
   <213> artificial sequence
<220>
   <223> Synthetic Construct
<400> 80
   Ala Tyr Gln Ser Ile Ile Ala Asn Glu Glu Glu Arg Glu Lys Asn Phe
   1               5                   10                  15
   Ala Ser Ser Lys Lys Asp Gly Ser Tyr Thr Asp Leu Leu
               20                  25
<210> 81
   <211> 193
   <212> DNA
   <213> artificial sequence
<220>
   <223> c-Jun self dimerization inhibitory peptide (SP12) with vector sequence
<220>
   <221> CDS
   <222> (1)..(123)
<400> 81
   agg tca gac tac aag gac gac gac gac aag gct tat caa gag tcc acc      48
   Arg Ser Asp Tyr Lys Asp Asp Asp Asp Lys Ala Tyr Gln Glu Ser Thr
   1               5                   10                  15
   aaa gcg ctg gtg gaa ggt ggc gcg gat ctg atc ctg att gaa acc gtt      96
   Lys Ala Leu Val Glu Gly Gly Ala Asp Leu Ile Leu Ile Glu Thr Val
               20                  25                  30
   ctt gtc gtc gtc gtc ctt gta gtc tga cctggtacca attgatgcat           143
   Leu Val Val Val Val Leu Val Val
           35                  40
   cgataccggt actagtcgga ccgcatatgc ccgggcgtac cgcggccgct              193
<210> 82
   <211> 40
   <212> PRT
   <213> artificial sequence
<220>
   <223> Synthetic Construct
<400> 82
   Arg Ser Asp Tyr Lys Asp Asp Asp Asp Lys Ala Tyr Gln Glu Ser Thr
   1               5                   10                  15
   Lys Ala Leu Val Glu Gly Gly Ala Asp Leu Ile Leu Ile Glu Thr Val
               20                  25                  30
   Leu Val Val Val Val Leu Val Val
           35                  40
<210> 83
   <211> 163
   <212> DNA
   <213> artificial sequence
<220>
   <223> c-Jun self dimerization inhibitory peptide (SP12)
<220>
   <221> CDS
   <222> (1)..(93)
<400> 83
   gct tat caa gag tcc acc aaa gcg ctg gtg gaa ggt ggc gcg gat ctg       48
   Ala Tyr Gln Glu Ser Thr Lys Ala Leu Val Glu Gly Gly Ala Asp Leu
   1               5                   10                  15
   atc ctg att gaa acc gtt ctt gtc gtc gtc gtc ctt gta gtc tga           93
   Ile Leu Ile Glu Thr Val Leu Val Val Val Val Leu Val Val
               20                  25                  30
   cctggtacca attgatgcat cgataccggt actagtcgga ccgcatatgc ccgggcgtac    153
   cgcggccgct                                                           163
<210> 84
   <211> 30
   <212> PRT
   <213> artificial sequence
<220>
   <223> Synthetic Construct
<400> 84
   Ala Tyr Gln Glu Ser Thr Lys Ala Leu Val Glu Gly Gly Ala Asp Leu
   1               5                   10                  15
   Ile Leu Ile Glu Thr Val Leu Val Val Val Val Leu Val Val
               20                  25                  30
<210> 85
   <211> 273
   <212> DNA
   <213> artificial sequence
<220>
   <223> c-Jun self dimerization inhibitory peptide (SP15) with vector sequence
<220>
   <221> CDS
   <222> (1)..(273)
<220>
   <221> misc_feature
   <222> (247)..(247)
   <223> n is a, c, g, or t
<400> 85
   agg tca gac tac aag gac gac gac gac aag act tat caa tca atc aat      48
   Arg Ser Asp Tyr Lys Asp Asp Asp Asp Lys Thr Tyr Gln Ser Ile Asn
   1               5                   10                  15
   ggc cca gaa aat aaa gtg aaa atg tat ttt ttg aat gat tta aat ttc      96
   Gly Pro Glu Asn Lys Val Lys Met Tyr Phe Leu Asn Asp Leu Asn Phe
               20                  25                  30
   tct aga cgc gat gct gga ttt aaa gca aga aaa gat gca cgg gac att     144
   Ser Arg Arg Asp Ala Gly Phe Lys Ala Arg Lys Asp Ala Arg Asp Ile
           35                  40                  45
   gct tca gat tat gaa aac att tct gtt gtt aac att cct cta tgg ggt     192
   Ala Ser Asp Tyr Glu Asn Ile Ser Val Val Asn Ile Pro Leu Trp Gly
       50                  55                  60
   gga gta gtc cag aga att att agt tct gtt aag ctt agt aca ttt ctc     240
   Gly Val Val Gln Arg Ile Ile Ser Ser Val Lys Leu Ser Thr Phe Leu
   65                  70                  75                  80
   tgc ggt ntt gaa aat aaa gat gtt tta att ttc                         273
   Cys Gly Xaa Glu Asn Lys Asp Val Leu Ile Phe
                   85                  90
<210> 86
   <211> 91
   <212> PRT
   <213> artificial sequence
<220>
   <221> misc feature
   <222> (83)..(83)
   <223> The 'Xaa' at location 83 stands for Ile, Val, Leu, or Phe.
<220>
   <223> Synthetic Construct
<400> 86
   Arg Ser Asp Tyr Lys Asp Asp Asp Asp Lys Thr Tyr Gln Ser Ile Asn
   1               5                   10                  15
   Gly Pro Glu Asn Lys Val Lys Met Tyr Phe Leu Asn Asp Leu Asn Phe
               20                  25                  30
   Ser Arg Arg Asp Ala Gly Phe Lys Ala Arg Lys Asp Ala Arg Asp Ile
           35                  40                  45
   Ala Ser Asp Tyr Glu Asn Ile Ser Val Val Asn Ile Pro Leu Trp Gly
       50                  55                  60
   Gly Val Val Gln Arg Ile Ile Ser Ser Val Lys Leu Ser Thr Phe Leu
   65                  70                  75                  80
   Cys Gly Xaa Glu Asn Lys Asp Val Leu Ile Phe
                   85                  90
<210> 87
   <211> 243
   <212> DNA
   <213> artificial sequence
<220>
   <223> c-Jun self dimerization inhibitory peptide (SP15)
<220>
   <221> CDS
   <222> (1)..(243)
<220>
   <221> misc_feature
   <222> (217)..(217)
   <223> n is a, c, g, or t
<400> 87
   act tat caa tca atc aat ggc cca gaa aat aaa gtg aaa atg tat ttt       48
   Thr Tyr Gln Ser Ile Asn Gly Pro Glu Asn Lys Val Lys Met Tyr Phe
   1               5                   10                  15
   ttg aat gat tta aat ttc tct aga cgc gat gct gga ttt aaa gca aga       96
   Leu Asn Asp Leu Asn Phe Ser Arg Arg Asp Ala Gly Phe Lys Ala Arg
               20                  25                  30
   aaa gat gca cgg gac att gct tca gat tat gaa aac att tct gtt gtt      144
   Lys Asp Ala Arg Asp Ile Ala Ser Asp Tyr Glu Asn Ile Ser Val Val
           35                  40                  45
   aac att cct cta tgg ggt gga gta gtc cag aga att att agt tct gtt      192
   Asn Ile Pro Leu Trp Gly Gly Val Val Gln Arg Ile Ile Ser Ser Val
       50                  55                  60
   aag ctt agt aca ttt ctc tgc ggt ntt gaa aat aaa gat gtt tta att      240
   Lys Leu Ser Thr Phe Leu Cys Gly Xaa Glu Asn Lys Asp Val Leu Ile
   65                  70                  75                  80
   ttc                                                                  243
   Phe
<210> 88
   <211> 81
   <212> PRT
   <213> artificial sequence
<220>
   <221> misc feature
   <222> (73)..(73)
   <223> The 'Xaa' at location 73 stands for Ile, Val, Leu, or Phe.
<220>
   <223> Synthetic Construct
<400> 88
   Thr Tyr Gln Ser Ile Asn Gly Pro Glu Asn Lys Val Lys Met Tyr Phe
   1               5                   10                  15
   Leu Asn Asp Leu Asn Phe Ser Arg Arg Asp Ala Gly Phe Lys Ala Arg
               20                  25                  30
   Lys Asp Ala Arg Asp Ile Ala Ser Asp Tyr Glu Asn Ile Ser Val Val
           35                  40                  45
   Asn Ile Pro Leu Trp Gly Gly Val Val Gln Arg Ile Ile Ser Ser Val
       50                  55                  60
   Lys Leu Ser Thr Phe Leu Cys Gly Xaa Glu Asn Lys Asp Val Leu Ile
   65                  70                  75                  80
   Phe
<210> 89
   <211> 320
   <212> DNA
   <213> artificial sequence
<220>
   <223> c-Jun self dimerization inhibitory peptide (SP18) with vector sequence
<220>
   <221> CDS
   <222> (1)..(54)
<400> 89
   agg tca gac tac aag gac gac gac gac aag gct tat caa tca atc ata       48
   Arg Ser Asp Tyr Lys Asp Asp Asp Asp Lys Ala Tyr Gln Ser Ile Ile
   1               5                   10                  15
   cat tga ctacaaggac gacgacgaca aggcttatca atcaatcaat ggggccctgc       104
   His
   tgaagattca acgttcttcg cctctccttg cttttgaata tcttcgatta tgatttgttc    164
   acattcaatg cctaatagcc gtttttcttg tcgtcgtcgt ccttgtagtc tgacctggta    224
   ccaattgatg catcgatacc ggtactagtc ggaccgcata tgcggccgct cgagcatgca    284
   tctagagggc cctattctat agtgtcacct aaatgc                              320
<210> 90
   <211> 17
   <212> PRT
   <213> artificial sequence
<220>
   <223> Synthetic Construct
<400> 90
   Arg Ser Asp Tyr Lys Asp Asp Asp Asp Lys Ala Tyr Gln Ser Ile Ile
   1               5                   10                  15
   His
<210> 91
   <211> 290
   <212> DNA
   <213> artificial sequence
<220>
   <223> c-Jun self dimerization inhibitory peptide (SP18)
<220>
   <221> CDS
   <222> (1)..(24)
<400> 91
   gct tat caa tca atc ata cat tga ctacaaggac gacgacgaca aggcttatca      54
   Ala Tyr Gln Ser Ile Ile His
   1               5
   atcaatcaat ggggccctgc tgaagattca acgttcttcg cctctccttg cttttgaata    114
   tcttcgatta tgatttgttc acattcaatg cctaatagcc gtttttcttg tcgtcgtcgt    174
   ccttgtagtc tgacctggta ccaattgatg catcgatacc ggtactagtc ggaccgcata    234
   tgcggccgct cgagcatgca tctagagggc cctattctat agtgtcacct aaatgc        290
<210> 92
   <211> 7
   <212> PRT
   <213> artificial sequence
<220>
   <223> Synthetic Construct
<400> 92
   Ala Tyr Gln Ser Ile Ile His
   1               5
<210> 93
   <211> 211
   <212> DNA
   <213> artificial sequence
<220>
   <223> c-Jun self dimerization inhibitory peptide (SP20) with vector sequence
<220>
   <221> CDS
   <222> (1)..(90)
<400> 93
   agg tca gac tac aag gac gac gac gac aag att tat tca tca att cta       48
   Arg Ser Asp Tyr Lys Asp Asp Asp Asp Lys Ile Tyr Ser Ser Ile Leu
   1               5                   10                  15
   tgg ggg aca aaa tgg tgc gtt tta ttg gta ata aca ccc taa               90
   Trp Gly Thr Lys Trp Cys Val Leu Leu Val Ile Thr Pro
               20                  25
   tctatagaga tggtgattga ttgataagcc ttctcgtcgt cgtccttgta gtctgacctg    150
   gtaccaattg atgcatcgat accggtacta gtcggaccgc atatgcccgg gcgtaccgcg    210
   g                                                                    211
<210> 94
   <211> 29
   <212> PRT
   <213> artificial sequence
<220>
   <223> Synthetic Construct
<400> 94
   Arg Ser Asp Tyr Lys Asp Asp Asp Asp Lys Ile Tyr Ser Ser Ile Leu
   1               5                   10                  15
   Trp Gly Thr Lys Trp Cys Val Leu Leu Val Ile Thr Pro
               20                  25
<210> 95
   <211> 181
   <212> DNA
   <213> artificial sequence
<220>
   <223> c-Jun self dimerization inhibitory peptide (SP20)
<220>
   <221> CDS
   <222> (1)..(60)
<400> 95
   att tat tca tca att cta tgg ggg aca aaa tgg tgc gtt tta ttg gta      48
   Ile Tyr Ser Ser Ile Leu Trp Gly Thr Lys Trp Cys Val Leu Leu Val
   1               5                   10                  15
   ata aca ccc taa tctatagaga tggtgattga ttgataagcc ttctcgtcgt         100
   Ile Thr Pro
   cgtccttgta gtctgacctg gtaccaattg atgcatcgat accggtacta gtcggaccgc   160
   atatgcccgg gcgtaccgcg g                                              181
<210> 96
   <211> 19
   <212> PRT
   <213> artificial sequence
<220>
   <223> Synthetic Construct
<400> 96
   Ile Tyr Ser Ser Ile Leu Trp Gly Thr Lys Trp Cys Val Leu Leu Val
   1               5                   10                  15
   Ile Thr Pro
<210> 97
   <211> 120
   <212> DNA
   <213> artificial sequence
<220>
   <223> c-Jun self dimerization inhibitory peptide (SP21) with vector
<220>
   <221> CDS
   <222> (1)..(105)
<220>
   <221> misc_feature
   <222> (53)..(53)
   <223> n is a, c, g, or t
<400> 97
   agg tca gac tac aag gac gac gac gac aag atc att att tat att ttc       48
   Arg Ser Asp Tyr Lys Asp Asp Asp Asp Lys Ile Ile Ile Tyr Ile Phe
   1               5                   10                  15
   ctt anc atc tct aat agc atc aaa aac atc ttc gac aat atg ggt aaa       96
   Leu Xaa Ile Ser Asn Ser Ile Lys Asn Ile Phe Asp Asn Met Gly Lys
               20                  25                  30
   atc aga taa ctccatcata tcaag                                          120
   Ile Arg
<210> 98
   <211> 34
   <212> PRT
   <213> artificial sequence
<220>
   <221> misc feature
   <222> (18)..(18)
   <223> The 'Xaa' at location 18 stands for Asn, Ser, Thr, or Ile.
<220>
   <223> Synthetic Construct
<400> 98
   Arg Ser Asp Tyr Lys Asp Asp Asp Asp Lys Ile Ile Ile Tyr Ile Phe
   1               5                   10                  15
   Leu Xaa Ile Ser Asn Ser Ile Lys Asn Ile Phe Asp Asn Met Gly Lys
               20                  25                  30
   Ile Arg
<210> 99
   <211> 90
   <212> DNA
   <213> artificial sequence
<220>
   <223> c-Jun self dimerization inhibitory peptide (SP21)
<220>
   <221> CDS
   <222> (1)..(75)
<220>
   <221> misc feature
   <222> (23)..(23)
   <223> n is a, c, g, or t
<400> 99
   atc att att tat att ttc ctt anc atc tct aat agc atc aaa aac atc       48
   Ile Ile Ile Tyr Ile Phe Leu Xaa Ile Ser Asn Ser Ile Lys Asn Ile
   1               5                   10                  15
   ttc gac aat atg ggt aaa atc aga taa ctccatcata tcaag                  90
   Phe Asp Asn Met Gly Lys Ile Arg
               20
<210> 100
   <211> 24
   <212> PRT
   <213> artificial sequence
<220>
   <221> misc_feature
   <222> (8)..(8)
   <223> The 'Xaa' at location 8 stands for Asn, Ser, Thr, or Ile.
<220>
   <223> Synthetic Construct
<400> 100
   Ile Ile Ile Tyr Ile Phe Leu Xaa Ile Ser Asn Ser Ile Lys Asn Ile
   1               5                   10                  15
   Phe Asp Asn Met Gly Lys Ile Arg
               20
<210> 101
   <211> 143
   <212> DNA
   <213> artificial sequence
<220>
   <223> c-Jun self dimerization inhibitory peptide (SP22) with vector
<220>
   <221> CDS
   <222> (1)..(141)
<400> 101
   agg tca gac tac aag gac gac gac gac aag aag gac tcc ata cgg cgg        48
   Arg Ser Asp Tyr Lys Asp Asp Asp Asp Lys Lys Asp Ser Ile Arg Arg
   1               5                   10                  15
   cgc ggc gag aat att tcc tcg cag gaa gtc gag gcc gtc ctc atg tcg        96
   Arg Gly Glu Asn Ile Ser Ser Gln Glu Val Glu Ala Val Leu Met Ser
               20                  25                  30
   cat ccc gaa gtc gtc aat gcc gcg gtc tac ccc gta cgc ggc gat ct        143
   His Pro Glu Val Val Asn Ala Ala Val Tyr Pro Val Arg Gly Asp
           35                  40                  45
<210> 102
   <211> 47
   <212> PRT
   <213> artificial sequence
<220>
   <223> Synthetic Construct
<400> 102
   Arg Ser Asp Tyr Lys Asp Asp Asp Asp Lys Lys Asp Ser Ile Arg Arg
   1               5                   10                  15
   Arg Gly Glu Asn Ile Ser Ser Gln Glu Val Glu Ala Val Leu Met Ser
               20                  25                  30
   His Pro Glu Val Val Asn Ala Ala Val Tyr Pro Val Arg Gly Asp
           35                  40                  45
<210> 103
   <211> 113
   <212> DNA
   <213> artificial sequence
<220>
   <223> c-Jun self dimerization inhibitory peptide (SP22)
<220>
   <221> CDS
   <222> (1)..(111)
<400> 103
   aag gac tcc ata cgg cgg cgc ggc gag aat att tcc tcg cag gaa gtc        48
   Lys Asp Ser Ile Arg Arg Arg Gly Glu Asn Ile Ser Ser Gln Glu Val
   1               5                   10                  15
   gag gcc gtc ctc atg tcg cat ccc gaa gtc gtc aat gcc gcg gtc tac        96
   Glu Ala Val Leu Met Ser His Pro Glu Val Val Asn Ala Ala Val Tyr
               20                  25                  30
   ccc gta cgc ggc gat ct                                                113
   Pro Val Arg Gly Asp
           35
<210> 104
   <211> 37
   <212> PRT
   <213> artificial sequence
<220>
   <223> Synthetic Construct
<400> 104
   Lys Asp Ser Ile Arg Arg Arg Gly Glu Asn Ile Ser Ser Gln Glu Val
   1               5                   10                  15
   Glu Ala Val Leu Met Ser His Pro Glu Val Val Asn Ala Ala Val Tyr
               20                  25                  30
   Pro Val Arg Gly Asp
           35
<210> 105
   <211> 192
   <212> DNA
   <213> artificial sequence
<220>
   <223> c-Jun self dimerization inhibitory peptide (SP24) with vector sequence
<220>
   <221> CDS
   <222> (1)..(111)
<400> 105
   agg tca gac tac aag gac gac gac gac aag cta tat caa tca cta ctc       48
   Arg Ser Asp Tyr Lys Asp Asp Asp Asp Lys Leu Tyr Gln Ser Leu Leu
   1               5                   10                  15
   act gct acc aaa gaa ttg ctt ttt gtc gcg cct gta gca aaa gca ttc       96
   Thr Ala Thr Lys Glu Leu Leu Phe Val Ala Pro Val Ala Lys Ala Phe
               20                  25                  30
   aca tcg tgt gat tga ttgataagcc ttctcgtcgt cgtccttgta gtctgacctg      151
   Thr Ser Cys Asp
           35
   gtaccaattg atgcatcgat accggtacta gtcggaccgc a                         192
<210> 106
   <211> 36
   <212> PRT
   <213> artificial sequence
<220>
   <223> Synthetic Construct
<400> 106
   Arg Ser Asp Tyr Lys Asp Asp Asp Asp Lys Leu Tyr Gln Ser Leu Leu
   1               5                   10                  15
   Thr Ala Thr Lys Glu Leu Leu Phe Val Ala Pro Val Ala Lys Ala Phe
               20                  25                  30
   Thr Ser Cys Asp
           35
<210> 107
   <211> 162
   <212> DNA
   <213> artificial sequence
<220>
   <223> c-Jun self dimerization inhibitory peptide (SP24)
<220>
   <221> CDS
   <222> (1)..(81)
<400> 107
   cta tat caa tca cta ctc act gct acc aaa gaa ttg ctt ttt gtc gcg       48
   Leu Tyr Gln Ser Leu Leu Thr Ala Thr Lys Glu Leu Leu Phe Val Ala
   1               5                   10                  15
   cct gta gca aaa gca ttc aca tcg tgt gat tga ttgataagcc ttctcgtcgt    101
   Pro Val Ala Lys Ala Phe Thr Ser Cys Asp
               20                  25
   cgtccttgta gtctgacctg gtaccaattg atgcatcgat accggtacta gtcggaccgc    161
   a                                                                    162
<210> 108
   <211> 26
   <212> PRT
   <213> artificial sequence
<220>
   <223> Synthetic Construct
<400> 108
   Leu Tyr Gln Ser Leu Leu Thr Ala Thr Lys Glu Leu Leu Phe Val Ala
   1               5                   10                  15
   Pro Val Ala Lys Ala Phe Thr Ser Cys Asp
               20                  25
<210> 109
   <211> 236
   <212> DNA
   <213> artificial sequence
<220>
   <223> c-Jun self dimerization inhibitory peptide (SP29) with vector sequence

<220>
   <221> CDS
   <222> (1)..(72)
<400> 109
   agg tca gac tac aag gac tac tgg tgg ggt cct ttc att ccc ccc ttt      48
   Arg Ser Asp Tyr Lys Asp Tyr Trp Trp Gly Pro Phe Ile Pro Pro Phe
   1               5                   10                  15
   ttc tgg aga cta aat aaa atc tga tattatatcg actctagagt cgcggccgca    102
   Phe Trp Arg Leu Asn Lys Ile
               20
   attcttaatt aattcattac ttgtacagct cgtccatgcc gagagtgatc ccggcggcgg   162
   tcacgaactc cagcaggacc atgtgatcgc gcttctcgtt ggggtctttg ctcagggcgg   222
   actgggtgct cagg                                                     236
<210> 110
   <211> 23
   <212> PRT
   <213> artificial sequence
<220>
   <223> Synthetic Construct
<400> 110
   Arg Ser Asp Tyr Lys Asp Tyr Trp Trp Gly Pro Phe Ile Pro Pro Phe
   1               5                   10                  15
   Phe Trp Arg Leu Asn Lys Ile
               20
<210> 111
   <211> 218
   <212> DNA
   <213> artificial sequence
<220>
   <223> c-Jun self dimerization inhibitory peptide (SP29)
<220>
   <221> CDS
   <222> (1)..(42)
<400> 111
   tac tgg tgg ggt cct ttc att ccc ccc ttt ttc tgg aga cta              42
   Tyr Trp Trp Gly Pro Phe Ile Pro Pro Phe Phe Trp Arg Leu
   1               5                   10
   aataaaatct gatattatat cgactctaga gtcgcggccg caattcttaa ttaattcatt   102
   acttgtacag ctcgtccatg ccgagagtga tcccggcggc ggtcacgaac tccagcagga   162
   ccatgtgatc gcgcttctcg ttggggtctt tgctcagggc ggactgggtg ctcagg       218
<210> 112
   <211> 14
   <212> PRT
   <213> artificial sequence
<220>
   <223> Synthetic Construct
<400> 112
   Tyr Trp Trp Gly Pro Phe Ile Pro Pro Phe Phe Trp Arg Leu
   1               5                   10
<210> 113
   <211> 412
   <212> DNA
   <213> artificial sequence
<220>
   <223> c-Jun self dimerization inhibitory peptide (SP30) with vector sequence
<220>
   <221> CDS
   <222> (1)..(186)
<400> 113
   agg tca gac tac aag gac gac gac gac aag gtc tac gcc tac ttc ggt       48
   Arg Ser Asp Tyr Lys Asp Asp Asp Asp Lys Val Tyr Ala Tyr Phe Gly
   1               5                   10                  15
   aac acc ggc gat gtt gtc gaa gta ggc gta gac ctt gta ggt atc gcc       96
   Asn Thr Gly Asp Val Val Glu Val Gly Val Asp Leu Val Gly Ile Ala
               20                  25                  30
   ggc gtt gcc cac gct cag gcc gct gac ccg cag ggc cag cag caa cag      144
   Gly Val Ala His Ala Gln Ala Ala Asp Pro Gln Gly Gln Gln Gln Gln
           35                  40                  45
   ggc cag cag gcc ggc cag gag gaa cag gcc gac acc gat tga              186
   Gly Gln Gln Ala Gly Gln Glu Glu Gln Ala Asp Thr Asp
       50                  55                  60
   ttgataagcc ttgtcgtcgt cgtccttgta gtctgacctg gtaccaattg atgcatcgat    246
   accggtacta gtcggaccgc atatgcccgg gcgtaccgcg gccgctcgag gcatgcatct    306
   agagggccgc atcatgtaat tagttatgtc acgcttacat tcacgccctc cccccacatc    366
   cgctctaacc gaaaaggaag gagttagaca acctgaagtc taggtc                   412
<210> 114
   <211> 61
   <212> PRT
   <213> artificial sequence
<220>
   <223> Synthetic Construct
<400> 114
   Arg Ser Asp Tyr Lys Asp Asp Asp Asp Lys Val Tyr Ala Tyr Phe Gly
   1               5                   10                  15
   Asn Thr Gly Asp Val Val Glu Val Gly Val Asp Leu Val Gly Ile Ala
               20                  25                  30
   Gly Val Ala His Ala Gln Ala Ala Asp Pro Gln Gly Gln Gln Gln Gln
           35                  40                  45
   Gly Gln Gln Ala Gly Gln Glu Glu Gln Ala Asp Thr Asp
       50                  55                  60
<210> 115
   <211> 382
   <212> DNA
   <213> artificial sequence
<220>
   <223> c-Jun self dimerization inhibitory peptide (SP30)
<220>
   <221> CDS
   <222> (1)..(156)
<400> 115
   gtc tac gcc tac ttc ggt aac acc ggc gat gtt gtc gaa gta ggc gta       48
   Val Tyr Ala Tyr Phe Gly Asn Thr Gly Asp Val Val Glu Val Gly Val
   1               5                   10                  15
   gac ctt gta ggt atc gcc ggc gtt gcc cac gct cag gcc gct gac ccg       96
   Asp Leu Val Gly Ile Ala Gly Val Ala His Ala Gln Ala Ala Asp Pro
               20                  25                  30
   cag ggc cag cag caa cag ggc cag cag gcc ggc cag gag gaa cag gcc      144
   Gln Gly Gln Gln Gln Gln Gly Gln Gln Ala Gly Gln Glu Glu Gln Ala
           35                  40                  45
   gac acc gat tga ttgataagcc ttgtcgtcgt cgtccttgta gtctgacctg          196
   Asp Thr Asp
       50
   gtaccaattg atgcatcgat accggtacta gtcggaccgc atatgcccgg gcgtaccgcg    256
   gccgctcgag gcatgcatct agagggccgc atcatgtaat tagttatgtc acgcttacat    316
   tcacgccctc cccccacatc cgctctaacc gaaaaggaag gagttagaca acctgaagtc    376
   taggtc                                                               382
<210> 116
   <211> 51
   <212> PRT
   <213> artificial sequence
<220>
   <223> Synthetic Construct
<400> 116
   Val Tyr Ala Tyr Phe Gly Asn Thr Gly Asp Val Val Glu Val Gly Val
   1               5                   10                  15
   Asp Leu Val Gly Ile Ala Gly Val Ala His Ala Gln Ala Ala Asp Pro
               20                  25                  30
   Gln Gly Gln Gln Gln Gln Gly Gln Gln Ala Gly Gln Glu Glu Gln Ala
           35                  40                  45
   Asp Thr Asp
       50
<210> 117
   <211> 213
   <212> DNA
   <213> artificial sequence
<220>
   <223> c-Jun self dimerization inhibitory peptide (SP32) with vector sequence
<220>
   <221> CDS
   <222> (1)..(66)
<400> 117
   agg tca gac tac aag gac gac gac gac aat acc ccc cac tcc tcc gat      48
   Arg Ser Asp Tyr Lys Asp Asp Asp Asp Asn Thr Pro His Ser Ser Asp
   1               5                   10                  15
   ggc cac aat aat ccc taa aatctcagtg tttcctccag tttttgctag             96
   Gly His Asn Asn Pro
               20
   aatcataggc tggtaaatta cttcagtgat tccttctaca aagctaaaca atgataactg   156
   attgattgat aagccttgtc gtcgtcgtcc ttgtagtctg acctggtacc aattgat      213
<210> 118
   <211> 21
   <212> PRT
   <213> artificial sequence
<220>
   <223> Synthetic Construct
<400> 118
   Arg Ser Asp Tyr Lys Asp Asp Asp Asp Asn Thr Pro His Ser Ser Asp
   1               5                   10                  15
   Gly His Asn Asn Pro
               20
<210> 119
   <211> 186
   <212> DNA
   <213> artificial sequence
<220>
   <223> c-Jun self dimerization inhibitory peptide (SP32)
<220>
   <221> CDS
   <222> (1)..(39)
<400> 119
   aat acc ccc cac tcc tcc gat ggc cac aat aat ccc taa aatctcagtg        49
   Asn Thr Pro His Ser Ser Asp Gly His Asn Asn Pro
   1               5                   10
   tttcctccag tttttgctag aatcataggc tggtaaatta cttcagtgat tccttctaca    109
   aagctaaaca atgataactg attgattgat aagccttgtc gtcgtcgtcc ttgtagtctg    169
   acctggtacc aattgat                                                   186
<210> 120
   <211> 12
   <212> PRT
   <213> artificial sequence
<220>
   <223> Synthetic Construct
<400> 120
   Asn Thr Pro His Ser Ser Asp Gly His Asn Asn Pro
   1               5                   10
<210> 121
   <211> 205
   <212> DNA
   <213> artificial sequence
<220>
   <223> c-Jun self dimerization inhibitory peptide (SP33) with vector sequence
<220>
   <221> CDS
   <222> (1)..(60)
<220>
   <221> misc_feature
   <222> (192)..(192)
   <223> n is a, c, g, or t
<400> 121
   agg tca gac tac aag gac gac gac gac aag gct tat caa tca atc aaa       48
   Arg Ser Asp Tyr Lys Asp Asp Asp Asp Lys Ala Tyr Gln Ser Ile Lys
   1               5                   10                  15
   tgg cca atg taa attgtcggtg cgccaggaaa gagcgtcggt ttgtgtttgt           100
   Trp Pro Met
   cgatgatttt aagtgtttcg agcggatcaa acttaggaag aagaatcatt taacacctgt    160
   tacagaaggg cttgtcgtcg tcgtccttgt antctgacct gaatt                     205
<210> 122
   <211> 19
   <212> PRT
   <213> artificial sequence
<220>
   <223> Synthetic Construct
<400> 122
   Arg Ser Asp Tyr Lys Asp Asp Asp Asp Lys Ala Tyr Gln Ser Ile Lys
   1               5                   10                  15
   Trp Pro Met
<210> 123
   <211> 175
   <212> DNA
   <213> artificial sequence
<220>
   <223> c-Jun self dimerization inhibitory peptide (SP33)
<220>
   <221> CDS
   <222> (1)..(30)
<220>
   <221> misc_feature
   <222> (162)..(162)
   <223> n is a, c, g, or t
<400> 123
   gct tat caa tca atc aaa tgg cca atg taa attgtcggtg cgccaggaaa         50
   Ala Tyr Gln Ser Ile Lys Trp Pro Met
   1               5
   gagcgtcggt ttgtgtttgt cgatgatttt aagtgtttcg agcggatcaa acttaggaag    110
   aagaatcatt taacacctgt tacagaaggg cttgtcgtcg tcgtccttgt antctgacct    170
   gaatt                                                                 175
<210> 124
   <211> 9
   <212> PRT
   <213> artificial sequence
<220>
   <223> Synthetic Construct
<400> 124
   Ala Tyr Gln Ser Ile Lys Trp Pro Met
   1               5
<210> 125
   <211> 225
   <212> DNA
   <213> artificial sequence
<220>
   <223> c-Jun self dimerization inhibitory peptide (SP34) with vector sequence
<220>
   <221> CDS
   <222> (1)..(120)
<400> 125
   agg tca gac tac aag gac gac gac gac aag gct tat caa tca ata aat      48
   Arg Ser Asp Tyr Lys Asp Asp Asp Asp Lys Ala Tyr Gln Ser Ile Asn
   1               5                   10                  15
   tcg tca cca gta ttg cct gaa aat agt caa gaa tta tca ctt cac tta      96
   Ser Ser Pro Val Leu Pro Glu Asn Ser Gln Glu Leu Ser Leu His Leu
               20                  25                  30
   aag caa cac gta aca aaa tca tga aagaatatat caaaagtaga tgaattagca    150
   Lys Gln His Val Thr Lys Ser
           35
   agaaaattac aagaagaaga taaaataaag ggtgtagaag aaaacaataa agatgaatta   210
   atgcagggtg atgat                                                    225
<210> 126
   <211> 39
   <212> PRT
   <213> artificial sequence
<220>
   <223> Synthetic Construct
<400> 126
   Arg Ser Asp Tyr Lys Asp Asp Asp Asp Lys Ala Tyr Gln Ser Ile Asn
   1               5                   10                  15
   Ser Ser Pro Val Leu Pro Glu Asn Ser Gln Glu Leu Ser Leu His Leu
               20                  25                  30
   Lys Gln His Val Thr Lys Ser
           35
<210> 127
   <211> 195
   <212> DNA
   <213> artificial sequence
<220>
   <223> c-Jun self dimerization inhibitory peptide (SP34)
<220>
   <221> CDS
   <222> (1)..(90)
<400> 127
   gct tat caa tca ata aat tcg tca cca gta ttg cct gaa aat agt caa       48
   Ala Tyr Gln Ser Ile Asn Ser Ser Pro Val Leu Pro Glu Asn Ser Gln
   1               5                   10                  15
   gaa tta tca ctt cac tta aag caa cac gta aca aaa tca tga               90
   Glu Leu Ser Leu His Leu Lys Gln His Val Thr Lys Ser
               20                  25
   aagaatatat caaaagtaga tgaattagca agaaaattac aagaagaaga taaaataaag    150
   ggtgtagaag aaaacaataa agatgaatta atgcagggtg atgat                    195
<210> 128
   <211> 29
   <212> PRT
   <213> artificial sequence
<220>
   <223> Synthetic Construct
<400> 128
   Ala Tyr Gln Ser Ile Asn Ser Ser Pro Val Leu Pro Glu Asn Ser Gln
   1               5                   10                  15
   Glu Leu Ser Leu His Leu Lys Gln His Val Thr Lys Ser
               20                  25
<210> 129
   <211> 245
   <212> DNA
   <213> artificial sequence
<220>
   <223> c-Jun self dimerization inhibitory peptide (SP35) with vector sequence
<220>
   <221> CDS
   <222> (1)..(87)
<220>
   <221> misc_feature
   <222> (234)..(234)
   <223> n is a, c, g, or t
<400> 129
   agg tca gac tac aag gac gac gac gac aag gct tat caa tca atc cgg      48
   Arg Ser Asp Tyr Lys Asp Asp Asp Asp Lys Ala Tyr Gln Ser Ile Arg
   1               5                   10                  15
   tct gga ggg ata gag tct agt tcg aaa agg gag agg taa gggtgggaat       97
   Ser Gly Gly Ile Glu Ser Ser Ser Lys Arg Glu Arg
               20                  25
   gaccctaagg acttacaatc caaacgaaac cttcttctct attcttcacg agtttgtgaa   157
   gttccttaag aggaggagac tacttcaaga ggccatagac ttgtcgtcgt cgtccttgta   217
   gtctgacctg gtaccanttg atgcatcg                                      245
<210> 130
   <211> 28
   <212> PRT
   <213> artificial sequence
<220>
   <223> Synthetic Construct
<400> 130
   Arg Ser Asp Tyr Lys Asp Asp Asp Asp Lys Ala Tyr Gln Ser Ile Arg
   1               5                   10                  15
   Ser Gly Gly Ile Glu Ser Ser Ser Lys Arg Glu Arg
               20                  25
<210> 131
   <211> 215
   <212> DNA
   <213> artificial sequence
<220>
   <223> c-Jun self dimerization inhibitory peptide (SP35)
<220>
   <221> CDS
   <222> (1)..(57)
<220>
   <221> misc_feature
   <222> (204)..(204)
   <223> n is a, c, g, or t
<400> 131
   gct tat caa tca atc cgg tct gga ggg ata gag tct agt tcg aaa agg        48
   Ala Tyr Gln Ser Ile Arg Ser Gly Gly Ile Glu Ser Ser Ser Lys Arg
   1               5                   10                  15
   gag agg taa gggtgggaat gaccctaagg acttacaatc caaacgaaac                97
   Glu Arg
   cttcttctct attcttcacg agtttgtgaa gttccttaag aggaggagac tacttcaaga    157
   ggccatagac ttgtcgtcgt cgtccttgta gtctgacctg gtaccanttg atgcatcg      215
<210> 132
   <211> 18
   <212> PRT
   <213> artificial sequence
<220>
   <223> Synthetic Construct
<400> 132
   Ala Tyr Gln Ser Ile Arg Ser Gly Gly Ile Glu Ser Ser Ser Lys Arg
   1               5                   10                  15
   Glu Arg
<210> 133
   <211> 117
   <212> DNA
   <213> artificial sequence
<220>
   <223> c-Jun self dimerization inhibitory peptide (SP36) with vector sequence
<220>
   <221> CDS
   <222> (1)..(78)
<400> 133
   agg tca gac tac aag gac gac gac gac aag gga cta caa gga cga cga        48
   Arg Ser Asp Tyr Lys Asp Asp Asp Asp Lys Gly Leu Gln Gly Arg Arg
   1               5                   10                  15
   cga caa ggt tat caa tca atc aag cca tga ttgatctccg atatatgaat          98
   Arg Gln Gly Tyr Gln Ser Ile Lys Pro
               20                  25
   tcaggtcaga ctacaagga                                                  117
<210> 134
   <211> 25
   <212> PRT
   <213> artificial sequence
<220>
   <223> Synthetic Construct
<400> 134
   Arg Ser Asp Tyr Lys Asp Asp Asp Asp Lys Gly Leu Gln Gly Arg Arg
   1               5                   10                  15
   Arg Gln Gly Tyr Gln Ser Ile Lys Pro
               20                  25
<210> 135
   <211> 87
   <212> DNA
   <213> artificial sequence
<220>
   <223> c-Jun self dimerization inhibitory peptide (SP36)
<220>
   <221> CDS
   <222> (1)..(48)
<400> 135
   gga cta caa gga cga cga cga caa ggt tat caa tca atc aag cca tga       48
   Gly Leu Gln Gly Arg Arg Arg Gln Gly Tyr Gln Ser Ile Lys Pro
   1               5                   10                  15
   ttgatctccg atatatgaat tcaggtcaga ctacaagga                            87
<210> 136
   <211> 15
   <212> PRT
   <213> artificial sequence
<220>
   <223> Synthetic Construct
<400> 136
   Gly Leu Gln Gly Arg Arg Arg Gln Gly Tyr Gln Ser Ile Lys Pro
   1               5                   10                  15
<210> 137
   <211> 220
   <212> DNA
   <213> artificial sequence
<220>
   <223> c-Jun self dimerization inhibitory peptide (SP45) with vector sequence
<220>
   <221> CDS
   <222> (1)..(219)
<220>
   <221> misc_feature
   <222> (184)..(184)
   <223> n is a, c, g, or t
<400> 137
   agg tca gac tac aag gac gac gac gac aag gct tat caa tca atc ggc      48
   Arg Ser Asp Tyr Lys Asp Asp Asp Asp Lys Ala Tyr Gln Ser Ile Gly
   1               5                   10                  15
   agc atc tgg aac agc tgc caa tgc atg agt ttc tgg tgc gca ttc gtg      96
   Ser Ile Trp Asn Ser Cys Gln Cys Met Ser Phe Trp Cys Ala Phe Val
               20                  25                  30
   cgc agc tgt tat ggg cct ggg cgc ggc tgg atg aag ccg aag cgt cgg     144
   Arg Ser Cys Tyr Gly Pro Gly Arg Gly Trp Met Lys Pro Lys Arg Arg
           35                  40                  45
   cgc gta ccg gga ttg aag tct tgt cgt cgt cgt cct tgt ngt ctg acc     192
   Arg Val Pro Gly Leu Lys Ser Cys Arg Arg Arg Pro Cys Xaa Leu Thr
       50                  55                  60
   tgg tac caa ttg atg cat cga tac cgg t                               220
   Trp Tyr Gln Leu Met His Arg Tyr Arg
   65                  70
<210> 138
   <211> 73
   <212> PRT
   <213> artificial sequence
<220>
   <221> misc feature
   <222> (62)..(62)
   <223> The 'Xaa' at location 62 stands for Ser, Gly, Arg, or Cys.
<220>
   <223> Synthetic Construct
<400> 138
   Arg Ser Asp Tyr Lys Asp Asp Asp Asp Lys Ala Tyr Gln Ser Ile Gly
   1               5                   10                  15
   Ser Ile Trp Asn Ser Cys Gln Cys Met Ser Phe Trp Cys Ala Phe Val
               20                  25                  30
   Arg Ser Cys Tyr Gly Pro Gly Arg Gly Trp Met Lys Pro Lys Arg Arg
           35                  40                  45
   Arg Val Pro Gly Leu Lys Ser Cys Arg Arg Arg Pro Cys Xaa Leu Thr
       50                  55                  60
   Trp Tyr Gln Leu Met His Arg Tyr Arg
   65                  70
<210> 139
   <211> 190
   <212> DNA
   <213> artificial sequence
<220>
   <223> c-Jun self dimerization inhibitory peptide (SP45)
<220>
   <221> CDS
   <222> (1)..(189)
<220>
   <221> misc_feature
   <222> (154)..(154)
   <223> n is a, c, g, or t
<400> 139
   gct tat caa tca atc ggc agc atc tgg aac agc tgc caa tgc atg agt       48
   Ala Tyr Gln Ser Ile Gly Ser Ile Trp Asn Ser Cys Gln Cys Met Ser
   1               5                   10                  15
   ttc tgg tgc gca ttc gtg cgc agc tgt tat ggg cct ggg cgc ggc tgg       96
   Phe Trp Cys Ala Phe Val Arg Ser Cys Tyr Gly Pro Gly Arg Gly Trp
               20                  25                  30
   atg aag ccg aag cgt cgg cgc gta ccg gga ttg aag tct tgt cgt cgt      144
   Met Lys Pro Lys Arg Arg Arg Val Pro Gly Leu Lys Ser Cys Arg Arg
           35                  40                  45
   cgt cct tgt ngt ctg acc tgg tac caa ttg atg cat cga tac cgg t        190
   Arg Pro Cys Xaa Leu Thr Trp Tyr Gln Leu Met His Arg Tyr Arg
       50                  55                  60
<210> 140
   <211> 63
   <212> PRT
   <213> artificial sequence
<220>
   <221> misc_feature
   <222> (52)..(52)
   <223> The 'Xaa' at location 52 stands for Ser, Gly, Arg, or Cys.
<220>
   <223> Synthetic Construct
<400> 140
   Ala Tyr Gln Ser Ile Gly Ser Ile Trp Asn Ser Cys Gln Cys Met Ser
   1               5                   10                  15
   Phe Trp Cys Ala Phe Val Arg Ser Cys Tyr Gly Pro Gly Arg Gly Trp
               20                  25                  30
   Met Lys Pro Lys Arg Arg Arg Val Pro Gly Leu Lys Ser Cys Arg Arg
           35                  40                  45
   Arg Pro Cys Xaa Leu Thr Trp Tyr Gln Leu Met His Arg Tyr Arg
       50                  55                  60
<210> 141
   <211> 153
   <212> DNA
   <213> artificial sequence
<220>
   <223> c-Jun self dimerization inhibitory peptide (SP54) with vector sequence
<220>
   <221> CDS
   <222> (1)..(153)
<220>
   <221> misc feature
   <222> (47)..(47)
   <223> n is a, c, g, or t
<400> 141
   agg tca gac tac aag gac gac gac gac aag gct tat caa tca ttc cnc        48
   Arg Ser Asp Tyr Lys Asp Asp Asp Asp Lys Ala Tyr Gln Ser Phe Xaa
   1               5                   10                  15
   ttg gca ggc tac cac ggc gac act tcg aga aca ttt cta gtg ggt tcg        96
   Leu Ala Gly Tyr His Gly Asp Thr Ser Arg Thr Phe Leu Val Gly Ser
               20                  25                  30
   gta tcc gca act gcc cga aaa tta gtt gaa gcg act caa gaa acg atg       144
   Val Ser Ala Thr Ala Arg Lys Leu Val Glu Ala Thr Gln Glu Thr Met
           35                  40                  45
   att gat tat                                                            153
   Ile Asp Tyr
       50
<210> 142
   <211> 51
   <212> PRT
   <213> artificial sequence
<220>
   <221> misc feature
   <222> (16)..(16)
   <223> The 'Xaa' at location 16 stands for His, Arg, Pro, or Leu.
<220>
   <223> Synthetic Construct
<400> 142
   Arg Ser Asp Tyr Lys Asp Asp Asp Asp Lys Ala Tyr Gln Ser Phe Xaa
   1               5                   10                  15
   Leu Ala Gly Tyr His Gly Asp Thr Ser Arg Thr Phe Leu Val Gly Ser
               20                  25                  30
   Val Ser Ala Thr Ala Arg Lys Leu Val Glu Ala Thr Gln Glu Thr Met
           35                  40                  45
   Ile Asp Tyr
       50
<210> 143
   <211> 123
   <212> DNA
   <213> artificial sequence
<220>
   <223> c-Jun self dimerization inhibitory peptide (SP54)
<220>
   <221> CDS
   <222> (1)..(123)
<220>
   <221> misc_feature
   <222> (17)..(17)
   <223> n is a, c, g, or t
<400> 143
   gct tat caa tca ttc cnc ttg gca ggc tac cac ggc gac act tcg aga       48
   Ala Tyr Gln Ser Phe Xaa Leu Ala Gly Tyr His Gly Asp Thr Ser Arg
   1               5                   10                  15
   aca ttt cta gtg ggt tcg gta tcc gca act gcc cga aaa tta gtt gaa       96
   Thr Phe Leu Val Gly Ser Val Ser Ala Thr Ala Arg Lys Leu Val Glu
               20                  25                  30
   gcg act caa gaa acg atg att gat tat                                  123
   Ala Thr Gln Glu Thr Met Ile Asp Tyr
           35                  40
<210> 144
   <211> 41
   <212> PRT
   <213> artificial sequence
<220>
   <221> misc_feature
   <222> (6)..(6)
   <223> The 'Xaa' at location 6 stands for His, Arg, Pro, or Leu.
<220>
   <223> Synthetic Construct
<400> 144
   Ala Tyr Gln Ser Phe Xaa Leu Ala Gly Tyr His Gly Asp Thr Ser Arg
   1               5                   10                  15
   Thr Phe Leu Val Gly Ser Val Ser Ala Thr Ala Arg Lys Leu Val Glu
               20                  25                  30
   Ala Thr Gln Glu Thr Met Ile Asp Tyr
           35                  40
<210> 145
   <211> 323
   <212> DNA
   <213> artificial sequence
<220>
   <223> c-Jun self dimerization inhibitory peptide (SP58) with vector sequence
<220>
   <221> CDS
   <222> (1)..(222)
<400> 145
   agg tca gac tac aag gac gac gac gac aag gct tat caa tca atc atg       48
   Arg Ser Asp Tyr Lys Asp Asp Asp Asp Lys Ala Tyr Gln Ser Ile Met
   1               5                   10                  15
   gca gtg gct gcc cag cag ccg gtc gcg ttc ctg gta ggc cgc cag cgt       96
   Ala Val Ala Ala Gln Gln Pro Val Ala Phe Leu Val Gly Arg Gln Arg
               20                  25                  30
   cgc cgc ggt cag gta gga atc gac tcc ggc gat cag cac ctt cga aca      144
   Arg Arg Gly Gln Val Gly Ile Asp Ser Gly Asp Gln His Leu Arg Thr
           35                  40                  45
   ccc ctg ttc cat gag ctt tgt cgt cgt cgt cct tgt agt ctg gcc tgg      192
   Pro Leu Phe His Glu Leu Cys Arg Arg Arg Pro Cys Ser Leu Ala Trp
       50                  55                  60
   tac caa ttg atg cat cga tac cgg tac tag tcggaccgca tatgcccggg        242
   Tyr Gln Leu Met His Arg Tyr Arg Tyr
   65                  70
   cgtaccgcgg ccgctcgagg catgcatcta gagggccgca tcatgtaatt agttatgtca    302
   cgcttacatt cacgccctcc c                                              323
<210> 146
   <211> 73
   <212> PRT
   <213> artificial sequence
<220>
   <223> Synthetic Construct
<400> 146
   Arg Ser Asp Tyr Lys Asp Asp Asp Asp Lys Ala Tyr Gln Ser Ile Met
   1               5                   10                  15
   Ala Val Ala Ala Gln Gln Pro Val Ala Phe Leu Val Gly Arg Gln Arg
               20                  25                  30
   Arg Arg Gly Gln Val Gly Ile Asp Ser Gly Asp Gln His Leu Arg Thr
           35                  40                  45
   Pro Leu Phe His Glu Leu Cys Arg Arg Arg Pro Cys Ser Leu Ala Trp
       50                  55                  60
   Tyr Gln Leu Met His Arg Tyr Arg Tyr
   65                  70
<210> 147
   <211> 293
   <212> DNA
   <213> artificial sequence
<220>
   <223> c-Jun self dimerization inhibitory peptide (SP58)
<220>
   <221> CDS
   <222> (1)..(192)
<400> 147
   gct tat caa tca atc atg gca gtg gct gcc cag cag ccg gtc gcg ttc       48
   Ala Tyr Gln Ser Ile Met Ala Val Ala Ala Gln Gln Pro Val Ala Phe
   1               5                   10                  15
   ctg gta ggc cgc cag cgt cgc cgc ggt cag gta gga atc gac tcc ggc       96
   Leu Val Gly Arg Gln Arg Arg Arg Gly Gln Val Gly Ile Asp Ser Gly
               20                  25                  30
   gat cag cac ctt cga aca ccc ctg ttc cat gag ctt tgt cgt cgt cgt      144
   Asp Gln His Leu Arg Thr Pro Leu Phe His Glu Leu Cys Arg Arg Arg
           35                  40                  45
   cct tgt agt ctg gcc tgg tac caa ttg atg cat cga tac cgg tac tag      192
   Pro Cys Ser Leu Ala Trp Tyr Gln Leu Met His Arg Tyr Arg Tyr
       50                  55                  60
   tcggaccgca tatgcccggg cgtaccgcgg ccgctcgagg catgcatcta gagggccgca    252
   tcatgtaatt agttatgtca cgcttacatt cacgccctcc c                        293
<210> 148
   <211> 63
   <212> PRT
   <213> artificial sequence
<220>
   <223> Synthetic Construct
<400> 148
   Ala Tyr Gln Ser Ile Met Ala Val Ala Ala Gln Gln Pro Val Ala Phe
   1               5                   10                  15
   Leu Val Gly Arg Gln Arg Arg Arg Gly Gln Val Gly Ile Asp Ser Gly
               20                  25                  30
   Asp Gln His Leu Arg Thr Pro Leu Phe His Glu Leu Cys Arg Arg Arg
           35                  40                  45
   Pro Cys Ser Leu Ala Trp Tyr Gln Leu Met His Arg Tyr Arg Tyr
       50                  55                  60
<210> 149
   <211> 253
   <212> DNA
   <213> artificial sequence
<220>
   <223> c-Jun self dimerization inhibitory peptide (SP60) with vector sequence
<220>
   <221> CDS
   <222> (1)..(84)
<400> 149
   agg tca gac tac aag gac gac gac gac aag gct aat caa ttg ccc aaa       48
   Arg Ser Asp Tyr Lys Asp Asp Asp Asp Lys Ala Asn Gln Leu Pro Lys
   1               5                   10                  15
   ata ctt gct gga cgg ctt ata ttt ata aag tgc taa ctgcgcttga            94
   Ile Leu Ala Gly Arg Leu Ile Phe Ile Lys Cys
               20                  25
   ttgattgata agcttctcgt cgtcgtcctt gtagtctgac ctggtaccaa ttgatgcatc    154
   gataccggta ctagtcggac cgcatatgcc cgggcgtacc gcggccgctc gaggcatgca    214
   tctagagggc cgcatcatgt aattagttat gtcacgctt                           253
<210> 150
   <211> 27
   <212> PRT
   <213> artificial sequence
<220>
   <223> Synthetic Construct
<400> 150
   Arg Ser Asp Tyr Lys Asp Asp Asp Asp Lys Ala Asn Gln Leu Pro Lys
   1               5                   10                  15
   Ile Leu Ala Gly Arg Leu Ile Phe Ile Lys Cys
               20                  25
<210> 151
   <211> 223
   <212> DNA
   <213> artificial sequence
<220>
   <223> c-Jun self dimerization inhibitory peptide (SP60)
<220>
   <221> CDS
   <222> (1)..(54)
<400> 151
   gct aat caa ttg ccc aaa ata ctt gct gga cgg ctt ata ttt ata aag      48
   Ala Asn Gln Leu Pro Lys Ile Leu Ala Gly Arg Leu Ile Phe Ile Lys
   1               5                   10                  15
   tgc taa ctgcgcttga ttgattgata agcttctcgt cgtcgtcctt gtagtctgac      104
   Cys
   ctggtaccaa ttgatgcatc gataccggta ctagtcggac cgcatatgcc cgggcgtacc    164
   gcggccgctc gaggcatgca tctagagggc cgcatcatgt aattagttat gtcacgctt     223
<210> 152
   <211> 17
   <212> PRT
   <213> artificial sequence
<220>
   <223> Synthetic Construct
<400> 152
   Ala Asn Gln Leu Pro Lys Ile Leu Ala Gly Arg Leu Ile Phe Ile Lys
   1               5                   10                  15
   Cys
<210> 153
   <211> 231
   <212> DNA
   <213> artificial sequence
<220>
   <223> c-Jun self dimerization inhibitory peptide (SP66) with vector sequence
<220>
   <221> CDS
   <222> (1)..(225)
<400> 153
   agg tca gac tac aag gac gac gac gac aag gct tat caa tca atc ata        48
   Arg Ser Asp Tyr Lys Asp Asp Asp Asp Lys Ala Tyr Gln Ser Ile Ile
   1               5                   10                  15
   ggg gcg gga aaa tca acg cta atc aaa gca tta act ggc gta tac cac        96
   Gly Ala Gly Lys Ser Thr Leu Ile Lys Ala Leu Thr Gly Val Tyr His
               20                  25                  30
   gcc gat cgc ggc acc atc tgg ctg gaa ggc cag gct atc tca ccg aaa       144
   Ala Asp Arg Gly Thr Ile Trp Leu Glu Gly Gln Ala Ile Ser Pro Lys
           35                  40                  45
   aat acc gcc cac gcg caa caa tgt cgt cgt cgt cct tgt agt ctg acc       192
   Asn Thr Ala His Ala Gln Gln Cys Arg Arg Arg Pro Cys Ser Leu Thr
       50                  55                  60
   tgg tac caa ttg atg cat cga tac cgg tac tag tcggac                    231
   Trp Tyr Gln Leu Met His Arg Tyr Arg Tyr
   65                  70
<210> 154
   <211> 74
   <212> PRT
   <213> artificial sequence
<220>
   <223> Synthetic Construct
<400> 154
   Arg Ser Asp Tyr Lys Asp Asp Asp Asp Lys Ala Tyr Gln Ser Ile Ile
   1               5                   10                  15
   Gly Ala Gly Lys Ser Thr Leu Ile Lys Ala Leu Thr Gly Val Tyr His
               20                  25                  30
   Ala Asp Arg Gly Thr Ile Trp Leu Glu Gly Gln Ala Ile Ser Pro Lys
           35                  40                  45
   Asn Thr Ala His Ala Gln Gln Cys Arg Arg Arg Pro Cys Ser Leu Thr
       50                  55                  60
   Trp Tyr Gln Leu Met His Arg Tyr Arg Tyr
   65                  70
<210> 155
   <211> 201
   <212> DNA
   <213> artificial sequence
<220>
   <223> c-Jun self dimerization inhibitory peptide (SP66)
<220>
   <221> CDS
   <222> (1)..(195)
<400> 155
   gct tat caa tca atc ata ggg gcg gga aaa tca acg cta atc aaa gca      48
   Ala Tyr Gln Ser Ile Ile Gly Ala Gly Lys Ser Thr Leu Ile Lys Ala
   1               5                   10                  15
   tta act ggc gta tac cac gcc gat cgc ggc acc atc tgg ctg gaa ggc      96
   Leu Thr Gly Val Tyr His Ala Asp Arg Gly Thr Ile Trp Leu Glu Gly
               20                  25                  30
   cag gct atc tca ccg aaa aat acc gcc cac gcg caa caa tgt cgt cgt      144
   Gln Ala Ile Ser Pro Lys Asn Thr Ala His Ala Gln Gln Cys Arg Arg
           35                  40                  45
   cgt cct tgt agt ctg acc tgg tac caa ttg atg cat cga tac cgg tac      192
   Arg Pro Cys Ser Leu Thr Trp Tyr Gln Leu Met His Arg Tyr Arg Tyr
       50                  55                  60
   tag tcggac                                                           201
<210> 156
   <211> 64
   <212> PRT
   <213> artificial sequence
<220>
   <223> Synthetic Construct
<400> 156
   Ala Tyr Gln Ser Ile Ile Gly Ala Gly Lys Ser Thr Leu Ile Lys Ala
   1               5                   10                  15
   Leu Thr Gly Val Tyr His Ala Asp Arg Gly Thr Ile Trp Leu Glu Gly
               20                  25                  30
   Gln Ala Ile Ser Pro Lys Asn Thr Ala His Ala Gln Gln Cys Arg Arg
           35                  40                  45
   Arg Pro Cys Ser Leu Thr Trp Tyr Gln Leu Met His Arg Tyr Arg Tyr
       50                  55                  60

<210> 157
   <211> 281
   <212> DNA
   <213> artificial sequence
<220>
   <223> c-Jun self dimerization inhibitory peptide (SP71) with vector sequence
<220>
   <221> CDS
   <222> (1)..(90)
<220>
   <221> misc_feature
   <222> (136)..(136)
   <223> n is a, c, g, or t
<400> 157
   agg tca gac tac aag gac ccc ttt ttc tgg aga cta aat aaa atc ttt       48
   Arg Ser Asp Tyr Lys Asp Pro Phe Phe Trp Arg Leu Asn Lys Ile Phe
   1               5                   10                  15
   tat ttt atc gac tct aga gtc gcg gcc gca att ctt aat taa               90
   Tyr Phe Ile Asp Ser Arg Val Ala Ala Ala Ile Leu Asn
               20                  25
   ttcattactt gtacagctcg tccatgccga gagtgatccc ggcggnggtc acgaactcca    150
   gcaggaccat gtgatcgcgc ttctcgttgg ggtctttgct cagggcggac tgggtgctca    210
   ggtagtggtt gtcgggcagc agcacggggc cgtcgccgat gggggtgttc tgctggtagt    270
   ggtcggcgag c                                                          281
<210> 158
   <211> 29
   <212> PRT
   <213> artificial sequence
<220>
   <223> Synthetic Construct
<400> 158
   Arg Ser Asp Tyr Lys Asp Pro Phe Phe Trp Arg Leu Asn Lys Ile Phe
   1               5                   10                  15
   Tyr Phe Ile Asp Ser Arg Val Ala Ala Ala Ile Leu Asn
               20                  25
<210> 159
   <211> 263
   <212> DNA
   <213> artificial sequence
<220>
   <223> c-Jun self dimerization inhibitory peptide (SP71)
<220>
   <221> CDS
   <222> (1)..(72)
<220>
   <221> misc_feature
   <222> (118)..(118)
   <223> n is a, c, g, or t
<400> 159
   ccc ttt ttc tgg aga cta aat aaa atc ttt tat ttt atc gac tct aga        48
   Pro Phe Phe Trp Arg Leu Asn Lys Ile Phe Tyr Phe Ile Asp Ser Arg
   1               5                   10                  15
   gtc gcg gcc gca att ctt aat taa ttcattactt gtacagctcg tccatgccga      102
   Val Ala Ala Ala Ile Leu Asn
               20
   gagtgatccc ggcggnggtc acgaactcca gcaggaccat gtgatcgcgc ttctcgttgg    162
   ggtctttgct cagggcggac tgggtgctca ggtagtggtt gtcgggcagc agcacggggc    222
   cgtcgccgat gggggtgttc tgctggtagt ggtcggcgag c                        263
<210> 160
   <211> 23
   <212> PRT
   <213> artificial sequence
<220>
   <223> Synthetic Construct
<400> 160
   Pro Phe Phe Trp Arg Leu Asn Lys Ile Phe Tyr Phe Ile Asp Ser Arg
   1               5                   10                  15
   Val Ala Ala Ala Ile Leu Asn
               20
<210> 161
   <211> 153
   <212> DNA
   <213> artificial sequence
<220>
   <223> c-Jun self dimerization inhibitory peptides (SP72, SP73, SP76, SP77) with vector sequence
<220>
   <221> CDS
   <222> (1)..(153)
<400> 161
   agg tca gac tac aag gac gac gac gac aag gag ctc aga tct cag ctg        48
   Arg Ser Asp Tyr Lys Asp Asp Asp Asp Lys Glu Leu Arg Ser Gln Leu
   1               5                   10                  15
   ggc ccg gta cca att gat gca tcg ata ccg gta cta gtc gga ccg cat        96
   Gly Pro Val Pro Ile Asp Ala Ser Ile Pro Val Leu Val Gly Pro His
               20                  25                  30
   atg ccc ggg cgt acc gcg gcc gct cga ggc atg cat cta gag ggc cgc       144
   Met Pro Gly Arg Thr Ala Ala Ala Arg Gly Met His Leu Glu Gly Arg
           35                  40                  45
   atc atg taa                                                           153
   Ile Met
       50
<210> 162
   <211> 50
   <212> PRT
   <213> artificial sequence
<220>
   <223> Synthetic Construct
<400> 162
   Arg Ser Asp Tyr Lys Asp Asp Asp Asp Lys Glu Leu Arg Ser Gln Leu
   1               5                   10                  15
   Gly Pro Val Pro Ile Asp Ala Ser Ile Pro Val Leu Val Gly Pro His
               20                  25                  30
   Met Pro Gly Arg Thr Ala Ala Ala Arg Gly Met His Leu Glu Gly Arg
           35                  40                  45
   Ile Met
       50
<210> 163
   <211> 123
   <212> DNA
   <213> artificial sequence
<220>
   <223> c-Jun self dimerization inhibitory peptides (SP72, SP73, SP76, SP77)
<220>
   <221> CDS
   <222> (1)..(123)
<400> 163
   gag ctc aga tct cag ctg ggc ccg gta cca att gat gca tcg ata ccg       48
   Glu Leu Arg Ser Gln Leu Gly Pro Val Pro Ile Asp Ala Ser Ile Pro
   1               5                   10                  15
   gta cta gtc gga ccg cat atg ccc ggg cgt acc gcg gcc gct cga ggc       96
   Val Leu Val Gly Pro His Met Pro Gly Arg Thr Ala Ala Ala Arg Gly
               20                  25                  30
   atg cat cta gag ggc cgc atc atg taa                                  123
   Met His Leu Glu Gly Arg Ile Met
           35                  40
<210> 164
   <211> 40
   <212> PRT
   <213> artificial sequence
<220>
   <223> Synthetic Construct
<400> 164
   Glu Leu Arg Ser Gln Leu Gly Pro Val Pro Ile Asp Ala Ser Ile Pro
   1               5                   10                  15
   Val Leu Val Gly Pro His Met Pro Gly Arg Thr Ala Ala Ala Arg Gly
               20                  25                  30
   Met His Leu Glu Gly Arg Ile Met
           35                  40
<210> 165
   <211> 60
   <212> DNA
   <213> artificial sequence
<220>
   <223> c-Jun self dimerization inhibitory peptide (SP79) with vector sequence
<220>
   <221> CDS
   <222> (1)..(60)
<400> 165
   agg tca gac tac aag gac gac gac gac aag gct tat caa tca atc aaa      48
   Arg Ser Asp Tyr Lys Asp Asp Asp Asp Lys Ala Tyr Gln Ser Ile Lys
   1               5                   10                  15
   tgg cca atg taa                                                      60
   Trp Pro Met
<210> 166
   <211> 19
   <212> PRT
   <213> artificial sequence
<220>
   <223> Synthetic Construct
<400> 166
   Arg Ser Asp Tyr Lys Asp Asp Asp Asp Lys Ala Tyr Gln Ser Ile Lys
   1               5                   10                  15
   Trp Pro Met
<210> 167
   <211> 30
   <212> DNA
   <213> artificial sequence
<220>
   <223> c-Jun self dimerization inhibitory peptide (SP79)
<220>
   <221> CDS
   <222> (1)..(30)
<400> 167
   gct tat caa tca atc aaa tgg cca atg taa                              30
   Ala Tyr Gln Ser Ile Lys Trp Pro Met
   1               5
<210> 168
   <211> 9
   <212> PRT
   <213> artificial sequence
<220>
   <223> Synthetic Construct
<400> 168
   Ala Tyr Gln Ser Ile Lys Trp Pro Met
   1               5
<210> 169
   <211> 111
   <212> DNA
   <213> artificial sequence
<220>
   <223> c-Jun self dimerization inhibitory peptide (SP80) with vector sequence
<220>
   <221> CDS
   <222> (1)..(111)
<400> 169
   agg tca gac tac aag gac gac gac gac aag gct tat caa tca tcc act      48
   Arg Ser Asp Tyr Lys Asp Asp Asp Asp Lys Ala Tyr Gln Ser Ser Thr
   1               5                   10                  15
   ggt agt gct gcc atc ctc ttc aat ttc cgt cga atg ggg atc gtg ata      96
   Gly Ser Ala Ala Ile Leu Phe Asn Phe Arg Arg Met Gly Ile Val Ile
               20                  25                  30
   ata att cag atc taa                                                 111
   Ile Ile Gln Ile
           35
<210> 170
   <211> 36
   <212> PRT
   <213> artificial sequence
<220>
   <223> Synthetic Construct
<400> 170
   Arg Ser Asp Tyr Lys Asp Asp Asp Asp Lys Ala Tyr Gln Ser Ser Thr
   1               5                   10                  15
   Gly Ser Ala Ala Ile Leu Phe Asn Phe Arg Arg Met Gly Ile Val Ile
               20                  25                  30
   Ile Ile Gln Ile
           35
<210> 171
   <211> 81
   <212> DNA
   <213> artificial sequence
<220>
   <223> c-Jun self dimerization inhibitory peptide (SP80)
<220>
   <221> CDS
   <222> (1)..(81)
<400> 171
   gct tat caa tca tcc act ggt agt gct gcc atc ctc ttc aat ttc cgt 48
   Ala Tyr Gln Ser Ser Thr Gly Ser Ala Ala Ile Leu Phe Asn Phe Arg
   1               5                   10                  15
   cga atg ggg atc gtg ata ata att cag atc taa 81
   Arg Met Gly Ile Val Ile Ile Ile Gln Ile
               20                  25
<210> 172
   <211> 26
   <212> PRT
   <213> artificial sequence
<220>
   <223> Synthetic Construct
<400> 172
   Ala Tyr Gln Ser Ser Thr Gly Ser Ala Ala Ile Leu Phe Asn Phe Arg
   1               5                   10                  15
   Arg Met Gly Ile Val Ile Ile Ile Gln Ile
               20                  25
<210> 173
   <211> 207
   <212> DNA
   <213> artificial sequence
<220>
   <223> c-Jun self dimerization inhibitory peptide (SP54-1) with vector sequence
<220>
   <221> CDS
   <222> (1)..(207)
<220>
   <221> misc feature
   <222> (47)..(47)
   <223> n is a, c, g, or t
<400> 173
   agg tca gac tac aag gac gac gac gac aag gct tat caa tca ttc cnc       48
   Arg Ser Asp Tyr Lys Asp Asp Asp Asp Lys Ala Tyr Gln Ser Phe Xaa
   1               5                   10                  15
   ttg gca ggc tac cac ggc gac act tcg aga aca ttt cta gtg ggt tcg       96
   Leu Ala Gly Tyr His Gly Asp Thr Ser Arg Thr Phe Leu Val Gly Ser
               20                  25                  30
   gta tcc gca act gcc cga aaa tta gtt gaa gcg act caa gaa acg atg      144
   Val Ser Ala Thr Ala Arg Lys Leu Val Glu Ala Thr Gln Glu Thr Met
           35                  40                  45
   att gat tat act tgt cgt cgt cgt cct tgt agt ctg acc tgg tac caa      192
   Ile Asp Tyr Thr Cys Arg Arg Arg Pro Cys Ser Leu Thr Trp Tyr Gln
       50                  55                  60
   ttg atg cat cga tac                                                  207
   Leu Met His Arg Tyr
   65
<210> 174
   <211> 69
   <212> PRT
   <213> artificial sequence
<220>
   <221> misc feature
   <222> (16)..(16)
   <223> The 'Xaa' at location 16 stands for His, Arg, Pro, or Leu.
<220>
   <223> Synthetic Construct
<400> 174
   Arg Ser Asp Tyr Lys Asp Asp Asp Asp Lys Ala Tyr Gln Ser Phe Xaa
   1               5                   10                  15
   Leu Ala Gly Tyr His Gly Asp Thr Ser Arg Thr Phe Leu Val Gly Ser
               20                  25                  30
   Val Ser Ala Thr Ala Arg Lys Leu Val Glu Ala Thr Gln Glu Thr Met
           35                  40                  45
   Ile Asp Tyr Thr Cys Arg Arg Arg Pro Cys Ser Leu Thr Trp Tyr Gln
       50                  55                  60
   Leu Met His Arg Tyr
   65
<210> 175
   <211> 177
   <212> DNA
   <213> artificial sequence
<220>
   <223> c-Jun self dimerization inhibitory peptide (SP54-1)
<220>
   <221> CDS
   <222> (1)..(177)
<220>
   <221> misc feature
   <222> (17)..(17)
   <223> n is a, c, g, or t
<400> 175
   gct tat caa tca ttc cnc ttg gca ggc tac cac ggc gac act tcg aga      48
   Ala Tyr Gln Ser Phe Xaa Leu Ala Gly Tyr His Gly Asp Thr Ser Arg
   1               5                   10                  15
   aca ttt cta gtg ggt tcg gta tcc gca act gcc cga aaa tta gtt gaa      96
   Thr Phe Leu Val Gly Ser Val Ser Ala Thr Ala Arg Lys Leu Val Glu
               20                  25                  30
   gcg act caa gaa acg atg att gat tat act tgt cgt cgt cgt cct tgt      144
   Ala Thr Gln Glu Thr Met Ile Asp Tyr Thr Cys Arg Arg Arg Pro Cys
           35                  40                  45
   agt ctg acc tgg tac caa ttg atg cat cga tac                          177
   Ser Leu Thr Trp Tyr Gln Leu Met His Arg Tyr
       50                  55
<210> 176
   <211> 59
   <212> PRT
   <213> artificial sequence
<220>
   <221> misc_feature
   <222> (6)..(6)
   <223> The 'Xaa' at location 6 stands for His, Arg, Pro, or Leu.
<220>
   <223> Synthetic Construct
<400> 176
   Ala Tyr Gln Ser Phe Xaa Leu Ala Gly Tyr His Gly Asp Thr Ser Arg
   1               5                   10                  15
   Thr Phe Leu Val Gly Ser Val Ser Ala Thr Ala Arg Lys Leu Val Glu
               20                  25                  30
   Ala Thr Gln Glu Thr Met Ile Asp Tyr Thr Cys Arg Arg Arg Pro Cys
           35                  40                  45
   Ser Leu Thr Trp Tyr Gln Leu Met His Arg Tyr
       50                  55
<210> 177
   <211> 165
   <212> DNA
   <213> artificial sequence
<220>
   <223> c-Jun self dimerization inhibitory peptide (SP66-1) with vector sequence
<220>
   <221> CDS
   <222> (1)..(165)
<400> 177
   agg tca gac tac aag gac gac gac gac aag gct tat caa tca atc ata       48
   Arg Ser Asp Tyr Lys Asp Asp Asp Asp Lys Ala Tyr Gln Ser Ile Ile
   1               5                   10                  15
   ggg gcg gga aaa tca acg cta atc aaa gca tta act ggc gta tac cac       96
   Gly Ala Gly Lys Ser Thr Leu Ile Lys Ala Leu Thr Gly Val Tyr His
               20                  25                  30
   gcc gat cgc ggc acc atc tgg ctg gaa ggc cag gct atc tca ccg aaa      144
   Ala Asp Arg Gly Thr Ile Trp Leu Glu Gly Gln Ala Ile Ser Pro Lys
           35                  40                  45
   aat acc gcc cac gcg caa caa                                          165
   Asn Thr Ala His Ala Gln Gln
       50                  55
<210> 178
   <211> 55
   <212> PRT
   <213> artificial sequence
<220>
   <223> Synthetic Construct
<400> 178
   Arg Ser Asp Tyr Lys Asp Asp Asp Asp Lys Ala Tyr Gln Ser Ile Ile
   1               5                   10                  15
   Gly Ala Gly Lys Ser Thr Leu Ile Lys Ala Leu Thr Gly Val Tyr His
               20                  25                  30
   Ala Asp Arg Gly Thr Ile Trp Leu Glu Gly Gln Ala Ile Ser Pro Lys
           35                  40                  45
   Asn Thr Ala His Ala Gln Gln
       50                  55
<210> 179
   <211> 165
   <212> DNA
   <213> artificial sequence
<220>
   <223> c-Jun self dimerization inhibitory peptide (SP66-1)
<220>
   <221> CDS
   <222> (1)..(135)
<400> 179
   agg tca gac tac aag gac gac gac gac aag gct tat caa tca atc ata       48
   Arg Ser Asp Tyr Lys Asp Asp Asp Asp Lys Ala Tyr Gln Ser Ile Ile
   1               5                   10                  15
   ggg gcg gga aaa tca acg cta atc aaa gca tta act ggc gta tac cac       96
   Gly Ala Gly Lys Ser Thr Leu Ile Lys Ala Leu Thr Gly Val Tyr His
               20                  25                  30
   gcc gat cgc ggc acc atc tgg ctg gaa ggc cag gct atc tcaccgaaaa       145
   Ala Asp Arg Gly Thr Ile Trp Leu Glu Gly Gln Ala Ile
           35                  40                  45
   ataccgccca cgcgcaacaa                                                 165
<210> 180
   <211> 45
   <212> PRT
   <213> artificial sequence
<220>
   <223> Synthetic Construct
<400> 180
   Arg Ser Asp Tyr Lys Asp Asp Asp Asp Lys Ala Tyr Gln Ser Ile Ile
   1               5                   10                  15
   Gly Ala Gly Lys Ser Thr Leu Ile Lys Ala Leu Thr Gly Val Tyr His
               20                  25                  30
   Ala Asp Arg Gly Thr Ile Trp Leu Glu Gly Gln Ala Ile
           35                  40                  45
<210> 181
   <211> 29
   <212> PRT
   <213> artificial sequence
<220>
   <223> retro-inverted peptide analogue of SEQ ID NO: 132 comprising a TAT protein targeting domain fused to the retro-inverted peptide moiety by a C-terminal glycine residue, wherein the complete sequence of SEQ ID NO: 132 has been reversed and wherein every amino acid residue other than glycine has been inverted (i.e., substituted for the corresponding D-amino acid residue.
<400> 181
   Arg Glu Arg Arg Ser Ser Ser Gln Ile Gly Gly Ser Arg Ile Ser Gln
   1               5                   10                  15
   Tyr Ala Gly Arg Arg Arg Gln Arg Arg Lys Lys Arg Gly
               20                  25
<210> 182
   <211> 26
   <212> PRT
   <213> artificial sequence
<220>
   <223> retro-inverted peptide analogue of SEQ ID NO: 136 comprising a TAT protein targeting domain fused to the retro-inverted peptide moiety by a C-terminal glycine residue, wherein the complete sequence of SEQ ID NO: 136 has been reversed and wherein every amino acid residue other than glycine has been inverted (i.e., substituted for the corresponding D-amino acid residue.
<400> 182
   Pro Lys Ile Ser Gln Tyr Gly Gln Arg Arg Arg Gly Gln Leu Gly Gly
   1               5                   10                  15
   Arg Arg Arg Gln Arg Arg Lys Lys Arg Gly
               20                  25